(19) Europäisches Patentamt
European Patent Office
Office européen des brevets

(11) **EP 4 455 128 A1**

(12) **EUROPEAN PATENT APPLICATION**
published in accordance with Art. 153(4) EPC

(43) Date of publication:
**30.10.2024 Bulletin 2024/44**

(21) Application number: **22911340.2**

(22) Date of filing: **22.12.2022**

(51) International Patent Classification (IPC):
***C07D 307/46*** *(2006.01)* ***C07D 333/16*** *(2006.01)*
***G02B 1/04*** *(2006.01)* ***G02B 5/30*** *(2006.01)*
***H05B 33/02*** *(2006.01)* ***H10K 50/00*** *(2023.01)*

(52) Cooperative Patent Classification (CPC):
**C07D 307/46; C07D 333/16; C07D 333/28; C07D 333/32; C07D 339/06; C08G 63/00; C08G 63/137; C08G 63/19; C08G 63/547; C08G 63/682; C08G 63/685; C08G 63/688; C08G 63/78; C08G 69/00; C08G 69/44;** (Cont.)

(86) International application number:
**PCT/JP2022/047334**

(87) International publication number:
**WO 2023/120638 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**22.12.2021 JP 2021207755**
**25.03.2022 JP 2022050388**
**04.04.2022 JP 2022062204**
**25.07.2022 JP 2022117673**
**21.09.2022 JP 2022150482**
**25.10.2022 JP 2022170362**
**29.11.2022 JP 2022190544**
**15.12.2022 JP 2022200615**
**19.12.2022 JP 2022202410**

(71) Applicants:
- **Tosoh Corporation**
  **Yamaguchi 746-8501 (JP)**
- **Sagami Chemical Research Institute**
  **Ayase-shi**
  **Kanagawa 252-1193 (JP)**

(72) Inventors:
- **ICHIJO, Hiroki**
  **Yokkaichi-shi, Mie 510-8540 (JP)**
- **SAKASHITA, Ryuichi**
  **Yokkaichi-shi, Mie 510-8540 (JP)**
- **MURAKAMI, Noritake**
  **Yokkaichi-shi, Mie 510-8540 (JP)**
- **SHICHIDA, Yuki**
  **Yokkaichi-shi, Mie 510-8540 (JP)**
- **INABA, Ayato**
  **Yokkaichi-shi, Mie 510-8540 (JP)**
- **KITAGAWA, Takahiro**
  **Yokkaichi-shi, Mie 510-8540 (JP)**
- **YOKOI, Taiyo**
  **Ayase-shi, Kanagawa 252-1193 (JP)**
- **IBE, Kouta**
  **Ayase-shi, Kanagawa 252-1193 (JP)**
- **KOBAYASHI, Osamu**
  **Ayase-shi, Kanagawa 252-1193 (JP)**

(74) Representative: **Novagraaf International SA**
**Chemin de l'Echo 3**
**1213 Onex, Geneva (CH)**

(54) **MAIN CHAIN POLYMER, OPTICAL FILM, METHOD FOR PRODUCING MAIN CHAIN POLYMER, METHOD FOR PRODUCING OPTICAL FILM, AND MULTILAYER FILM**

(57) A main chain polymer according to this embodiment comprises, in its polymer main chain, a photoreactive reverse wavelength dispersion unit that exhibits both the functions of photoreactivity and reverse wavelength dispersion of birefringence, wherein the photoreactive reverse wavelength dispersion unit has a structure represented by chemical formula (1) below.

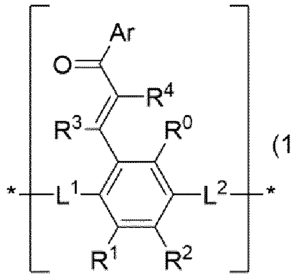

(1)

[In chemical formula (1) above, $L^1$ and $L^2$ may be the same or different from each other, each representing a carbonyl group, an ester linkage, an amide linkage, an ether linkage or a single bond. * represents a site of binding with another structure in the main chain polymer. Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent. R°, $R^1$, $R^2$, $R^3$ and $R^4$ each independently represent a hydrogen atom, a halogen atom or a C1 to C8 alkyl group.]

(52) Cooperative Patent Classification (CPC): (Cont.)
**C08G 85/00; C08K 5/00; C08L 67/02;
C08L 77/12; C08L 101/12; G02B 1/04;
G02B 5/30; H05B 33/02; H10K 50/00**

## Description

### Technical Field

The present invention relates to a main chain polymer, a composition, an optical film and production methods.

### Background Art

OLED displays and liquid-crystal displays are widely used, for example in types of smart devices, computer monitors and TVs, as important display devices in the multimedia society. These displays, furthermore, are made using numerous optical films for improved display characteristics. The films play a crucial role in, for example, contrast improvement and color compensation when viewed from the front or diagonally.

A typical example of an optical film relating to OLED displays and liquid-crystal displays is a retardation film. A retardation film combined with a polarizing plate can be used as an anti-reflection layer in types of displays. In this application, there is a particular need for a retardation film that exhibits greater in-plane retardation in longer-wavelength ranges, or a film having reverse wavelength dispersion properties (hereinafter also expressed as a reverse wavelength dispersion film). For example, when a reverse wavelength dispersion film is used in the application of a circular polarizing plate for OLEDs, it is preferred that its retardation be approximately 1/4 of the measuring wavelength $\lambda$. To be more specific, it is preferred that the ratio between the retardation at 450 nm and the retardation at 550 nm, Re (450)/Re (550), be close to 0.80 to 0.89.

As for source materials for the production of reverse wavelength dispersion films, various polymerizable liquid-crystal compounds having reverse wavelength dispersion properties are under development. In the production of a reverse wavelength dispersion film, such a polymerizable liquid-crystal compound is applied onto an alignment film created through photoalignment treatment, created through rubbing treatment or photoalignment treatment in advance (e.g., see PTL 1 to 3), and then cured, for example with light or heat, to form a film. In other words, a step of forming an alignment film is essential (e.g., PTL 4). The polymerizable liquid-crystal compound having reverse wavelength dispersion properties, furthermore, is produced through multistep synthesis (e.g., PTL 5). In addition, there is a proposal to form a retardation film using a side chain acrylate resin, without requiring an alignment film (e.g., see PTL 6 and 7). Side chain liquid-crystal acrylate resins containing a photoreactive group, however, have drawbacks such as high cost due to the need for multiple steps in monomer synthesis and low temperature tolerances. Linear-chain liquid-crystal polyester resins containing a photoreactive group, with which optical films and retardation films can be formed through polarized ultraviolet irradiation and heating treatment without requiring an alignment film, are expected to enable the production of optical films and retardation films superior in heat resistance from inexpensive monomers. Linear-chain liquid-crystal polyester resins, however, have the disadvantage that while they have excellent heat resistance, they require high processing temperatures, 200 degrees or above (e.g., PTL 8 to 10). When a linear-chain liquid-crystal polyester resin is employed as an optical film and a retardation film, therefore, the issue has arisen that inexpensive generalpurpose resin-film support substrates, such as polyethylene terephthalate (temperature tolerance, approximately 160°C), polyethylene terenaphthalate (temperature tolerance, approximately 200°C) and cycloolefin polymers (temperature tolerances, approximately 160°C), cannot be used.

### Citation List

### Patent Literature

PTL 1: Japanese Unexamined Patent Application Publication No. 8-160430
PTL 2: Japanese Unexamined Patent Application Publication (Translation of PCT Application) No. 2003-505561
PTL 3: International Publication No. 2010-150748
PTL 4: Japanese Patent No. 6172556
PTL 5: Japanese Patent No. 6754845
PTL 6: Japanese Unexamined Patent Application Publication No. 2002-226858
PTL 7: International Publication Pamphlet No. 2014/017497
PTL 8: Japanese Unexamined Patent Application Publication No. 2018-188529
PTL 9: Japanese Unexamined Patent Application Publication No. 2021-028375
PTL 10: Japanese Unexamined Patent Application Publication No. 2021-028384

Summary of Invention

Technical Problem

As mentioned in the Background Art above, when a polymerizable liquid-crystal compound having reverse wavelength dispersion properties is used as a source material for the production of a reverse wavelength dispersion film, a step of forming a liquid-crystal alignment film is essential. This can hardly be considered advantageous in the process of manufacturing a reverse wavelength dispersion film.

In addition, polymerizable liquid-crystal compounds having reverse wavelength dispersion properties used as source materials for the production of reverse wavelength dispersion films often require synthesis through multiple steps; they are inferior in economic efficiency.

In the present invention, therefore, the inventors proceeded with research with the goal of providing a reverse wavelength dispersion film that requires no step of forming an alignment film and can be formed through the application of a polymer.

Solution to Problem

After extensive research to solve the above problem, the inventors found that a particular photoreactive structure develops reverse wavelength dispersion of birefringence, and that the use of a polymer comprising a photoreactive reverse wavelength dispersion unit that exhibits both of these functions of photoreactivity and reverse wavelength dispersion of birefringence (hereinafter also referred to as a photoreactive reverse wavelength dispersion unit A or unit A) or a resin composition comprising a polymer having a particular photoreactive group and a particular additive enables the formation of a reverse wavelength dispersion retardation film without requiring an alignment film. Based on these findings, the inventors completed the present invention.

The present invention was proposed based on such findings. Specifically, it has the following configurations.

A main chain polymer comprising, in a polymer main chain thereof, a photoreactive reverse wavelength dispersion unit that exhibits both functions of photoreactivity and reverse wavelength dispersion of birefringence, wherein the photoreactive reverse wavelength dispersion unit has a structure represented by chemical formula (1) below.

[Chem. 1]

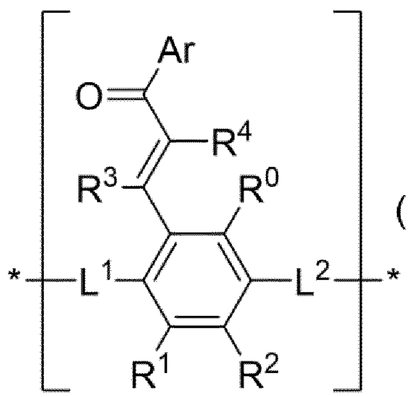

(1)

[In chemical formula (1) above, $L^1$ and $L^2$ may be the same or different from each other, each representing a carbonyl group, an ester linkage, an amide linkage, an ether linkage or a single bond.

* represents a site of binding with another structure in the main chain polymer.

Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.

$R^0$, $R^1$, $R^2$, $R^3$ and $R^4$ each independently represent a hydrogen atom, a halogen atom, a C1 to C8 alkyl group or a group represented by chemical formula (Z1) below.]

[Chem. 2]

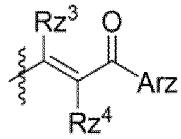

(Z1)

[In chemical formula (Z1) above, $Rz^3$ and $Rz^4$ each independently represent a hydrogen atom, a halogen atom or a C1 to C8 alkyl group.

Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.

A wavy-lined portion represents a site of binding with a portion in formula (1) excluding R°, $R^1$, $R^2$, $R^3$ or $R^4$.]

A dihydroxy compound represented by chemical formula (1') below.

[Chem. 3]

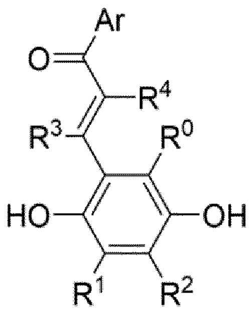

(1')

[In chemical formula (1') above, R°, $R^1$, $R^2$, $R^3$ and $R^4$ each independently represent a hydrogen atom, a halogen atom or a C1 to C8 alkyl group or a group represented by chemical formula (Z1) below. Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.]

[Chem. 4]

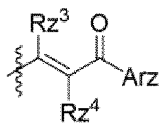

(Z1)

[In chemical formula (Z1) above, $Rz^3$ and $Rz^4$ each independently represent a hydrogen atom, a halogen atom or a C1 to C8 alkyl group.
Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.
A wavy-lined portion represents a site of binding with a portion in formula (1) excluding R°, $R^1$, $R^2$, $R^3$ or $R^4$.]

A method for producing a dihydroxy compound, the method comprising allowing a dihydroxy compound indicated by chemical formula (3) below and a ketone indicated by chemical formula (4') below to react together to give a dihydroxy compound represented by chemical formula (1') below.

[Chem. 5]

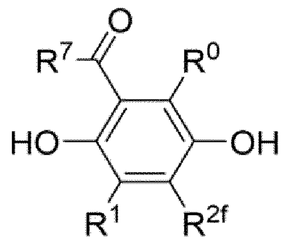

**(3)**

[In chemical formula (3) above, R°, $R^1$ and $R^7$ each independently represent a hydrogen atom, a halogen atom or a C1 to C8 alkyl group.
$R^{2f}$ represents a hydrogen atom, a halogen atom, a C1 to C8 alkyl group or a formyl group.]

[Chem. 6]

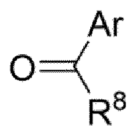

**(4')**

[In chemical formula (4') above, $R^8$ represents a C1 to C8 alkyl group or a C1 to C6 haloalkyl group.
Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system and annulated aromatic ring system optionally having a substituent.]

[Chem. 7]

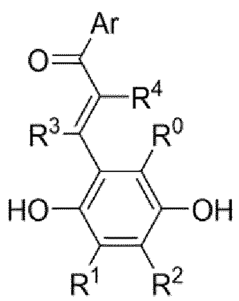

**(1')**

[In chemical formula (1') above, $R^0$, $R^1$, $R^2$, $R^3$ and $R^4$ each independently represent a hydrogen atom, a halogen atom or a C1 to C8 alkyl group or a group represented by chemical formula (Z1) below. Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.]

[Chem. 8]

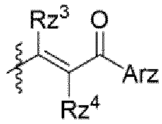

(Z1)

[In chemical formula (Z1) above, $Rz^3$ and $Rz^4$ each independently represent a hydrogen atom, a halogen atom or a C1 to C8 alkyl group.
Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent. A wavy-lined portion represents a site of binding with a portion in formula (1) excluding R°, $R^1$, $R^2$, $R^3$ or $R^4$.]

Advantageous Effects of Invention

According to the present invention, there can be provided a reverse wavelength dispersion film that requires no step of forming an alignment film and can be formed through the application of a polymer.

Description of Embodiments

In the following, the present invention will be described in detail. Although the description of requirements provided below may be based on a typical embodiment or specific example, the present invention is not limited to such an embodiment. It should be noted that a numerical range expressed using ("from" and) "to" herein indicates a range that includes the values before and after "to" as the lower and upper limits.

In the present description, a structure enclosed by brackets in a polymer structure represents a repeat unit in the polymer structure.

In the present description, a number placed at the lower right of brackets in a polymer structure represents the relative amount of the repeat unit in the polymer structure.

In the present description, the direction of binding of a divalent group or chemical structure presented by way of example (e.g., an ester linkage or a repeat unit in a polymer structure) is not particularly restricted as long as it is chemically acceptable.

An aspect of the present invention is a main chain polymer comprising, in its polymer main chain, a photoreactive reverse wavelength dispersion unit A, which exhibits both functions of photoreactivity and reverse wavelength dispersion of birefringence (hereinafter also expressed as the polymer according to the present invention).

An example of a photoreactive reverse wavelength dispersion unit A is a structure indicated by chemical formula (1) below.

[Chem. 9]

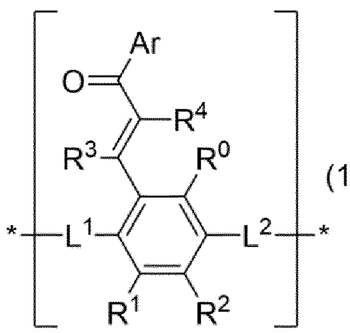

(1)

[In formula (1), $L^1$ and $L^2$ may be the same or different from each other, each representing a carbonyl group, an ester linkage, an amide linkage, an ether linkage or a single bond.
* represents a site of binding with another structure in the main chain polymer.

Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system and annulated aromatic ring system optionally having a substituent.

R°, $R^1$, $R^2$, $R^3$ and $R^4$ each independently represent a hydrogen atom, a halogen atom, a C1 to C8 alkyl group or a group represented by chemical formula (Z1) below.]

[Chem. 10]

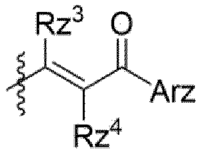

(Z1)

[In chemical formula (Z1) above, $Rz^3$ and $Rz^4$ each independently represent a hydrogen atom, a halogen atom or a C1 to C8 alkyl group.

Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent. The wavy-lined portion represents a site of binding with the portion in formula (1) excluding R°, $R^1$, $R^2$, $R^3$ or $R^4$.]

In formula (1), R°, $R^1$, $R^2$, $R^3$ and $R^4$ each independently represent a hydrogen atom, a halogen atom, a C1 to C8 alkyl group or a group represented by chemical formula (Z1). Examples of halogen atoms include a chlorine atom, a bromine atom, an iodine atom and a fluorine atom, and examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group.

In formula (Z1), $Rz^3$ and $Rz^4$ each independently represent a hydrogen atom, a halogen atom or a C1 to C8 alkyl group. Examples of halogen atoms include a chlorine atom, a bromine atom, an iodine atom and a fluorine atom, and examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group.

In formula (Z1), for use as $Rz^3$ and $Rz^4$, a hydrogen atom, a methyl group, an ethyl group, a propyl group, an isopropyl group, a butyl group, an isobutyl group, a sec-butyl group and a tert-butyl group are preferred because they ensure that the polymer according to the present invention will exhibit good optical characteristics. A hydrogen atom, a methyl group and an ethyl group are particularly preferred.

Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system and annulated aromatic ring system optionally having a substituent.

Examples of monocyclic aromatic ring systems at Arz whose ring-constituting atoms are atoms selected from the group consisting of a carbon atom, a nitrogen atom, an oxygen atom and a sulfur atom and that may have a substituent include benzene, pyridine, pyrazine, pyrimidine, pyridazine, furan, pyrrole, imidazole, thiophene, pyrazole, oxazole, isoxazole, thiazole and triazole.

Examples of polycyclic aromatic ring systems at Arz whose ring-constituting atoms are atoms selected from the group consisting of a carbon atom, a nitrogen atom, an oxygen atom and a sulfur atom and that may have a substituent include biphenyl, terphenyl, bipyridine, bithiophene and bifuran.

Examples of annulated aromatic ring systems at Arz whose ring-constituting atoms are atoms selected from the group consisting of a carbon atom, a nitrogen atom, an oxygen atom and a sulfur atom and that may have a substituent include naphthalene, anthracene, phenanthrene, quinoline, benzofuran, benzimidazole, benzothiophene, indole, indazole, benzoxazole, benzothiazole and benzotriazole.

For use as Arz, a monocyclic aromatic ring system whose ring-constituting atoms are atoms selected from the group consisting of a carbon atom, a nitrogen atom, an oxygen atom and a sulfur atom and that may have a substituent is preferred because of easy availability. More preferably, this aromatic ring system is a benzene ring, a furan ring, a thiophene ring, a thiazole ring or an oxazole ring because this ensures that the polymer according to the present invention

will exhibit good optical characteristics. A benzene ring or thiophene ring is particularly preferred because they ensure that the polymer according to the present invention will exhibit better optical characteristics.

For use as R°, $R^1$, $R^2$, $R^3$ and $R^4$, a hydrogen atom, a methyl group, an ethyl group, a propyl group, an isopropyl group, a butyl group, an isobutyl group, a sec-butyl group, a tert-butyl group and the groups represented by chemical formulae (Z1-1) to (Z1-4) below are preferred because they ensure that the polymer according to the present invention will exhibit good optical characteristics. A hydrogen atom, a methyl group and an ethyl group are particularly preferred.

[Chem. 11]

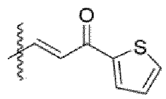
(Z1-1)

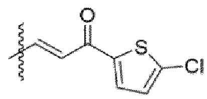
(Z1-2)

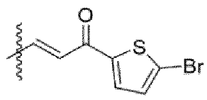
(Z1-3)

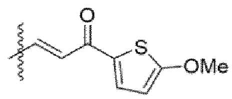
(Z1-4)

In formula (1), $L^1$ and $L^2$ may be the same or different from each other, each representing a carbonyl group, an ester linkage, an amide linkage, an ether linkage or a single bond.

For use as $L^1$ and $L^2$, a carbonyl group, an ester linkage or an ether linkage is preferred.

In formula (1), Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system and annulated aromatic ring system optionally having a substituent.

In this context, examples of substituents in the monocyclic aromatic ring system, polycyclic aromatic ring system and annulated aromatic ring system include a C1 to C8 alkyl group, a halogen atom, a C1 to C4 alkoxy group and a C2 to C4 acyl group.

Examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group.

Examples of halogen atoms include a fluorine atom, a chlorine atom, a bromine atom and an iodine atom.

Examples of C1 to C4 alkoxy groups include a methoxy group, an ethoxy group, a propyloxy group, an isopropyloxy group, a butyloxy group, an isobutyloxy group, a sec-butyloxy group and a tert-butyloxy group.

Examples of C2 to C4 acyl groups include an acetyl group, a propionyl group and a butyryl group.

Examples of monocyclic aromatic ring systems at Ar whose ring-constituting atoms are atoms selected from the group consisting of a carbon atom, a nitrogen atom, an oxygen atom and a sulfur atom and that may have a substituent include benzene, pyridine, pyrazine, pyrimidine, pyridazine, furan, pyrrole, imidazole, thiophene, pyrazole, oxazole, isoxazole, thiazole and triazole.

Examples of polycyclic aromatic ring systems at Ar whose ring-constituting atoms are atoms selected from the group consisting of a carbon atom, a nitrogen atom, an oxygen atom and a sulfur atom and that may have a substituent include biphenyl, terphenyl, bipyridine, bithiophene and bifuran.

Examples of annulated aromatic ring systems at Ar whose ring-constituting atoms are atoms selected from the group consisting of a carbon atom, a nitrogen atom, an oxygen atom and a sulfur atom and that may have a substituent include naphthalene, anthracene, phenanthrene, quinoline, benzofuran, benzimidazole, benzothiophene, indole, indazole, benzoxazole, benzothiazole and benzotriazole.

For use as Ar, a monocyclic aromatic ring system whose ring-constituting atoms are atoms selected from the group consisting of a carbon atom, a nitrogen atom, an oxygen atom and a sulfur atom and that may have a substituent is preferred because of easy availability. More preferably, this aromatic ring system is a benzene ring, a furan ring, a thiophene ring, a thiazole ring or an oxazole ring because this ensures that the polymer according to the present invention will exhibit good optical characteristics. A benzene ring or thiophene ring is particularly preferred because they ensure that the polymer according to the present invention will exhibit better optical characteristics.

Preferred examples of Ars include the structures indicated by chemical formulae (Ar-1) to (Ar-7) below.

[Chem. 12]

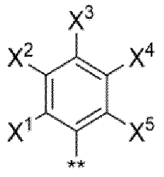
(Ar-1)

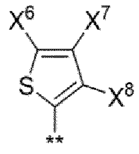
(Ar-2)

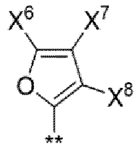
(Ar-3)

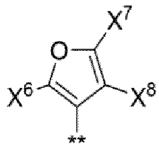
(Ar-4)

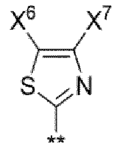
(Ar-5)

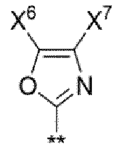
(Ar-6)

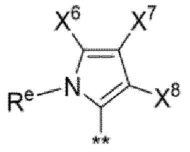
(Ar-7)

[In chemical formulae (Ar-1) to (Ar-7) above, $X^1$, $X^2$, $X^3$, $X^4$, $X^5$, $X^6$, $X^7$ and $X^8$ each independently represent a hydrogen atom a C1 to C8 alkyl group, a C1 to C6 alkoxy group, a halogen atom, a nitro group, a cyano group, a C1 to C6 alkylthio group or a C2 to C8 dialkylamino group.

$R^e$ represents a hydrogen atom or a C1 to C10 alkyl group.

** represents a site of binding with the other portion in chemical formula (1) above, excluding Ar.]

In formulae (Ar-1) to (Ar-7), $X^1$, $X^2$, $X^3$, $X^4$, $X^5$, $X^6$, $X^7$ and $X^8$ each independently represent a hydrogen atom, a C1 to C8 alkyl group, a C1 to C6 alkoxy group, a halogen atom, a nitro group, a cyano group, a C1 to C6 alkylthio group or a C2 to C8 dialkylamino group. Examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group, examples of C1 to C6 alkoxy groups include a methoxy group, an ethoxy group, a propyloxy group, an isopropyloxy group, a butyloxy group, an isobutyloxy group, a sec-butyloxy group, a tert-butyloxy group, a pentyloxy group and a hexyloxy group, examples of halogen atoms include a fluorine atom, a chlorine atom, a bromine atom and an iodine atom, examples of C1 to C6 alkylthio groups include a methylthio group, an ethylthio group, a propylthio group, an isopropylthio group, a butylthio group, an isobutylthio group, a sec-butylthio group, a tert-butylthio group, a pentylthio group and a hexylthio group, and examples of C2 to C8 dialkylamino groups include a dimethylamino group, a diethylamino group, a dipropylamino group and a dibutylamino group.

For $X^1$, $X^2$, $X^3$, $X^4$, $X^5$, $X^6$, $X^7$ and $X^8$, it is preferred that each be a hydrogen atom, a C1 to C8 alkyl group, a C1 to C6 alkoxy group or a halogen atom because this leads to superior optical characteristics of the polymer according to the present invention. It is particularly preferred that each be a hydrogen atom, a methyl group, a methoxy group or a halogen atom because of the ease of introduction.

In (Ar-1) to (Ar-7), $R^e$ represents a hydrogen atom or a C1 to C10 alkyl group. Examples of C1 to C10 alkyl groups include a methyl group, an ethyl group, a propyl group, an isopropyl group, a butyl group, an isobutyl group, a sec-butyl group, a tert-butyl group, a pentyl group, a hexyl group, a heptyl group, an octyl group, a nonyl group and a decyl group.

For $R^e$, it is preferred that it be a hydrogen atom, a methyl group or a C1 to C4 alkyl group because this leads to superior optical characteristics of the polymer according to the present invention. It is particularly preferred that $R^e$ be a hydrogen atom, a methyl group, an ethyl group, a propyl group, a butyl group, an isobutyl group, a sec-butyl group or a tert-butyl group.

Specific examples of photoreactive reverse wavelength dispersion units A indicated by general formula (1) include the photoreactive reverse wavelength dispersion units A indicated by chemical formulae (1-1-1) to (1-7-4) below.

[Chem. 13]

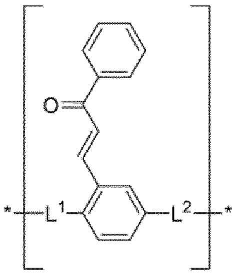

(1-1-1)

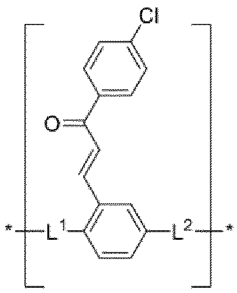

(1-1-2)

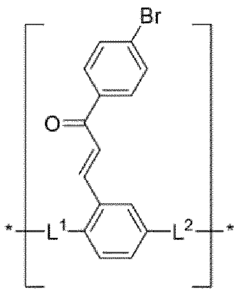

(1-1-3)

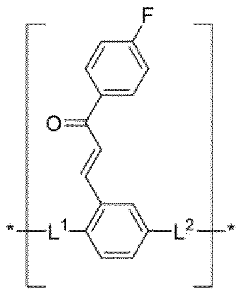

(1-1-4)

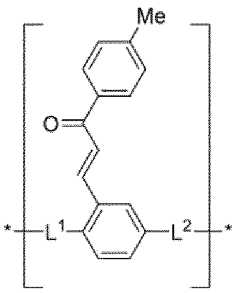

(1-1-5)

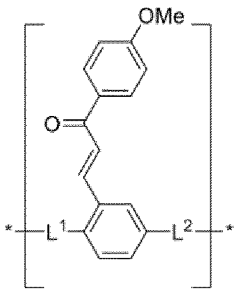

(1-1-6)

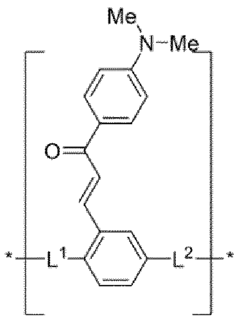

(1-1-7)

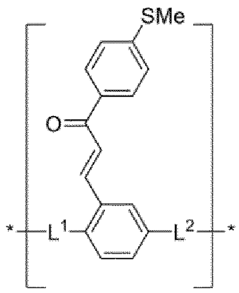

(1-1-8)

[Chem. 14]

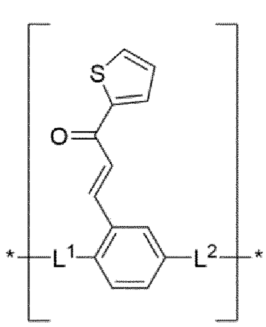

(1-2-1)

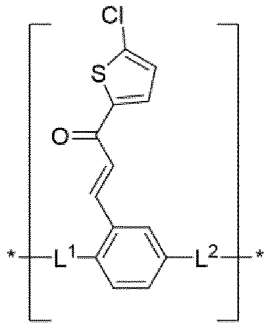

(1-2-2)

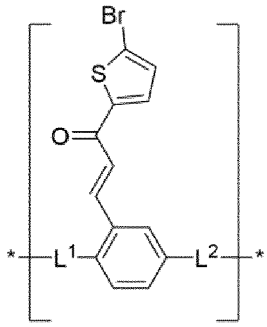

(1-2-3)

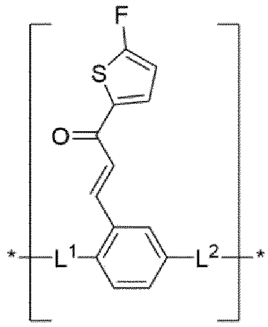

(1-2-4)

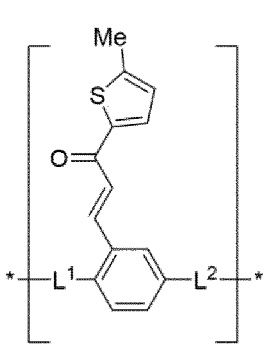

(1-2-5)

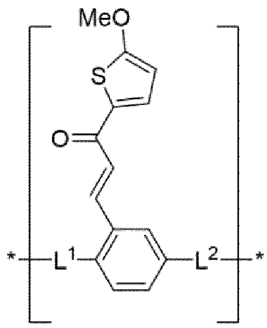

(1-2-6)

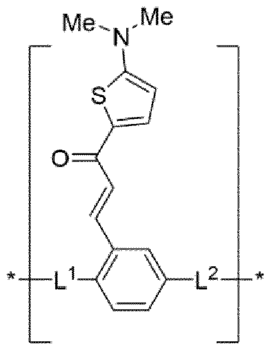

(1-2-7)

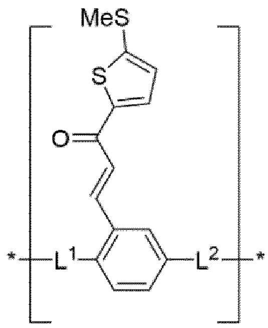

(1-2-8)

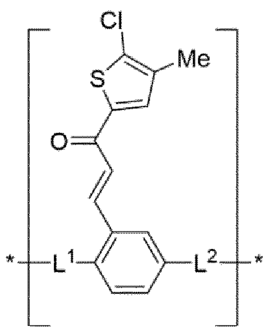

(1-2-9)

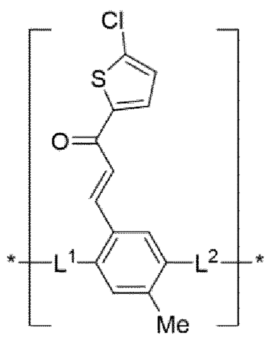

(1-2-10)

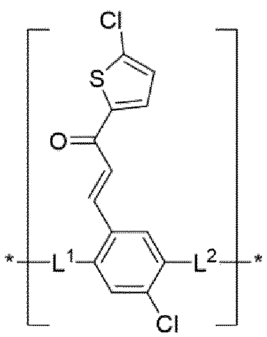

(1-2-11)

[Chem. 15]

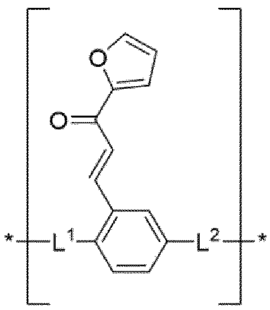

(1-3-1)

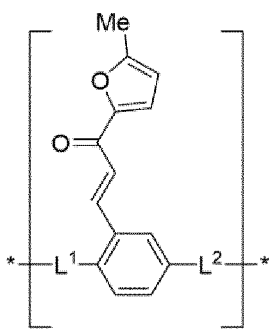

(1-3-2)

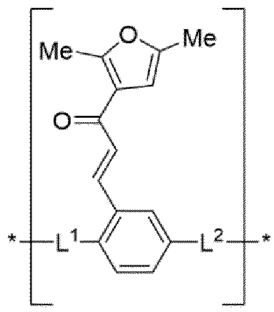

(1-3-3)

[Chem. 16]

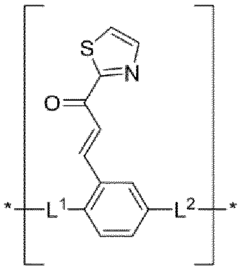

(1-4-1)

[Chem. 17]

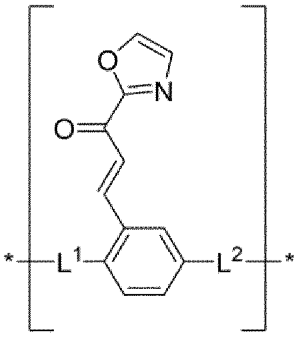

(1-5-1)

[Chem. 18]

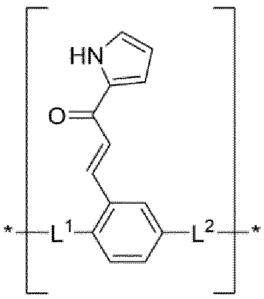

(1-6-1)

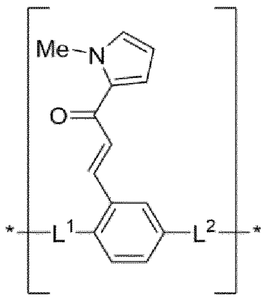

(1-6-2)

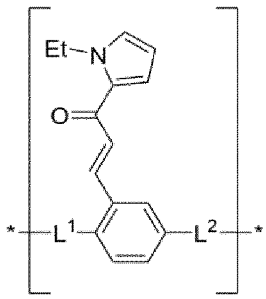

(1-6-3)

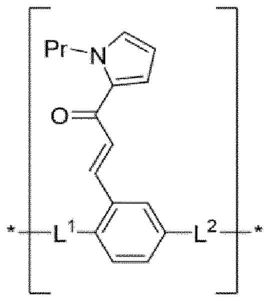

(1-6-4)

[Chem. 19]

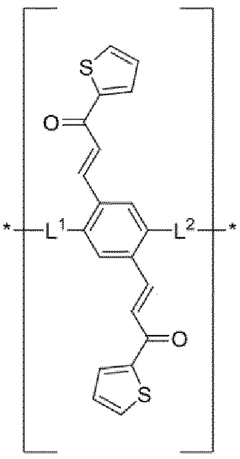
(1-7-1)

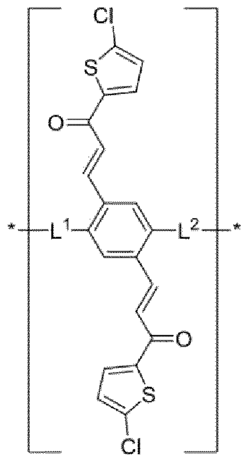
(1-7-2)

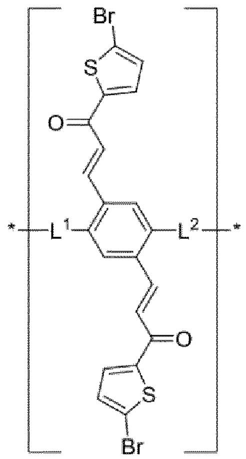
(1-7-3)

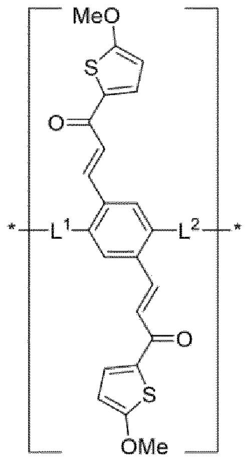
(1-7-4)

[In formulae (1-1-1) to (1-7-4), $L^1$ and $L^2$ may be the same or different from each other, each representing a carbonyl group, an ester linkage, an amide linkage, an ether linkage or a single bond.
* represents a site of binding with another structure in the main chain polymer.]

Of these (1-1-1) to (1-7-4), particularly preferred units are (1-1-1) to (1-2-11), (1-6-1) to (1-6-4) and (1-7-1) to (1-7-4), with especially preferred ones being (1-2-1) to (1-2-8) and (1-7-1) to (1-7-3).

The polymer according to the present invention preferably further comprises at least one type of repeat unit selected from the group consisting of chemical formulae (2A), (2B), (2C) and (2D) below.

[Chem. 20]

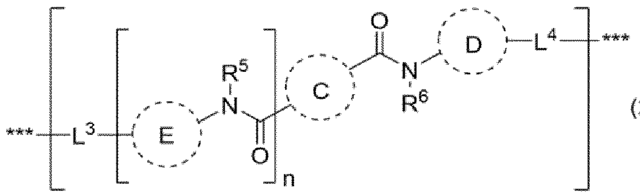
(2A)

[In chemical formula (2A) above, ring C, ring D and ring E each independently represent a ring system selected from the group consisting of a monocyclic aromatic ring system, a polycyclic aromatic ring system, an annulated aromatic ring system and an aliphatic hydrocarbon ring system whose ring-constituting atoms are atoms selected from the group consisting of a carbon atom, a nitrogen atom, an oxygen atom and a sulfur atom, with the monocyclic aromatic ring system, polycyclic aromatic ring system, annulated aromatic ring system and aliphatic hydrocarbon ring system optionally having a substituent.
$R^5$ and $R^6$ may be the same or different from each other, each representing a group selected from the group consisting of a hydrogen atom, a substituted or unsubstituted C1 to C20 alkyl group, a substituted or unsubstituted C3 to C8 cycloalkyl group and a substituted or unsubstituted C3 to C12 aromatic group.
n is 0 or 1.
$L^3$ and $L^4$ may be the same or different from each other, each representing a carbonyl group, an ester linkage, an amide linkage, an ether linkage or a single bond.
*** represents a site of binding with another structure in the main chain polymer.]

In formula (2A), ring C, ring D and ring E each independently represent a ring system selected from the group consisting of a monocyclic aromatic ring system, a polycyclic aromatic ring system, an annulated aromatic ring system and an aliphatic hydrocarbon ring system whose ring-constituting atoms are atoms selected from the group consisting of a carbon atom, a nitrogen atom, an oxygen atom and a sulfur atom, with the monocyclic aromatic ring system, polycyclic aromatic ring system, annulated aromatic ring system and aliphatic hydrocarbon ring system optionally having a substituent.

Specific examples of monocyclic aromatic ring systems at ring C whose ring-constituting atoms are atoms selected from the group consisting of a carbon atom, a nitrogen atom, an oxygen atom and a sulfur atom and that may

have a substituent include benzene, pyridine, pyrazine, pyrimidine, pyridazine, furan, pyrrole, imidazole, thiophene, pyrazole, oxazole, isoxazole, thiazole and triazole.

Examples of polycyclic aromatic ring systems at ring C whose ring-constituting atoms are atoms selected from the group consisting of a carbon atom, a nitrogen atom, an oxygen atom and a sulfur atom and that may have a substituent include biphenyl, terphenyl, bipyridine, bithiophene and bifuran.

Examples of annulated aromatic ring systems at ring C whose ring-constituting atoms are atoms selected from the group consisting of a carbon atom, a nitrogen atom, an oxygen atom and a sulfur atom and that may have a substituent include naphthalene, anthracene, phenanthrene, quinoline, benzofuran, benzimidazole, benzothiophene, indole, indazole, benzoxazole, benzothiazole and benzotriazole.

Specific examples of aliphatic hydrocarbon ring systems at ring C whose ring-constituting atoms are atoms selected from the group consisting of a carbon atom, a nitrogen atom, an oxygen atom and a sulfur atom and that may have a substituent include cyclopentane, cyclohexane and tricyclo[5.2.1.0(2,6)]decane.

For use as ring C, a monocyclic aromatic ring system whose ring-constituting atoms are atoms selected from the group consisting of a carbon atom, a nitrogen atom, an oxygen atom and a sulfur atom and that may have a substituent is preferred because of easy availability of source carboxylic acids. It is particularly preferred that ring C be a benzene ring because of the ease of introduction.

In formula (2A), ring D and ring E each independently represent a ring system selected from the group consisting of a monocyclic aromatic ring system, a polycyclic aromatic ring system, an annulated aromatic ring system and an aliphatic hydrocarbon ring system whose ring-constituting atoms are atoms selected from the group consisting of a carbon atom, a nitrogen atom, an oxygen atom and a sulfur atom, with the monocyclic aromatic ring system, polycyclic aromatic ring system, annulated aromatic ring system and aliphatic hydrocarbon ring system optionally having a substituent.

Examples of specific rings D and E are, independently for each ring, the same as for ring C. A monocyclic aromatic ring system whose ring-constituting atoms are atoms selected from the group consisting of a carbon atom, a nitrogen atom, an oxygen atom and a sulfur atom is preferred. It is particularly preferred that rings D and E be benzene rings because of the ease of introduction.

In this context, examples of substituents in the monocyclic aromatic ring system, polycyclic aromatic ring system, annulated aromatic ring system and aliphatic hydrocarbon ring system include a C1 to C8 alkyl group, a halogen atom, a C1 to C4 alkoxy group and a C2 to C4 acyl group. Examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group, examples of halogen atoms include a fluorine atom, a chlorine atom, a bromine atom and an iodine atom, examples of C1 to C4 alkoxy groups include a methoxy group, an ethoxy group, a propyloxy group, an isopropyloxy group, a butyloxy group, an isobutyloxy group, a sec-butyloxy group and a tert-butyloxy group, and examples of C2 to C4 acyl groups include an acetyl group, a propionyl group and a butyryl group.

In formula (2A), $R^5$ and $R^6$ may be the same or different from each other, each representing a group selected from the group consisting of a hydrogen atom, a substituted or unsubstituted C1 to C20 alkyl group, a substituted or unsubstituted C3 to C8 cycloalkyl group and a substituted or unsubstituted C3 to C12 aromatic group.

Examples of C1 to C20 alkyl groups include a methyl group, an ethyl group, a propyl group, an isopropyl group, a butyl group, an isobutyl group, a sec-butyl group, a tert-butyl group, a pentyl group, a hexyl group, a heptyl group, an octyl group, a nonyl group, a decyl group and an icosyl group. Of these, a methyl group, an ethyl group, a propyl group, an isopropyl group, a butyl group, an isobutyl group, a sec-butyl group and a tert-butyl group are particularly preferred, with a methyl group and an ethyl group being especially preferred because of the ease of introduction.

Examples of C3 to C8 cycloalkyl groups include a cyclopropyl group, a cyclobutyl group, a cyclopentyl group, a cyclohexyl group, a cycloheptyl group and a cyclooctyl group.

Examples of C3 to C12 aromatic groups include a phenyl group, a naphthyl group, a biphenylyl group and a pyridyl group.

[Chem. 21]

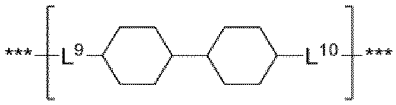 (2B)

[In chemical formula (2B) above, $L^9$ and $L^{10}$ may be the same or different from each other, each representing a carbonyl group, an ester linkage, an amide linkage, an ether linkage or a single bond.
*** represents a site of binding with another structure in the main chain polymer.]

[Chem. 22]

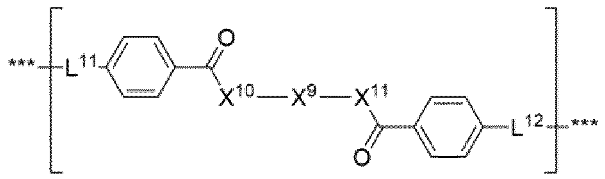

(2C)

[In chemical formula (2C) above, $X^9$ represents a C1 to C10 alkylene chain or a single bond.
$X^{10}$ represents -O- or -N($R^c$)-.
$X^{11}$ represents -O- or -N($R^d$)-.
$R^c$ and $R^d$ may be the same or different from each other, each representing a hydrogen atom or a C1 to C10 alkyl group.
$L^{11}$ and $L^{12}$ may be the same or different from each other, each representing a carbonyl group, an ester linkage, an amide linkage, an ether linkage or a single bond.
*** represents a site of binding with another structure in the main chain polymer.]

[Chem. 23]

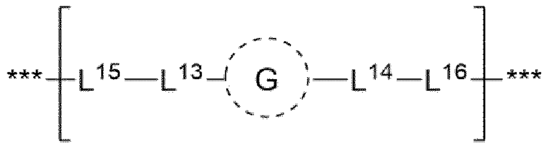

(2D)

[In chemical formula (2D) above, ring G represents a ring system selected from the group consisting of a monocyclic aromatic ring system, a polycyclic aromatic ring system, an annulated aromatic ring system, a spiro ring system and an aliphatic hydrocarbon ring system whose ring-constituting atoms are atoms selected from the group consisting of a carbon atom, a nitrogen atom, an oxygen atom and a sulfur atom, with the monocyclic aromatic ring system, polycyclic aromatic ring system, annulated aromatic ring system, spiro ring system and aliphatic hydrocarbon ring system optionally having a substituent.
$L^{13}$ and $L^{14}$ may be the same or different from each other, each representing a single bond or a C1 to C6 alkylene chain.
$L^{15}$ and $L^{16}$ may be the same or different from each other, each representing a carbonyl group, an ester linkage, an amide linkage, an ether linkage or a single bond.
*** represents a site of binding with another structure in the main chain polymer.]

In formula (2D), rings G each independently represent a ring system selected from the group consisting of a monocyclic aromatic ring system, a polycyclic aromatic ring system, an annulated aromatic ring system, a spiro ring system and an aliphatic hydrocarbon ring system whose ring-constituting atoms are atoms selected from the group consisting of a carbon atom, a nitrogen atom, an oxygen atom and a sulfur atom, with the monocyclic aromatic ring system, polycyclic aromatic ring system, annulated aromatic ring system, spiro ring system and aliphatic hydrocarbon ring system optionally having a substituent.

Specific examples of monocyclic aromatic ring systems at ring G whose ring-constituting atoms are atoms selected from the group consisting of a carbon atom, a nitrogen atom, an oxygen atom and a sulfur atom and that may have a substituent include benzene, pyridine, pyrazine, pyrimidine, pyridazine, furan, pyrrole, imidazole, thiophene, pyrazole, oxazole, isoxazole, thiazole and triazole.

Examples of polycyclic aromatic ring systems at ring G whose ring-constituting atoms are atoms selected from the group consisting of a carbon atom, a nitrogen atom, an oxygen atom and a sulfur atom and that may have a substituent include biphenyl, terphenyl, bipyridine, bithiophene and bifuran.

Examples of annulated aromatic ring systems at ring G whose ring-constituting atoms are atoms selected from the group consisting of a carbon atom, a nitrogen atom, an oxygen atom and a sulfur atom and that may have a substituent include naphthalene, anthracene, phenanthrene, quinoline, benzofuran, benzimidazole, benzothiophene, indole, indazole, benzoxazole, benzothiazole and benzotriazole.

An example of a spiro ring system at ring G whose ring-constituting atoms are atoms selected from the group consisting of a carbon atom, a nitrogen atom, an oxygen atom and a sulfur atom and that may have a substituent is the structure indicated by chemical formula (sp1-1) below.

[Chem. 24]

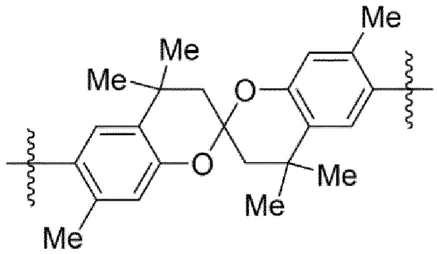

(sp1-1)

Examples of aliphatic hydrocarbon ring systems at ring G whose ring-constituting atoms are atoms selected from the group consisting of a carbon atom, a nitrogen atom, an oxygen atom and a sulfur atom and that may have a substituent include cyclopropane, cyclopentane, cyclohexane and tricyclo[5.2.1.0(2,6)]decane.

For use as ring G, a monocyclic aromatic ring system or aliphatic hydrocarbon ring system whose ring-constituting atoms are atoms selected from the group consisting of a carbon atom, a nitrogen atom, an oxygen atom and a sulfur atom is preferred because of easy availability. It is particularly preferred that ring G be a benzene ring, cyclohexane or tricyclo[5.2.1.0(2,6)]decane because this leads to good optical characteristics.

A preferred example of a repeat unit indicated by general formula (2A) is a repeat unit indicated by chemical formula (2'A) below.

[Chem. 25]

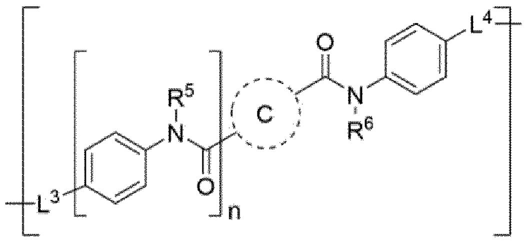

(2'A)

In formula (2'A), ring C, $R^5$, $R^6$, $L^3$, $L^4$ and n are synonymous with ring C, $R^5$, $R^6$, $L^3$, $L^4$ and n, respectively, in formula (2A).

Specific examples of repeat units indicated by general formula (2A) include the structures indicated by chemical formulae (2A-1-1) to (2A-2-20) below.

[Chem. 26]

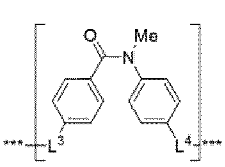
(2A-1-1)

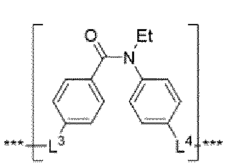
(2A-1-2)

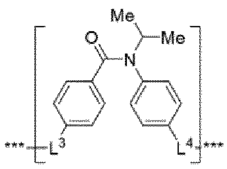
(2A-1-3)

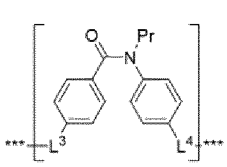
(2A-1-4)

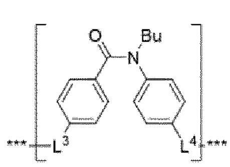
(2A-1-5)

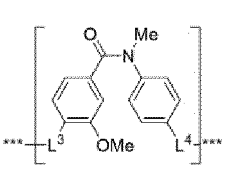
(2A-1-6)

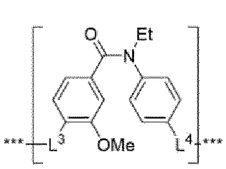
(2A-1-7)

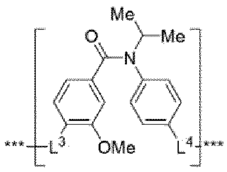
(2A-1-8)

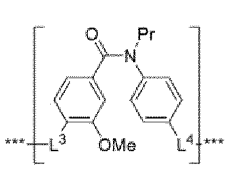
(2A-1-9)

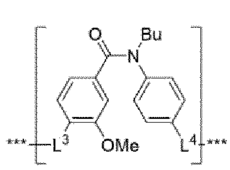
(2A-1-10)

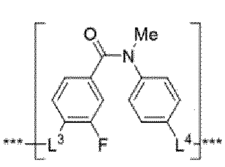
(2A-1-11)

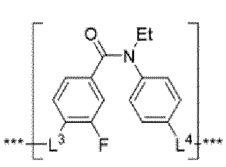
(2A-1-12)

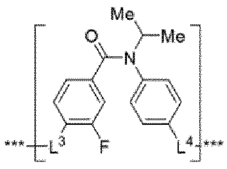
(2A-1-13)

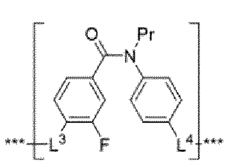
(2A-1-14)

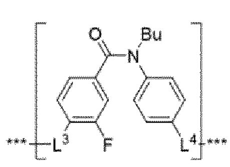
(2A-1-15)

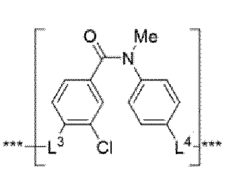
(2A-1-16)

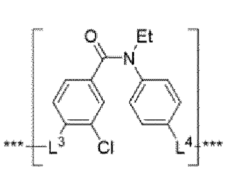
(2A-1-17)

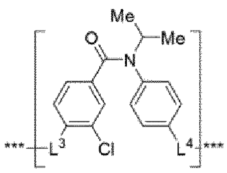
(2A-1-18)

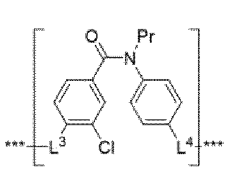
(2A-1-19)

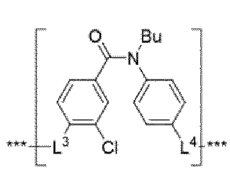
(2A-1-20)

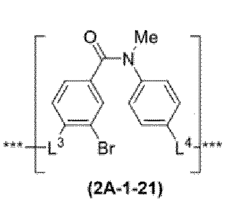
(2A-1-21)

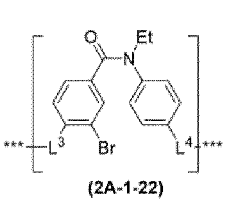
(2A-1-22)

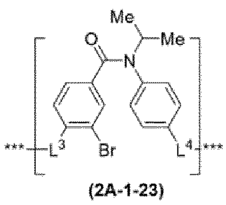
(2A-1-23)

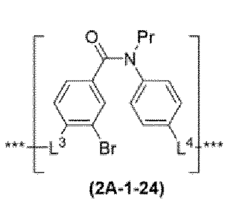
(2A-1-24)

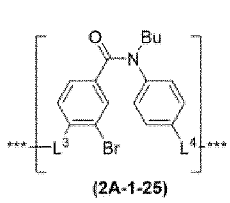
(2A-1-25)

[Chem. 27]

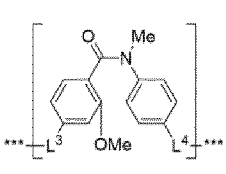
(2A-1-26)
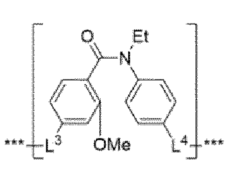
(2A-1-27)
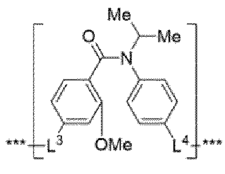
(2A-1-28)
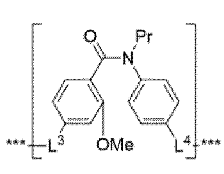
(2A-1-29)
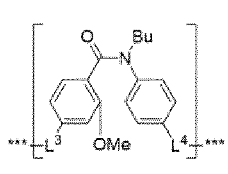
(2A-1-30)

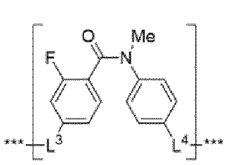
(2A-1-31)
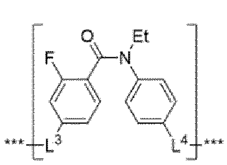
(2A-1-32)
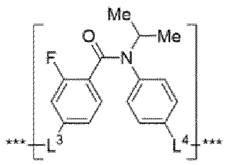
(2A-1-33)
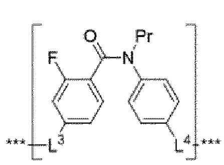
(2A-1-34)
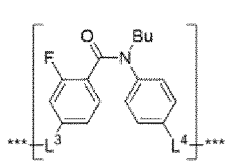
(2A-1-35)

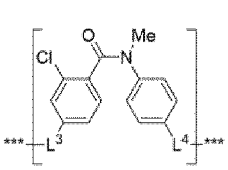
(2A-1-36)
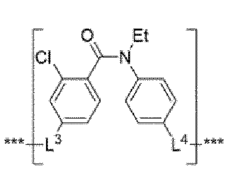
(2A-1-37)
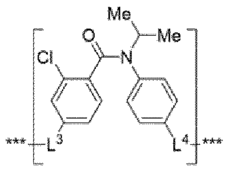
(2A-1-38)
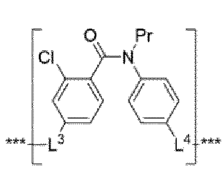
(2A-1-39)
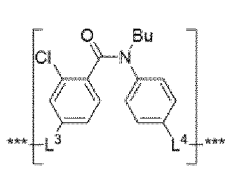
(2A-1-40)

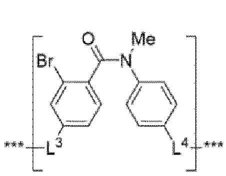
(2A-1-41)
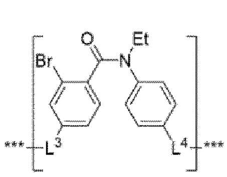
(2A-1-42)
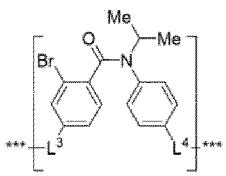
(2A-1-43)
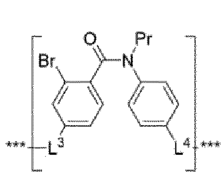
(2A-1-44)
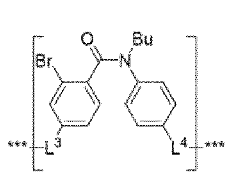
(2A-1-45)

[Chem. 28]

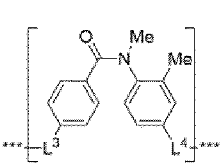
(2A-1-46)
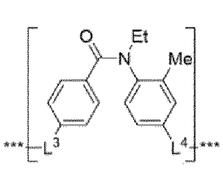
(2A-1-47)
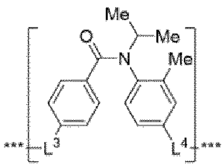
(2A-1-48)
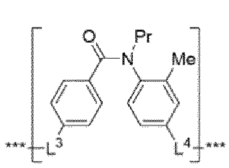
(2A-1-49)
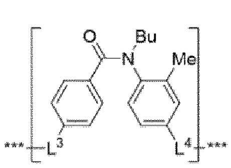
(2A-1-50)

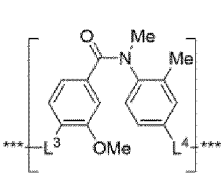
(2A-1-51)
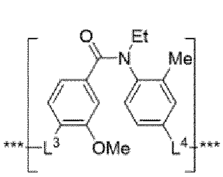
(2A-1-52)
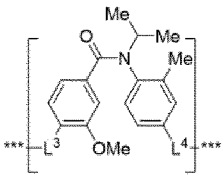
(2A-1-53)
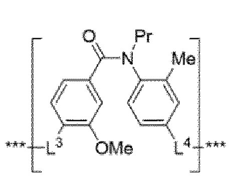
(2A-1-54)
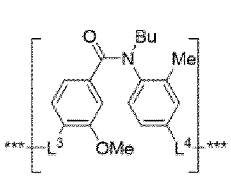
(2A-1-55)

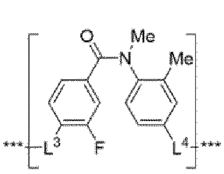
(2A-1-56)
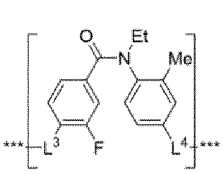
(2A-1-57)
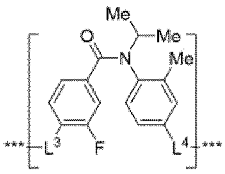
(2A-1-58)
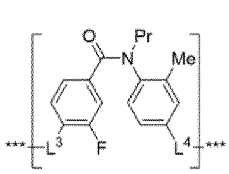
(2A-1-59)
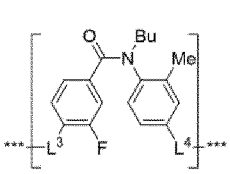
(2A-1-60)

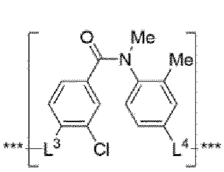
(2A-1-61)
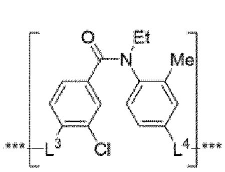
(2A-1-62)
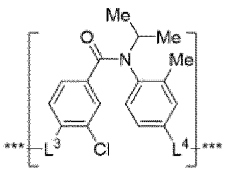
(2A-1-63)
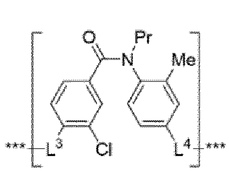
(2A-1-64)
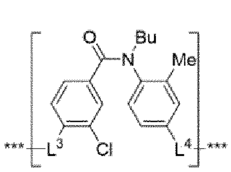
(2A-1-65)

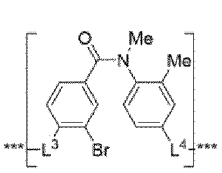
(2A-1-66)
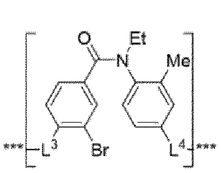
(2A-1-67)
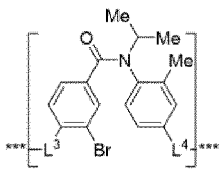
(2A-1-68)
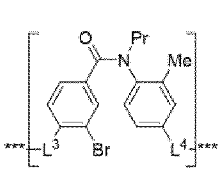
(2A-1-69)
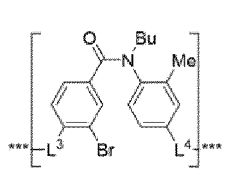
(2A-1-70)

[Chem. 29]

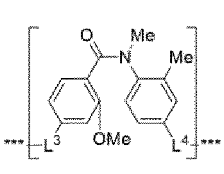
(2A-1-71)
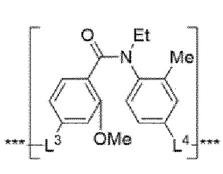
(2A-1-72)
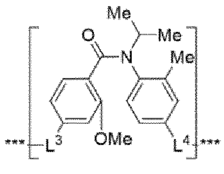
(2A-1-73)
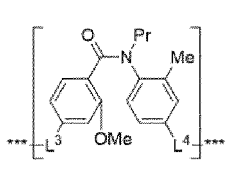
(2A-1-74)
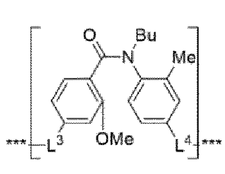
(2A-1-75)

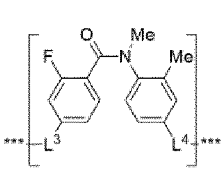
(2A-1-76)
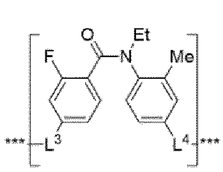
(2A-1-77)
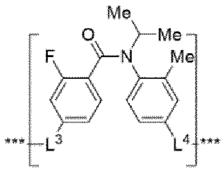
(2A-1-78)
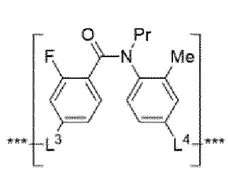
(2A-1-79)
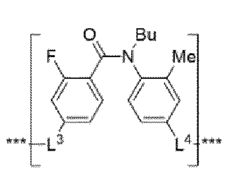
(2A-1-80)

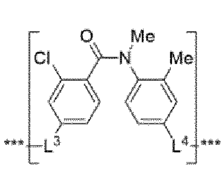
(2A-1-81)
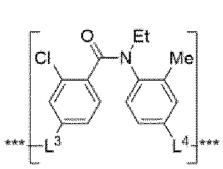
(2A-1-82)
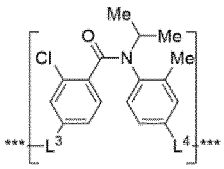
(2A-1-83)
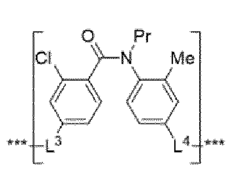
(2A-1-84)
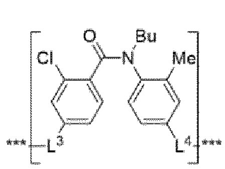
(2A-1-85)

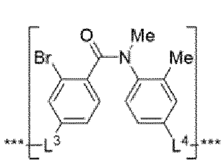
(2A-1-86)
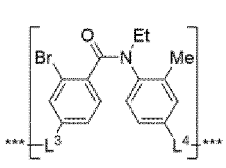
(2A-1-87)
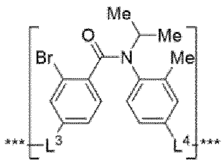
(2A-1-88)
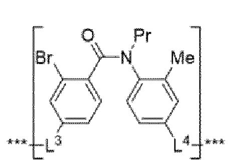
(2A-1-89)
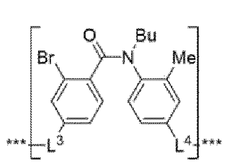
(2A-1-90)

[Chem. 30]

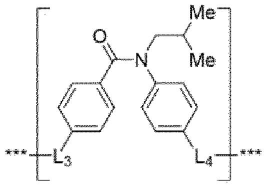
(2A-1-91)

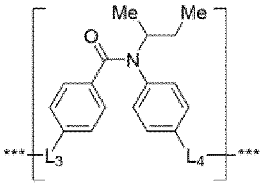
(2A-1-92)

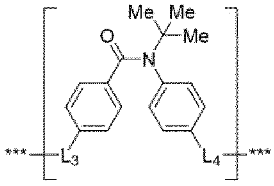
(2A-1-93)

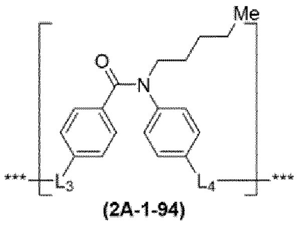
(2A-1-94)

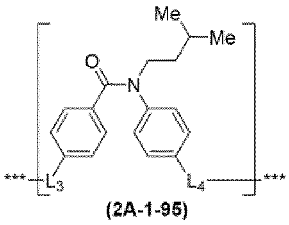
(2A-1-95)

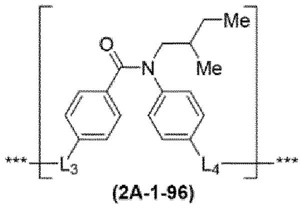
(2A-1-96)

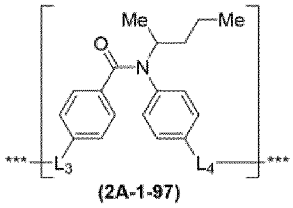
(2A-1-97)

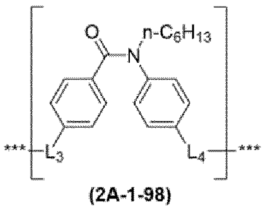
(2A-1-98)

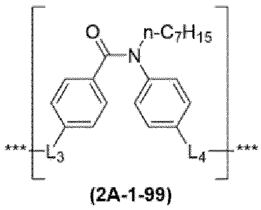
(2A-1-99)

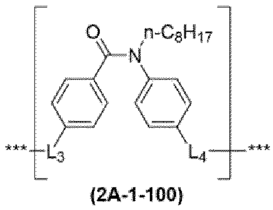
(2A-1-100)

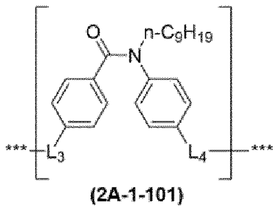
(2A-1-101)

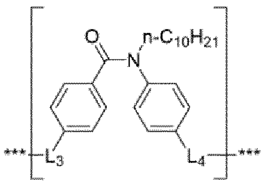
(2A-1-102)

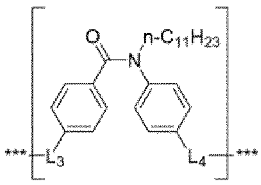
(2A-1-103)

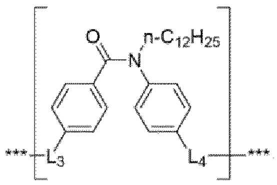
(2A-1-104)

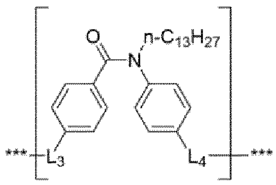
(2A-1-105)

[Chem. 31]

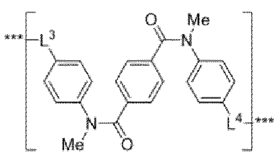
(2A-2-1)
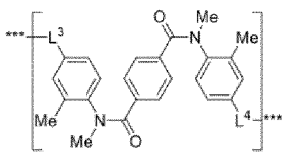
(2A-2-2)
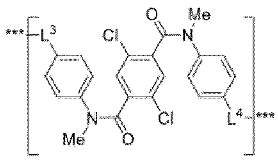
(2A-2-3)
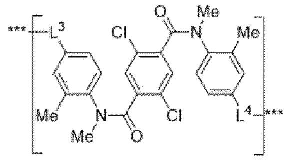
(2A-2-4)

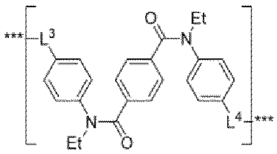
(2A-2-5)
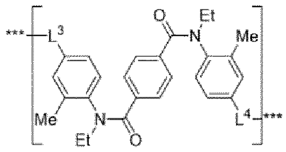
(2A-2-6)
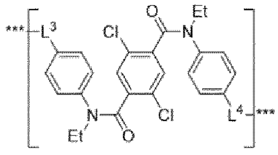
(2A-2-7)
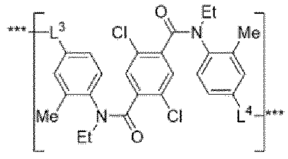
(2A-2-8)

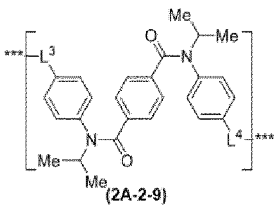
(2A-2-9)
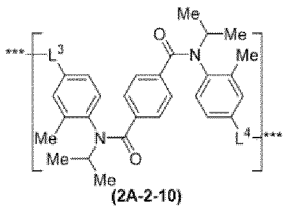
(2A-2-10)
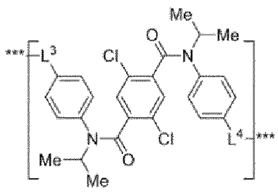
(2A-2-11)
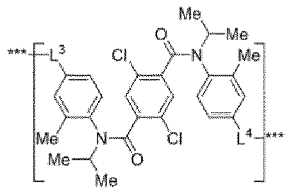
(2A-2-12)

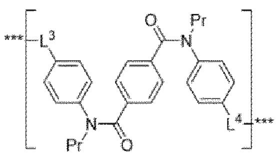
(2A-2-13)
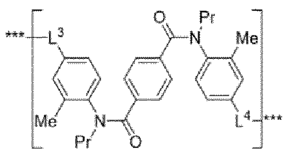
(2A-2-14)
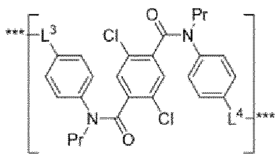
(2A-2-15)
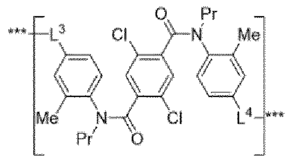
(2A-2-16)

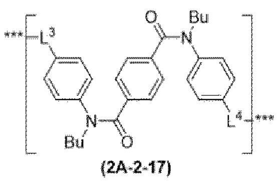
(2A-2-17)
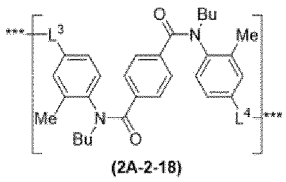
(2A-2-18)
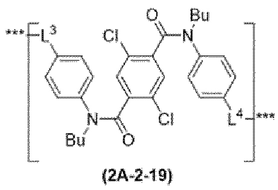
(2A-2-19)
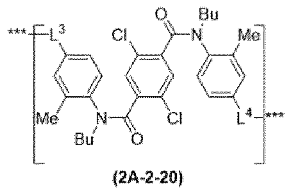
(2A-2-20)

[In formulae (2A-1-1) to (2A-2-20), $L^3$ and $L^4$ may be the same or different from each other, each representing a carbonyl group, an ester linkage, an amide linkage, an ether linkage or a single bond.]

Of these (2A-1-1) to (2A-2-20), particularly preferred units are (2A-1-1) to (2A-1-10), (2A-1-36) to (2A-1-40), (2A-1-51) to (2A-1-55), (2A-1-91) to (2A-1-105) and (2A-2-1) to (2A-2-4) because they allow for easier synthesis of a source monomer for the polymer according to the present invention, with (2A-1-1) to (2A-1-10), (2A-1-36) to (2A-1-40), (2A-1-91) to (2A-1-105), (2A-1-51) to (2A-1-55) and (2A-1-104) being especially preferred because they lead to superior optical characteristics of the polymer according to the present invention.

Specific examples of repeat units indicated by general formula (2B) include the structures indicated by chemical formulae (2B-1) to (2B-3) below.

[Chem. 32]

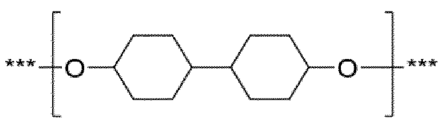
(2B-1)
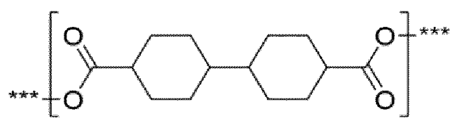
(2B-2)

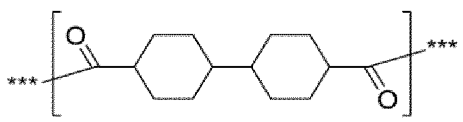
(2B-3)

Of these (2B-1) to (2B-3), (2B-1) is particularly preferred because it leads to superior optical characteristics of the polymer according to the present invention.

Specific examples of repeat units indicated by general formula (2C) include the structures indicated by chemical formulae (2C-1-1) to (2C-2-23) below.

[Chem. 33]

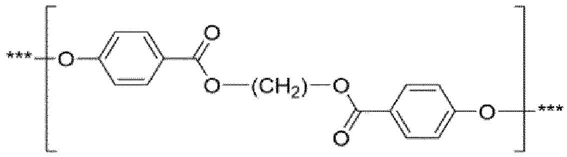

(2C-1-1)

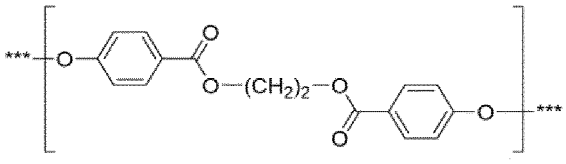

(2C-1-2)

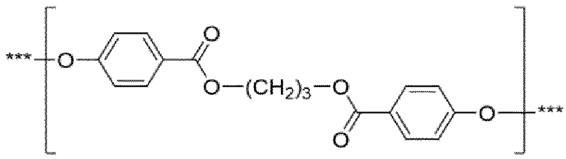

(2C-1-3)

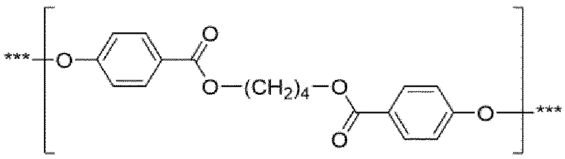

(2C-1-4)

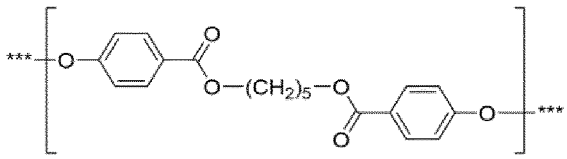

(2C-1-5)

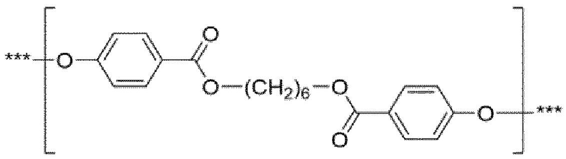

(2C-1-6)

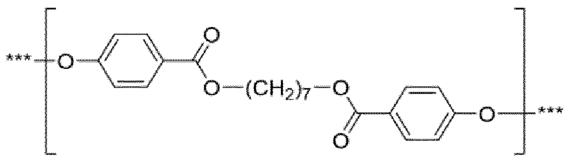

(2C-1-7)

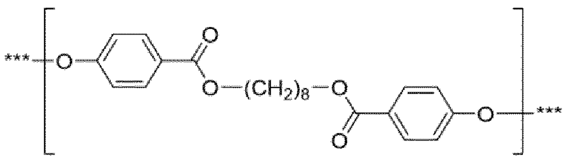

(2C-1-8)

[Chem. 34]

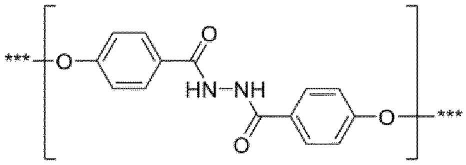
(2C-2-1)

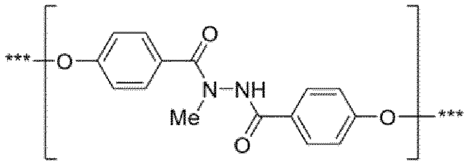
(2C-2-2)

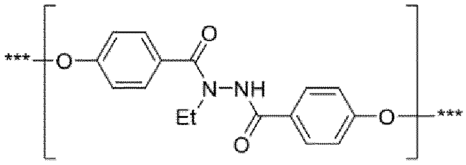
(2C-2-3)

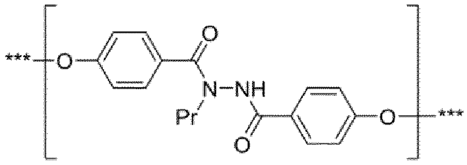
(2C-2-4)

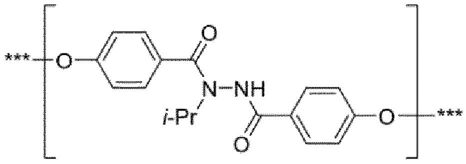
(2C-2-5)

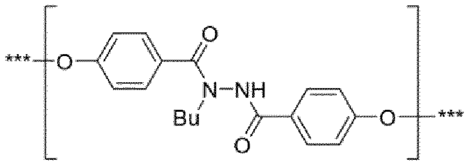
(2C-2-6)

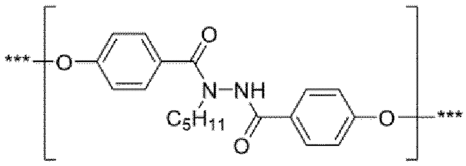
(2C-2-7)

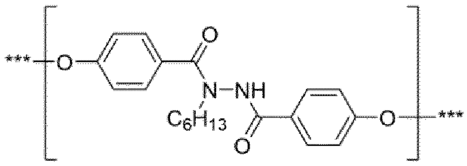
(2C-2-8)

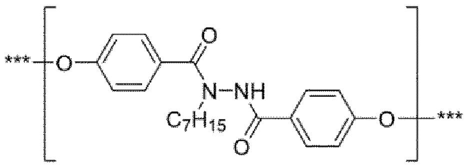
(2C-2-9)

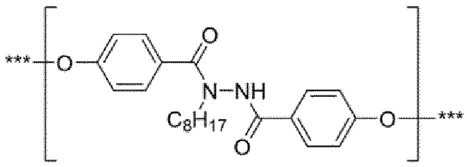
(2C-2-10)

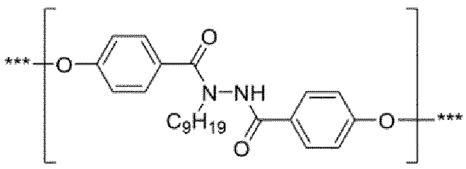
(2C-2-11)

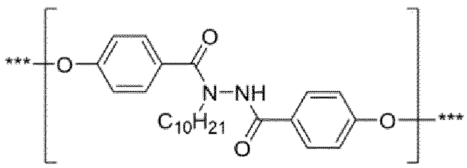
(2C-2-12)

[Chem. 35]

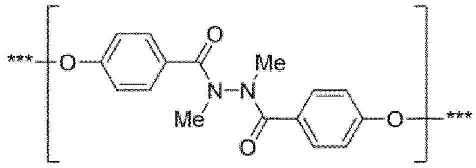

(2C-2-13)

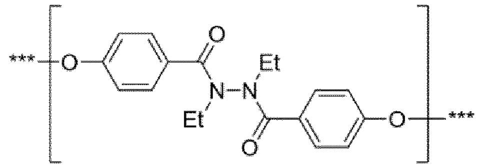

(2C-2-14)

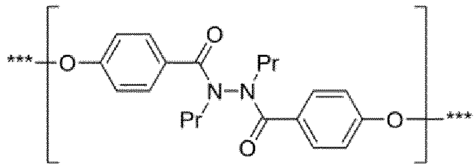

(2C-2-15)

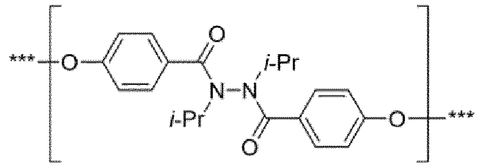

(2C-2-16)

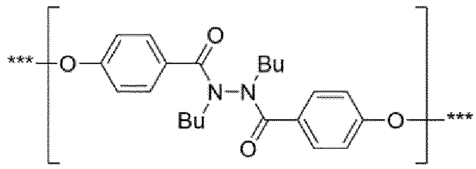

(2C-2-17)

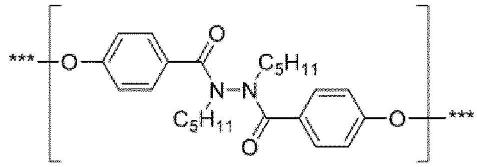

(2C-2-18)

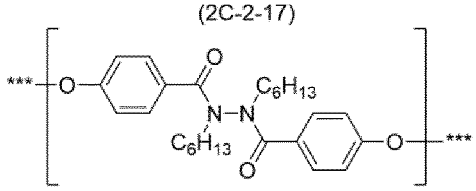

(2C-2-19)

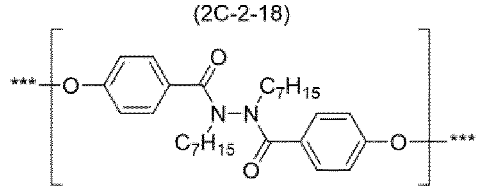

(2C-2-20)

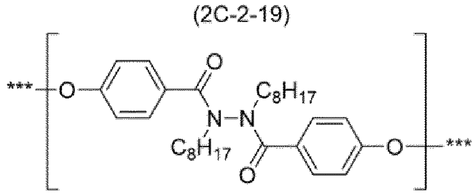

(2C-2-21)

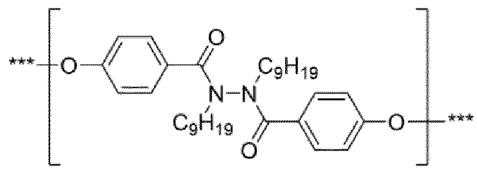

(2C-2-22)

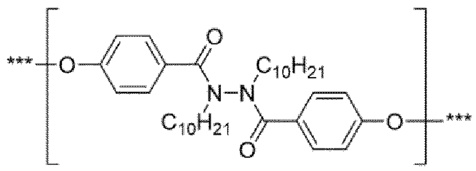

(2C-2-23)

Of these (2C-1-1) to (2C-2-23), (2C-1-1) to (2C-1-8), (2C-2-2) to (2C-2-6) and (2C-2-13) to (2C-2-19) are particularly preferred because they lead to superior optical characteristics of the polymer according to the present invention.

Specific examples of repeat units indicated by general formula (2D) include the structures indicated by chemical formulae (2D-1-1) to (2D-14-1) below.

[Chem. 36]

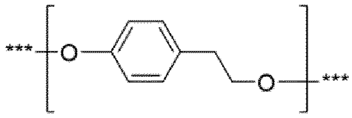
(2D-1-1)

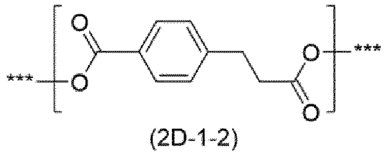
(2D-1-2)

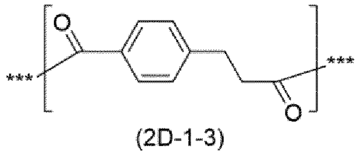
(2D-1-3)

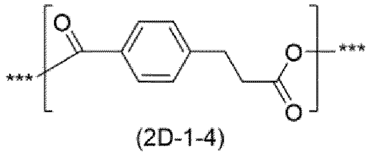
(2D-1-4)

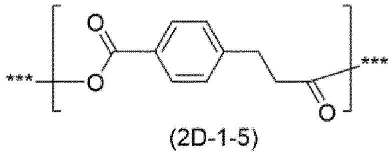
(2D-1-5)

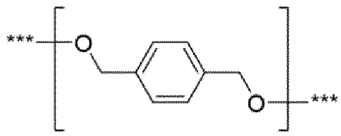
(2D-2-1)

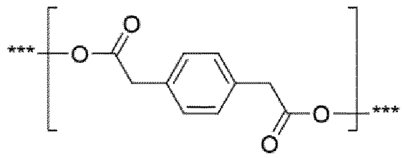
(2D-2-2)

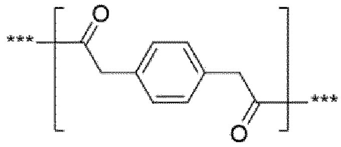
(2D-2-3)

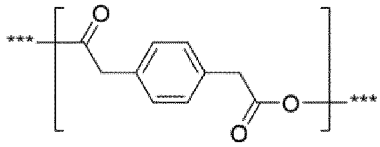
(2D-2-4)

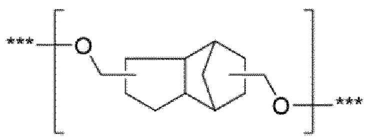
(2D-3-1)

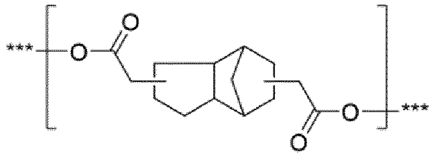
(2D-3-2)

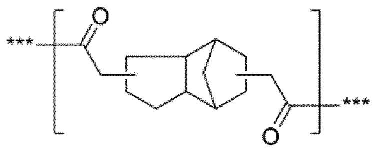
(2D-3-3)

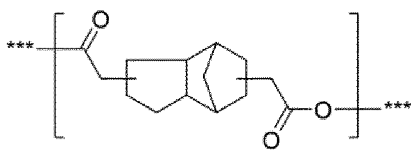
(2D-3-3)

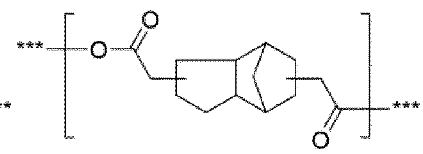
(2D-3-3)

[Chem. 37]

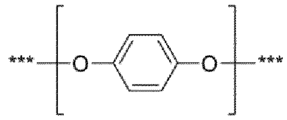
(2D-4-1)

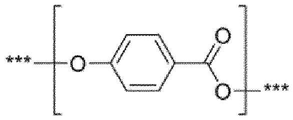
(2D-4-2)

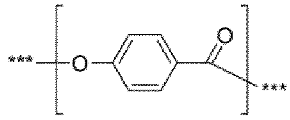
(2D-4-3)

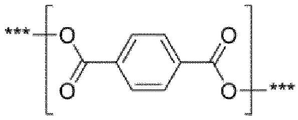
(2D-4-4)

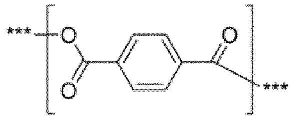
(2D-4-5)

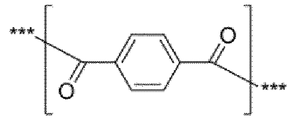
(2D-4-6)

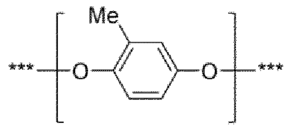
(2D-5-1)

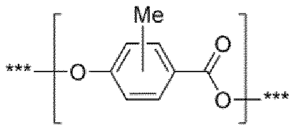
(2D-5-2)

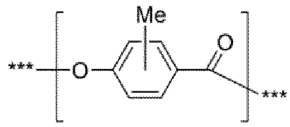
(2D-5-3)

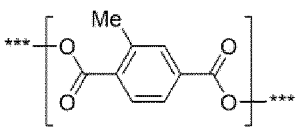
(2D-5-4)

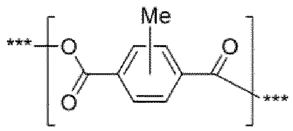
(2D-5-5)

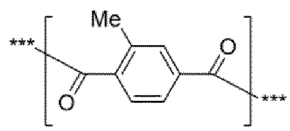
(2D-5-6)

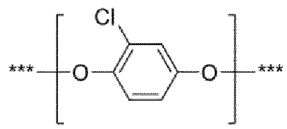
(2D-6-1)

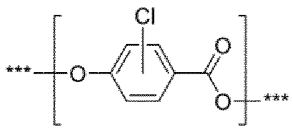
(2D-6-2)

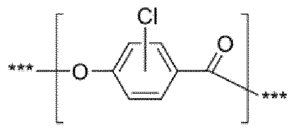
(2D-6-3)

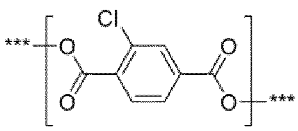
(2D-6-4)

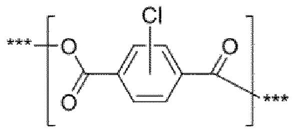
(2D-6-5)

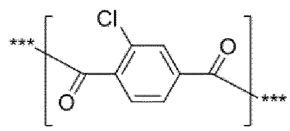
(2D-6-6)

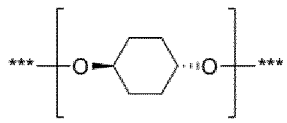
(2D-7-1)

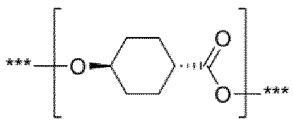
(2D-7-2)

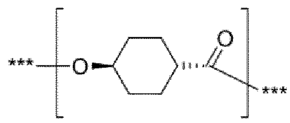
(2D-7-3)

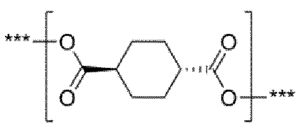
(2D-7-4)

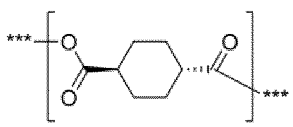
(2D-7-5)

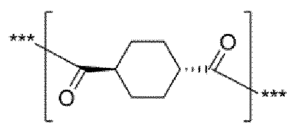
(2D-7-6)

[Chem. 38]

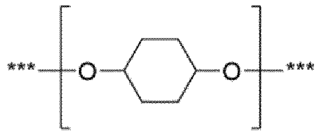
(2D-7-7)

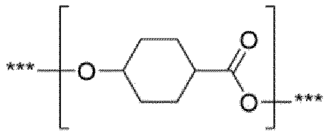
(2D-7-8)

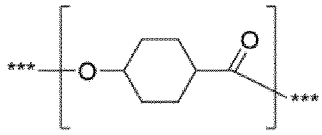
(2D-7-9)

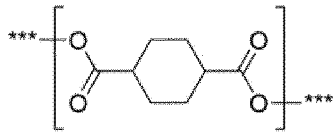
(2D-7-10)

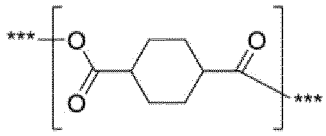
(2D-7-11)

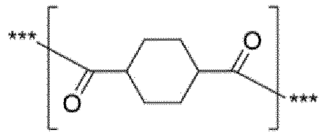
(2D-7-12)

[Chem. 39]

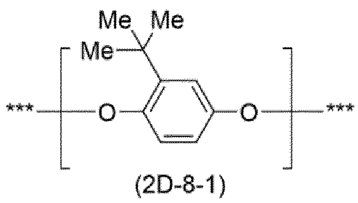
(2D-8-1)

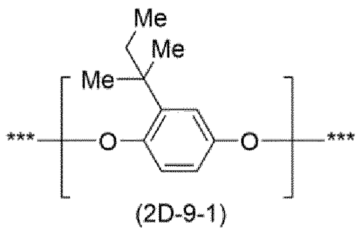
(2D-9-1)

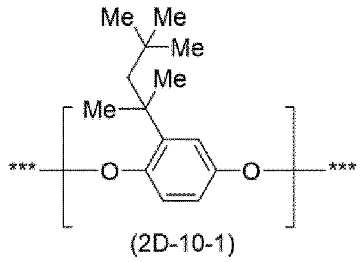
(2D-10-1)

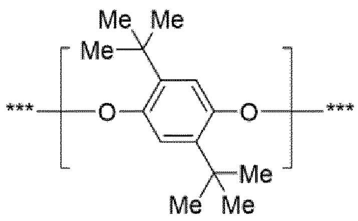
(2D-11-1)

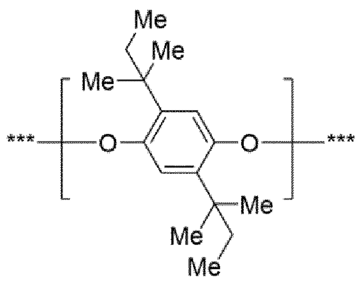
(2D-12-1)

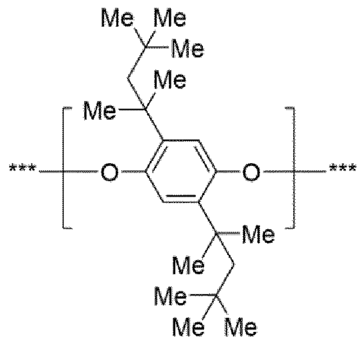
(2D-13-1)

[Chem. 40]

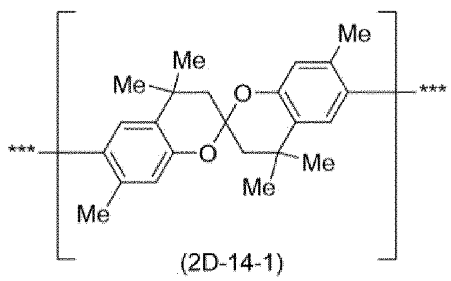
(2D-14-1)

Of these (2D-1-1) to (2D-14-1), (2D-1-1), (2D-2-1), (2D-3-1), (2D-7-3), (2D-7-4), (2D-7-6), (2D-8-1) to (2D-13-1) and (2D-14-1) are particularly preferred because they lead to superior optical characteristics of the polymer according to the present invention.

The polymer according to the present invention, furthermore, preferably contains at least one type of the repeat units indicated by chemical formulae (5-1) to (5-13) below for the purpose of the adjustment of the characteristics that the polymer develops.

[0134] [Chem. 41]

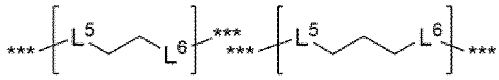

(5-1) (5-2)

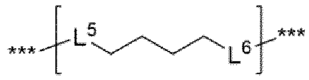

(5-3)

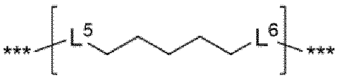

(5-4)

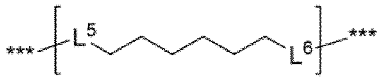

(5-5)

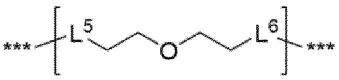

(5-6)

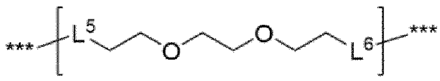

(5-7)

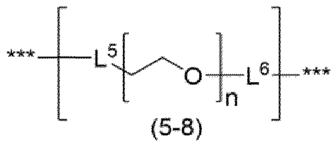

(5-8)

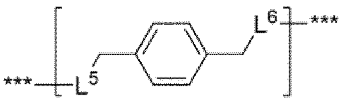

(5-9)

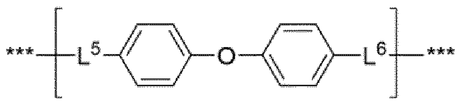

(5-10)

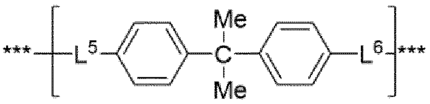

(5-11)

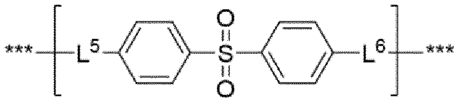

(5-12)

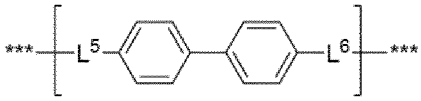

(5-13)

[In formulae (5-1) to (5-13), $L^5$ and $L^6$ may be the same or different from each other, each representing a carbonyl group, an ester linkage, an amide linkage, an ether linkage or a single bond.
n represents an integer of 4 to 500.
*** represents a site of binding with another structure in the main chain polymer.]

**[0082]** The polymer according to the present invention may contain structures indicated by chemical formulae (6-1) to (6-12) for the purpose of the adjustment of the characteristics that the polymer develops.

[0137] [Chem. 42]

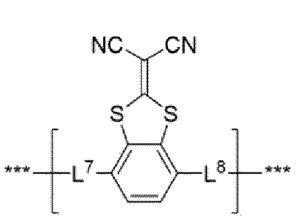
(6-1)

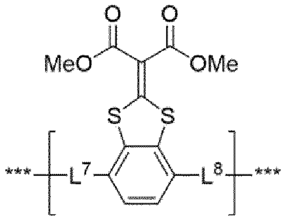
(6-2)

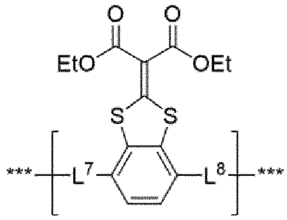
(6-3)

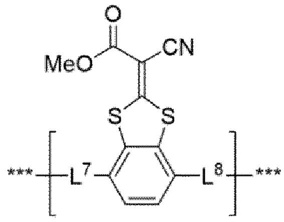
(6-4)

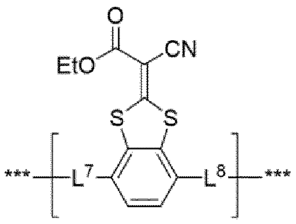
(6-5)

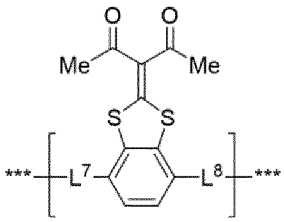
(6-6)

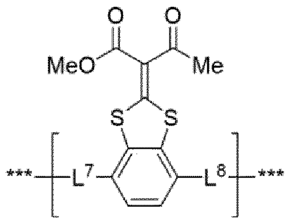
(6-7)

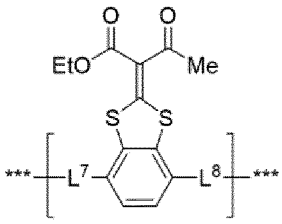
(6-8)

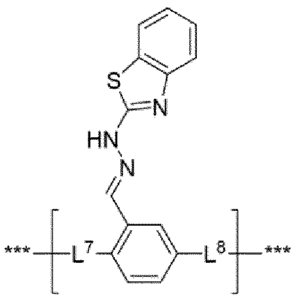
(6-9)

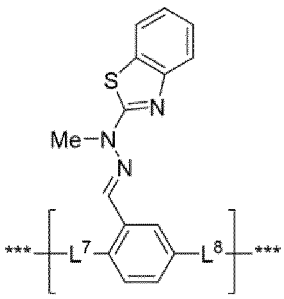
(6-10)

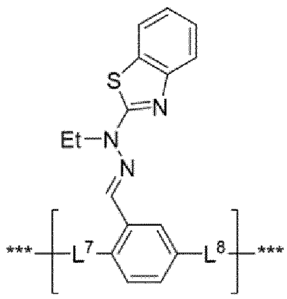
(6-11)

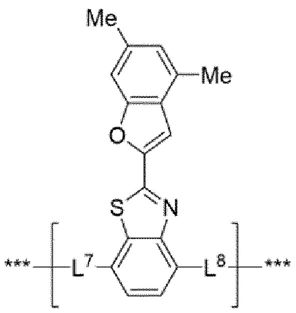
(6-12)

[In formulae (6-1) to (6-12), $L^7$ and $L^8$ may be the same or different from each other, each representing a carbonyl group, an ester linkage, an amide linkage, an ether linkage or a single bond.
*** represents a site of binding with another structure in the main chain polymer.]

**[0083]** Specific examples of polymers according to the present invention include the structures indicated by chemical formulae (P1-1-1-1-1) to (P7-12-1) below.

[Chem. 43]

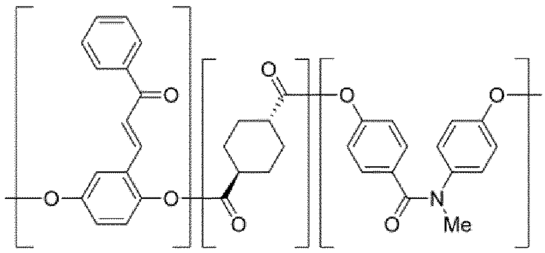

(P1-1-1-1-1)

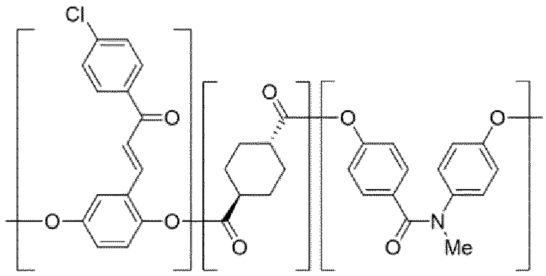

(P1-1-1-1-2)

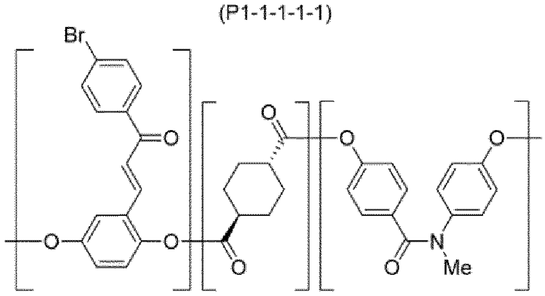

(P1-1-1-1-3)

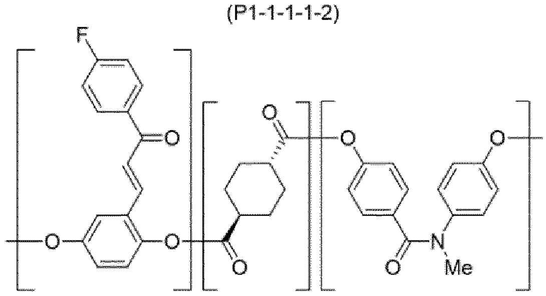

(P1-1-1-1-4)

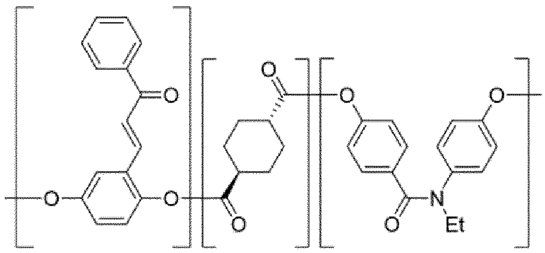

(P1-1-1-2-1)

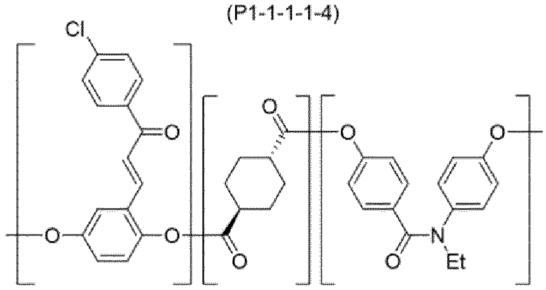

(P1-1-1-2-2)

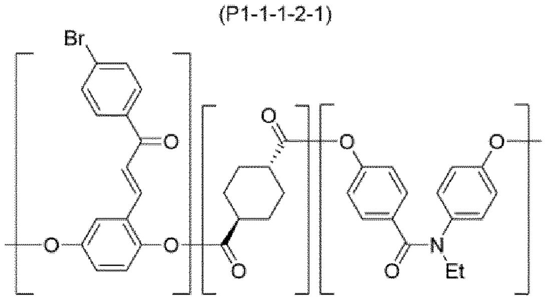

(P1-1-1-2-3)

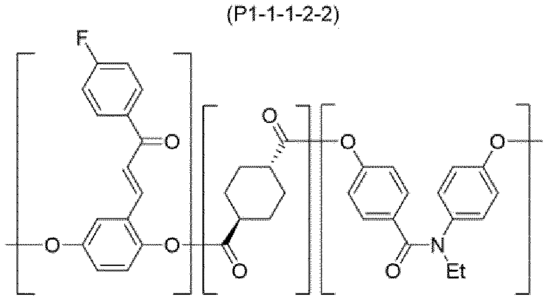

(P1-1-1-2-4)

[Chem. 44]

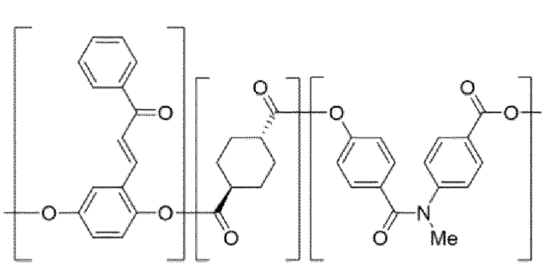

(P1-1-2-1-1)

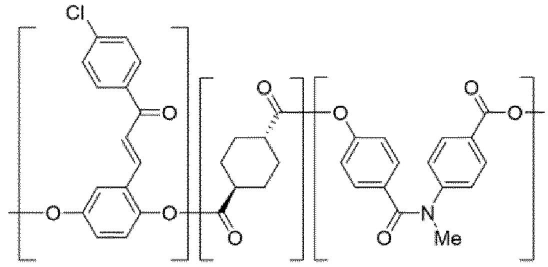

(P1-1-2-1-2)

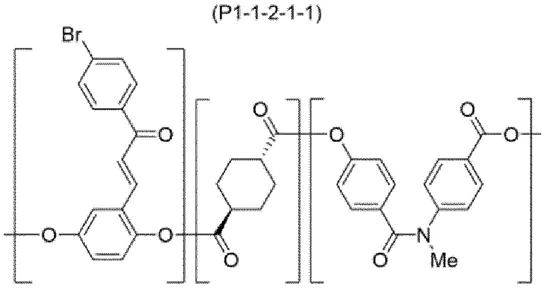

(P1-1-2-1-3)

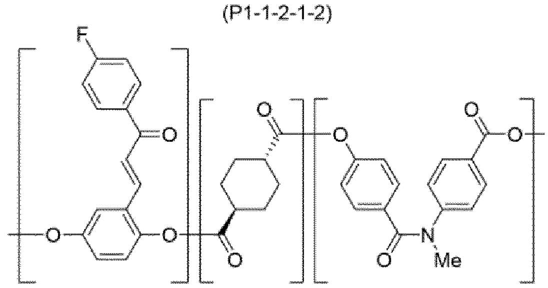

(P1-1-2-1-4)

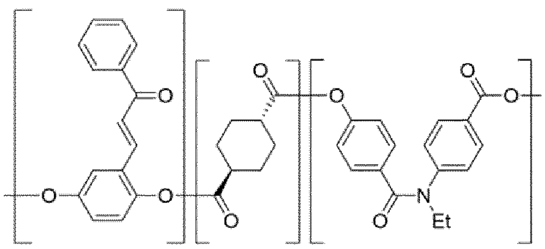

(P1-1-2-2-1)

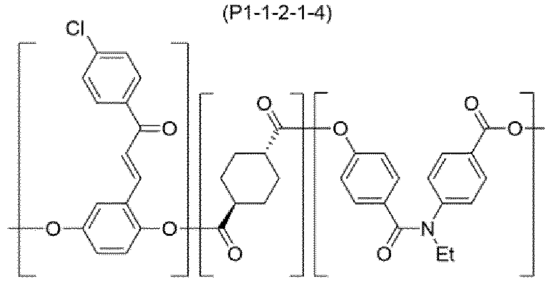

(P1-1-2-2-2)

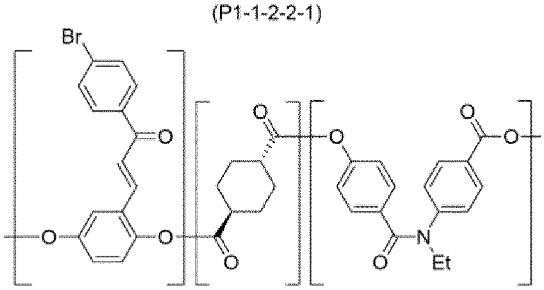

(P1-1-2-2-3)

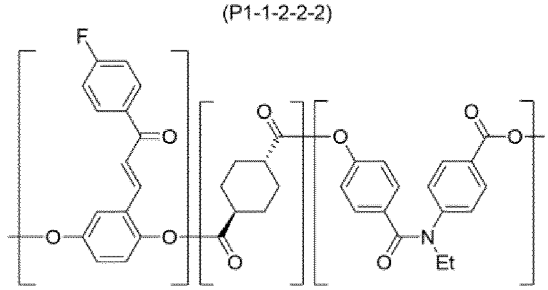

(P1-1-2-2-4)

[Chem. 45]

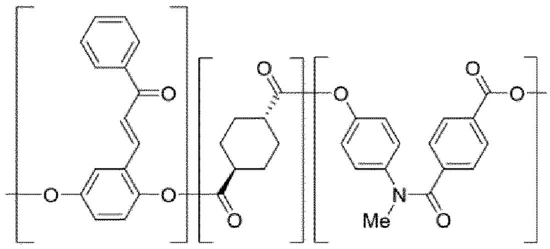

(P1-1-3-1-1)

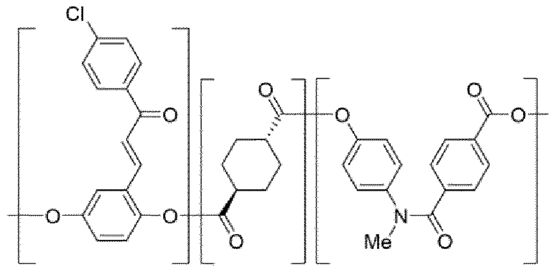

(P1-1-3-1-2)

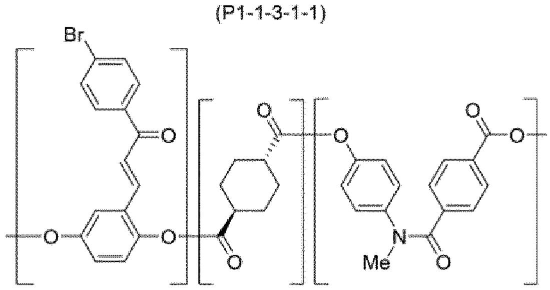

(P1-1-3-1-3)

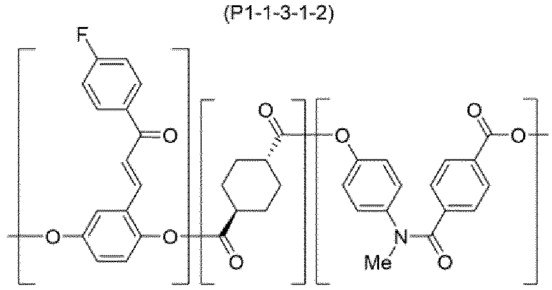

(P1-1-3-1-4)

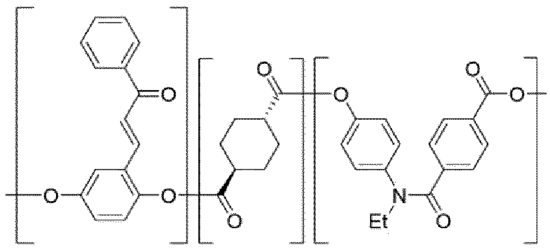

(P1-1-3-2-1)

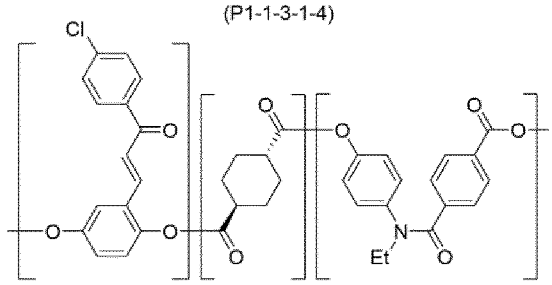

(P1-1-3-2-2)

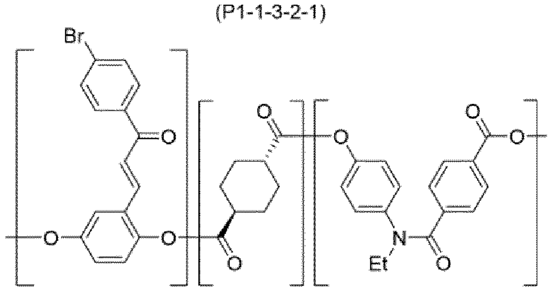

(P1-1-3-2-3)

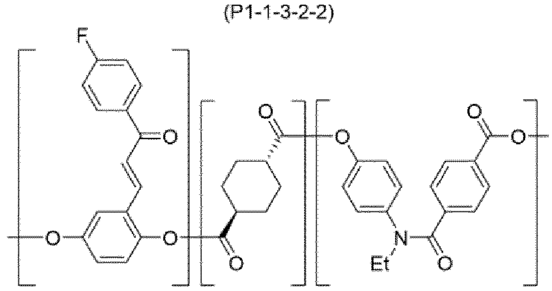

(P1-1-3-2-4)

[Chem. 46]

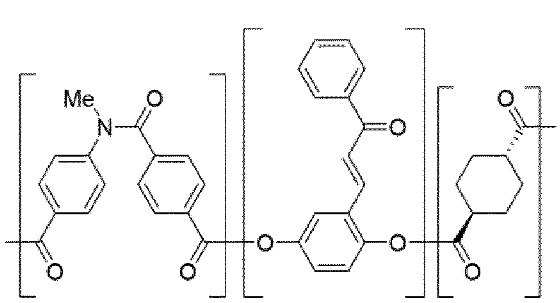

(P1-1-4-1-1)

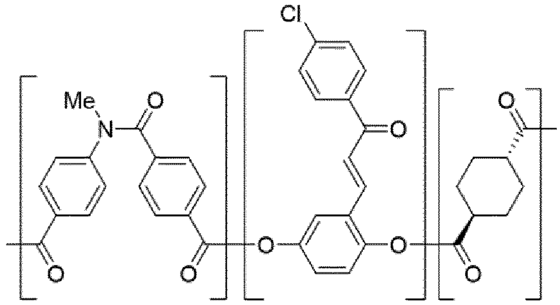

(P1-1-4-1-2)

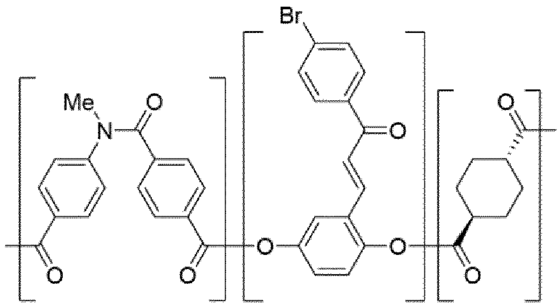

(P1-1-4-1-3)

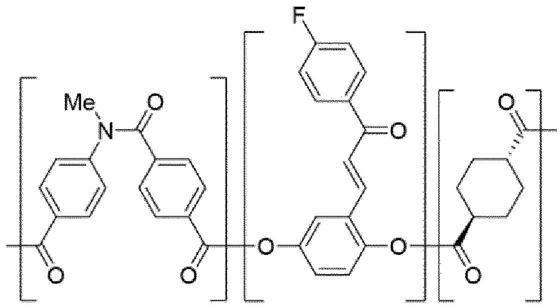

(P1-1-4-1-4)

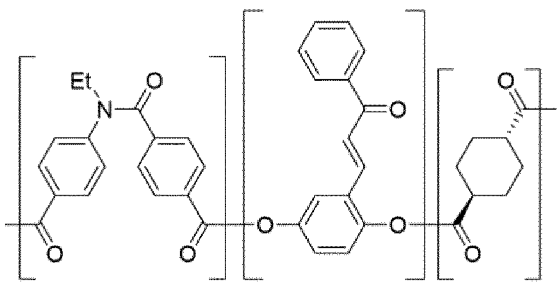

(P1-1-4-2-1)

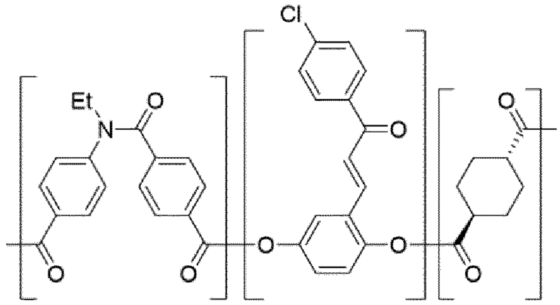

(P1-1-4-2-2)

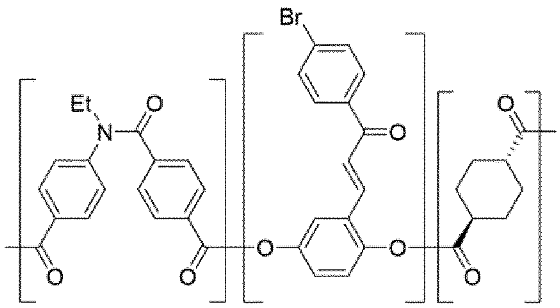

(P1-1-4-2-3)

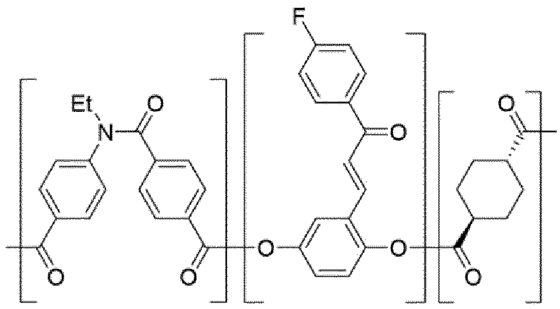

(P1-1-4-2-4)

[Chem. 47]

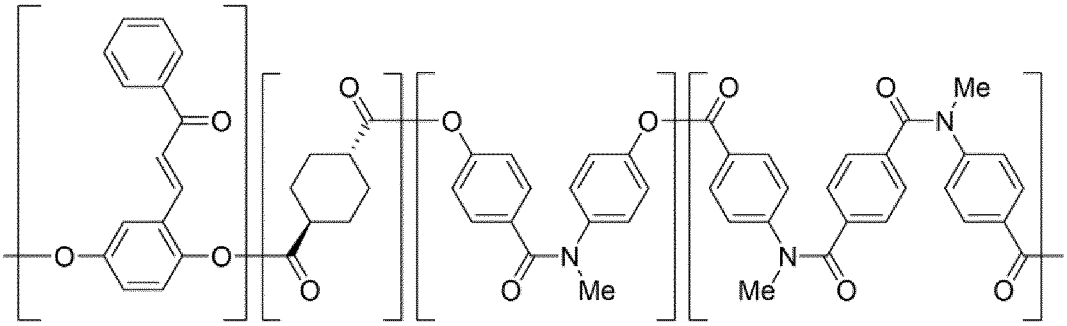

(P1-1-5-1-1)

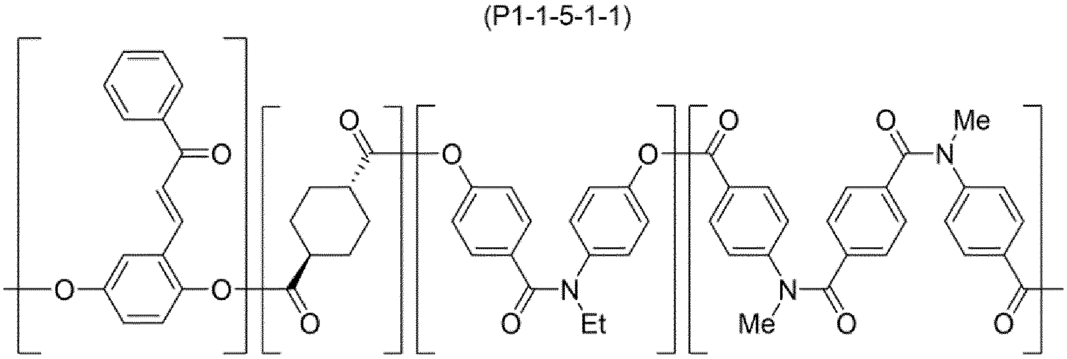

(P1-1-5-2-1)

[Chem. 48]

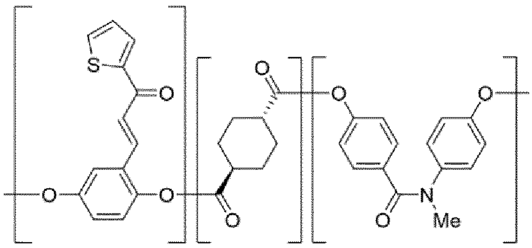

(P1-2-1-1-1)

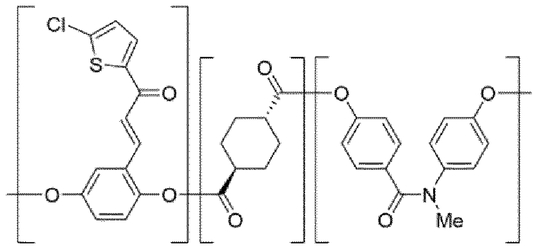

(P1-2-1-1-2)

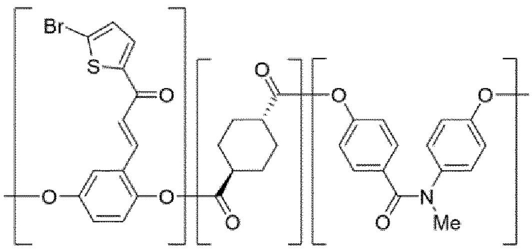

(P1-2-1-1-3)

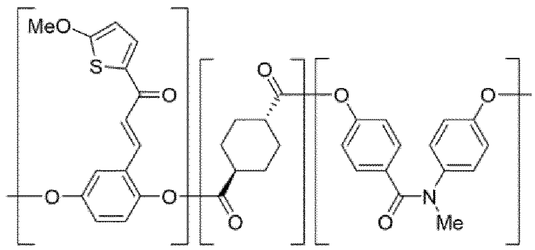

(P1-2-1-1-4)

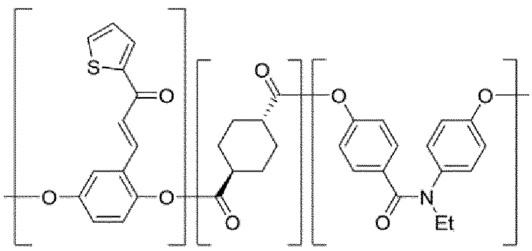

(P1-2-1-2-1)

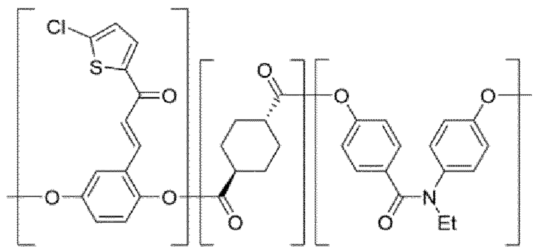

(P1-2-1-2-2)

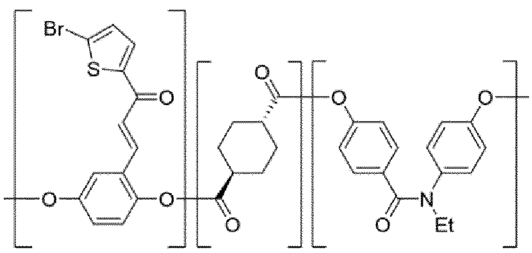

(P1-2-1-2-3)

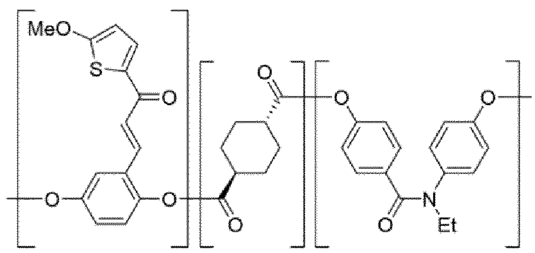

(P1-2-1-2-4)

[Chem. 49]

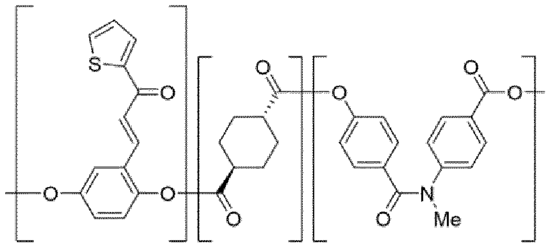

(P1-2-2-1-1)

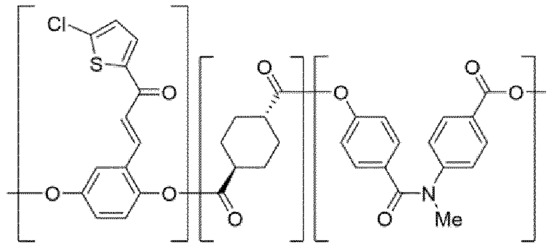

(P1-2-2-1-2)

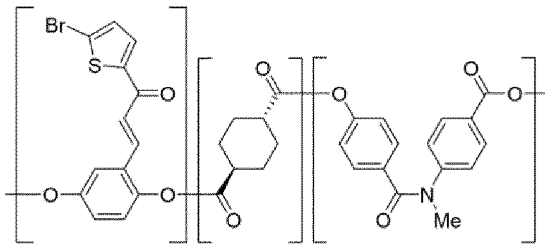

(P1-2-2-1-3)

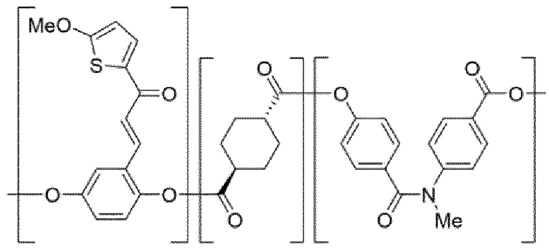

(P1-2-2-1-4)

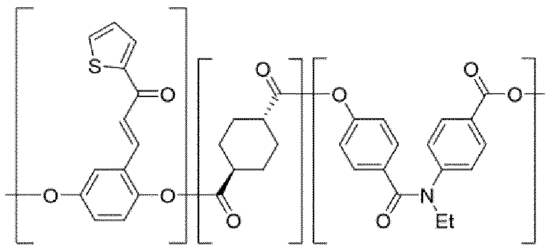

(P1-2-2-2-1)

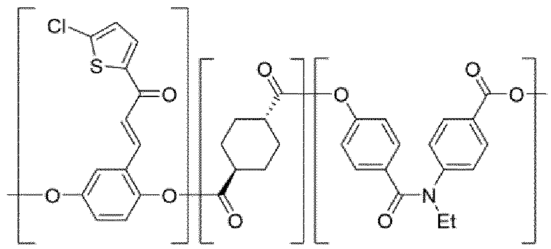

(P1-2-2-2-2)

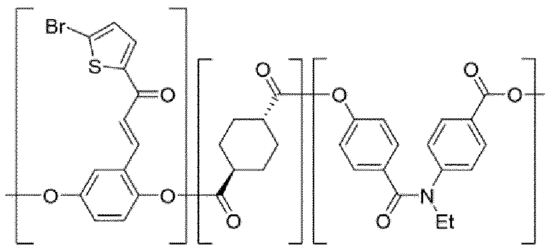

(P1-2-2-2-3)

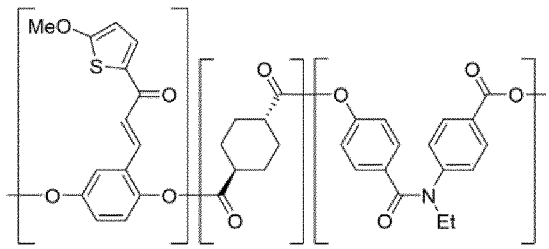

(P1-2-2-2-4)

[Chem. 50]

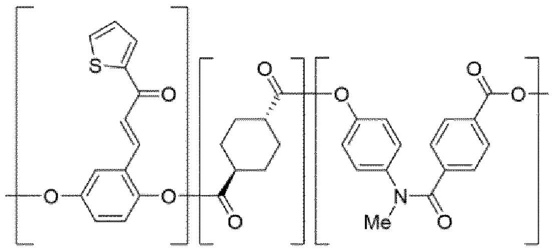

(P1-2-3-1-1)

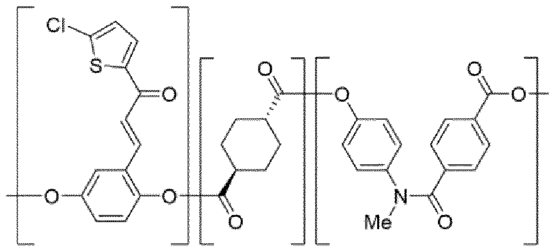

(P1-2-3-1-2)

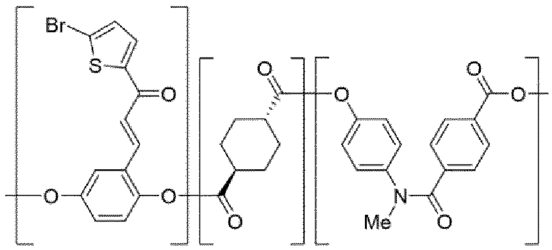

(P1-2-3-1-3)

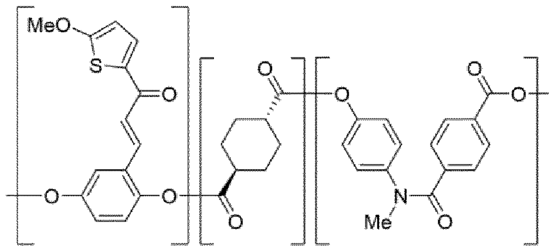

(P1-2-3-1-4)

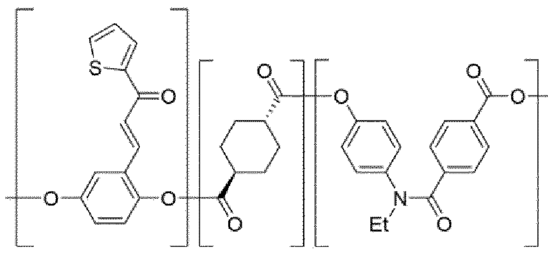

(P1-2-3-2-1)

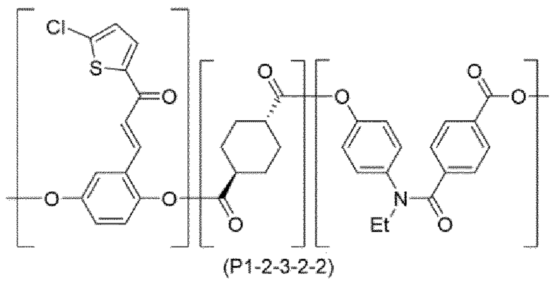

(P1-2-3-2-2)

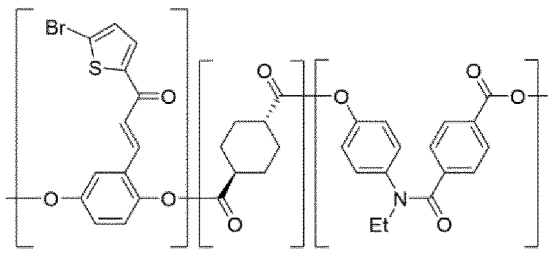

(P1-2-3-2-3)

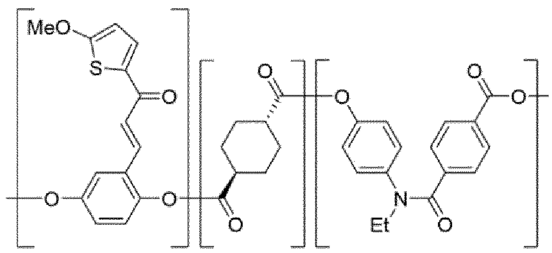

(P1-2-3-2-4)

[Chem. 51]

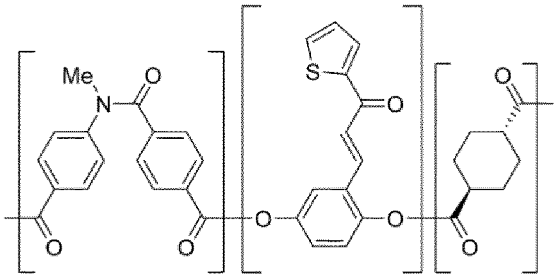
(P1-2-4-1-1)

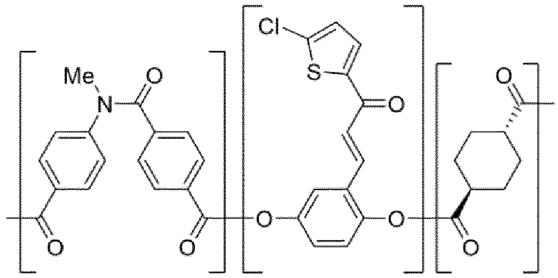
(P1-2-4-1-2)

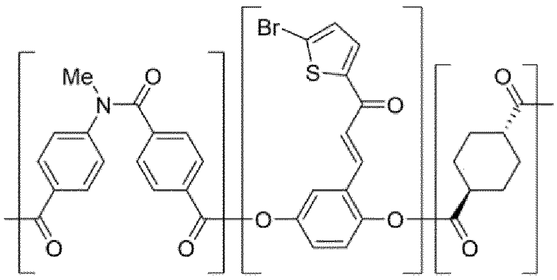
(P1-2-4-1-3)

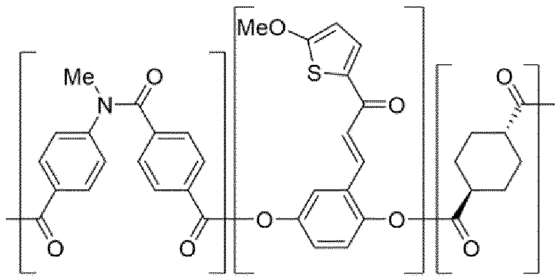
(P1-2-4-1-4)

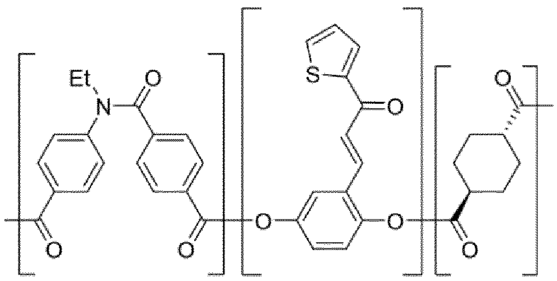
(P1-2-4-2-1)

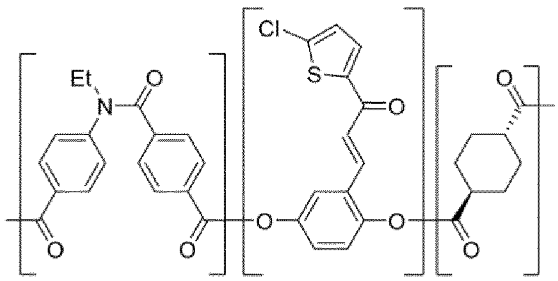
(P1-2-4-2-2)

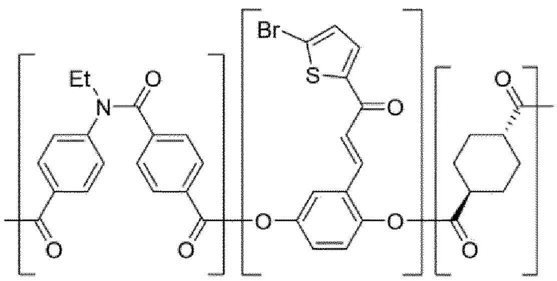
(P1-2-4-2-3)

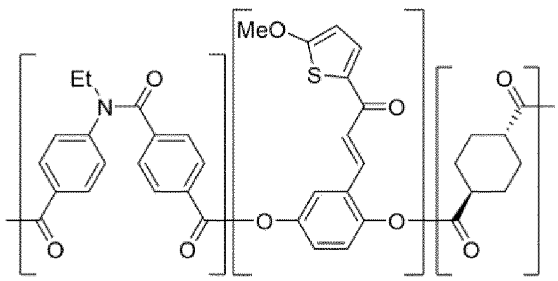
(P1-2-4-2-4)

[Chem. 52]

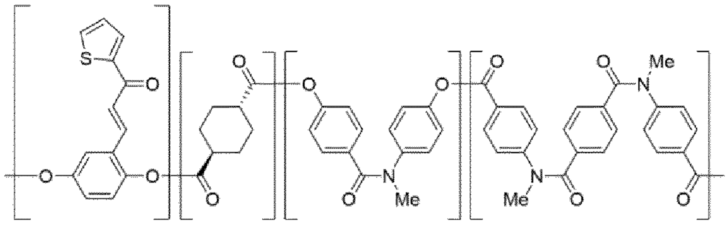

(P1-2-5-1-1)

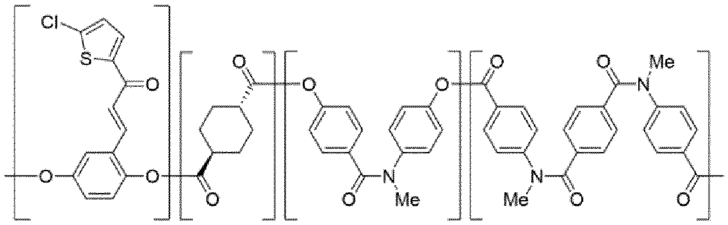

(P1-2-5-1-2)

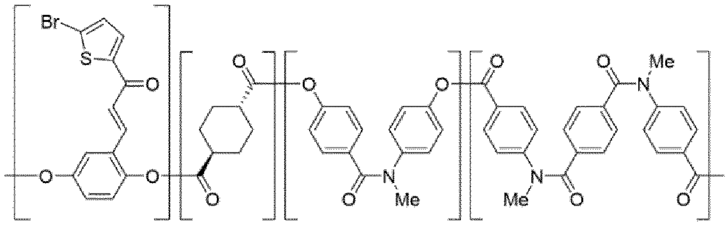

(P1-2-5-1-3)

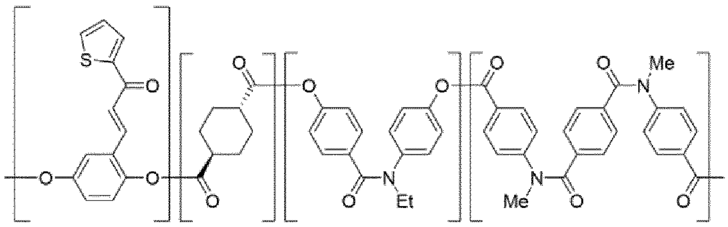

(P1-2-5-2-1)

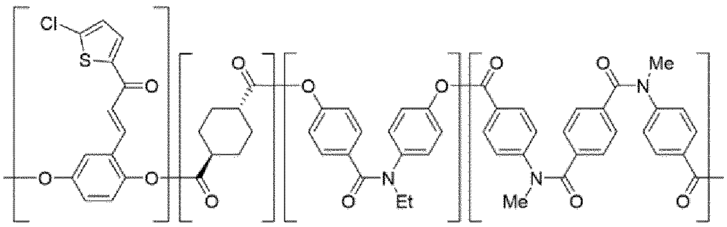

(P1-2-5-2-2)

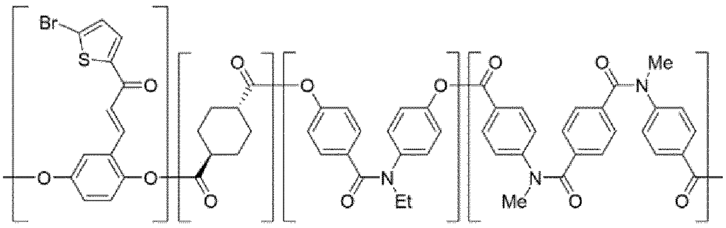

(P1-2-5-2-3)

[Chem. 53]

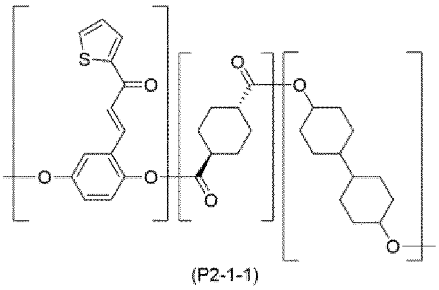

(P2-1-1)

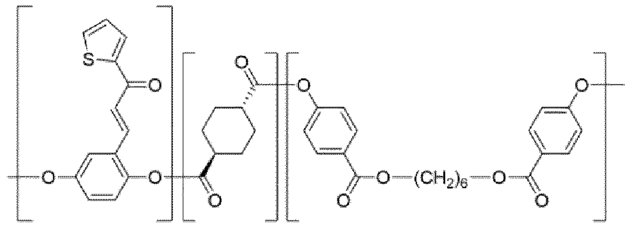

(P2-1-2)

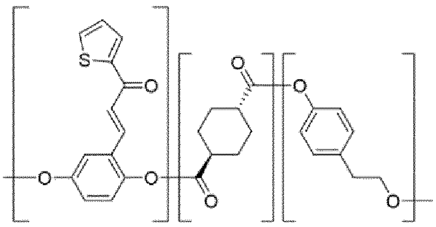

(P2-1-3)

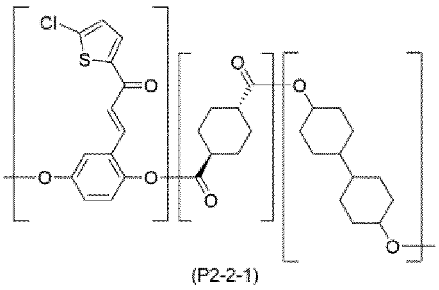

(P2-2-1)

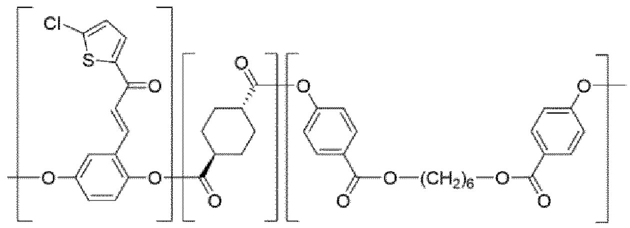

(P2-2-2)

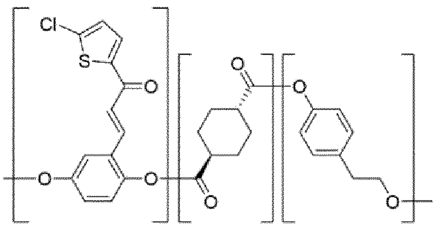

(P2-2-3)

[Chem. 54]

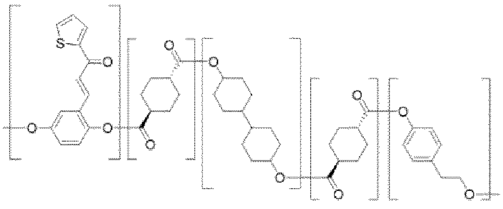

(P3-1-1)

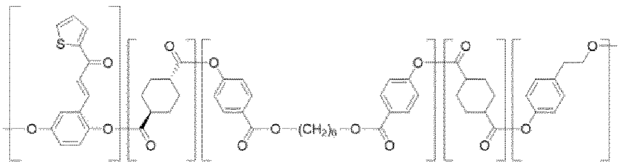

(P3-1-2)

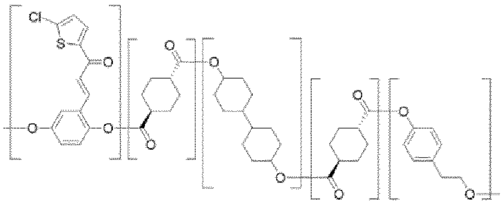

(P3-2-1)

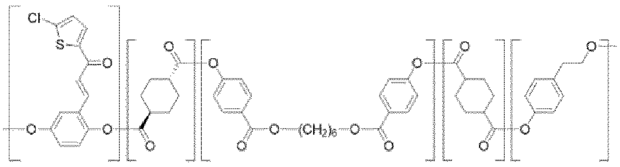

(P3-2-2)

[Chem. 55]

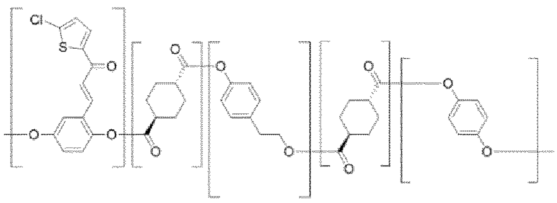

(P4-1-1)

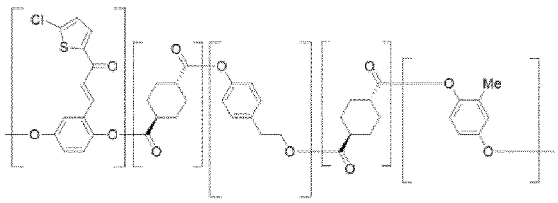

(P4-1-2)

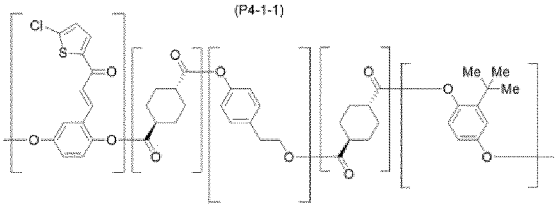

(P4-1-3)

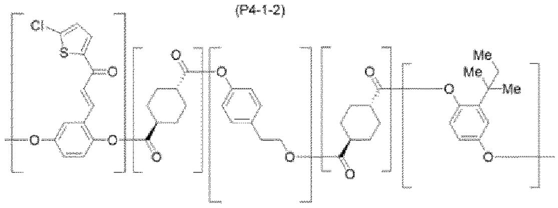

(P4-1-4)

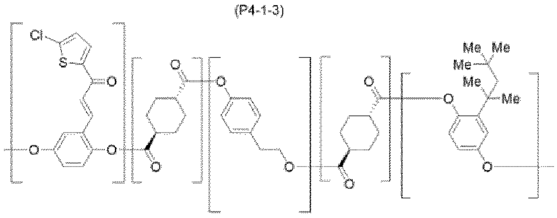

(P4-1-5)

[Chem. 56]

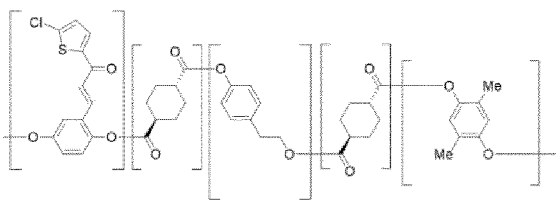

(P4-1-6)

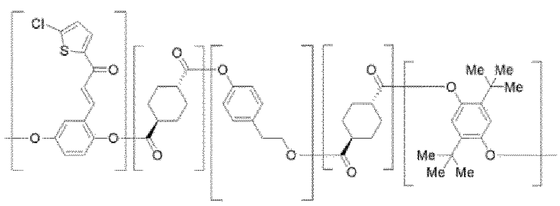

(P4-1-7)

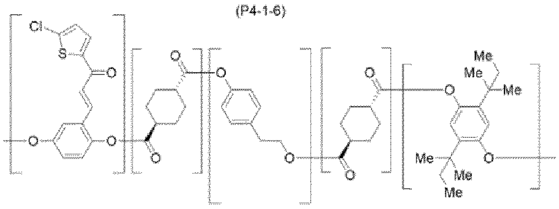

(P4-1-8)

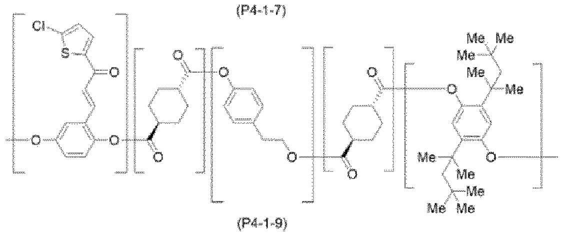

(P4-1-9)

[Chem. 57]

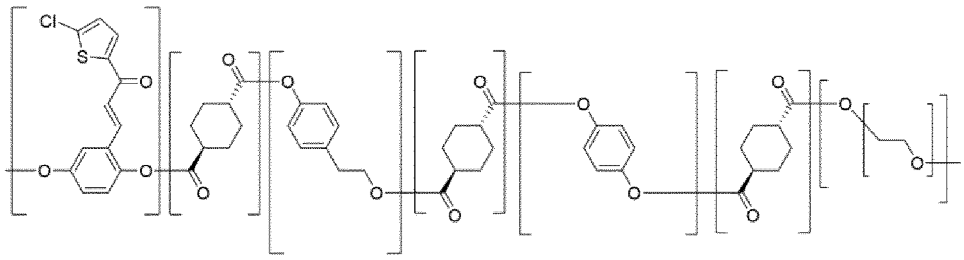

(P4-2-1)

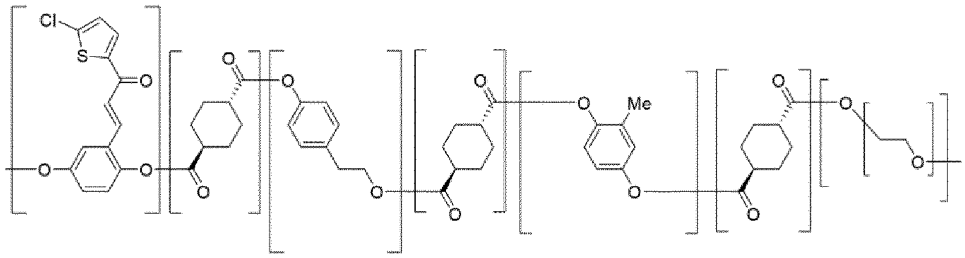

(P4-2-2)

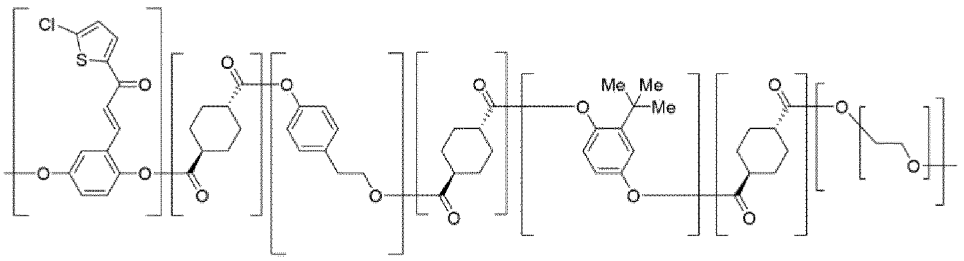

(P4-2-3)

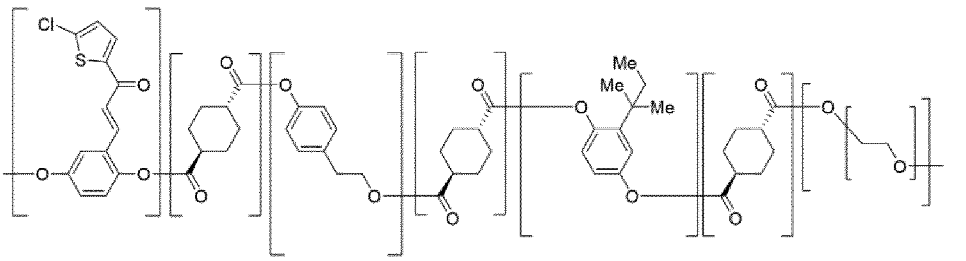

(P4-2-4)

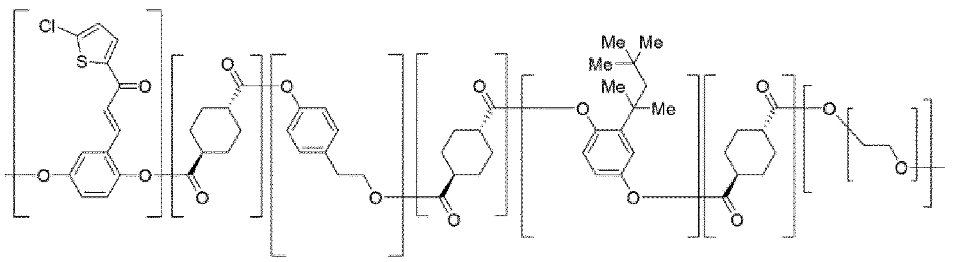

(P4-2-5)

[Chem. 58]

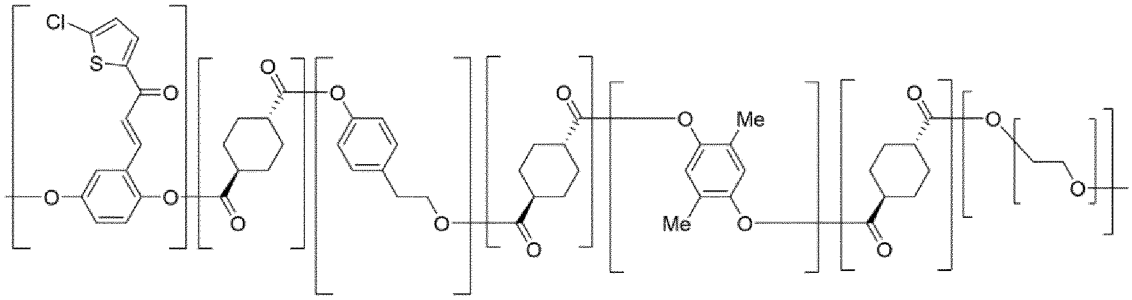

(P4-2-6)

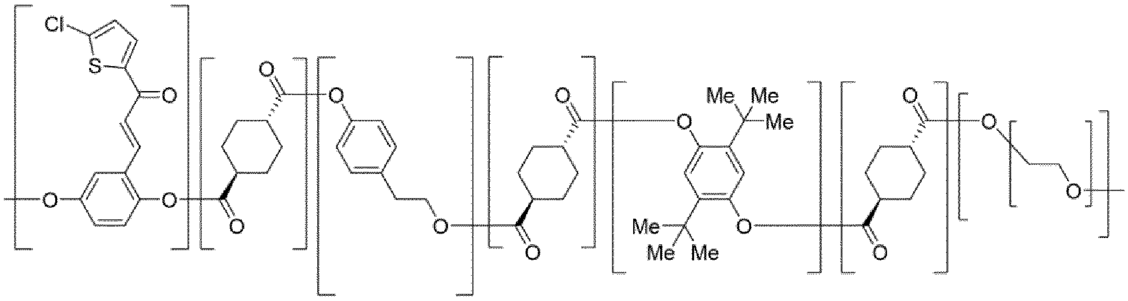

(P4-2-7)

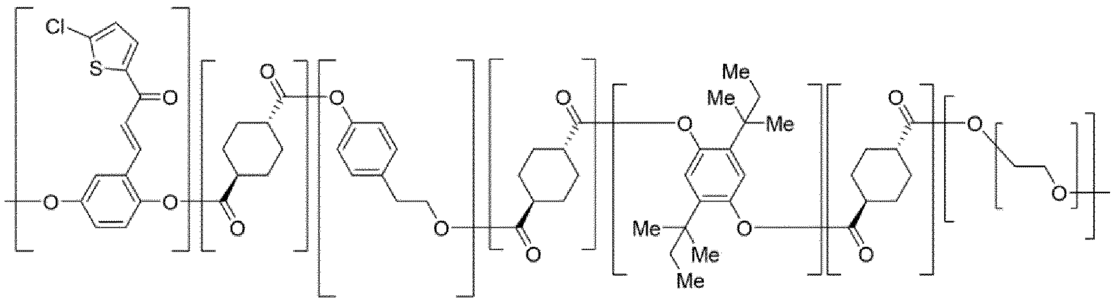

(P4-2-8)

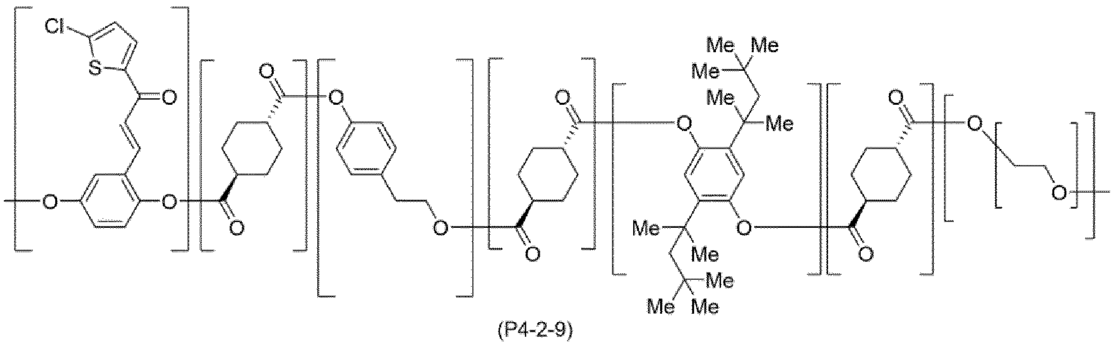

(P4-2-9)

[Chem. 59]

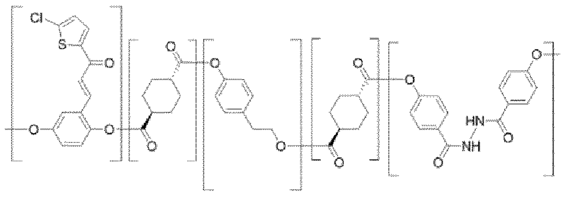

(P5-1-1)

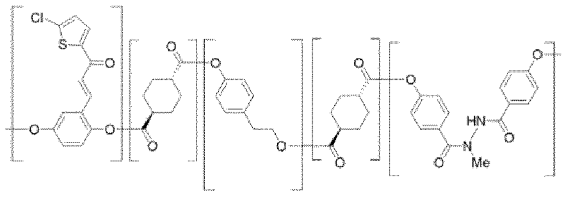

(P5-1-2)

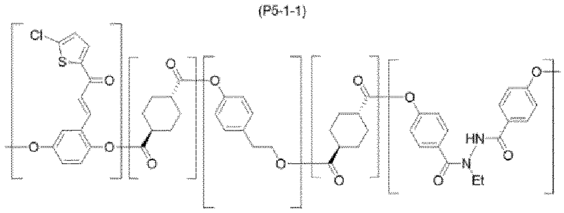

(P5-1-3)

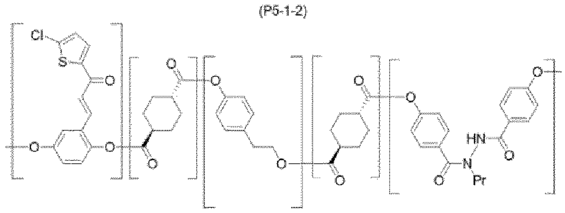

(P5-1-4)

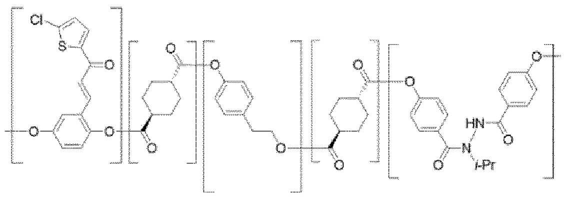

(P5-1-5)

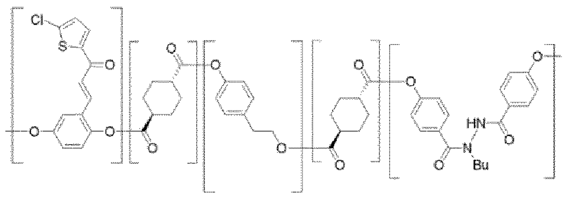

(P5-1-6)

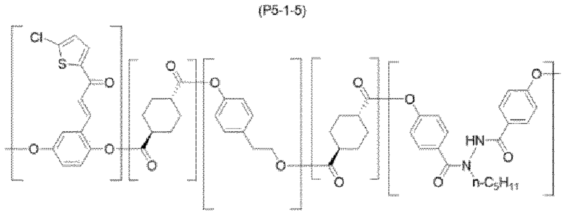

(P5-1-7)

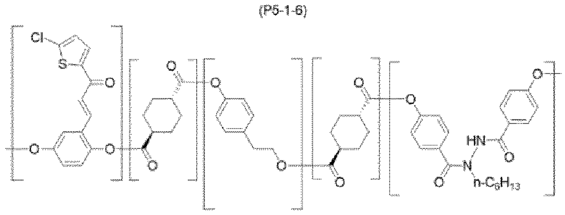

(P5-1-8)

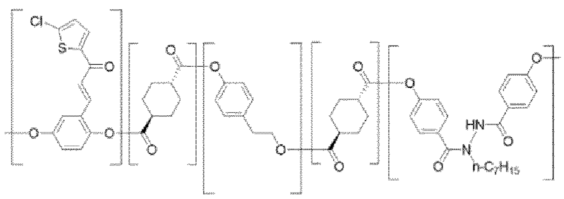

(P5-1-9)

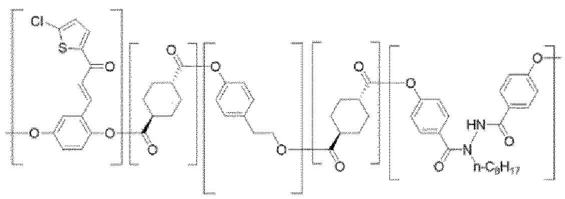

(P5-1-10)

[Chem. 60]

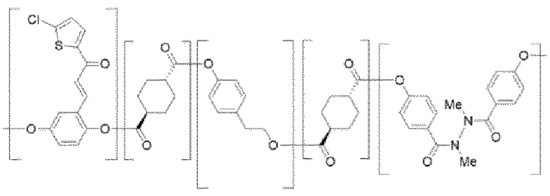

(P5-1-11)

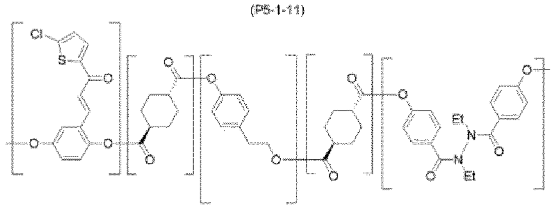

(P5-1-12)

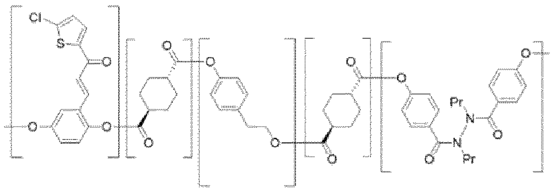

(P5-1-13)

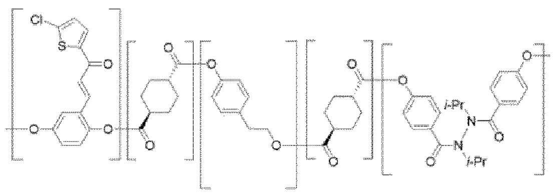

(P5-1-14)

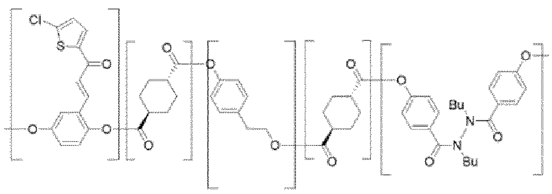

(P5-1-15)

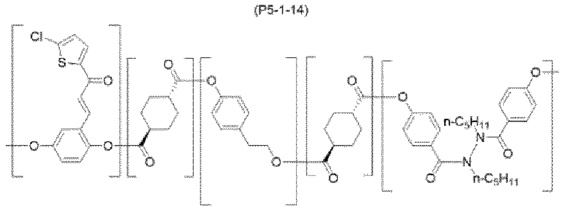

(P5-1-16)

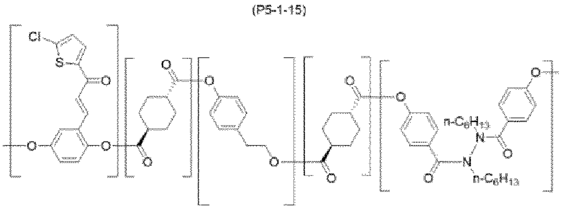

(P5-1-17)

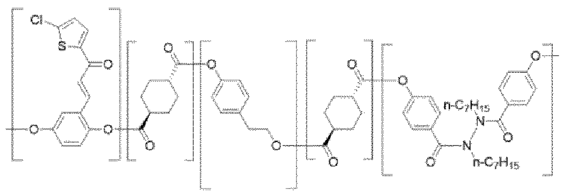

(P5-1-18)

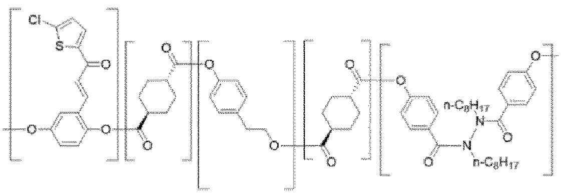

(P5-1-19)

[Chem. 61]

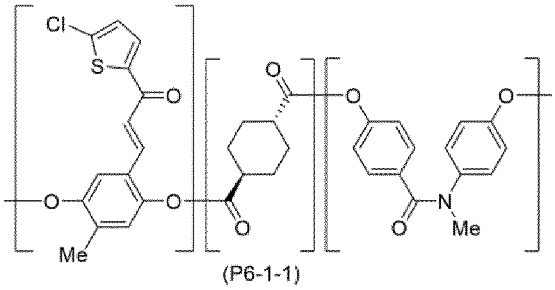

(P6-1-1)

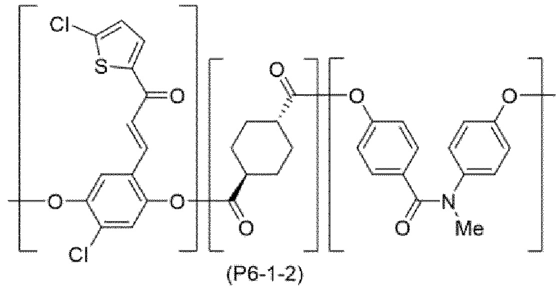

(P6-1-2)

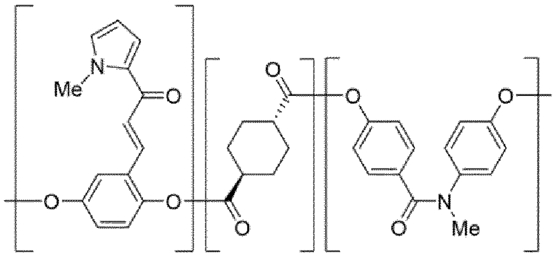

(P6-1-3)

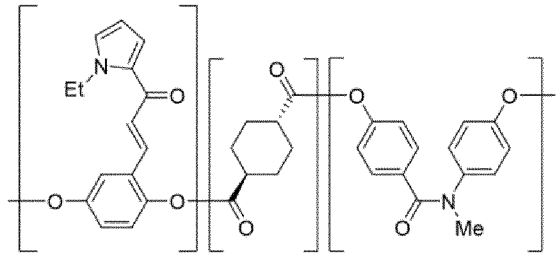

(P6-1-4)

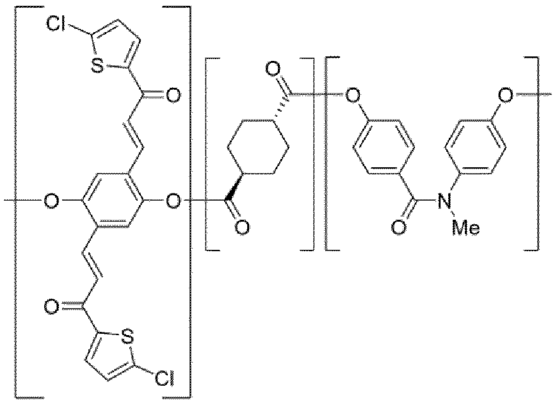

(P6-1-5)

[Chem. 62]

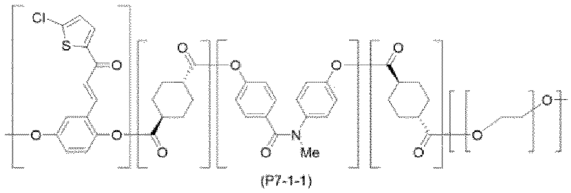
(P7-1-1)

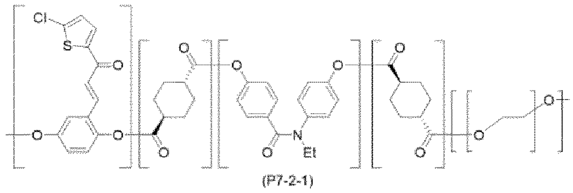
(P7-2-1)

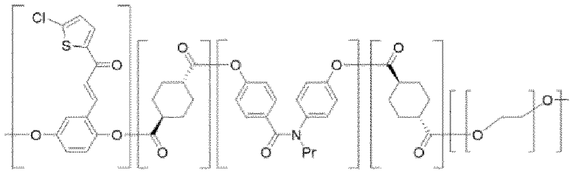
(P7-3-1)

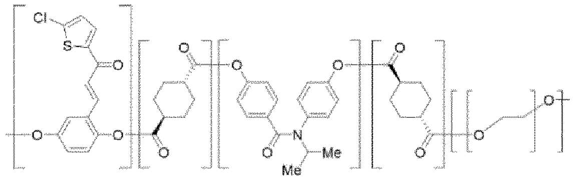
(P7-3-2)

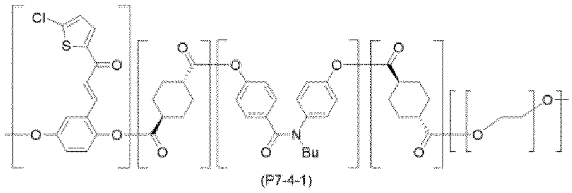
(P7-4-1)

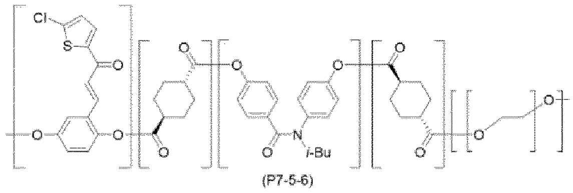
(P7-5-6)

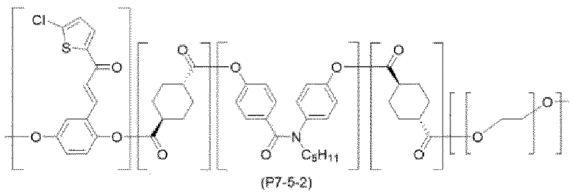
(P7-5-2)

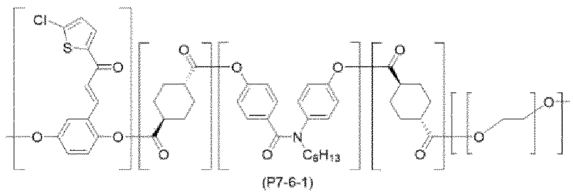
(P7-6-1)

[0160] [Chem. 63]

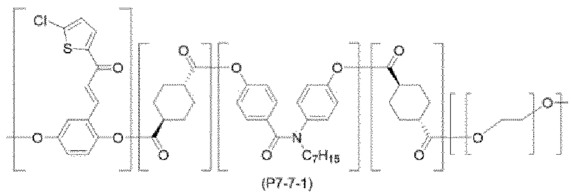
(P7-7-1)

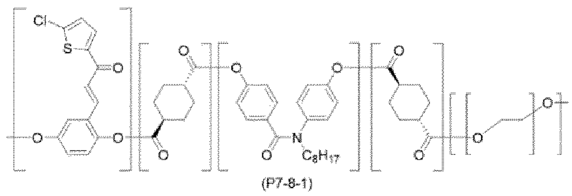
(P7-8-1)

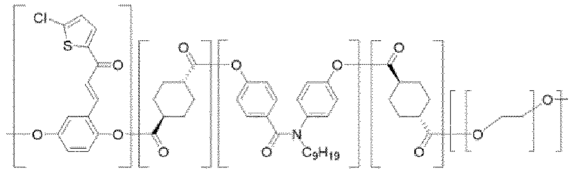
(P7-9-1)

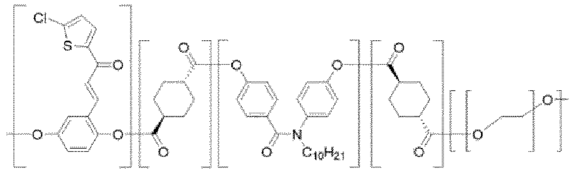
(P7-10-1)

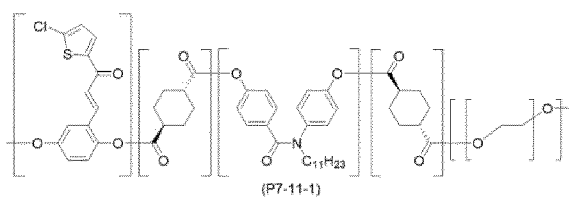
(P7-11-1)

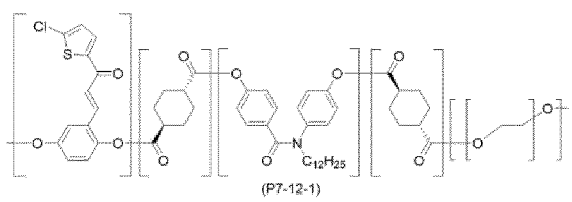
(P7-12-1)

Of these (P1-1-1-1-1) to (P7-12-1), particularly preferred structures are (P1-2-1-1-1) to (P1-2-5-1-4), (P2-2-1) to (P2-2-3), (P3-2-1) to (P3-2-2), (P4-1-1) to (P4-2-9) and (P5-1-1) to (P5-1-19) because they lead to good optical characteristics of the polymer according to the present invention.

For the polymer according to the present invention, the amount of the photoreactive reverse wavelength dispersion unit A in the polymer is from 1 mol% to 50 mol%, preferably from 5 mol% to 45 mol%, more preferably from 20 mol% to 45 mol%.

When the polymer according to the present invention contains repeat units represented by (2A), (2B), (2C) and (2D), the amount of the repeat units represented by (2A), (2B), (2C) and (2D) in the polymer is from 1 mol% to 99 mol%,

preferably from 10 mol% to 70 mol%, more preferably from 15 mol% to 60 mol%.

The weight-average molecular weight of the polymer according to the present invention is preferably 1,000 or more and 100,000 or less, particularly preferably 1,100 or more and 50,000 or less.

For the polymer according to the present invention, it is preferred that at least one of its ends be a monocarboxylic acid ester because this leads to a short-wavelength shift of the absorption band, thereby allowing for the provision of a film with a low yellowness index (YI) when the polymer is made into a film.

An example of a monocarboxylic acid ester is a structure indicated by chemical formula (2') below.

[Chem. 64]

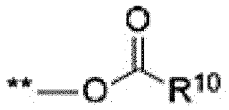

(2')

[Here, in chemical formula (2') above, $R^{10}$ represents a group selected from the group consisting of a substituted or unsubstituted C1 to C20 alkyl group, a substituted or unsubstituted C3 to C8 cycloalkyl group and a substituted or unsubstituted C3 to C12 aromatic group.
** represents a site of binding with another structure in the main chain polymer.]

In formula (2'), at $R^{10}$, examples of C1 to C20 alkyl groups include a methyl group, an ethyl group, a propyl group, an isopropyl group, a butyl group, an isobutyl group, a sec-butyl group, a tert-butyl group, a pentyl group, a hexyl group, a heptyl group, an octyl group, a nonyl group, a decyl group and an icosyl group. Of these, a methyl group, an ethyl group, a propyl group, an isopropyl group, a butyl group, an isobutyl group, a sec-butyl group and a tert-butyl group are particularly preferred, with a methyl group and an ethyl group being especially preferred because of the ease of introduction.

Examples of C3 to C8 cycloalkyl groups include a cyclopropyl group, a cyclobutyl group, a cyclopentyl group, a cyclohexyl group, a cycloheptyl group and a cyclooctyl group.

Examples of C3 to C12 aromatic groups include a phenyl group, a naphthyl group, a biphenylyl group and a pyridyl group.

Examples of monocarboxylic acid esters include the structures indicated by chemical formulae (2'-1) to (2'-37) below.

[Chem. 65]

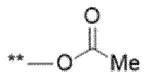
(2'-1)
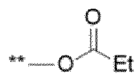
(2'-2)
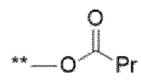
(2'-3)
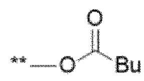
(2'-4)
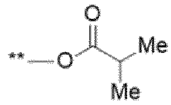
(2'-5)
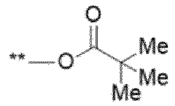
(2'-6)

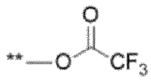
(2'-7)
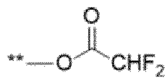
(2'-8)
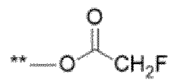
(2'-9)
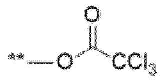
(2'-10)
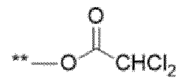
(2'-11)
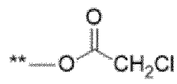
(2'-12)

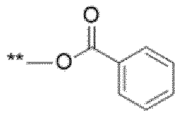
(2'-13)
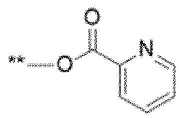
(2'-14)
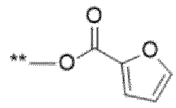
(2'-15)
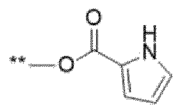
(2'-16)
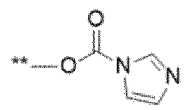
(2'-17)

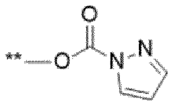
(2'-18)
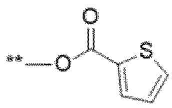
(2'-19)
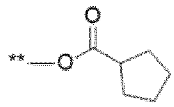
(2'-20)
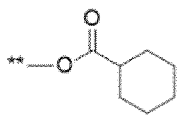
(2'-21)
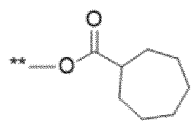
(2'-22)

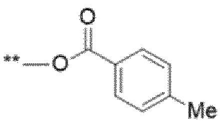
(2'-23)
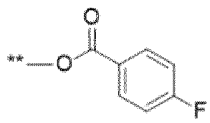
(2'-24)
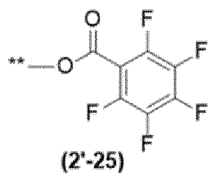
(2'-25)
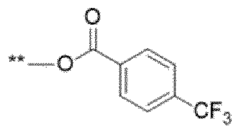
(2'-26)

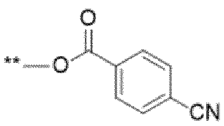
(2'-27)
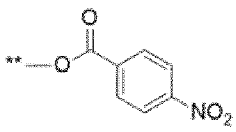
(2'-28)
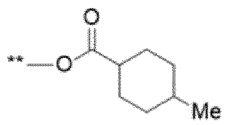
(2'-29)
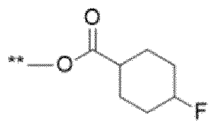
(2'-30)

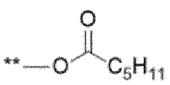
(2'-31)
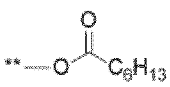
(2'-32)
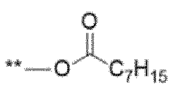
(2'-33)
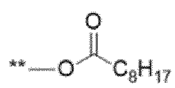
(2'-34)

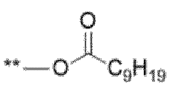
(2'-35)
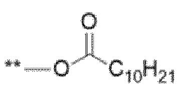
(2'-36)
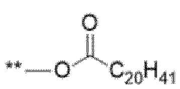
(2'-37)

[** represents a site of binding with another structure in the main chain polymer.]

Of these (2'-1) to (2'-37), particularly preferred structures are (2'-1) to (2'-13) and (2'-20) to (2'-37) because of the ease of introduction, with especially preferred ones being, for example, (2'-1) to (2'-6), (2'-13), (2'-20) to (2'-23), (2'-29) and (2'-31) to (2'-36).

For polymers according to the present invention at least one end of which is a monocarboxylic acid ester, specific examples include the structures indicated by chemical formulae (PA1-1-1-1-1) to (PA7-12-1) below.

[Chem. 66]

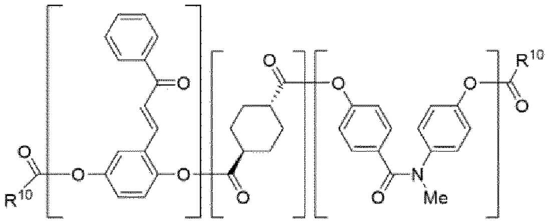

(PA1-1-1-1-1)

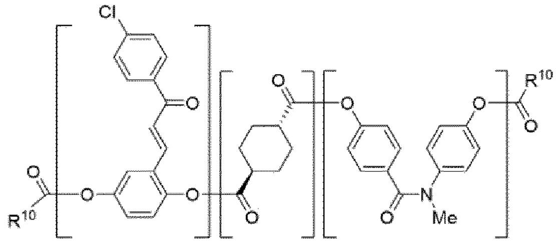

(PA1-1-1-1-2)

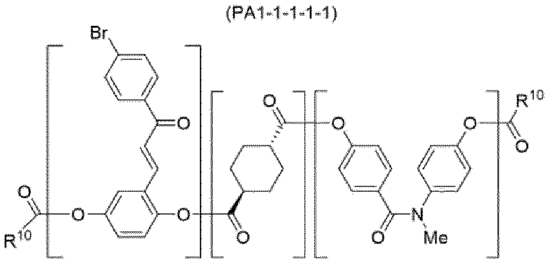

(PA1-1-1-1-3)

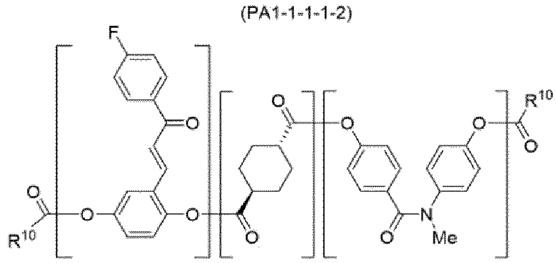

(PA1-1-1-1-4)

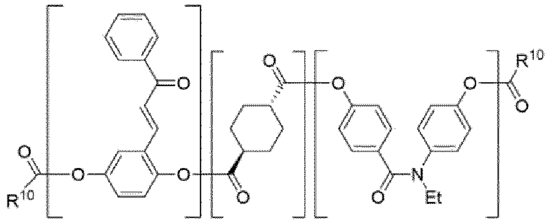

(PA1-1-1-2-1)

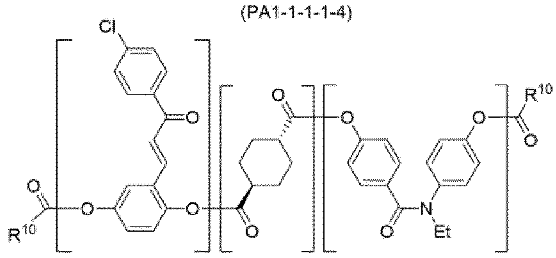

(PA1-1-1-2-2)

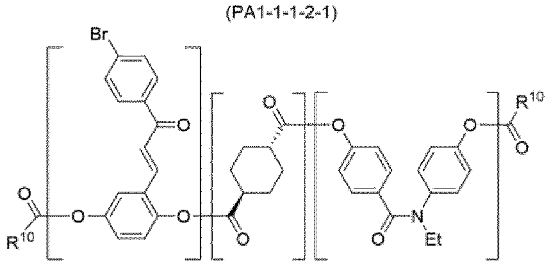

(PA1-1-1-2-3)

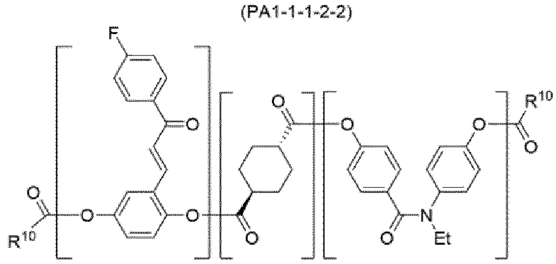

(PA1-1-1-2-4)

[Chem. 67]

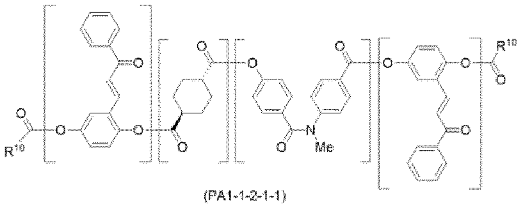

(PA1-1-2-1-1)

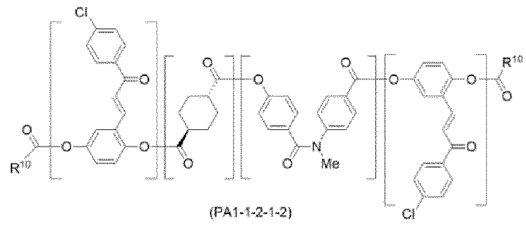

(PA1-1-2-1-2)

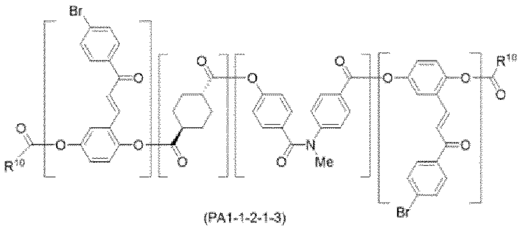

(PA1-1-2-1-3)

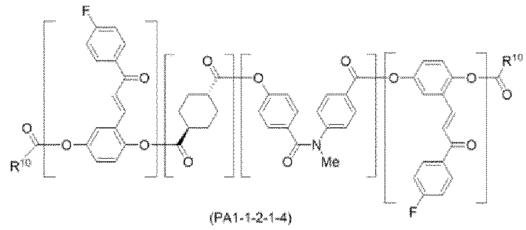

(PA1-1-2-1-4)

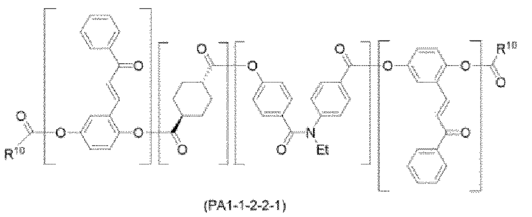

(PA1-1-2-2-1)

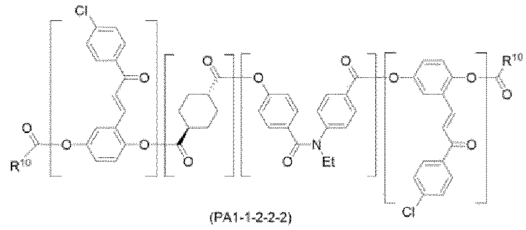

(PA1-1-2-2-2)

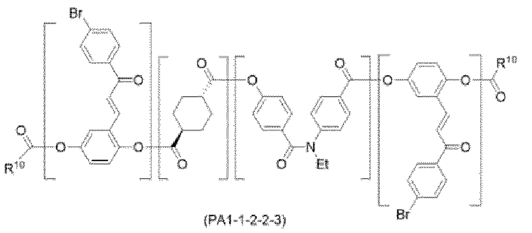

(PA1-1-2-2-3)

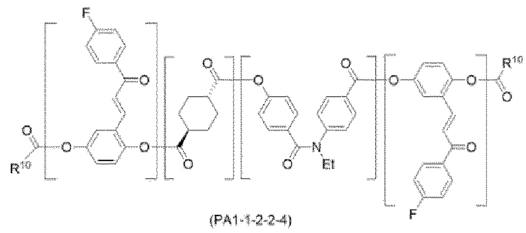

(PA1-1-2-2-4)

[Chem. 68]

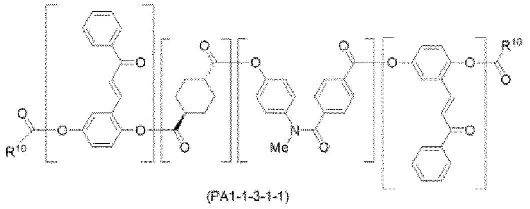

(PA1-1-3-1-1)

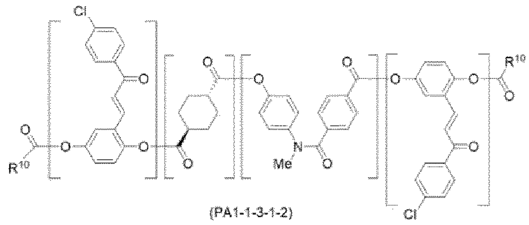

(PA1-1-3-1-2)

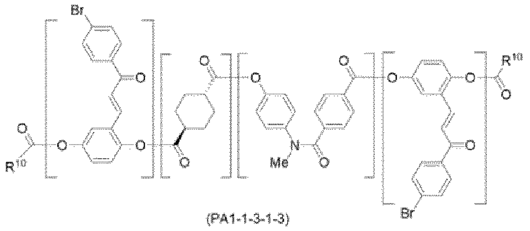

(PA1-1-3-1-3)

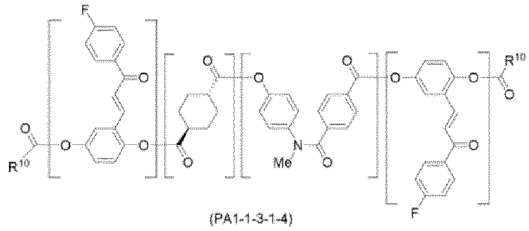

(PA1-1-3-1-4)

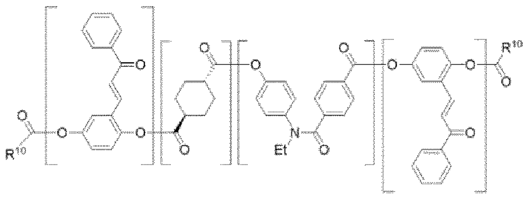

(PA1-1-3-2-1)

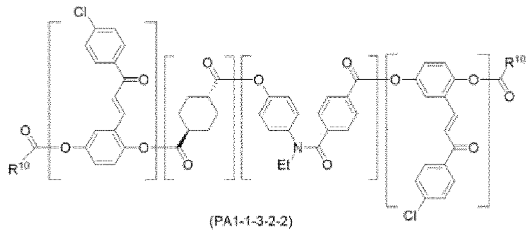

(PA1-1-3-2-2)

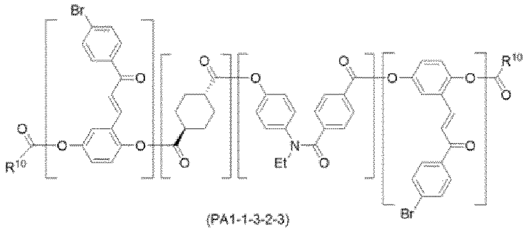

(PA1-1-3-2-3)

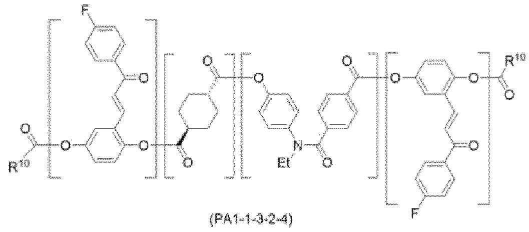

(PA1-1-3-2-4)

[Chem. 69]

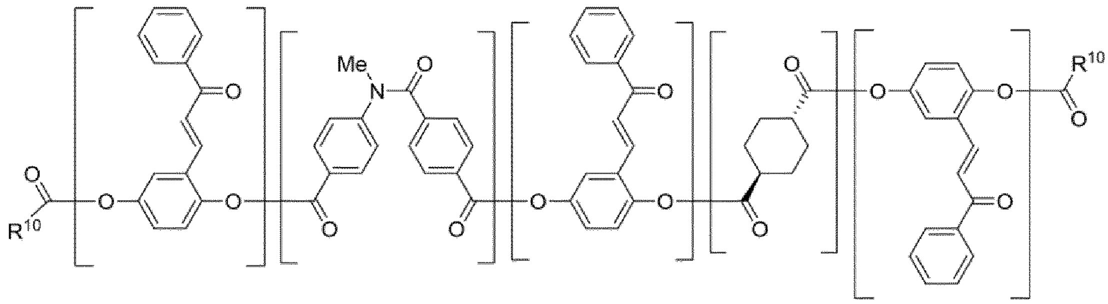

(PA1-1-4-1-1)

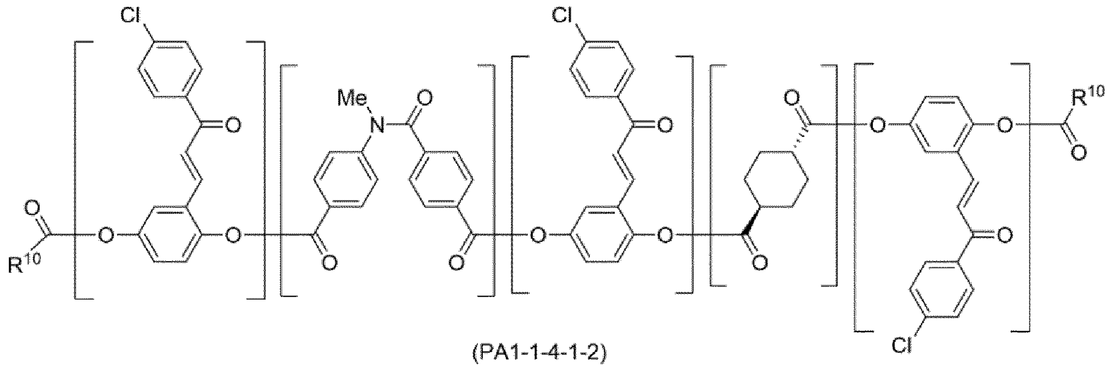

(PA1-1-4-1-2)

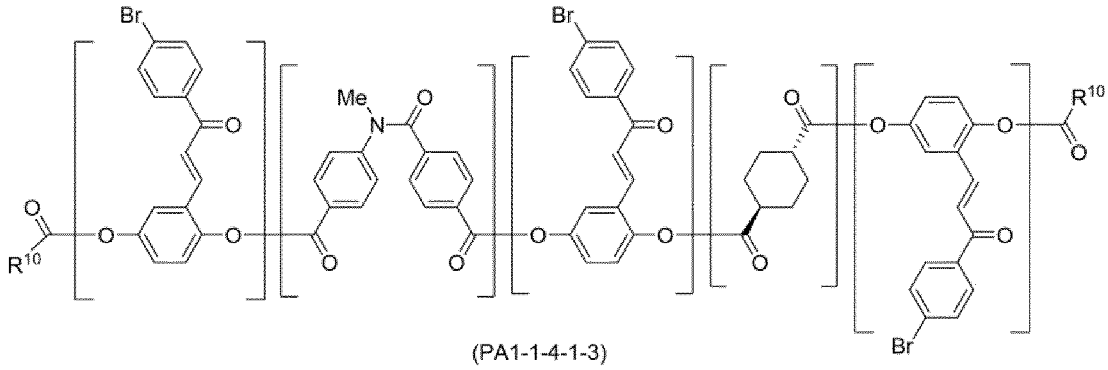

(PA1-1-4-1-3)

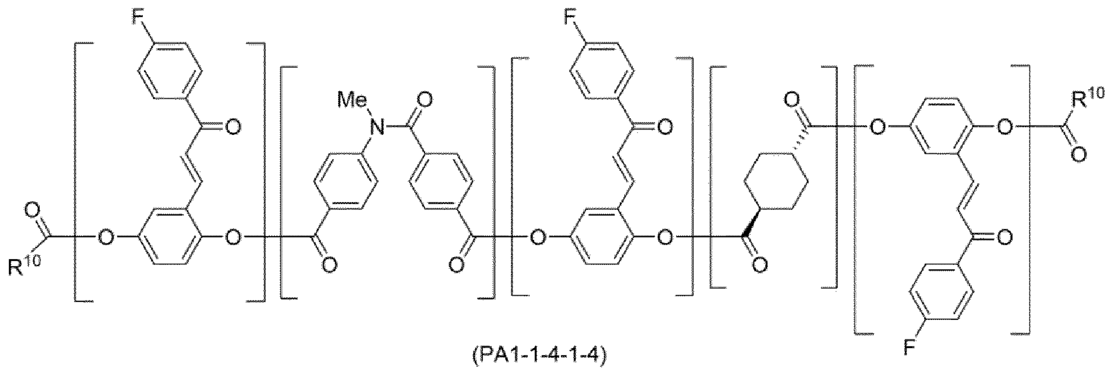

(PA1-1-4-1-4)

[Chem. 70]

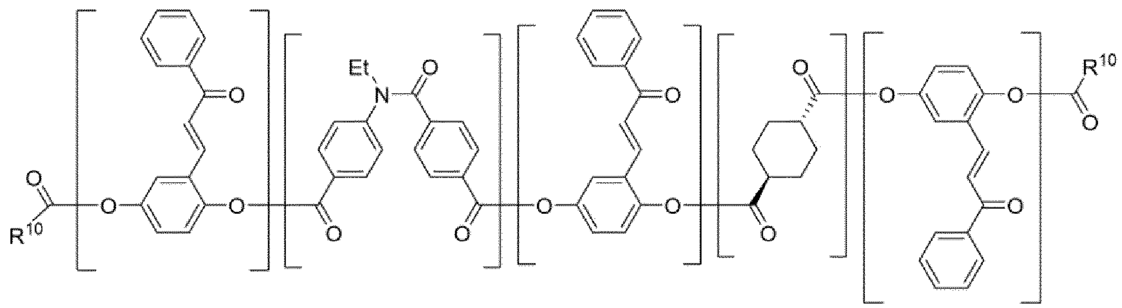

(PA1-1-4-2-1)

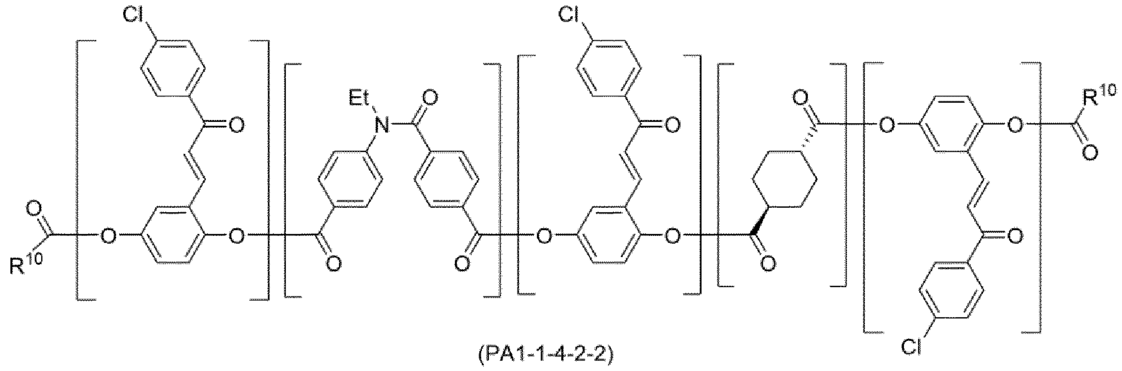

(PA1-1-4-2-2)

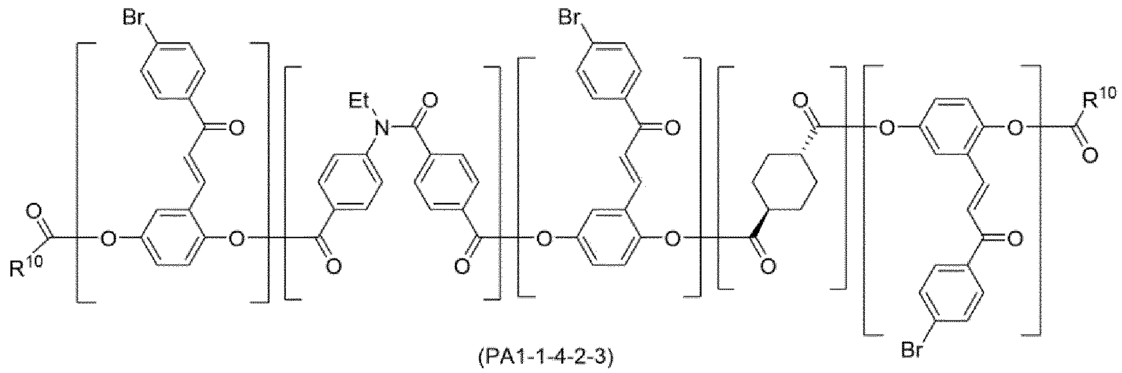

(PA1-1-4-2-3)

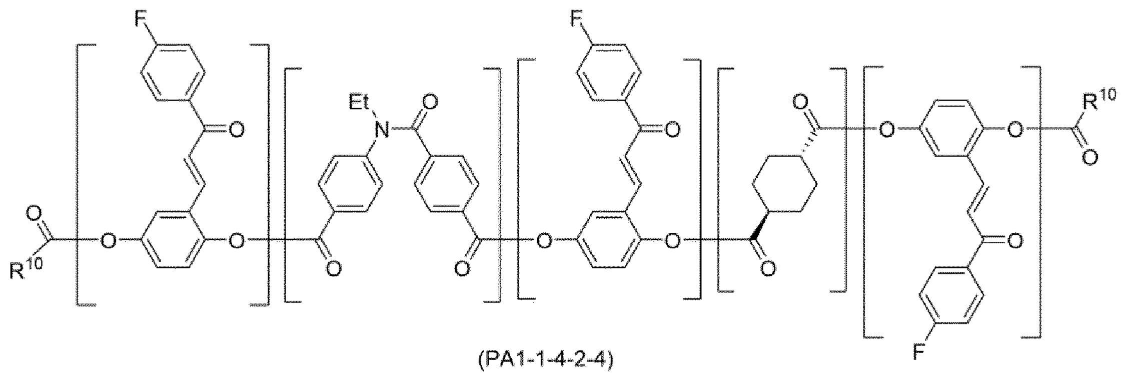

(PA1-1-4-2-4)

[Chem. 71]

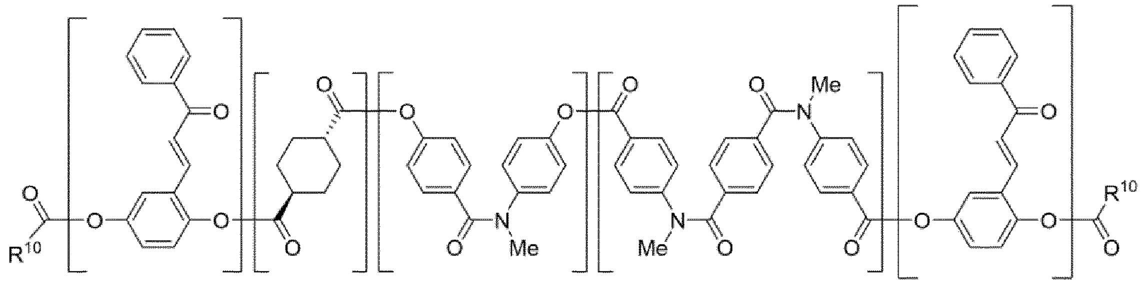

(PA1-1-5-1-1)

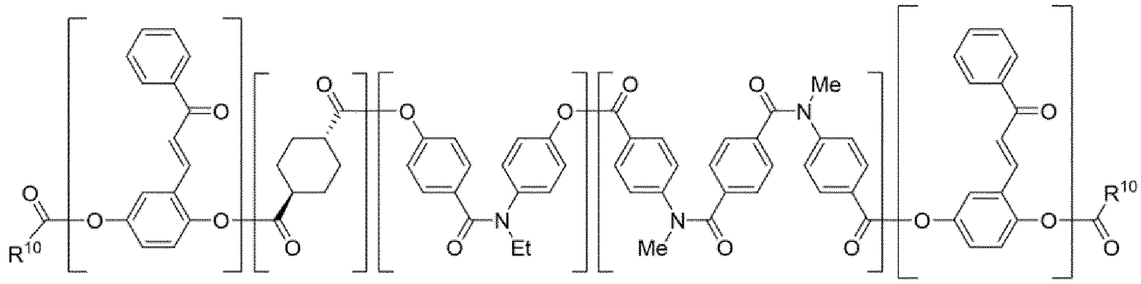

(PA1-1-5-2-1)

[Chem. 72]

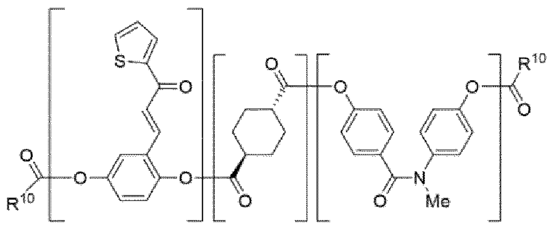

(PA1-2-1-1-1)

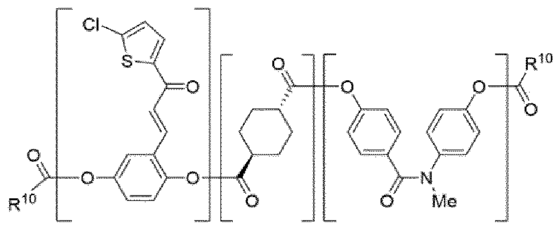

(PA1-2-1-1-2)

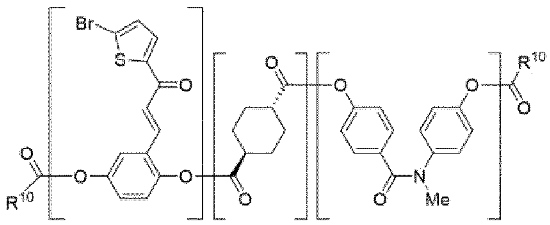

(PA1-2-1-1-3)

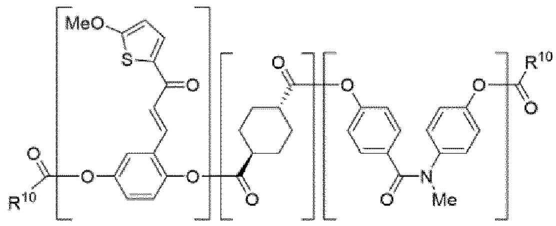

(PA1-2-1-1-4)

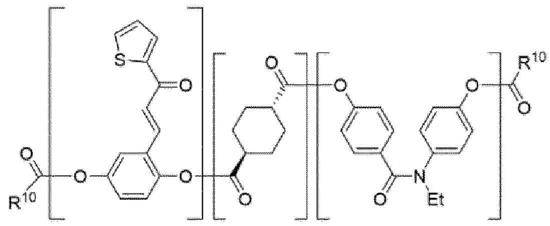

(PA1-2-1-2-1)

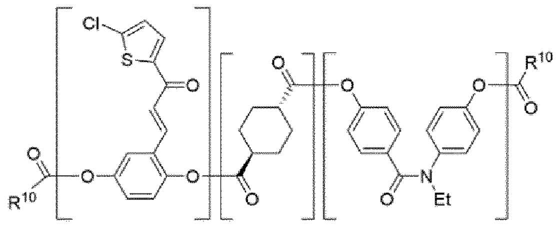

(PA1-2-1-2-2)

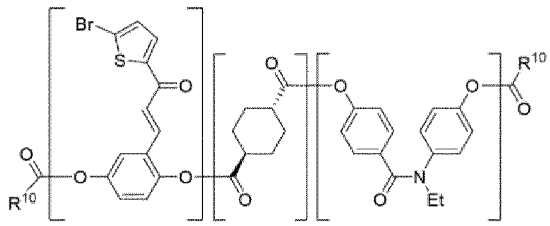

(PA1-2-1-2-3)

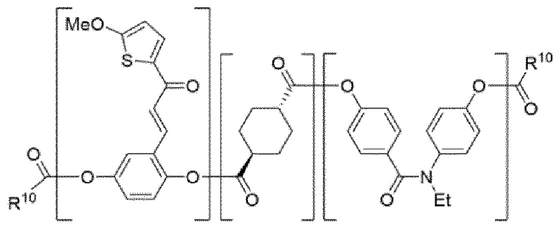

(PA1-2-1-2-4)

[Chem. 73]

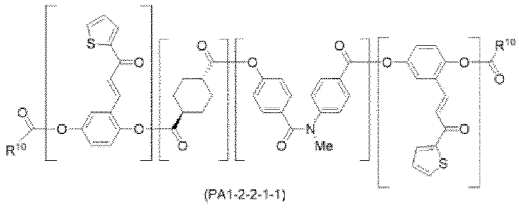

(PA1-2-2-1-1)

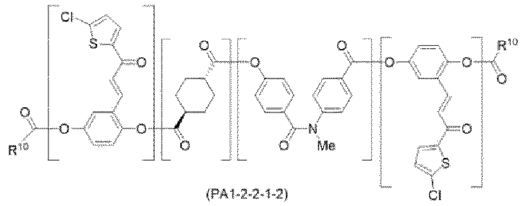

(PA1-2-2-1-2)

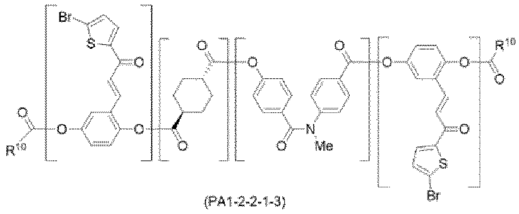

(PA1-2-2-1-3)

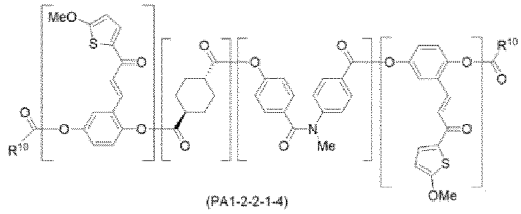

(PA1-2-2-1-4)

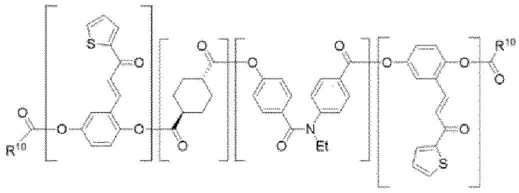

(PA1-2-2-2-1)

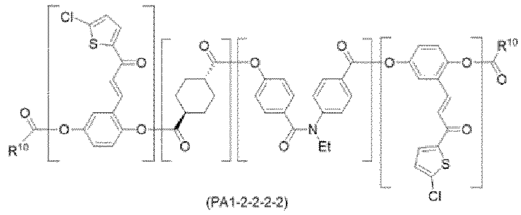

(PA1-2-2-2-2)

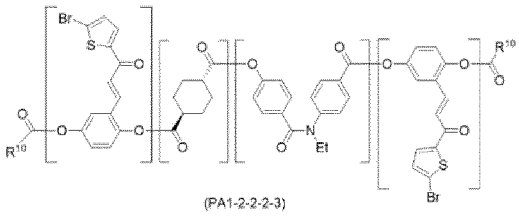

(PA1-2-2-2-3)

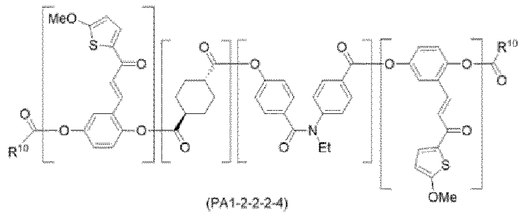

(PA1-2-2-2-4)

[Chem. 74]

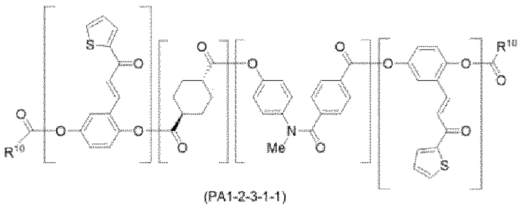

(PA1-2-3-1-1)

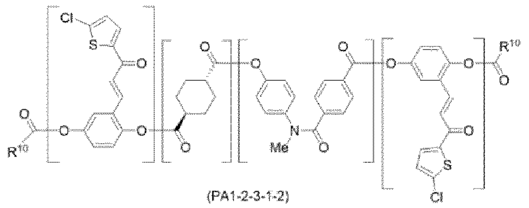

(PA1-2-3-1-2)

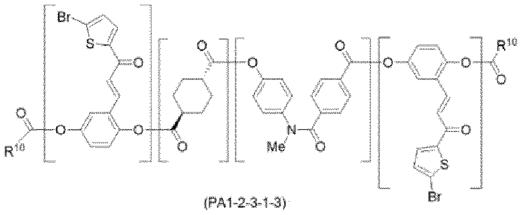

(PA1-2-3-1-3)

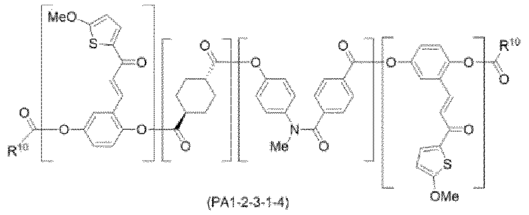

(PA1-2-3-1-4)

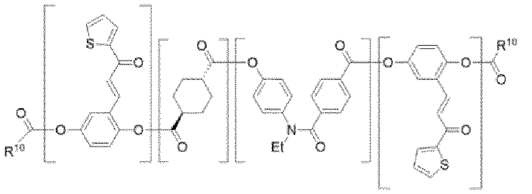

(PA1-2-3-2-1)

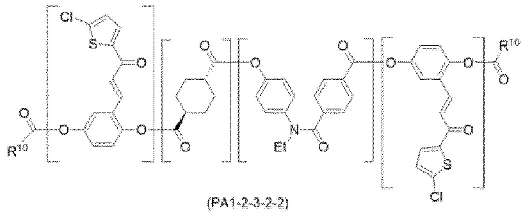

(PA1-2-3-2-2)

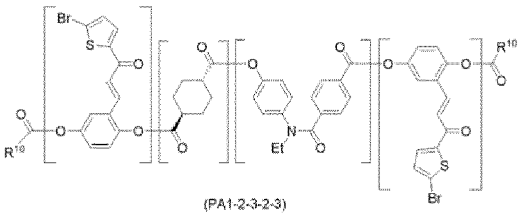

(PA1-2-3-2-3)

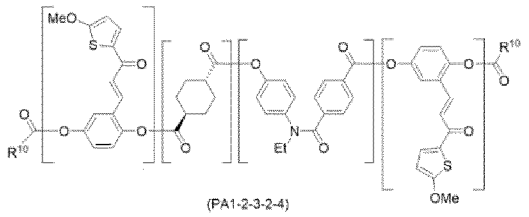

(PA1-2-3-2-4)

[Chem. 75]

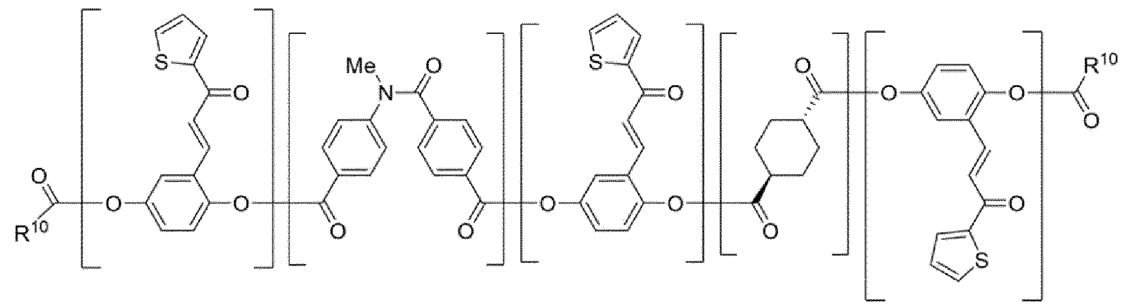

(PA1-2-4-1-1)

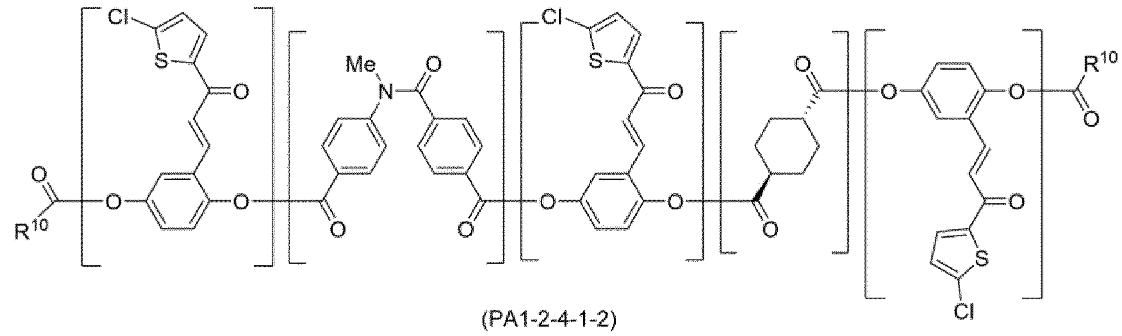

(PA1-2-4-1-2)

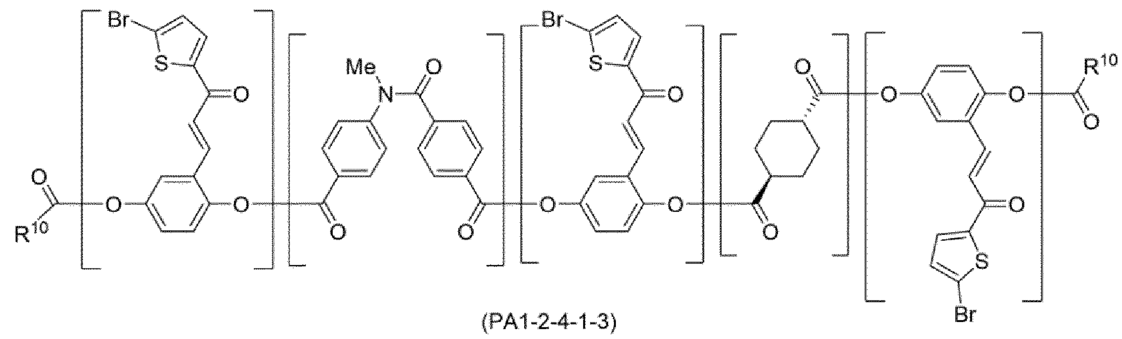

(PA1-2-4-1-3)

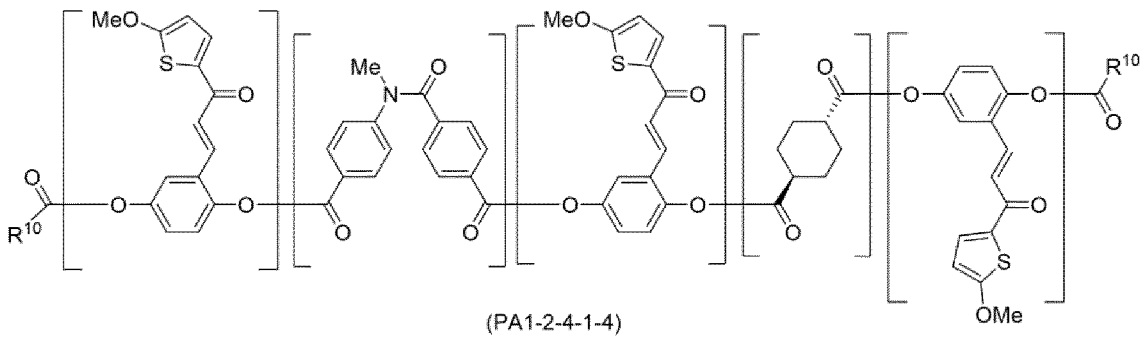

(PA1-2-4-1-4)

[Chem. 76]

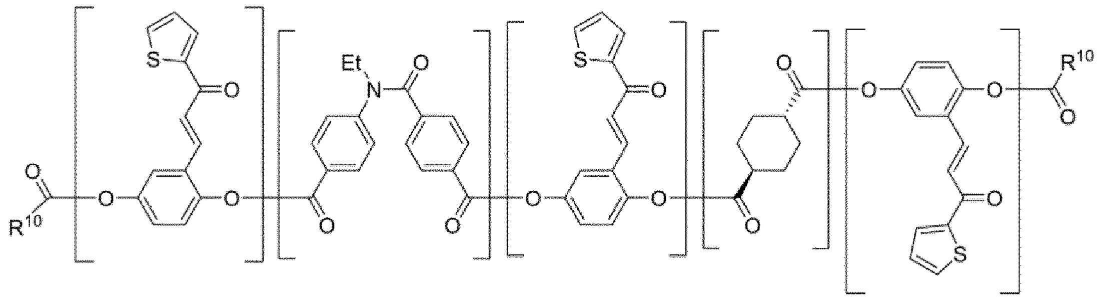

(PA1-2-4-2-1)

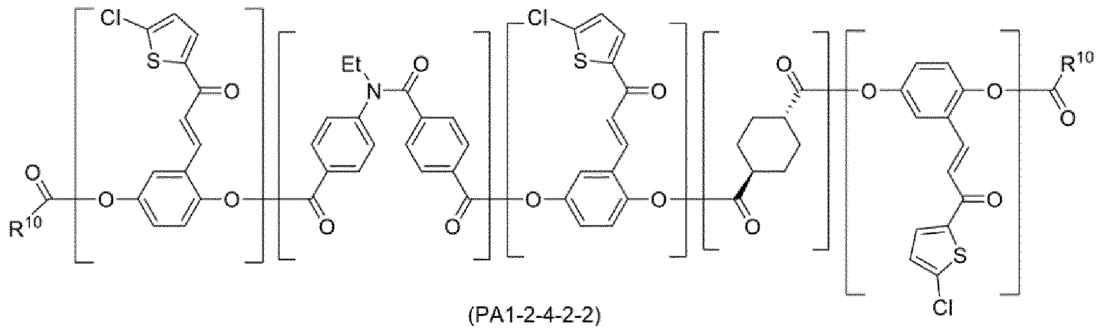

(PA1-2-4-2-2)

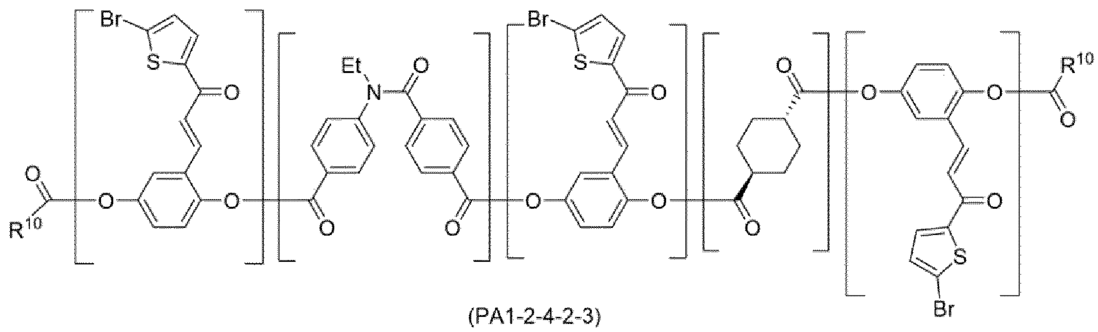

(PA1-2-4-2-3)

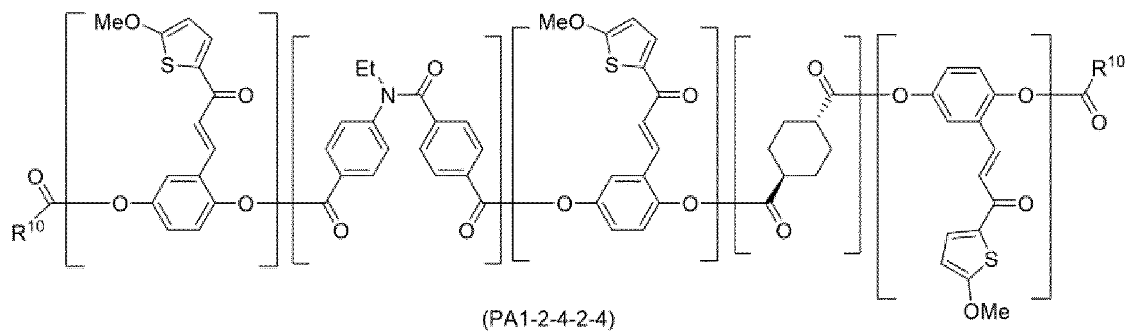

(PA1-2-4-2-4)

[Chem. 77]

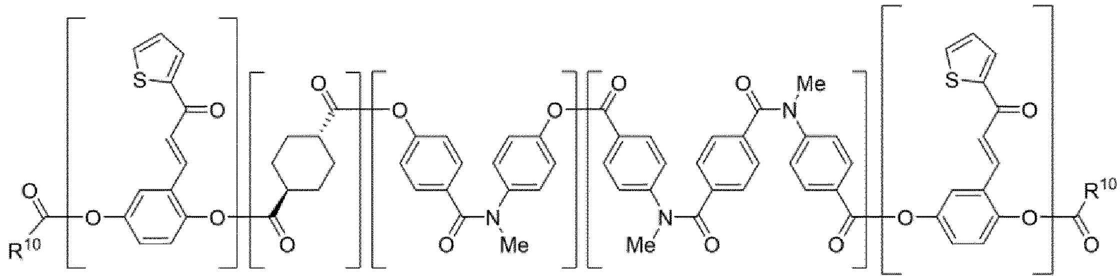

(PA1-2-5-1-1)

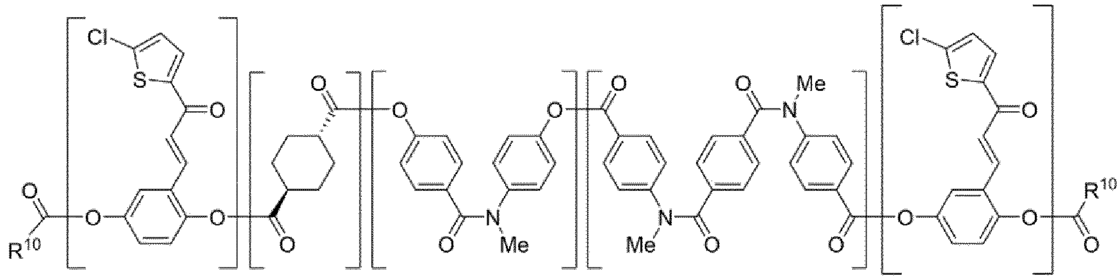

(PA1-2-5-1-2)

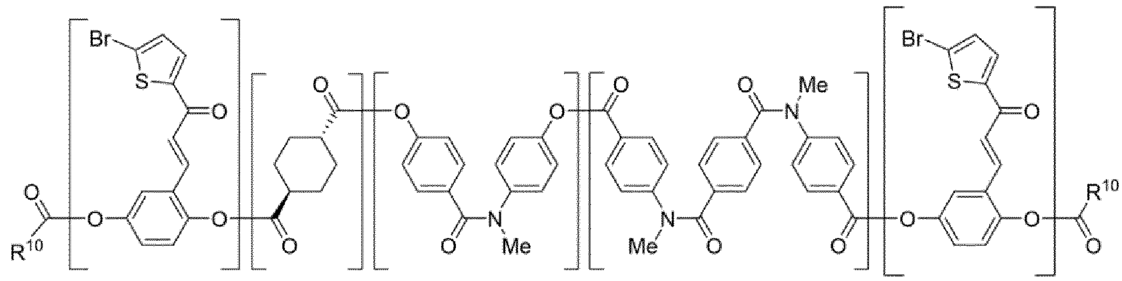

(PA1-2-5-1-3)

[Chem. 78]

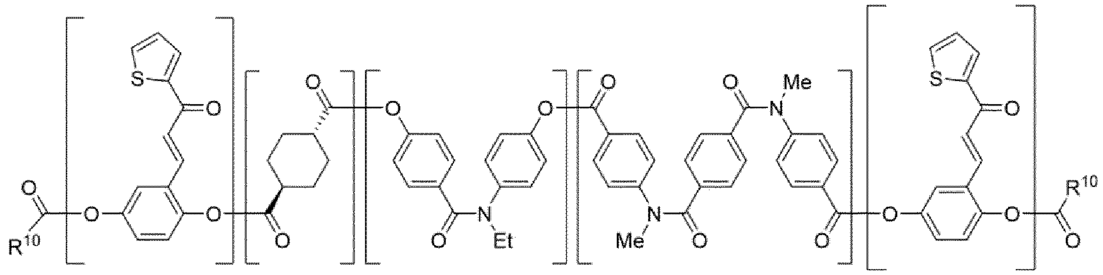

(PA1-2-5-2-1)

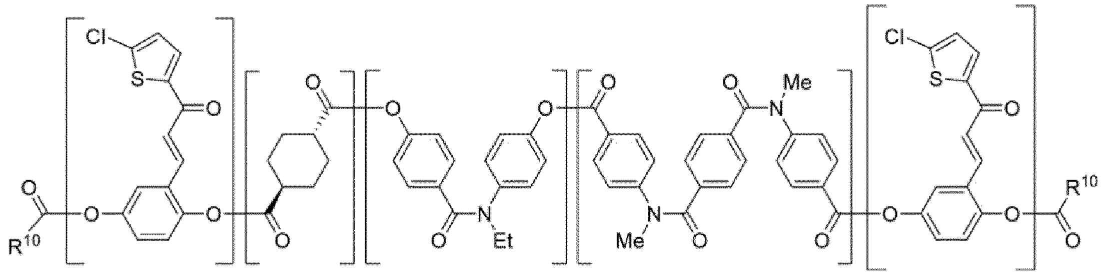

(PA1-2-5-2-2)

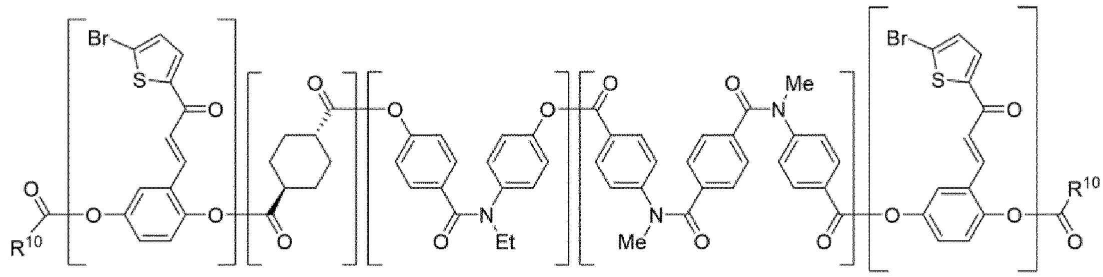

(PA1-2-5-2-3)

[Chem. 79]

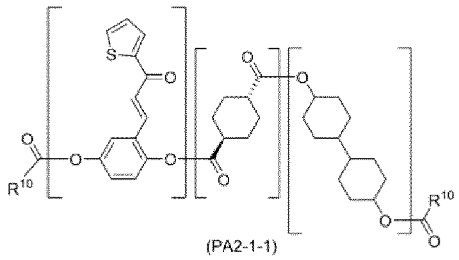

(PA2-1-1)

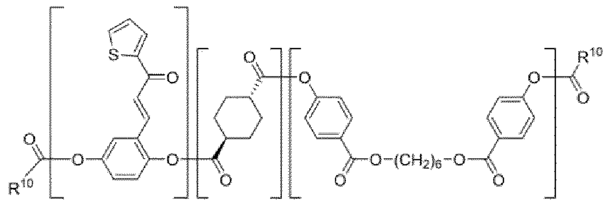

(PA2-1-2)

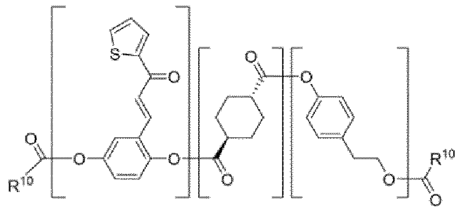

(PA2-1-3)

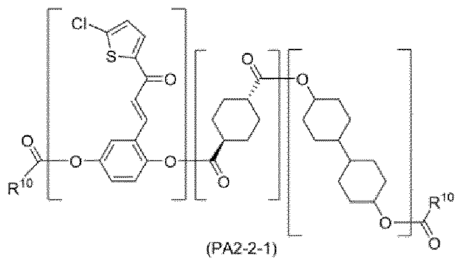

(PA2-2-1)

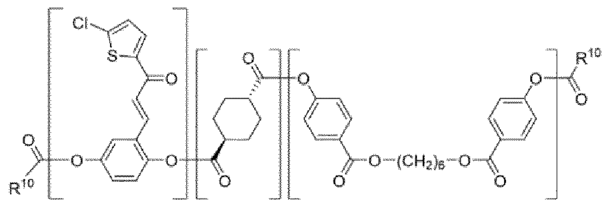

(PA2-2-2)

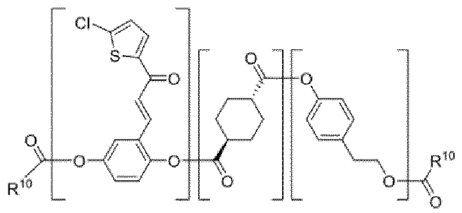

(PA2-2-3)

[Chem. 80]

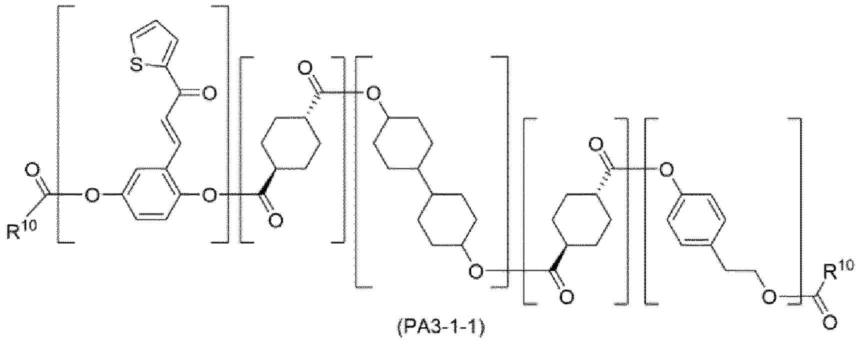

(PA3-1-1)

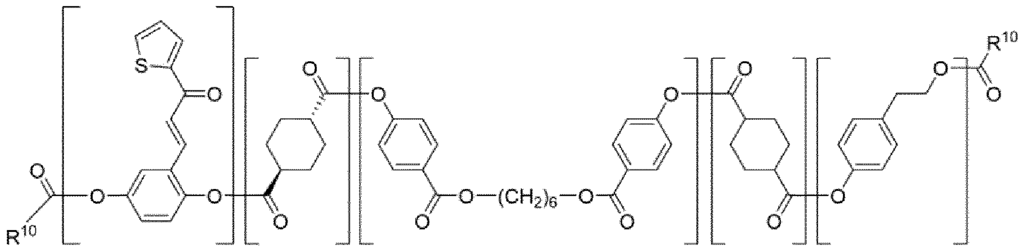

(PA3-1-2)

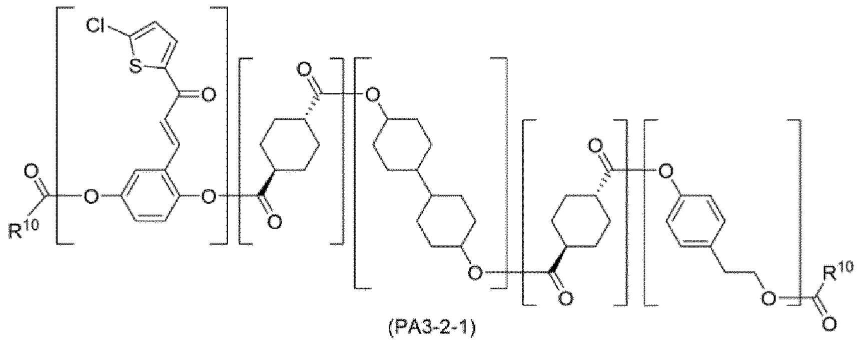

(PA3-2-1)

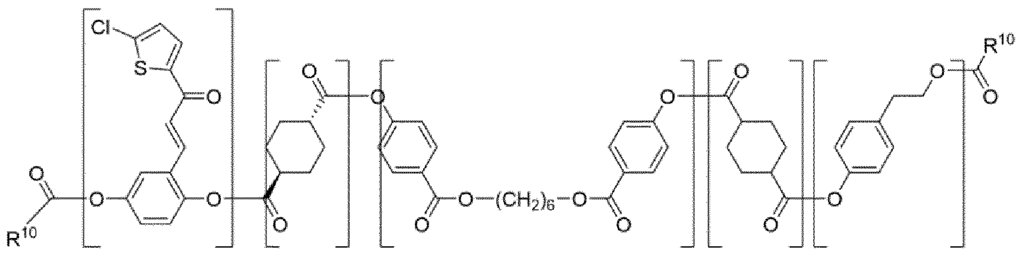

(PA3-2-2)

[Chem. 81]

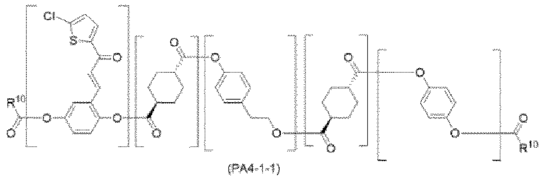

(PA4-1-1)

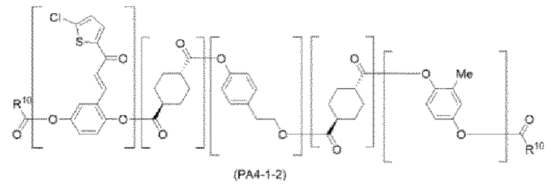

(PA4-1-2)

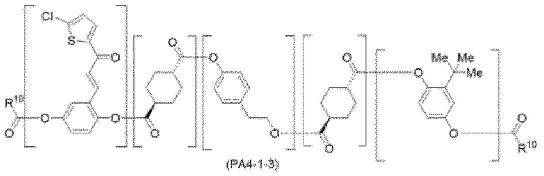

(PA4-1-3)

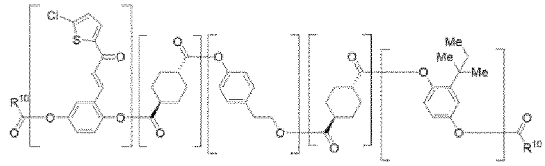

(PA4-1-4)

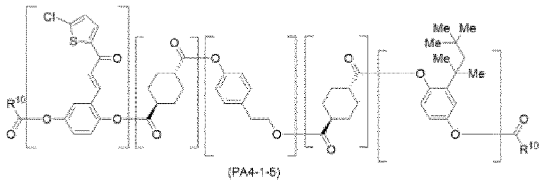

(PA4-1-5)

[Chem. 82]

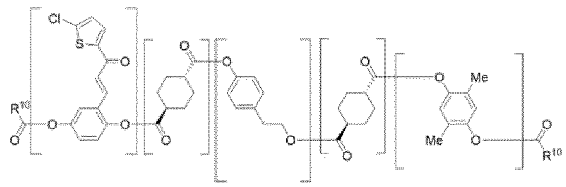

(PA4-1-6)

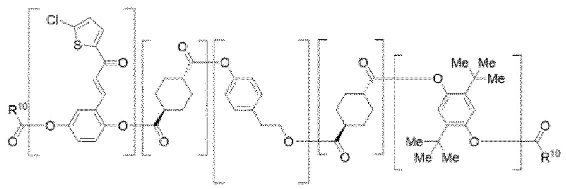

(PA4-1-7)

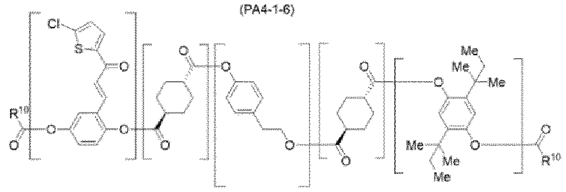

(PA4-1-8)

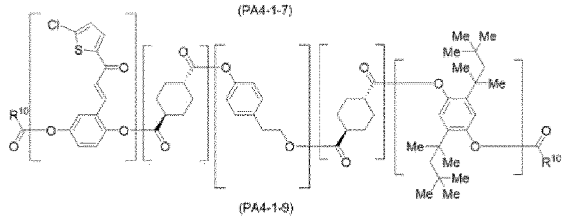

(PA4-1-9)

[Chem. 83]

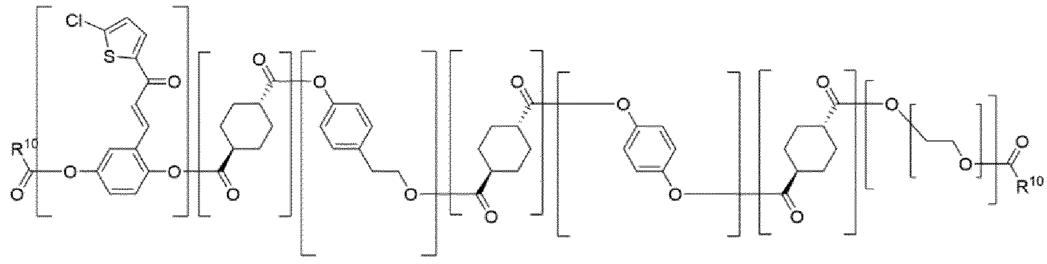

(PA4-2-1)

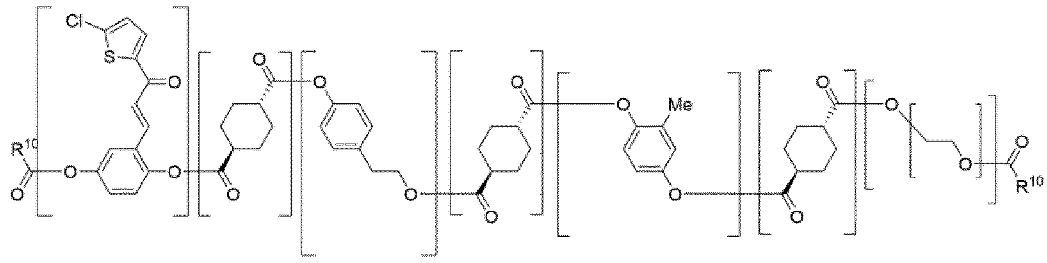

(PA4-2-2)

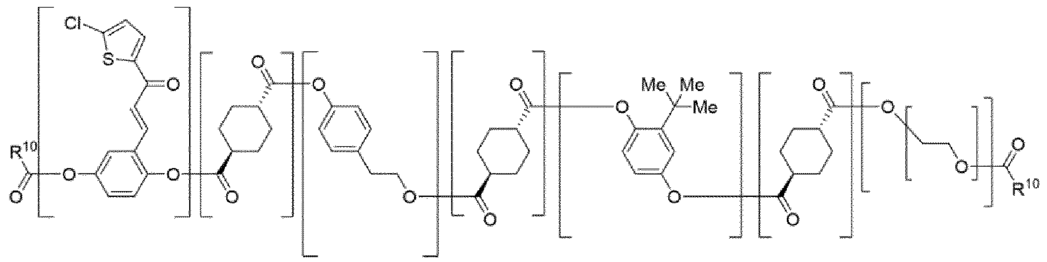

(PA4-2-3)

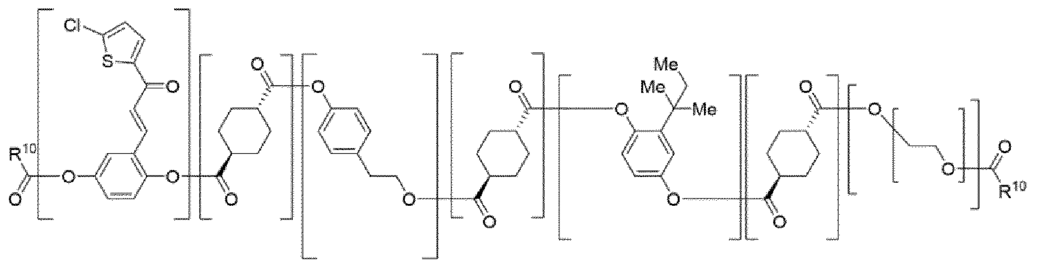

(PA4-2-4)

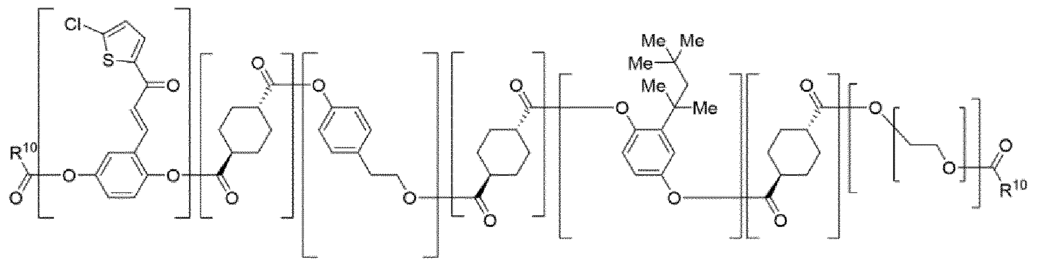

(PA4-2-5)

[Chem. 84]

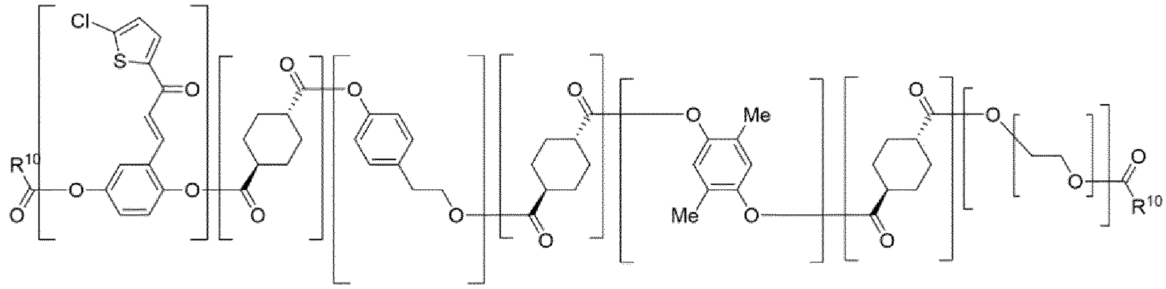

(PA4-2-6)

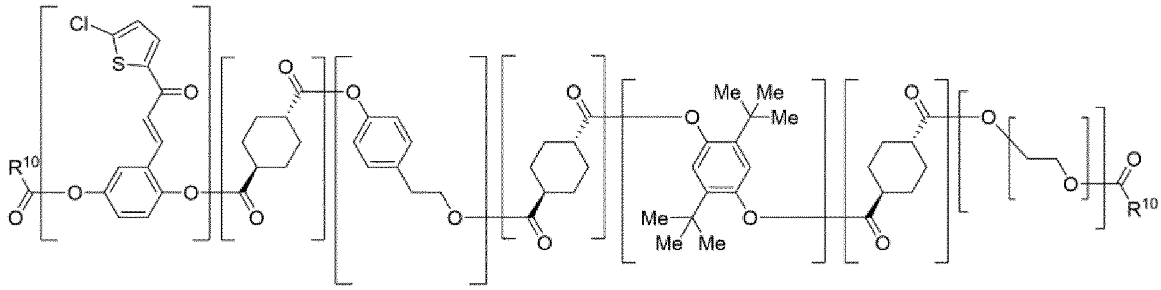

(PA4-2-7)

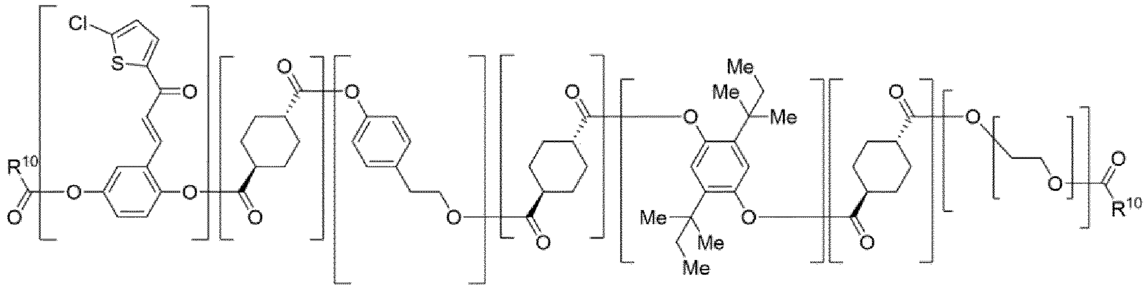

(PA4-2-8)

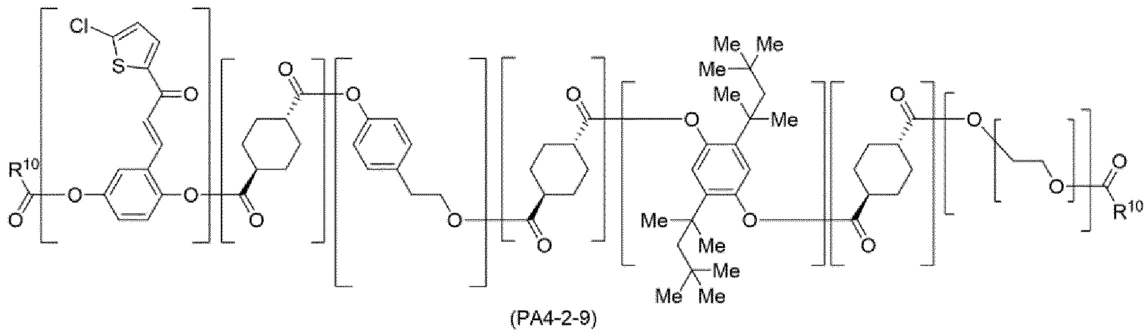

(PA4-2-9)

[Chem. 85]

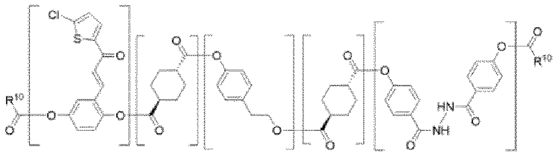

(PA5-1-1)

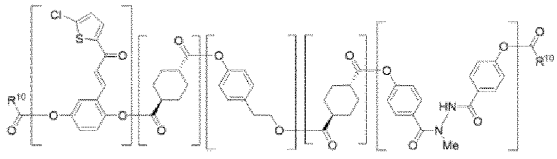

(PA5-1-2)

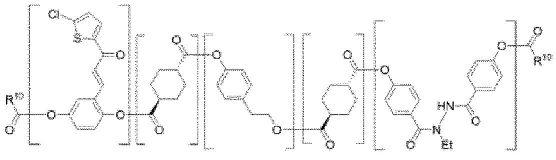

(PA5-1-3)

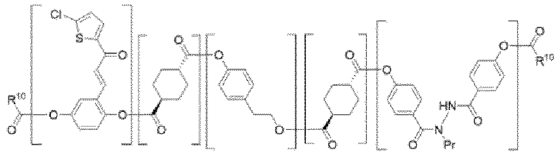

(PA5-1-4)

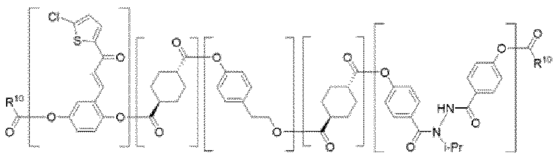

(PA5-1-5)

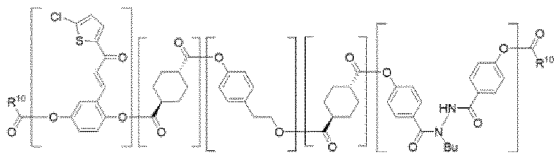

(PA5-1-6)

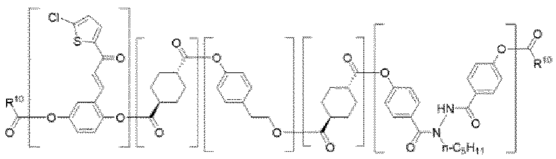

(PA5-1-7)

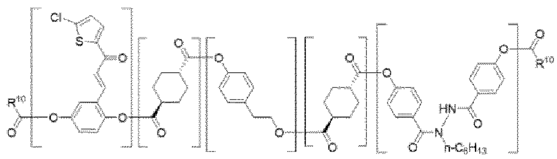

(PA5-1-8)

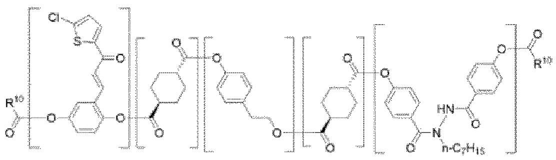

(PA5-1-9)

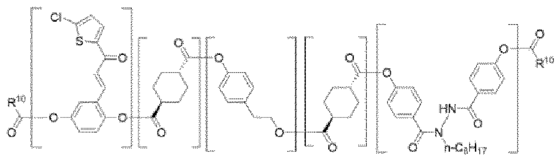

(PA5-1-10)

[Chem. 86]

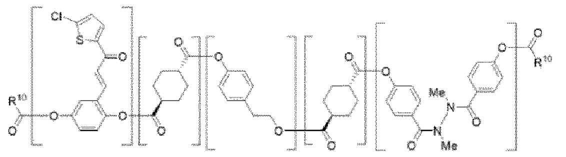

(PA5-1-11)

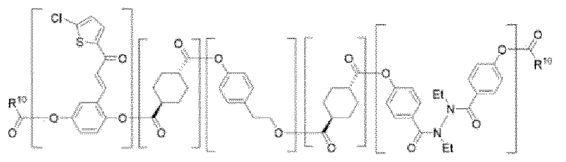

(PA5-1-12)

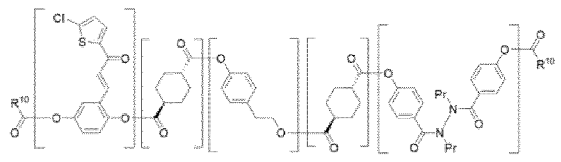

(PA5-1-13)

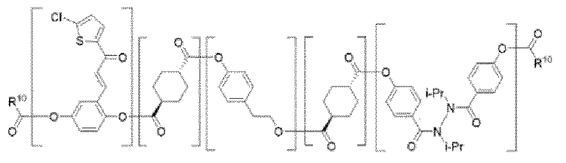

(PA5-1-14)

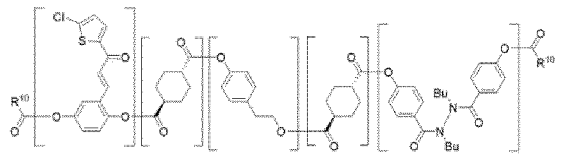

(PA5-1-15)

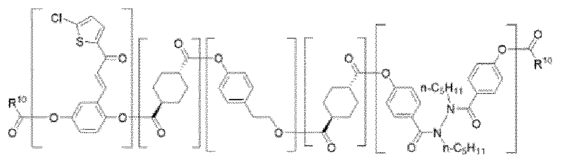

(PA5-1-16)

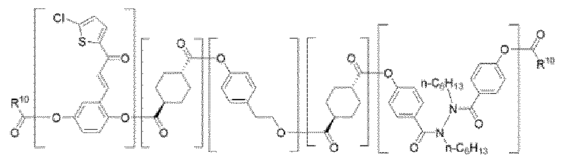

(PA5-1-17)

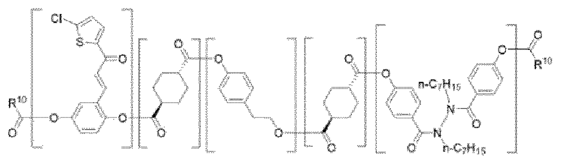

(PA5-1-18)

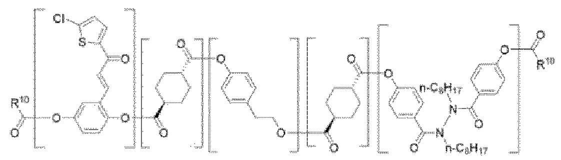

(PA5-1-19)

[Chem. 87]

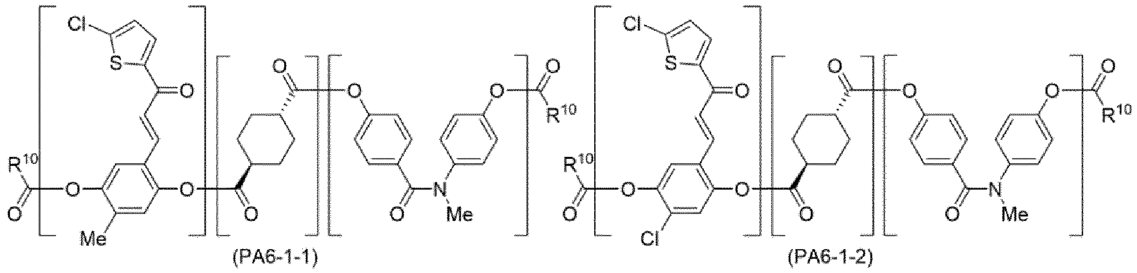

(PA6-1-1) (PA6-1-2)

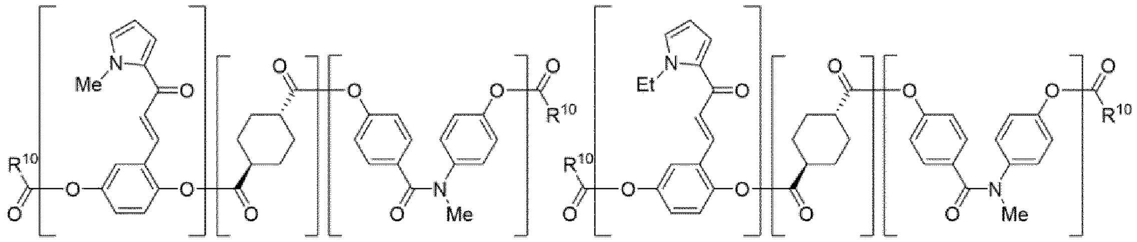

(PA6-1-3) (PA6-1-4)

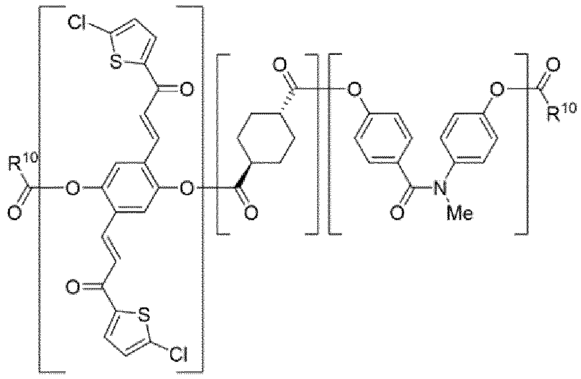

(PA6-1-5)

[Chem. 88]

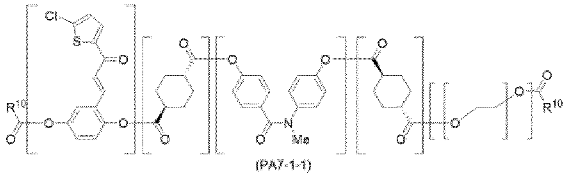
(PA7-1-1)

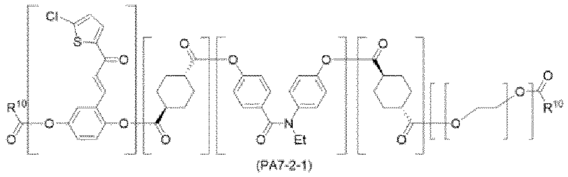
(PA7-2-1)

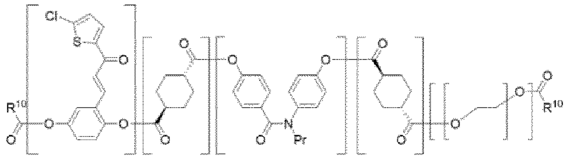
(PA7-3-1)

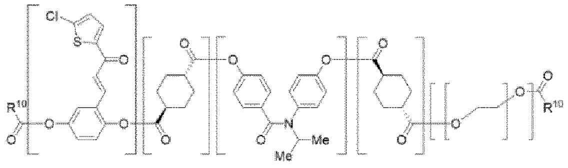
(PA7-3-2)

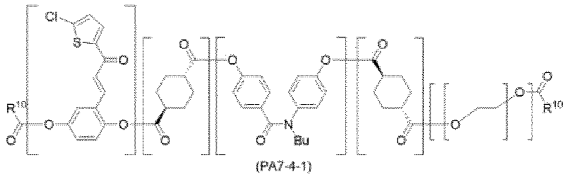
(PA7-4-1)

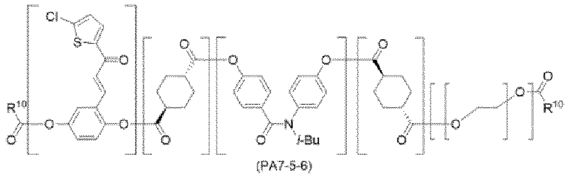
(PA7-5-6)

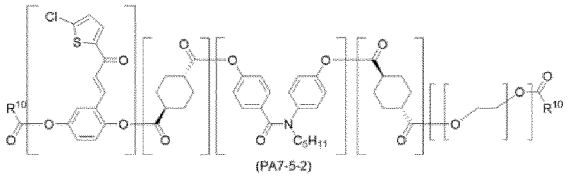
(PA7-5-2)

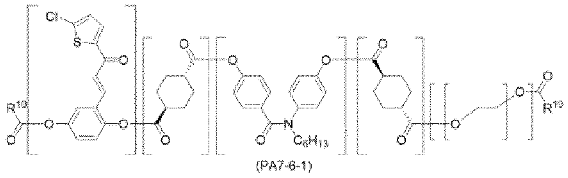
(PA7-6-1)

[Chem. 89]

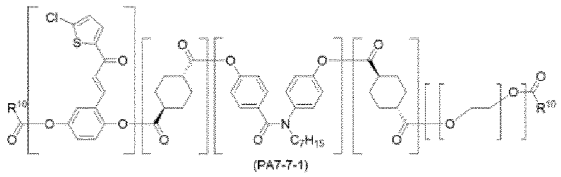
(PA7-7-1)

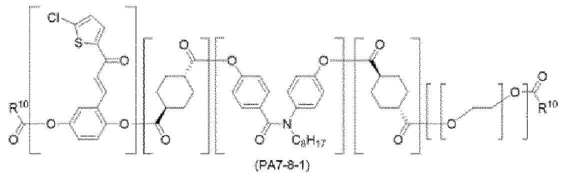
(PA7-8-1)

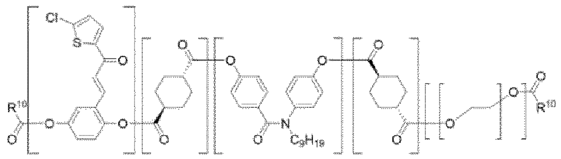
(PA7-9-1)

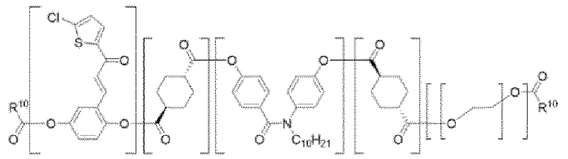
(PA7-10-1)

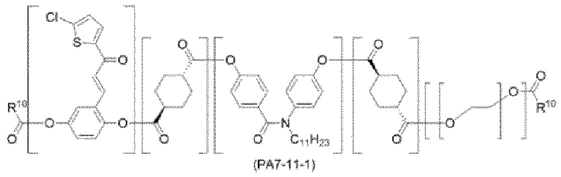
(PA7-11-1)

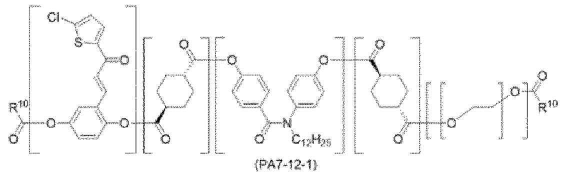
(PA7-12-1)

(In chemical formulae (PA1-1-1-1-1) to (PA7-12-1) above, $R^{10}$ is synonymous with $R^{10}$ in chemical formula (2') above.)

Of these chemical formulae (PA1-1-1-1-1) to (PA7-12-1), particularly preferred ones are chemical formulae (PA1-2-1-1-1) to (PA1-2-5-1-4), (PA2-2-1) to (PA2-2-3), (PA3-2-1) to (PA3-2-2), (PA4-1-1) to (PA5-1-19) and (PA7-1-1) to (PA7-12-1) because their optical characteristics are good.

As for the method for producing the polymer according to the present invention, the polymer can be produced by polymerizing a source composition that contains a dihydroxy compound indicated by chemical formula (1') below. In particular, it is especially preferred to incorporate one or more species selected from the group consisting of dicarboxylic acid dichlorides and dicarboxylic acids in the source composition and polymerize the composition. A method for producing the polymer according to the present invention is also an aspect of the present invention.

[Chem. 90]

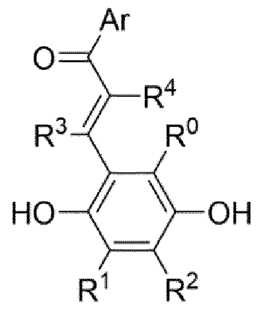

(1')

[In chemical formula (1') above, $R^0$, $R^1$, $R^2$, $R^3$ and $R^4$ each independently represent a hydrogen atom, a halogen atom, a C1 to C8 alkyl group or a group represented by chemical formula (Z1) below. Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.]

[Chem. 91]

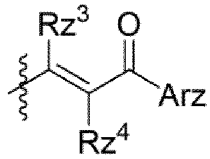

(Z1)

[In chemical formula (Z1) above, $Rz^3$ and $Rz^4$ each independently represent a hydrogen atom, a halogen atom or a C1 to C8 alkyl group.

Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent. The wavy-lined portion represents a site of binding with the portion in formula (1) excluding R°, $R^1$, $R^2$, $R^3$ or $R^4$.]

In formula (1'), R°, $R^1$, $R^2$, $R^3$ and $R^4$ each independently represent a hydrogen atom, a halogen atom, a C1 to C8 alkyl group or a group represented by formula (Z1). Examples of halogen atoms include a chlorine atom, a bromine atom, an iodine atom and a fluorine atom, and examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group.

In formula (Z1), $Rz^3$ and $Rz^4$ each independently represent a hydrogen atom, a halogen atom or a C1 to C8 alkyl group. Examples of halogen atoms include a chlorine atom, a bromine atom, an iodine atom and a fluorine atom, and examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group.

In formula (Z1), for use as $Rz^3$ and $Rz^4$, a hydrogen atom, a methyl group, an ethyl group, a propyl group, an isopropyl group, a butyl group, an isobutyl group, a sec-butyl group and a tert-butyl group are preferred because they ensure that the polymer according to the present invention will exhibit good optical characteristics. A hydrogen atom, a methyl group and an ethyl group are particularly preferred.

Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system and annulated aromatic ring system optionally having a substituent.

Examples of monocyclic aromatic ring systems at Arz whose ring-constituting atoms are atoms selected from the group consisting of a carbon atom, a nitrogen atom, an oxygen atom and a sulfur atom and that may have a substituent

include benzene, pyridine, pyrazine, pyrimidine, pyridazine, furan, pyrrole, imidazole, thiophene, pyrazole, oxazole, isoxazole, thiazole and triazole.

Examples of polycyclic aromatic ring systems at Arz whose ring-constituting atoms are atoms selected from the group consisting of a carbon atom, a nitrogen atom, an oxygen atom and a sulfur atom and that may have a substituent include biphenyl, terphenyl, bipyridine, bithiophene and bifuran.

Examples of annulated aromatic ring systems at Arz whose ring-constituting atoms are atoms selected from the group consisting of a carbon atom, a nitrogen atom, an oxygen atom and a sulfur atom and that may have a substituent include naphthalene, anthracene, phenanthrene, quinoline, benzofuran, benzimidazole, benzothiophene, indole, indazole, benzoxazole, benzothiazole and benzotriazole.

For use as Arz, a monocyclic aromatic ring system whose ring-constituting atoms are atoms selected from the group consisting of a carbon atom, a nitrogen atom, an oxygen atom and a sulfur atom and that may have a substituent is preferred because of easy availability. More preferably, this aromatic ring system is a benzene ring, a furan ring, a thiophene ring, a thiazole ring or an oxazole ring because this ensures that the polymer according to the present invention will exhibit good optical characteristics. A benzene ring or thiophene ring is particularly preferred because they ensure that the polymer according to the present invention will exhibit better optical characteristics.

For use as R°, $R^1$, $R^2$, $R^3$ and $R^4$, a hydrogen atom, a methyl group, an ethyl group, a propyl group, an isopropyl group, a butyl group, an isobutyl group, a sec-butyl group, a tert-butyl group and the groups represented by chemical formulae (Z1-1) to (Z1-4) below are preferred because optical characteristics after their introduction into the polymer according to the present invention will be superior. A hydrogen atom, a methyl group and an ethyl group are particularly preferred because of the ease of introduction.

[Chem. 92]

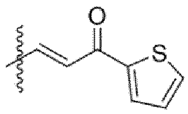
(Z1-1)

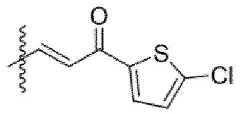
(Z1-2)

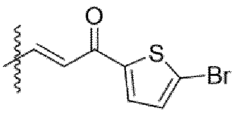
(Z1-3)

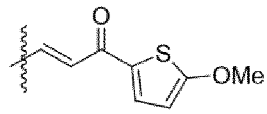
(Z1-4)

In chemical formula (1'), examples of Ars are the same Ars as in chemical formula (1). Preferred examples include the structures indicated by chemical formulae (Ar-1) to (Ar-7) below.

[Chem. 93]

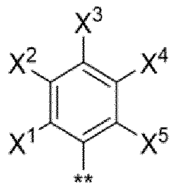
(Ar-1)

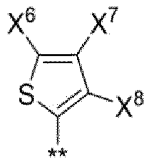
(Ar-2)

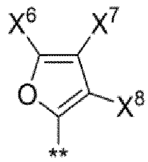
(Ar-3)

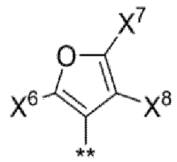
(Ar-4)

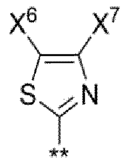
(Ar-5)

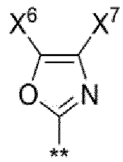
(Ar-6)

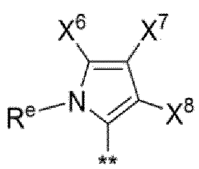
(Ar-7)

[In chemical formulae (Ar-1) to (Ar-7) above, $X^1$, $X^2$, $X^3$, $X^4$, $X^5$, $X^6$, $X^7$ and $X^8$ each independently represent a hydrogen atom or a C1 to C8 alkyl group, a C1 to C6 alkoxy group, a halogen atom, a nitro group, a cyano group, a C1 to C6 alkylthio group or a C2 to C8 dialkylamino group.

$R^e$ represents a hydrogen atom or a C1 to C10 alkyl group.

** represents a site of binding with the other portion in chemical formula (1) above, excluding Ar.]

In (Ar-1) to (Ar-7), $X^1$, $X^2$, $X^3$, $X^4$, $X^5$, $X^6$, $X^7$ and $X^8$ each independently represent a hydrogen atom, a C1 to

C8 alkyl group, a C1 to C6 alkoxy group, a halogen atom, a nitro group, a cyano group, a C1 to C6 alkylthio group or a C2 to C8 dialkylamino group. Examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group, examples of C1 to C6 alkoxy groups include a methoxy group, an ethoxy group, a propyloxy group, an isopropyloxy group, a butyloxy group, an isobutyloxy group, a sec-butyloxy group, a tert-butyloxy group, a pentyloxy group and a hexyloxy group, examples of halogen atoms include a fluorine atom, a chlorine atom, a bromine atom and an iodine atom, examples of C1 to C6 alkylthio groups include a methylthio group, an ethylthio group, a propylthio group, an isopropylthio group, a butylthio group, an isobutylthio group, a sec-butylthio group, a tert-butylthio group, a pentylthio group and a hexylthio group, and examples of C2 to C8 dialkylamino groups include a dimethylamino group, a diethylamino group, a dipropylamino group and a dibutylamino group.

For $X^1$, $X^2$, $X^3$, $X^4$, $X^5$, $X^6$, $X^7$ and $X^8$, it is preferred that each be a hydrogen atom, a C1 to C8 alkyl group, a C1 to C6 alkoxy group or a halogen atom because optical characteristics after their introduction into the polymer according to the present invention will be superior. It is particularly preferred that each be a hydrogen atom, a methyl group, a methoxy group or a halogen atom because of the ease of introduction.

For use as the dihydroxy compound indicated by chemical formula (1') above, the dihydroxy compounds represented by chemical formulae (1'-1-1) to (1'-7-4) below are preferred.

[Chem. 94]

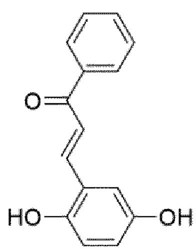

(1'-1-1)

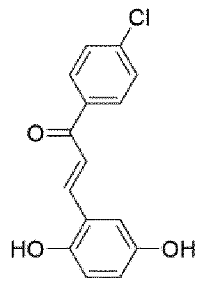

(1'-1-2)

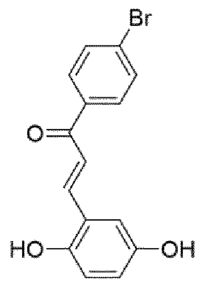

(1'-1-3)

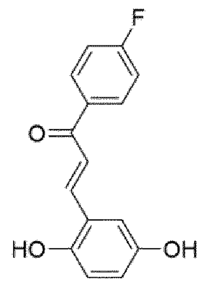

(1'-1-4)

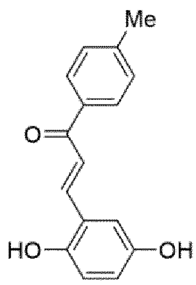

(1'-1-5)

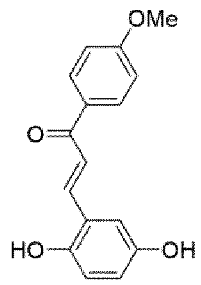

(1'-1-6)

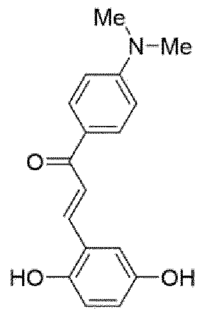

(1'-1-7)

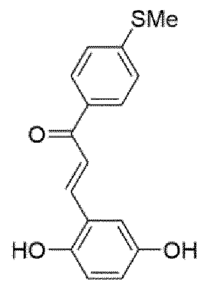

(1'-1-8)

[Chem. 95]

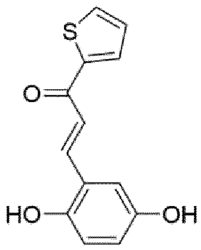
(1'-2-1)

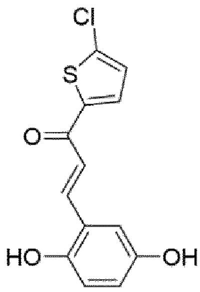
(1'-2-2)

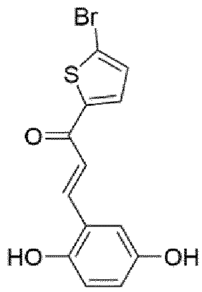
(1'-2-3)

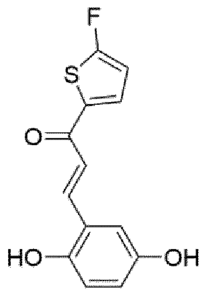
(1'-2-4)

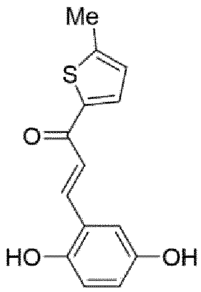
(1'-2-5)

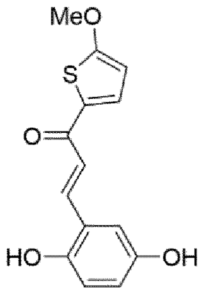
(1'-2-6)

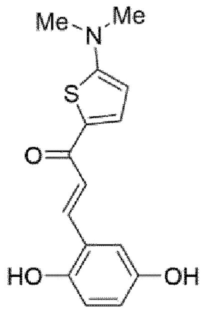
(1'-2-7)

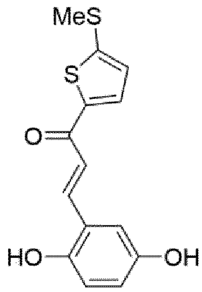
(1'-2-8)

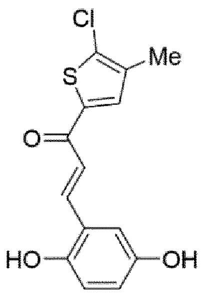
(1'-2-9)

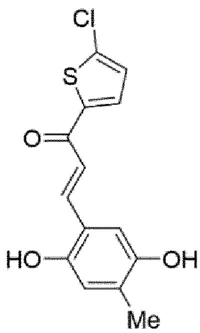
(1'-2-10)

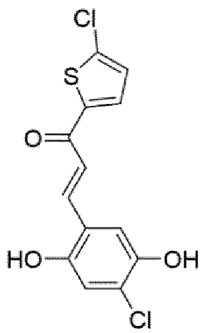
(1'-2-11)

[0225] [Chem. 96]

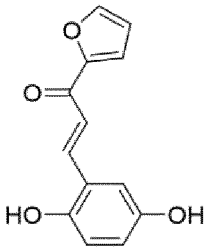
(1'-3-1)

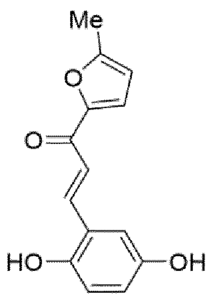
(1'-3-2)

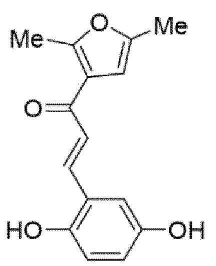
(1'-3-3)

[Chem. 97]

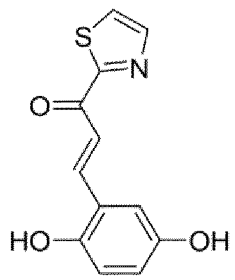

(1'-4-1)

[0227] [Chem. 98]

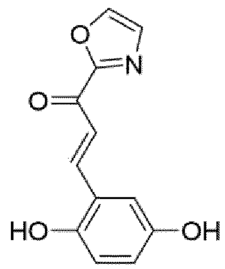

(1'-5-1)

[0228] [Chem. 99]

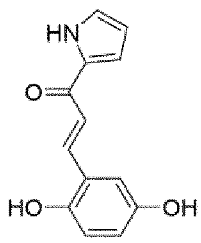

(1'-6-1)

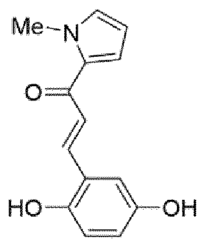

(1'-6-2)

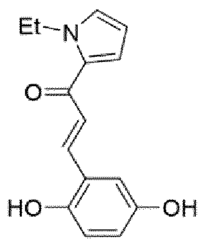

(1'-6-3)

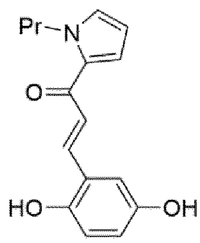

(1'-6-4)

[Chem. 100]

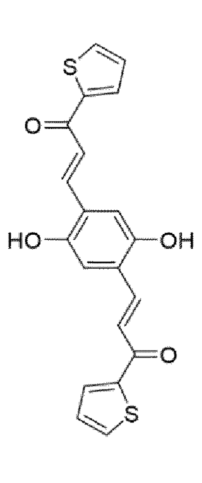

(1'-7-1)

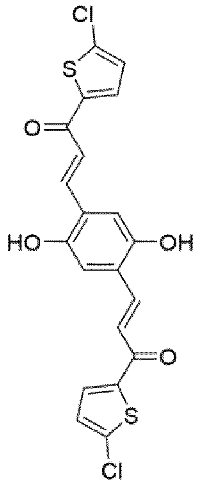

(1'-7-2)

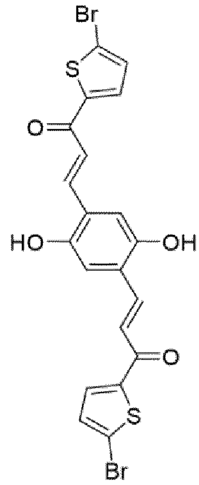

(1'-7-3)

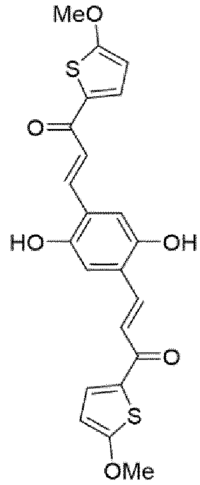

(1'-7-4)

Of these (1'-1-1) to (1'-7-4), particularly preferred compounds are (1'-2-1) to (1'-2-11), (1'-6-1) to (1'-6-4) and (1'-7-1) to (1'-7-4), with especially preferred ones being (1'-2-1) to (1'-2-6), (1'-2-1) to (1'-2-11), (1'-6-2) to (1'-6-3) and (1'-7-2).

Examples of dicarboxylic acids include aliphatic polycarboxylic acids (specifically, the saturated polycarboxylic acids containing 2 to 20 carbon atoms of oxalic acid, malonic acid, succinic acid, glutaric acid, adipic acid and sebacic acid, and the unsaturated polycarboxylic acids of maleic acid, fumaric acid and itaconic acid), alicyclic polycarboxylic acids (cyclobutanedicarboxylic acid, trans-1,4-cyclohexanedicarboxylic acid and cis-1,4-cyclohexanedicarboxylic acid) and aromatic polycarboxylic acids (terephthalic acid, isophthalic acid, orthophthalic acid, 4,4'-biphenyldicarboxylic acid, 4,4'-oxybis(benzoic acid) and 2,5-furandicarboxylic acid).

Examples of dicarboxylic acid dichlorides include the saturated carboxylic acid dichlorides containing 2 to 20 carbon atoms of oxalic acid dichloride, malonic acid dichloride, succinic acid dichloride, glutaric acid dichloride, adipic acid dichloride and sebacic acid dichloride, the unsaturated polycarboxylic acid dichlorides of fumaric acid dichloride and itaconic acid dichloride, the alicyclic carboxylic acid dichlorides of cyclobutanedicarboxylic acid dichloride, cyclopentanedicarboxylic acid dichloride, trans-1,4-cyclohexanedicarboxylic acid dichloride and cis-1,4-cyclohexanedicarboxylic acid dichloride and the aromatic polycarboxylic acid dichlorides of terephthalic acid dichloride, isophthalic acid dichloride, orthophthalic acid dichloride, 4,4'-biphenyldicarboxylic acid dichloride, 4,4'-oxybis(benzoyl acid chloride) and 2,5-furandicarboxylic acid dichloride.

In the method for producing the polymer according to the present invention, it is preferred to further incorporate compounds represented by chemical formulae (2"A), (2"B), (2"C) and (2"D) below in the source composition and polymerizing the composition.

[0234] [Chem. 101]

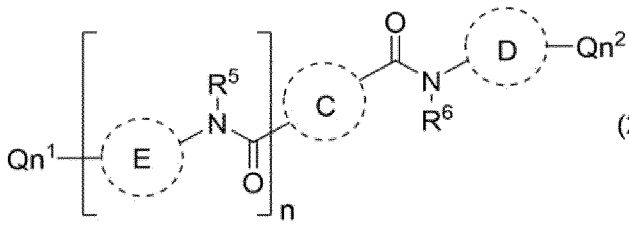

(2"A)

[In chemical formula (2"A) above, ring C, ring D and ring E each independently represent a ring system selected from the group consisting of a monocyclic aromatic ring system, a polycyclic aromatic ring system, an annulated aromatic ring system and an aliphatic hydrocarbon ring system whose ring-constituting atoms are atoms selected from the group consisting of a carbon atom, a nitrogen atom, an oxygen atom and a sulfur atom, with the monocyclic aromatic ring system, polycyclic aromatic ring system, annulated aromatic ring system and aliphatic hydrocarbon ring system optionally having a substituent.

$R^5$ and $R^6$ may be the same or different from each other, each representing a group selected from the group consisting of a hydrogen atom, a substituted or unsubstituted C1 to C20 alkyl group, a substituted or unsubstituted

C3 to C8 cycloalkyl group and a substituted or unsubstituted C3 to C12 aromatic group.

$Qn^1$ and $Qn^2$ may be the same or different from each other, each representing a hydroxy group, an amino group, a carboxy group or -C(=O)Cl.

n is 0 or 1.]

In formula (2"A), ring C, ring D and ring E each independently represent a ring system selected from the group consisting of a monocyclic aromatic ring system, a polycyclic aromatic ring system, an annulated aromatic ring system and an aliphatic hydrocarbon ring system whose ring-constituting atoms are atoms selected from the group consisting of a carbon atom, a nitrogen atom, an oxygen atom and a sulfur atom, with the monocyclic aromatic ring system, polycyclic aromatic ring system, annulated aromatic ring system and aliphatic hydrocarbon ring system optionally having a substituent.

Specific examples of monocyclic aromatic ring systems at ring C whose ring-constituting atoms are atoms selected from the group consisting of a carbon atom, a nitrogen atom, an oxygen atom and a sulfur atom and that may have a substituent include benzene, pyridine, pyrazine, pyrimidine, pyridazine, furan, pyrrole, imidazole, thiophene, pyrazole, oxazole, isoxazole, thiazole and triazole.

Examples of polycyclic aromatic ring systems at ring C whose ring-constituting atoms are atoms selected from the group consisting of a carbon atom, a nitrogen atom, an oxygen atom and a sulfur atom and that may have a substituent include biphenyl, terphenyl, bipyridine, bithiophene and bifuran.

Examples of annulated aromatic ring systems at ring C whose ring-constituting atoms are atoms selected from the group consisting of a carbon atom, a nitrogen atom, an oxygen atom and a sulfur atom and that may have a substituent include naphthalene, anthracene, phenanthrene, quinoline, benzofuran, benzimidazole, benzothiophene, indole, indazole, benzoxazole, benzothiazole and benzotriazole.

Specific examples of aliphatic hydrocarbon ring systems at ring C whose ring-constituting atoms are atoms selected from the group consisting of a carbon atom, a nitrogen atom, an oxygen atom and a sulfur atom and that may have a substituent include cyclopentane, cyclohexane and tricyclo[5.2.1.0(2,6)]decane.

For use as ring C, a monocyclic aromatic ring system whose ring-constituting atoms are atoms selected from the group consisting of a carbon atom, a nitrogen atom, an oxygen atom and a sulfur atom is preferred because of easy availability. It is particularly preferred that ring C be a benzene ring because of the ease of introduction.

In formula (2"A), ring D and ring E each independently represent a ring system selected from the group consisting of a monocyclic aromatic ring system, a polycyclic aromatic ring system, an annulated aromatic ring system and an aliphatic hydrocarbon ring system whose ring-constituting atoms are atoms selected from the group consisting of a carbon atom, a nitrogen atom, an oxygen atom and a sulfur atom, with the monocyclic aromatic ring system, polycyclic aromatic ring system, annulated aromatic ring system and aliphatic hydrocarbon ring system optionally having a substituent.

Examples of specific rings D and E are, independently for each ring, the same as for ring C because of the ease of introduction. A monocyclic aromatic ring system whose ring-constituting atoms are atoms selected from the group consisting of a carbon atom, a nitrogen atom, an oxygen atom and a sulfur atom is preferred. It is particularly preferred that rings D and E be benzene rings because of the ease of introduction.

In this context, examples of substituents in the monocyclic aromatic ring system, polycyclic aromatic ring system, annulated aromatic ring system and aliphatic hydrocarbon ring system include a C1 to C8 alkyl group, a halogen atom, a C1 to C4 alkoxy group and a C2 to C4 acyl group. Examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group, examples of halogen atoms include a fluorine atom, a chlorine atom, a bromine atom and an iodine atom, examples of C1 to C4 alkoxy groups include a methoxy group, an ethoxy group, a propyloxy group, an isopropyloxy group, a butyloxy group, an isobutyloxy group, a sec-butyloxy group and a tert-butyloxy group, and examples of C2 to C4 acyl groups include an acetyl group, a propionyl group and a butyryl group.

In formula (2"A), $R^5$ and $R^6$ may be the same or different from each other, each representing a group selected from the group consisting of a hydrogen atom, a substituted or unsubstituted C1 to C20 alkyl group, a substituted or unsubstituted C3 to C8 cycloalkyl group and a substituted or unsubstituted C3 to C12 aromatic group.

Examples of C1 to C20 alkyl groups include a methyl group, an ethyl group, a propyl group, an isopropyl group, a butyl group, an isobutyl group, a sec-butyl group, a tert-butyl group, a pentyl group, a hexyl group, a heptyl group, an octyl group, a nonyl group, a decyl group and an icosyl group. Of these, a methyl group, an ethyl group, a propyl group, an isopropyl group, a butyl group, an isobutyl group, a sec-butyl group and a tert-butyl group are particularly preferred because optical characteristics after their introduction into the polymer according to the present invention will be superior, with a methyl group and an ethyl group being especially preferred because of the ease of introduction.

Examples of C3 to C8 cycloalkyl groups include a cyclopropyl group, a cyclobutyl group, a cyclopentyl group, a cyclohexyl group, a cycloheptyl group and a cyclooctyl group.

Examples of C3 to C12 aromatic groups include a phenyl group, a naphthyl group, a biphenylyl group and a

pyridyl group.

In formula (2"A), $Qn^1$ and $Qn^2$ may be the same or different from each other, each representing a hydroxy group, an amino group, a carboxy group or - C(=O)Cl.

[Chem. 102]

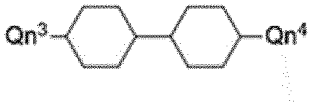

(2"B)

[In chemical formula (2"B) above, $Qn^3$ and $Qn^4$ may be the same or different from each other, each representing a hydroxy group, an amino group, a carboxy group or -C(=O)Cl.]

[Chem. 103]

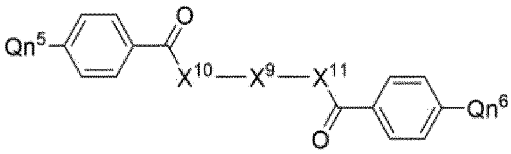

(2"C)

[In chemical formula (2"C) above, $X^9$ represents a C1 to C10 alkylene chain or a single bond.
$X^{10}$ represents -O- or -N($R^c$)-.
$X^{11}$ represents -O- or -N($R^d$)-.
$R^c$ and $R^d$ may be the same or different from each other, each representing a hydrogen atom or a C1 to C10 alkyl group.
$Qn^5$ and $Qn^6$ may be the same or different from each other, each representing a hydroxy group, an amino group, a carboxy group or -C(=O)Cl.]

[Chem. 104]

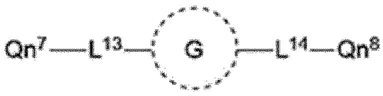

(2"D)

[In chemical formula (2"D) above, ring G represents a ring system selected from the group consisting of a monocyclic aromatic ring system, a polycyclic aromatic ring system, an annulated aromatic ring system, a spiro ring system and an aliphatic hydrocarbon ring system whose ring-constituting atoms are atoms selected from the group consisting of a carbon atom, a nitrogen atom, an oxygen atom and a sulfur atom, with the monocyclic aromatic ring system, polycyclic aromatic ring system, annulated aromatic ring system, spiro ring system and aliphatic hydrocarbon ring system optionally having a substituent.
$L^{13}$ and $L^{14}$ may be the same or different from each other, each representing a single bond or a C1 to C6 alkylene chain.
$Qn^7$ and $Qn^8$ may be the same or different from each other, each representing a hydroxy group, an amino group, a carboxy group or -C(=O)Cl.]

In formula (2"D), rings G each independently represent a ring system selected from the group consisting of a monocyclic aromatic ring system, a polycyclic aromatic ring system, an annulated aromatic ring system, a spiro ring system and an aliphatic hydrocarbon ring system whose ring-constituting atoms are atoms selected from the group consisting of a carbon atom, a nitrogen atom, an oxygen atom and a sulfur atom, with the monocyclic aromatic ring system, polycyclic aromatic ring system, annulated aromatic ring system, spiro ring system and aliphatic hydrocarbon ring system optionally having a substituent.

Specific examples of monocyclic aromatic ring systems at ring G whose ring-constituting atoms are atoms selected from the group consisting of a carbon atom, a nitrogen atom, an oxygen atom and a sulfur atom and that may have a substituent include benzene, pyridine, pyrazine, pyrimidine, pyridazine, furan, pyrrole, imidazole, thiophene, pyrazole, oxazole, isoxazole, thiazole and triazole.

Examples of polycyclic aromatic ring systems at ring G whose ring-constituting atoms are atoms selected from the group consisting of a carbon atom, a nitrogen atom, an oxygen atom and a sulfur atom and that may have a substituent include biphenyl, terphenyl, bipyridine, bithiophene and bifuran.

Examples of annulated aromatic ring systems at ring G whose ring-constituting atoms are atoms selected from the group consisting of a carbon atom, a nitrogen atom, an oxygen atom and a sulfur atom and that may have a substituent include naphthalene, anthracene, phenanthrene, quinoline, benzofuran, benzimidazole, benzothiophene, indole, indazole, benzoxazole, benzothiazole and benzotriazole.

An example of a spiro ring system at ring G whose ring-constituting atoms are atoms selected from the group consisting of a carbon atom, a nitrogen atom, an oxygen atom and a sulfur atom and that may have a substituent is the structure indicated by chemical formula (sp1-1) below.

[0261] [Chem. 105]

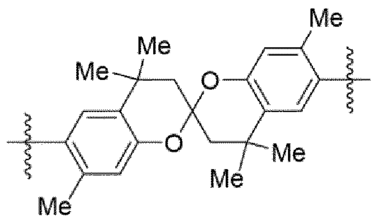

(sp1-1)

Examples of aliphatic hydrocarbon ring systems at ring G whose ring-constituting atoms are atoms selected from the group consisting of a carbon atom, a nitrogen atom, an oxygen atom and a sulfur atom and that may have a substituent include cyclopentane, cyclohexane and tricyclo[5.2.1.0(2,6)]decane.

For use as ring G, a monocyclic aromatic ring system or aliphatic hydrocarbon ring system whose ring-constituting atoms are atoms selected from the group consisting of a carbon atom, a nitrogen atom, an oxygen atom and a sulfur atom is preferred because of easy availability. It is particularly preferred that ring G be benzene, cyclohexane or tricyclo[5.2.1.0(2,6)]decane because this leads to good optical characteristics of the polymer.

Specific examples of compounds indicated by formula (2"A) include the structures indicated by chemical formulae (2"A-1-1) to (2"A-2-20) below.

[Chem. 106]

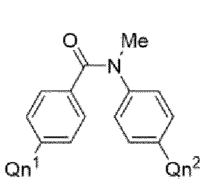
(2"A-1-1)

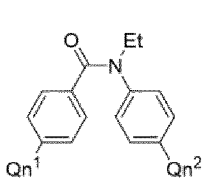
(2"A-1-2)

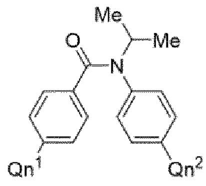
(2"A-1-3)

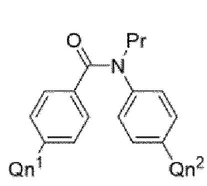
(2"A-1-4)

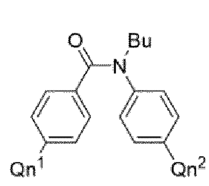
(2"A-1-5)

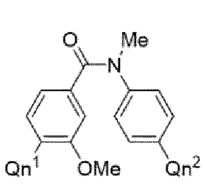
(2"A-1-6)

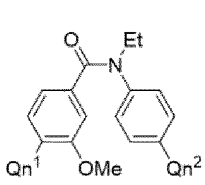
(2"A-1-7)

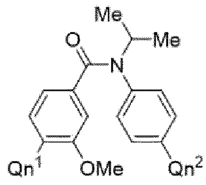
(2"A-1-8)

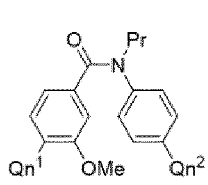
(2"A-1-9)

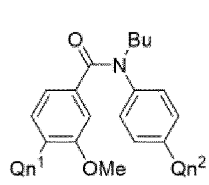
(2"A-1-10)

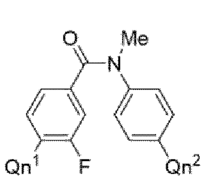
(2"A-1-11)

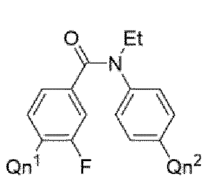
(2"A-1-12)

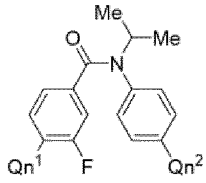
(2"A-1-13)

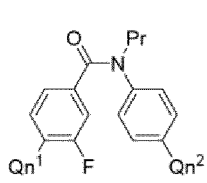
(2"A-1-14)

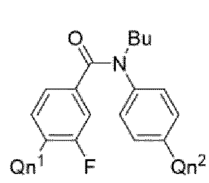
(2"A-1-15)

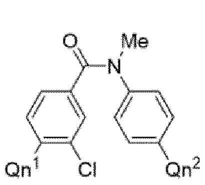
(2"A-1-16)

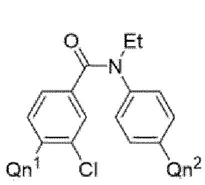
(2"A-1-17)

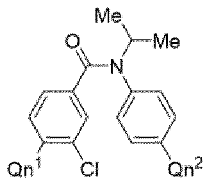
(2"A-1-18)

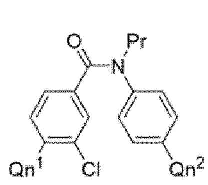
(2"A-1-19)

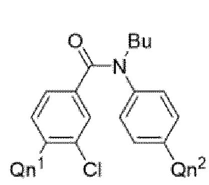
(2"A-1-20)

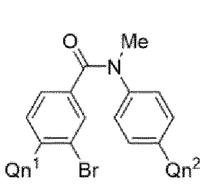
(2"A-1-21)

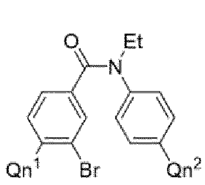
(2"A-1-22)

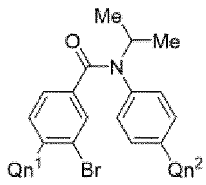
(2"A-1-23)

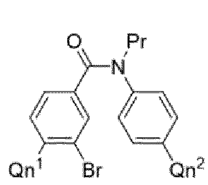
(2"A-1-24)

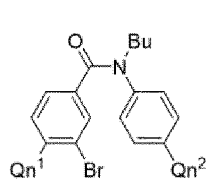
(2"A-1-25)

[Chem. 107]

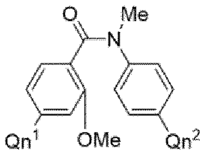
(2"A-1-26)

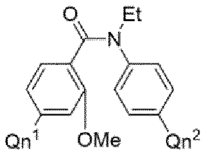
(2"A-1-27)

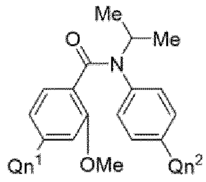
(2"A-1-28)

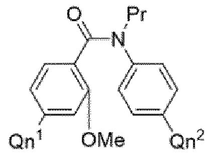
(2"A-1-29)

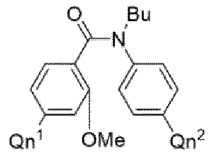
(2"A-1-30)

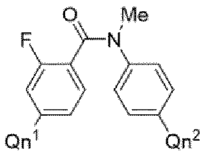
(2"A-1-31)

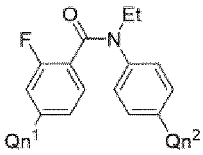
(2"A-1-32)

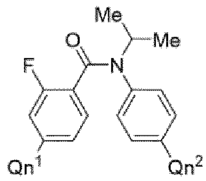
(2"A-1-33)

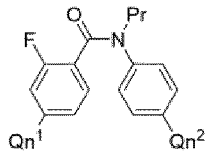
(2"A-1-34)

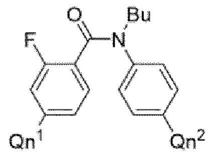
(2"A-1-35)

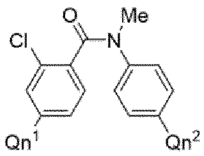
(2"A-1-36)

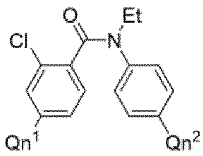
(2"A-1-37)

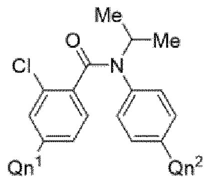
(2"A-1-38)

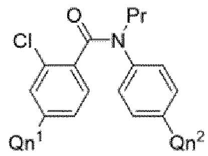
(2"A-1-39)

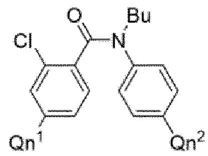
(2"A-1-40)

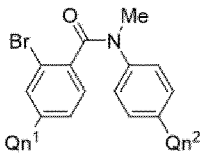
(2"A-1-41)

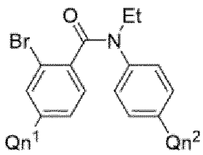
(2"A-1-42)

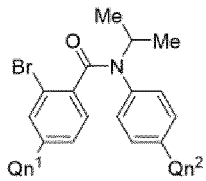
(2"A-1-43)

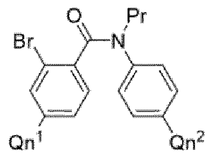
(2"A-1-44)

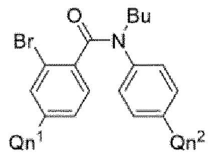
(2"A-1-45)

[Chem. 108]

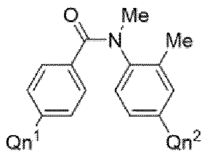
(2"A-1-46)

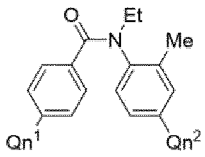
(2"A-1-47)

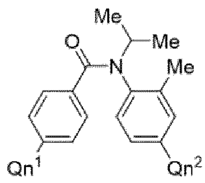
(2"A-1-48)

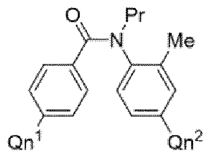
(2"A-1-49)

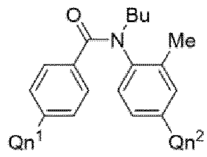
(2"A-1-50)

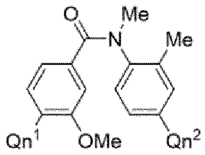
(2"A-1-51)

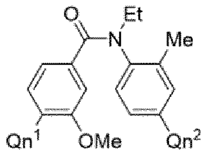
(2"A-1-52)

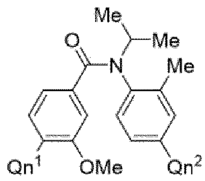
(2"A-1-53)

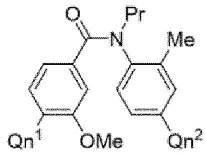
(2"A-1-54)

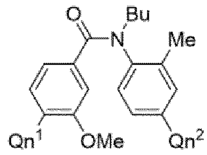
(2"A-1-55)

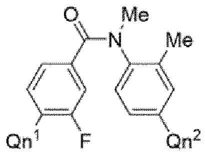
(2"A-1-56)

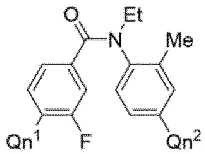
(2"A-1-57)

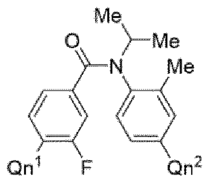
(2"A-1-58)

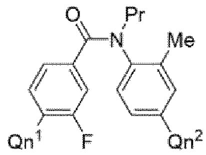
(2"A-1-59)

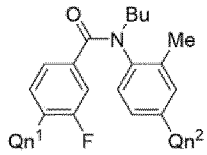
(2"A-1-60)

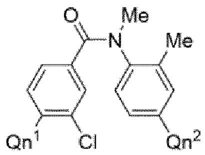
(2"A-1-61)

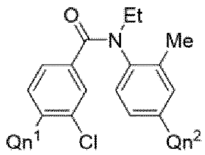
(2"A-1-62)

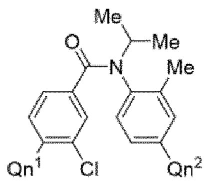
(2"A-1-63)

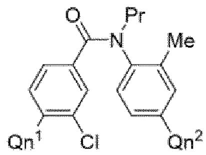
(2"A-1-64)

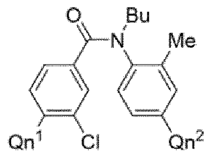
(2"A-1-65)

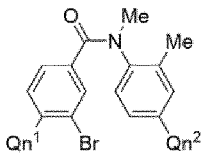
(2"A-1-66)

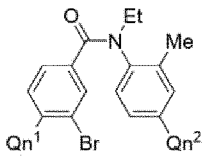
(2"A-1-67)

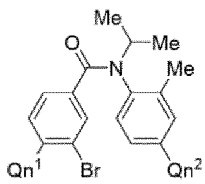
(2"A-1-68)

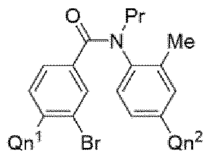
(2"A-1-69)

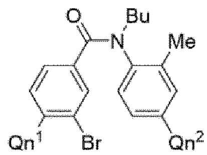
(2"A-1-70)

[Chem. 109]

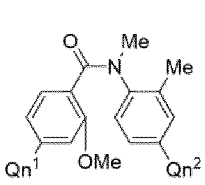

(2"A-1-71)

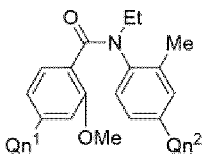

(2"A-1-72)

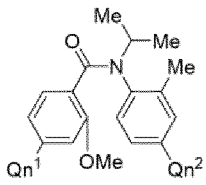

(2"A-1-73)

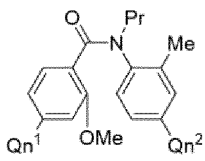

(2"A-1-74)

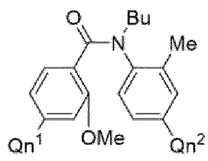

(2"A-1-75)

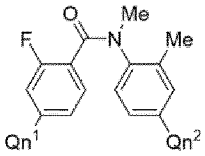

(2"A-1-76)

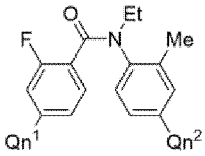

(2"A-1-77)

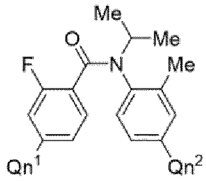

(2"A-1-78)

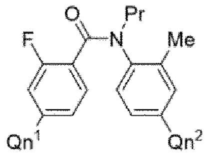

(2"A-1-79)

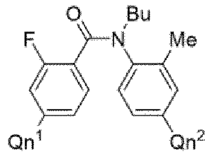

(2"A-1-80)

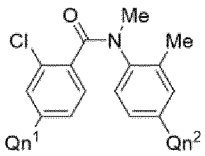

(2"A-1-81)

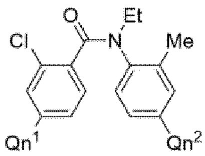

(2"A-1-82)

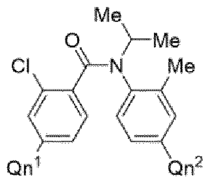

(2"A-1-83)

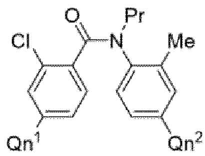

(2"A-1-84)

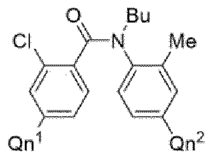

(2"A-1-85)

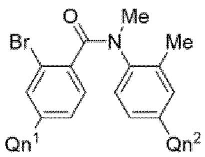

(2"A-1-86)

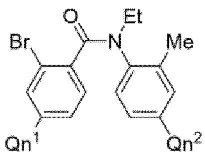

(2"A-1-87)

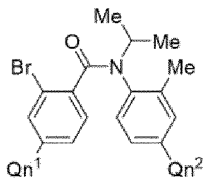

(2"A-1-88)

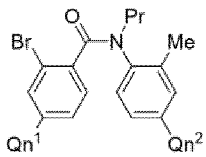

(2"A-1-89)

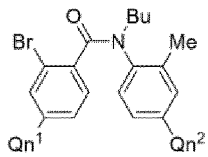

(2"A-1-90)

[Chem. 110]

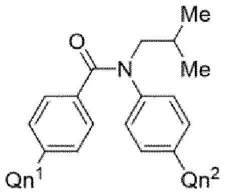

(2"A-1-91)

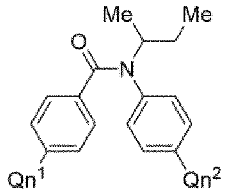

(2"A-1-92)

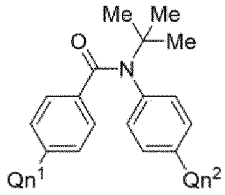

(2"A-1-93)

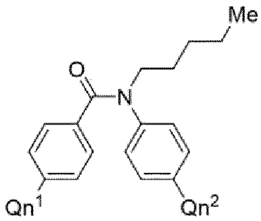

(2"A-1-94)

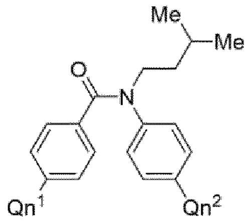

(2"A-1-95)

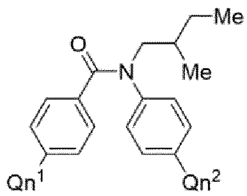

(2"A-1-96)

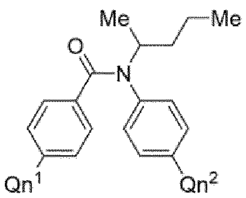

(2"A-1-97)

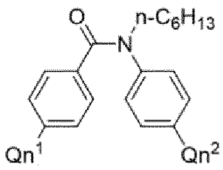

(2"A-1-98)

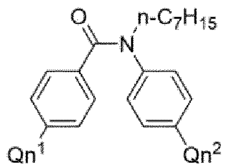

(2"A-1-99)

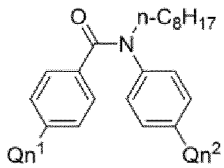

(2"A-1-100)

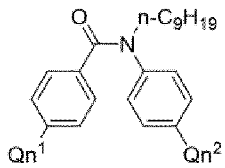

(2"A-1-101)

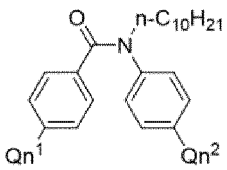

(2"A-1-102)

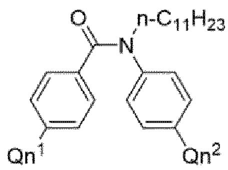

(2"A-1-103)

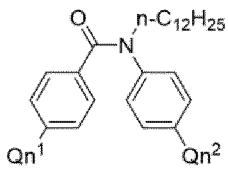

(2"A-1-104)

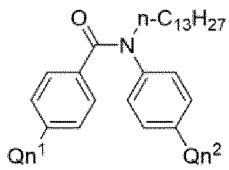

(2"A-1-105)

[Chem. 111]

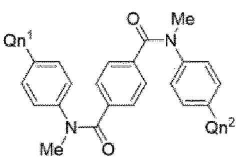
(2"A-2-1)

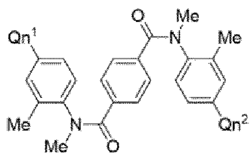
(2"A-2-2)

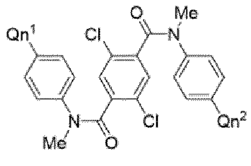
(2"A-2-3)

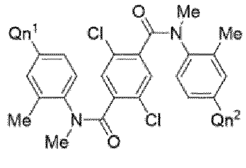
(2"A-2-4)

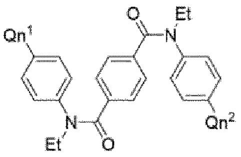
(2"A-2-5)

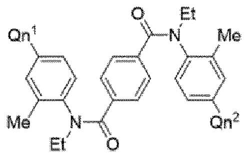
(2"A-2-6)

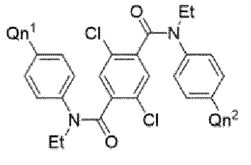
(2"A-2-7)

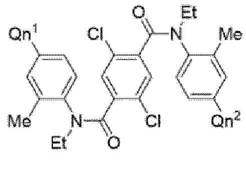
(2"A-2-8)

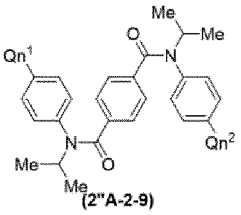
(2"A-2-9)

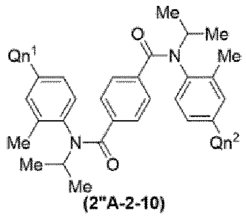
(2"A-2-10)

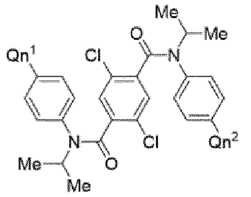
(2"A-2-11)

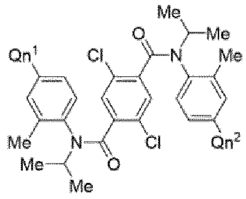
(2"A-2-12)

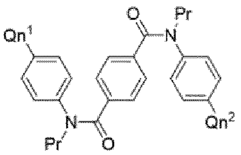
(2"A-2-13)

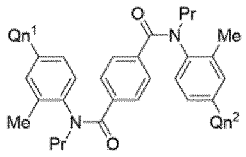
(2"A-2-14)

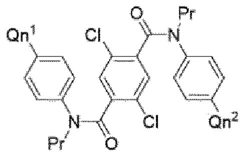
(2"A-2-15)

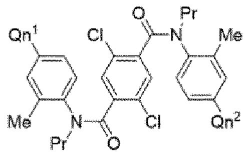
(2"A-2-16)

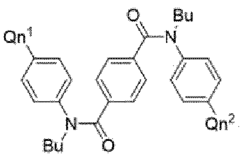
(2"A-2-17)

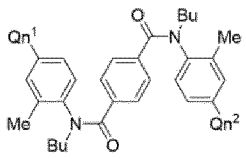
(2"A-2-18)

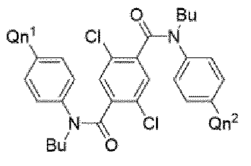
(2"A-2-19)

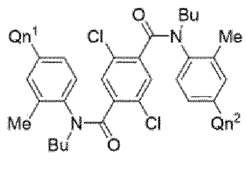
(2"A-2-20)

[In formulae (2"A-1-1) to (2"A-2-20), $Qn^1$ and $Qn^2$ may be the same or different from each other, each representing a hydroxy group, an amino group, a carboxy group or -C(=O)Cl.]

Of these (2'A-1-1) to (2"A-2-20), particularly preferred compounds are (2"A-1-1) to (2'A-1-10), (2'A-1-36) to (2'A-1-40), (2'A-1-51) to (2'A-1-55) and (2"A-2-1) to (2"A-2-4) because they allow for easier synthesis of the polymer according to the present invention, with (2"A-1-1) to (2'A-1-10), (2'A-1-36) to (2'A-1-40) and (2'A-1-51) to (2'A-1-55) being especially preferred because they lead to superior optical characteristics of the polymer according to the present invention.

Specific examples of compounds indicated by formula (2"B) include the structures indicated by chemical formulae (2"B-1) to (2"B-2) below.

[Chem. 112]

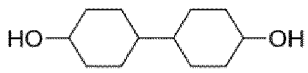
(2"B-1)

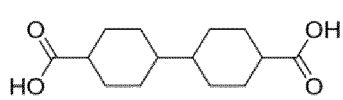
(2"B-2)

Of these (2"B-1) to (2"B-2), (2"B-1) is particularly preferred because it leads to superior optical characteristics of the polymer according to the present invention.

Specific examples of compounds indicated by formula (2"C) include the structures indicated by chemical formulae (2"C-1-1) to (2"C-2-23) below.

[Chem. 113]

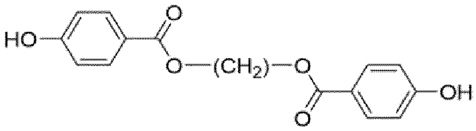

(2"C-1-1)

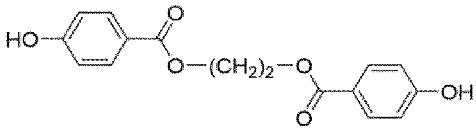

(2"C-1-2)

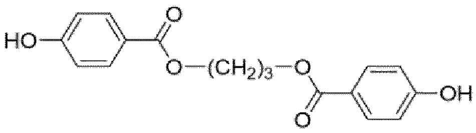

(2"C-1-3)

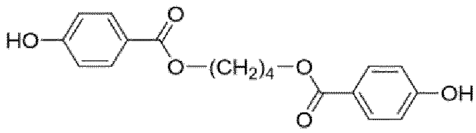

(2"C-1-4)

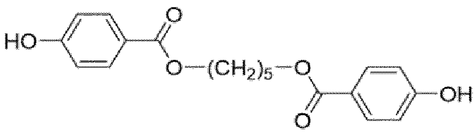

(2"C-1-5)

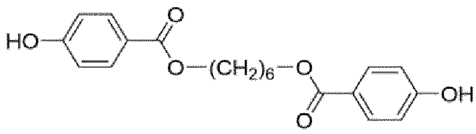

(2"C-1-6)

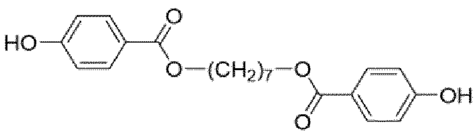

(2"C-1-7)

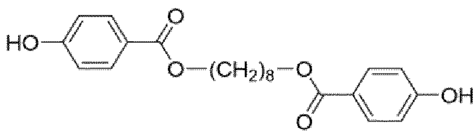

(2"C-1-8)

[Chem. 114]

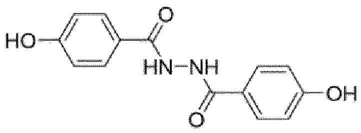

(2"C-2-1)

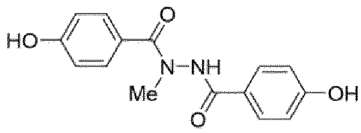

(2"C-2-2)

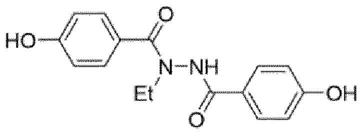

(2"C-2-3)

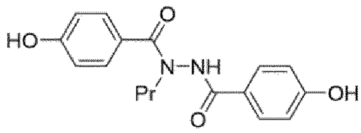

(2"C-2-4)

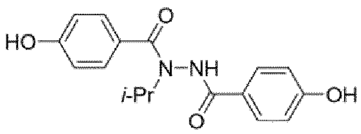

(2"C-2-5)

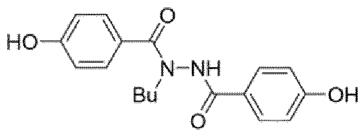

(2"C-2-6)

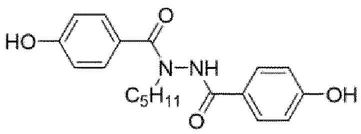

(2"C-2-7)

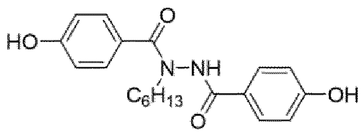

(2"C-2-8)

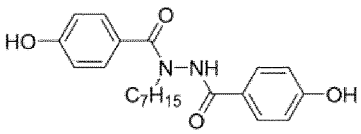

(2"C-2-9)

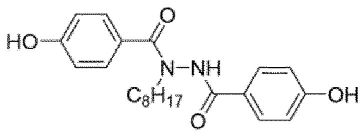

(2"C-2-10)

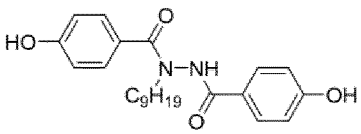

(2"C-2-11)

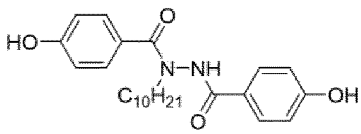

(2"C-2-12)

[Chem. 115]

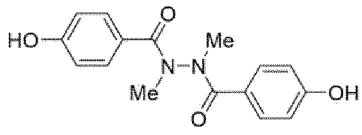

(2"C-2-13)

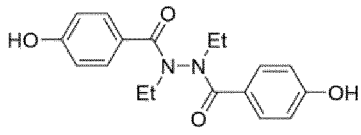

(2"C-2-14)

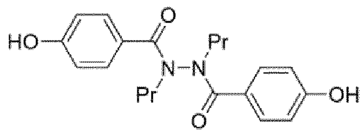

(2"C-2-15)

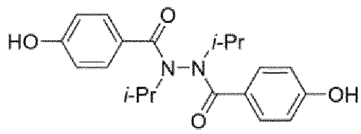

(2"C-2-16)

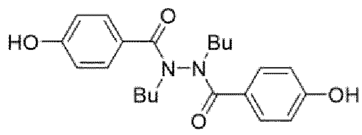

(2"C-2-17)

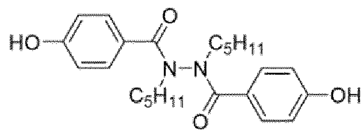

(2"C-2-18)

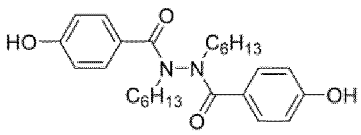

(2"C-2-19)

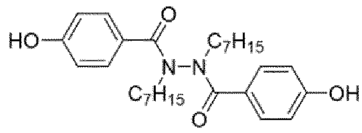

(2"C-2-20)

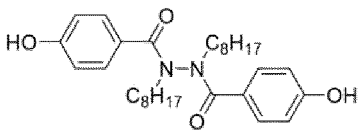

(2"C-2-21)

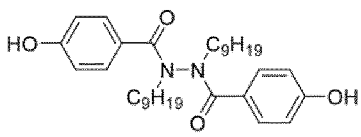

(2"C-2-22)

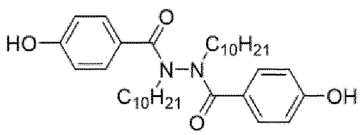

(2"C-2-23)

Of these (2"C-1-1) to (2"C-2-23), (2"C-1-4) to (2"C-1-8) and (2"C-2-1) to (2"C-2-23) are particularly preferred because they lead to superior optical characteristics of the polymer according to the present invention.

Specific examples of compounds indicated by formula (2"D) include the structures indicated by chemical formulae (2"D-1-1) to (2"D-14-1) below.

[Chem. 116]

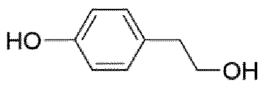

(2"D-1-1)

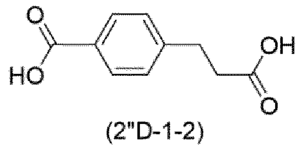

(2"D-1-2)

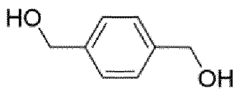

(2"D-2-1)

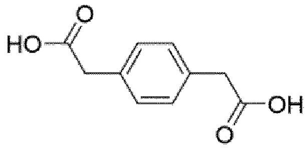

(2"D-2-2)

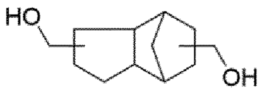

(2"D-3-1)

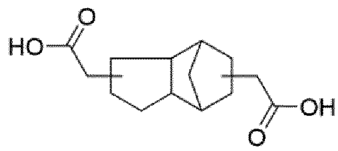

(2"D-3-2)

[Chem. 117]

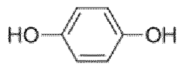

(2"D-4-1)

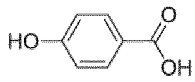

(2"D-4-2)

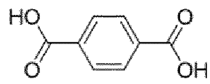

(2"D-4-3)

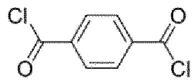

(2"D-4-4)

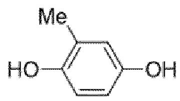

(2"D-5-1)

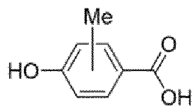

(2"D-5-2)

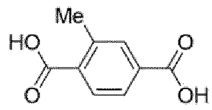

(2"D-5-3)

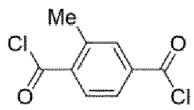

(2"D-5-4)

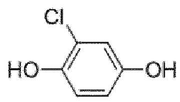

(2"D-6-1)

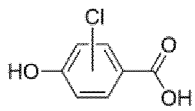

(2"D-6-2)

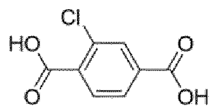

(2"D-6-3)

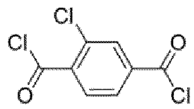

(2"D-6-4)

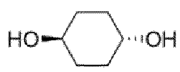

(2"D-7-1)

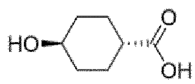

(2"D-7-2)

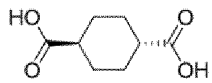

(2"D-7-3)

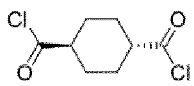

(2"D-7-4)

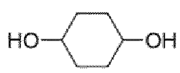

(2"D-7-5)

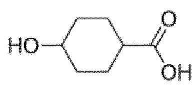

(2"D-7-6)

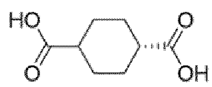

(2"D-7-7)

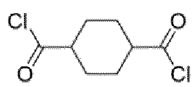

(2"D-7-8)

[Chem. 118]

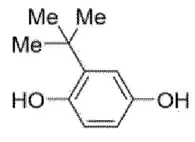

(2"D-8-1)

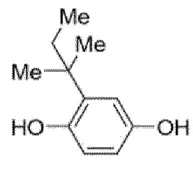

(2"D-9-1)

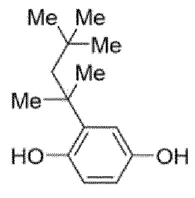

(2"D-10-1)

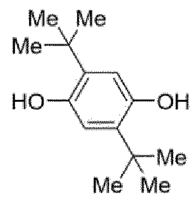

(2"D-11-1)

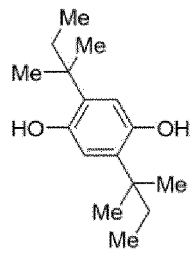

(2"D-12-1)

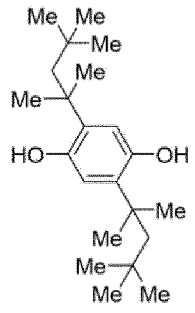

(2"D-13-1)

[Chem. 119]

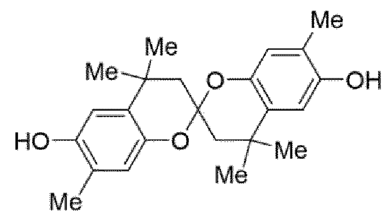

(2"D-14-1)

Of these (2"D-1-1) to (2"D-14-1),(2"D-1-1), (2"D-2-1), (2"D-3-1), (2"D-3-1) (2"D-4-3), (2"D-7-3), (2"D-7-4), (2"D-7-4) to (2"D-13-1) and (2"D-14-1) are particularly preferred because they lead to superior optical characteristics of the polymer according to the present invention.

In the method for producing the polymer according to the present invention, a polyhydroxy compound may be used as a source material for the purpose of the adjustment of the characteristics of the polymer that forms.

Examples of polyhydroxy compounds include the dihydroxy compounds of ethylene glycol, diethylene glycol, triethylene glycol, tetraethylene glycol, 1,3-propanediol, 1 ,2-propylene glycol, 1 ,4-butanediol, 2,2-butanediol, 2,3-butanediol, 2,4-dimethyl-2,4-pentanediol, 1,5-pentanediol, 1,6-hexanediol, 1,7-heptanediol, 2-methyl-1,3-propanediol, 3-methyl-1, 5-heptanediol, cyclopentanediol, cyclohexanediol, 1,8-octanediol, 1,9-nonanediol, 1,10-decanediol, 1,12-dodecanediol, hydroquinone, tetramethylhydroquinone, 4,4'-dihydroxybiphenyl, vanillyl alcohol, furan dimethanol, 2,2-bis(4-hydroxyphenyl)propane, 4,4'-(1,3-dimethylbutylidene)diphenol, 6,6'-dihydroxy-4,4,4',4',7,7'-hexamethyl-2,2'-spirobicromane, tricyclodecanedimethanol, 2,2'-dihydroxydiphenyl ether, 4,4'-methylenebis(2,6-dimethylphenol), 3,3',5,5'-tetramethylbiphenyl-4,4'-diol, 1,1-bis(4-hydroxyphenyl)cyclohexane, 2-butene-1,4-diol, 2,2-diisobutyl-1,3-propanediol, bis[4-(2-hydroxyethoxy)phenyl] sulfone, 2,2,4,4-tetramethyl-1,3-cyclobutanediol, 1,4-bis(3-hydroxyphenoxy)benzene, 4,4'-bicyclohexanol, bis(4-hydroxy-3-methylphenyl) sulfide, 2,2-diisoamyl-1,3-propanediol, 4,4'-dihydroxydiphenylmethane, dihydroxynaphthalene, 2,2-bis(3-cyclohexyl-4-hydroxyphenyl)propane, bis(4-hydroxyphenyl) sulfone, 1,4-benzenedimethanol, 3,9-bis(1,1-dimethyl-2-hydroxyethyl)-2,4,8,10-tetraoxaspiro[5.5]undecane, 4,4'-biphenyldimethanol, 1,3-bis(hexafluoro-$\alpha$-hydroxyisopropyl)benzene, 3,6-dihydroxybenzonorbornane, 2-benzyloxy-1,3-propanediol, 4,4'-dihydroxybenzophenone, 4,4'-dihydroxydiphenyl ether, 9,9-bis(4-hydroxyphenyl)fluorene, 1,8-bis(hydroxymethyl)anthracene, 1,4-bis[2-(4-hydroxyphenyl)-2-propyl]benzene, $\alpha$,$\alpha$'-bis(4-hydroxy-3,5-dimethylphenyl)-1,4-diisopropylbenzene, 2,2-bis(4-hydroxy-3,5-dimethylphenyl)propane, 2,2'-methylenebis(4-methylphenol), 1,3-bis(4-hydroxyphenoxy)benzene, 2,2-bis(4-hydroxyphenyl)butane, 1,1-bis(4-hydroxy-3-methylphenyl)cyclohexane, 2,2-bis(4-hydroxy-3-methylphenyl)propane, 2,2'-dihydroxybenzophenone, 2,2'-bis(hydroxymethyl)diphenyl ether, 7,7'-dihydroxy-4,4,4',4'-tetramethyl-2,2'-spirobicromane, 1,4-bis(hydroxymethyl)-2,3,5,6-tetramethylbenzene, 4,4'-ethylidenebisphenol, cyclohexanedimethanol, polyethylene glycol, 1,3-adamantanediol, 1-hydroxy-3-(hydroxymethyl)adamantane, 2,7-dihydroxy-9H-fluoren-9-one and polyethylene glycols with various molecular weights, the trihydroxy compounds of glycerol, trimethylolpropane, triethanolamine, 2,3,4,4'-tetrahydroxybenzophenone, 1,2,3-butanetriol and 2,6-bis(hydroxymethyl)-4-methylphenol and the tetrahydroxy compound of pentaerythritol, and examples of other polyhydroxy compounds include various saccharides.

The polymerization method is not particularly limited. Examples include polymerization methods known in the field, such as interfacial polymerization.

An example of a method for interfacial polymerization is the method of performing interfacial polymerization in a water-organic solvent bilayer system.

When interfacial polymerization is performed in a water-organic solvent bilayer system, it is preferred to use a phase transfer catalyst and a base for the purpose of accelerating the reaction.

Examples of phase transfer catalysts include the ammonium salts of benzyltriethylammonium chloride, benzyltriethylammonium bromide, tetrabutylammonium chloride, tetrabutylammonium bromide, tetraamylammonium chloride, tetraamylammonium bromide, tetraheptylammonium chloride, tetraheptylammonium bromide, dimethyldipalmitylammonium chloride and dimethyldipalmitylammonium bromide and the phosphonium salts of tetraethylphosphonium chloride, tetraethylphosphonium bromide, tributylhexylphosphonium chloride, tributylhexylphosphonium bromide, tetra-n-octylphosphonium chloride, tetra-n-octylphosphonium bromide, tributyl-n-octylphosphonium chloride, tributyl-n-octylphosphonium bromide, tetrabutylphosphonium chloride, tetrabutylphosphonium bromide, tetraphenylphosphonium bromide and tetraphenylphosphonium chloride.

Examples of bases include the alkali metal hydroxides of sodium hydroxide and potassium hydroxide, the alkali metal carbonates of sodium carbonate and potassium carbonate, the alkali metal hydride of sodium hydride, the alkali metal alkoxides of sodium methoxide and sodium ethoxide and tetrabutylammonium hydroxide.

The reaction temperature for the interfacial polymerization is preferably -30°C or above and 50°C or below, particularly preferably 0°C or above and 40°C or below. The duration of reaction is preferably 30 minutes or more and 24 hours or less.

As the organic solvent used for the interfacial polymerization, any organic solvent that does not harm the reaction can be used. Examples include ether solvents, such as dioxane, tetrahydrofuran (Hereinafter abbreviated as THF.), diethyl ether, diisopropyl ether, cyclopentyl methyl ether and dimethoxyethane; aromatic solvents, such as benzene, toluene, xylene, chlorobenzene, dichlorobenzene and pyridine; halogenated solvents, such as dichloromethane, chloroform and carbon tetrachloride; and amide solvents, such as N,N-dimethylformamide, N,N-dimethylacetamide and N-methyl-2-pyrrolidone. One such solvent alone or two or more mixed in any proportions may be used.

An example of a method for solution polymerization is the method of using at least one condensing agent in at least one organic solvent.

As the organic solvent used for solution polymerization, any organic solvent that does not harm the reaction can be used. Examples include ether solvents, such as dioxane, THF, diethyl ether, diisopropyl ether, cyclopentyl methyl ether and dimethoxyethane; aromatic solvents, such as benzene, toluene, xylene, chlorobenzene, dichlorobenzene and pyridine; halogenated solvents, such as dichloromethane, chloroform and carbon tetrachloride; amide solvents, such as N,N-dimethylformamide, N,N-dimethylacetamide and NMP; and ester solvents, such as ethyl acetate and butyl acetate. One such solvent alone or two or more mixed in any proportions may be used.

Examples of condensing agents used for solution polymerization include 1-[3-(dimethylamino)propyl]-3-ethylcarbodiimide, 1-[3-(dimethylamino)propyl]-3-ethylcarbodiimide hydrochloride, N,N'-diisopropylcarbodiimide, N,N'-dicyclohexylcarbodiimide, bis(2,6-diisopropylphenyl)carbodiimide, bis(trimethylsilyl)carbodiimide, 1-cyclohexyl-3-(2-morpholinoethyl)carbodiimide-p-toluenesulfonate, N,N'-di-tert-butylcarbodiimide and 1-[3-(dimethylamino)propyl]-3-ethylcarbodiimide methiodide. Of these, 1-[3-(dimethylamino)propyl]-3-ethylcarbodiimide, 1-[3-(dimethylamino)propyl]-3-ethylcarbodiimide hydrochloride, N,N'-diisopropylcarbodiimide and N,N'-dicyclohexylcarbodiimide are particularly preferred. Two or more of such ester-linkage-forming condensing agents may be used.

The reaction temperature for solution polymerization is preferably - 30°C or above and 90°C or below, particularly preferably 0°C or above and 60°C or below. The duration of reaction is preferably 30 minutes or more and 24 hours or less.

In solution polymerization, it is preferred to conduct the process with an added base to ensure that the reaction will proceed smoothly.

Examples of bases used include organic bases, inorganic bases, organometallic compounds, metal alkoxides, and metal amides.

Examples of organic bases include triethylamine, tributylamine, diisopropylethylamine, pyridine, 4-dimethylaminopyridine, piperidine, piperazine, pyrrolidine, morpholine, N-methylmorpholine, imidazole and N-methylimidazole.

Examples of inorganic bases include lithium hydroxide, sodium hydroxide, potassium hydroxide, calcium hydroxide, lithium carbonate, sodium carbonate, potassium carbonate, cesium carbonate, sodium hydrogencarbonate, potassium hydrogencarbonate, sodium hydride and potassium hydride.

Examples of organometallic compounds include normal butyllithium, sec-butyllithium, tert-butyllithium and phenyllithium.

Examples of metal alkoxides include sodium methoxide, sodium ethoxide, sodium tert-butoxide and potassium tert-butoxide.

Examples of metal amides include lithium amide, sodium amide, lithium diisopropylamide, lithium hexamethyldisilazide, sodium hexamethyldisilazide and potassium hexamethyldisilazide.

Among bases, organic bases are particularly preferred because they allow the reaction to proceed in a favorable manner and are inexpensive. Triethylamine, tributylamine, diisopropylethylamine, pyridine, 4-dimethylaminopyridine, piperidine, piperazine and pyrrolidine are more preferred.

The amount of base used is not particularly restricted. A solvating amount of base may be used.

The base may be used in liquid form, particle form or granule form or directly as a powdery solid depending on its properties. Solutions of them can also be used, and there is no specific restriction on their concentration.

Another aspect of the present invention relates to a dihydroxy compound represented by formula (1') below (hereinafter also referred to as the dihydroxy compound according to the present invention). The dihydroxy compound according to the present invention serves as a source material for the polymer according to the present invention.

[0311] [Chem. 120]

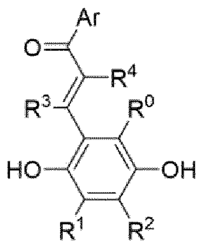

(1')

**[0171]** [In chemical formula (1') above, $R^0$, $R^1$, $R^2$, $R^3$ and $R^4$ each independently represent a hydrogen atom, a halogen atom, a C1 to C8 alkyl group or a group represented by chemical formula (Z1) below. Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.]

[0313] [Chem. 121]

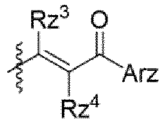

(Z1)

[In formula (Z1), $Rz^3$ and $Rz^4$ each independently represent a hydrogen atom, a halogen atom or a C1 to C8 alkyl group.

Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent. The wavy-lined portion represents a site of binding with the portion in formula (1) excluding $R^0$, $R^1$, $R^2$, $R^3$ or $R^4$.]

In formula (1'), R°, $R^1$, $R^2$, $R^3$ and $R^4$ each independently represent a hydrogen atom, a halogen atom, a C1 to C8 alkyl group or a group represented by chemical formula (Z1). Examples of halogen atoms include a chlorine atom, a bromine atom, an iodine atom and a fluorine atom, and examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group.

In formula (Z1), $Rz^3$ and $Rz^4$ each independently represent a hydrogen atom, a halogen atom or a C1 to C8 alkyl group. Examples of halogen atoms include a chlorine atom, a bromine atom, an iodine atom and a fluorine atom, and examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and

an octyl group.

In formula (Z1), for use as $Rz^3$ and $Rz^4$, a hydrogen atom, a methyl group, an ethyl group, a propyl group, an isopropyl group, a butyl group, an isobutyl group, a sec-butyl group and a tert-butyl group are preferred because they ensure that the polymer according to the present invention will exhibit good optical characteristics. A hydrogen atom, a methyl group and an ethyl group are particularly preferred.

Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system and annulated aromatic ring system optionally having a substituent.

Examples of monocyclic aromatic ring systems at Arz whose ring-constituting atoms are atoms selected from the group consisting of a carbon atom, a nitrogen atom, an oxygen atom and a sulfur atom and that may have a substituent include benzene, pyridine, pyrazine, pyrimidine, pyridazine, furan, pyrrole, imidazole, thiophene, pyrazole, oxazole, isoxazole, thiazole and triazole.

Examples of polycyclic aromatic ring systems at Arz whose ring-constituting atoms are atoms selected from the group consisting of a carbon atom, a nitrogen atom, an oxygen atom and a sulfur atom and that may have a substituent include biphenyl, terphenyl, bipyridine, bithiophene and bifuran.

Examples of annulated aromatic ring systems at Arz whose ring-constituting atoms are atoms selected from the group consisting of a carbon atom, a nitrogen atom, an oxygen atom and a sulfur atom and that may have a substituent include naphthalene, anthracene, phenanthrene, quinoline, benzofuran, benzimidazole, benzothiophene, indole, indazole, benzoxazole, benzothiazole and benzotriazole.

For use as Arz, a monocyclic aromatic ring system whose ring-constituting atoms are atoms selected from the group consisting of a carbon atom, a nitrogen atom, an oxygen atom and a sulfur atom and that may have a substituent is preferred because of easy availability. More preferably, this aromatic ring system is a benzene ring, a furan ring, a thiophene ring, a thiazole ring or an oxazole ring because this ensures that the polymer according to the present invention will exhibit good optical characteristics. A benzene ring or thiophene ring is particularly preferred because they ensure that the polymer according to the present invention will exhibit better optical characteristics.

For use as R°, $R^1$, $R^2$, $R^3$ and $R^4$, a hydrogen atom, a methyl group, an ethyl group, a propyl group, an isopropyl group, a butyl group, an isobutyl group, a sec-butyl group, a tert-butyl group and the groups represented by chemical formulae (Z1-1) to (Z1-4) below are preferred because optical characteristics after their introduction into the polymer according to the present invention will be superior. A hydrogen atom, a methyl group and an ethyl group are particularly preferred because of the ease of introduction.

[0324] [Chem. 122]

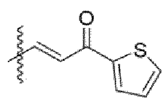
(Z1-1)

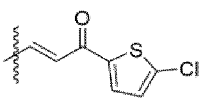
(Z1-2)

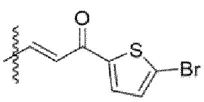
(Z1-3)

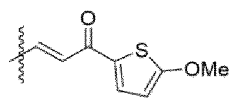
(Z1-4)

In formula (1'), Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system and annulated aromatic ring system optionally having a substituent.

In this context, examples of substituents in the monocyclic aromatic ring system, polycyclic aromatic ring system and annulated aromatic ring system include a C1 to C8 alkyl group, a halogen atom, a C1 to C4 alkoxy group and a C2 to C4 acyl group.

Examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group.

Examples of halogen atoms include a fluorine atom, a chlorine atom, a bromine atom and an iodine atom.

Examples of C1 to C4 alkoxy groups include a methoxy group, an ethoxy group, a propyloxy group, an isopropyloxy group, a butyloxy group, an isobutyloxy group, a sec-butyloxy group and a tert-butyloxy group.

Examples of C2 to C4 acyl groups include an acetyl group, a propionyl group and a butyryl group.

Examples of monocyclic aromatic ring systems at Ar whose ring-constituting atoms are atoms selected from the group consisting of a carbon atom, a nitrogen atom, an oxygen atom and a sulfur atom and that may have a substituent include benzene, pyridine, pyrazine, pyrimidine, pyridazine, furan, pyrrole, imidazole, thiophene, pyrazole, oxazole,

isoxazole, thiazole and triazole.

Examples of polycyclic aromatic ring systems at Ar whose ring-constituting atoms are atoms selected from the group consisting of a carbon atom, a nitrogen atom, an oxygen atom and a sulfur atom and that may have a substituent include biphenyl, terphenyl, bipyridine, bithiophene and bifuran.

Examples of annulated aromatic ring systems at Ar whose ring-constituting atoms are atoms selected from the group consisting of a carbon atom, a nitrogen atom, an oxygen atom and a sulfur atom and that may have a substituent include naphthalene, anthracene, phenanthrene, quinoline, benzofuran, benzimidazole, benzothiophene, indole, indazole, benzoxazole, benzothiazole and benzotriazole.

For use as Ar, a monocyclic aromatic ring system whose ring-constituting atoms are atoms selected from the group consisting of a carbon atom, a nitrogen atom, an oxygen atom and a sulfur atom and that may have a substituent is preferred because of easy availability. More preferably, this aromatic ring system is a benzene ring, a furan ring, a thiophene ring, a thiazole ring or an oxazole ring because this ensures that the polymer according to the present invention will exhibit good optical characteristics. A benzene ring or thiophene ring is particularly preferred because they ensure that the polymer according to the present invention will exhibit better optical characteristics.

Preferred examples of Ars include the structures indicated by chemical formulae (Ar-1) to (Ar-7) below.

[Chem. 123]

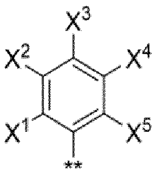

(Ar-1)

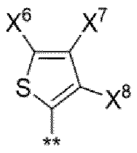

(Ar-2)

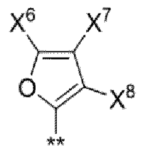

(Ar-3)

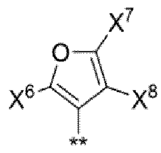

(Ar-4)

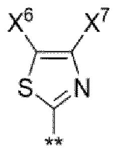

(Ar-5)

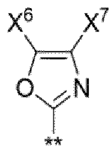

(Ar-6)

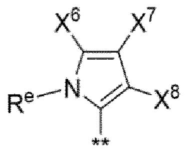

(Ar-7)

[In chemical formulae (Ar-1) to (Ar-7) above, $X^1$, $X^2$, $X^3$, $X^4$, $X^5$, $X^6$, $X^7$ and $X^8$ each independently represent a hydrogen atom, a C1 to C8 alkyl group, a C1 to C6 alkoxy group, a halogen atom, a nitro group, a cyano group, a C1 to C6 alkylthio group or a C2 to C8 dialkylamino group.

$R^e$ represents a hydrogen atom or a C1 to C10 alkyl group.

** represents a site of binding with the other portion in chemical formula (1') above, excluding Ar.]

In formulae (Ar-1) to (Ar-7), $X^1$, $X^2$, $X^3$, $X^4$, $X^5$, $X^6$, $X^7$ and $X^8$ each independently represent a hydrogen atom, a C1 to C8 alkyl group, a C1 to C6 alkoxy group, a halogen atom, a nitro group, a cyano group, a C1 to C6 alkylthio group or a C2 to C8 dialkylamino group. Examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group, examples of C1 to C6 alkoxy groups include a methoxy group, an ethoxy group, a propyloxy group, an isopropyloxy group, a butyloxy group, an isobutyloxy group, a sec-butyloxy group, a tert-butyloxy group, a pentyloxy group and a hexyloxy group, examples of halogen atoms include a fluorine atom, a chlorine atom, a bromine atom and an iodine atom, examples of C1 to C6 alkylthio groups include a methylthio group, an ethylthio group, a propylthio group, an isopropylthio group, a butylthio group, an isobutylthio group, a sec-butylthio group, a tert-butylthio group, a pentylthio group and a hexylthio group, and examples of C2 to C8 dialkylamino groups include a dimethylamino group, a diethylamino group, a dipropylamino group and a dibutylamino group.

For $X^1$, $X^2$, $X^3$, $X^4$, $X^5$, $X^6$, $X^7$ and $X^8$, it is preferred that each independently be a hydrogen atom, a C1 to C8 alkyl group, a C1 to C6 alkoxy group or a halogen atom, particularly preferably a hydrogen atom, a methyl group, a methoxy group or a halogen atom.

A preferred example of a dihydroxy compound according to the present invention is a dihydroxy compound indicated by chemical formula (1'-2) below.

[Chem. 124]

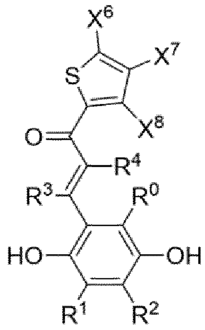

(1'-2)

[In chemical formula (1'-2) above, $R^0$, $R^1$, $R^2$, $R^3$ and $R^4$ each independently represent a hydrogen atom or a C1 to C8 alkyl group.

$X^6$, $X^7$ and $X^8$ each independently represent a hydrogen atom, a C1 to C8 alkyl group, a C1 to C6 alkoxy group, a halogen atom, a nitro group, a cyano group, a C1 to C6 alkylthio group or a C2 to C8 dialkylamino group.]

In formula (1'-2), R°, $R^1$, $R^2$, $R^3$ and $R^4$ each independently represent a hydrogen atom or a C1 to C8 alkyl group. Examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group.

For use as R°, $R^1$, $R^2$, $R^3$ and $R^4$, a hydrogen atom, a methyl group, an ethyl group, a propyl group, an isopropyl group, a butyl group, an isobutyl group, a sec-butyl group and a tert-butyl group are preferred because of the ease of introduction. A hydrogen atom, a methyl group and an ethyl group are particularly preferred because of the ease of introduction.

In formula (1'-2), $X^6$, $X^7$ and $X^8$ each independently represent a hydrogen atom, a C1 to C8 alkyl group, a C1 to C6 alkoxy group, a halogen atom, a nitro group, a cyano group, a C1 to C6 alkylthio group or a C2 to C8 dialkylamino group. Examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group, examples of C1 to C6 alkoxy groups include a methoxy group, an ethoxy group, a propyloxy group, an isopropyloxy group, a butyloxy group, an isobutyloxy group, a sec-butyloxy group, a tert-butyloxy group, a pentyloxy group and a hexyloxy group, examples of halogen atoms include a fluorine atom, a chlorine atom, a bromine atom and an iodine atom, examples of C1 to C6 alkylthio groups include a methylthio group, an ethylthio group, a propylthio group, an isopropylthio group, a butylthio group, an isobutylthio group, a sec-butylthio group, a tert-butylthio group, a pentylthio group and a hexylthio group, and examples of C2 to C8 dialkylamino groups include a dimethylamino group, a diethylamino group, a dipropylamino group and a dibutylamino group.

For $X^6$, $X^7$ and $X^8$, it is preferred that each be a hydrogen atom, a C1 to C8 alkyl group, a C1 to C6 alkoxy group or a halogen atom because this leads to superior optical characteristics after the introduction of the dihydroxy compound according to the present invention into the polymer according to the present invention. A hydrogen atom, a methyl group, a methoxy group, a chlorine atom, a bromine atom or a fluorine atom is particularly preferred because of the ease of introduction.

More preferred examples of dihydroxy compounds indicated by general formula (1') include the dihydroxy compounds indicated by chemical formulae (1'-2-1) to (1'-7-4) below.

[Chem. 125]

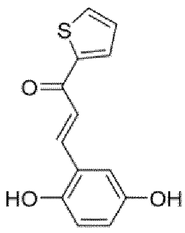
(1'-2-1)

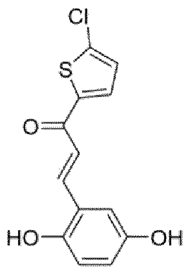
(1'-2-2)

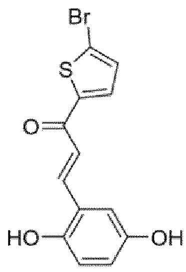
(1'-2-3)

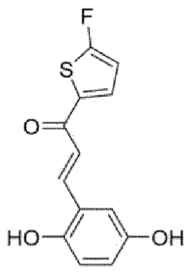
(1'-2-4)

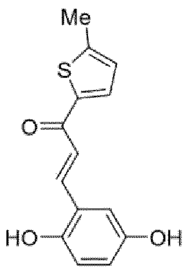
(1'-2-5)

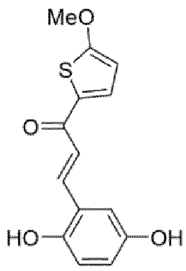
(1'-2-6)

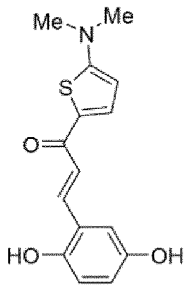
(1'-2-7)

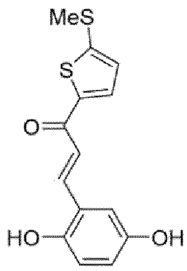
(1'-2-8)

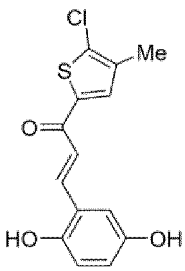
(1'-2-9)

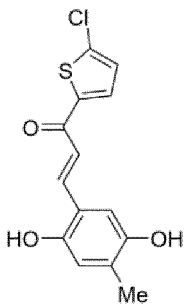
(1'-2-10)

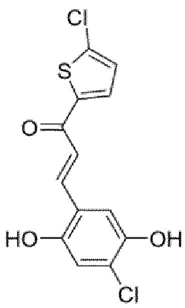
(1'-2-11)

[Chem. 126]

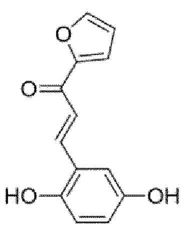
(1'-3-1)

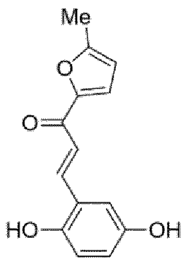
(1'-3-2)

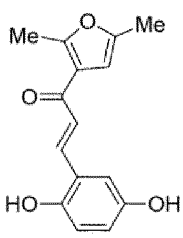
(1'-3-3)

[Chem. 127]

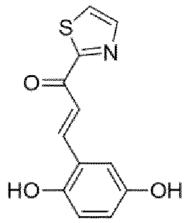

(1'-4-1)

[Chem. 128]

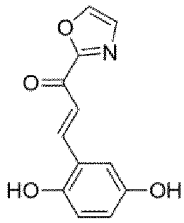

(1'-5-1)

[Chem. 129]

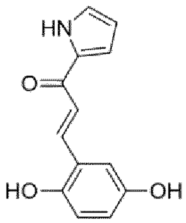

(1'-6-1)

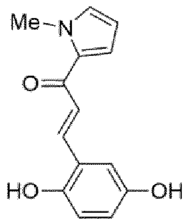

(1'-6-2)

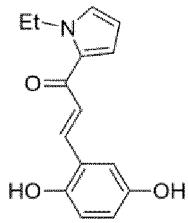

(1'-6-3)

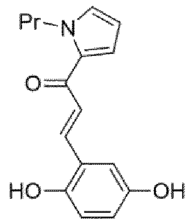

(1'-6-4)

[Chem. 130]

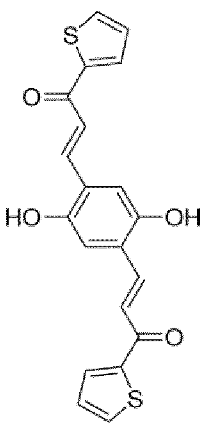

(1'-7-1)

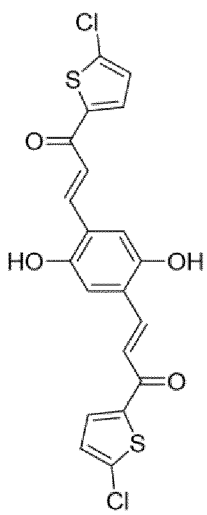

(1'-7-2)

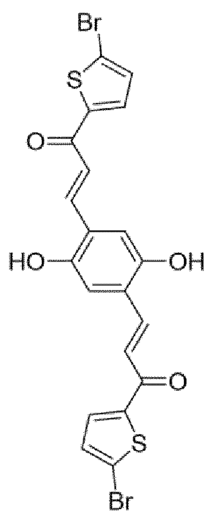

(1'-7-3)

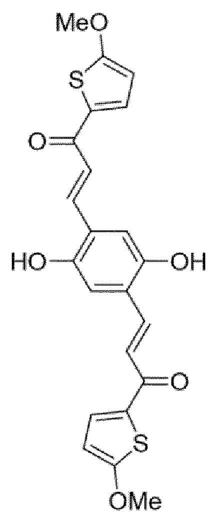

(1'-7-4)

Of these (1 '-2-1) to (1 '-7-4), particularly preferred compounds are (1'-2-2) to (1'-2-11), (1'-6-1) to (1'-6-4) and (1'-7-1) to (1'-7-4), with especially preferred ones being (1'-2-1) to (1'-2-6), (1'-2-1) to (1'-2-11), (1'-6-2) to (1'-6-3) and (1'-7-1).

A method for producing the dihydroxy compound according to the present invention, represented by formula (1'), is a method comprising allowing a dihydroxy compound indicated by chemical formula (3) below and a ketone indicated by chemical formula (4') below to react together.

[Chem. 131]

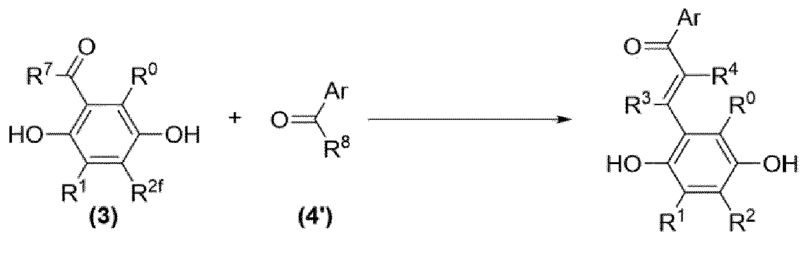

(3) (4') (1')

In chemical formula (3) above, R°, $R^1$ and $R^7$ each independently represent a hydrogen atom, a halogen atom or a C1 to C8 alkyl group.

$R^{2f}$ represents a hydrogen atom, a halogen atom, a C1 to C8 alkyl group or a formyl group.

In chemical formula (4') above, Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system and annulated aromatic ring system optionally having a substituent. $R^8$ represents a C1 to C8 alkyl group or a C1 to C6 haloalkyl group.

In chemical formula (1') above, Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system and annulated aromatic ring system optionally having a substituent. R°, $R^1$, $R^2$, $R^3$ and $R^4$ each independently represent a hydrogen atom, a halogen atom, a C1 to C8 alkyl group or a group represented by chemical formula (Z1) below.

[Chem. 132]

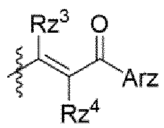

(Z1)

[In formula (Z1) above, $Rz^3$ and $Rz^4$ each independently represent a hydrogen atom, a halogen atom or a C1 to C8 alkyl group.

Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent. The wavy-lined portion represents a site of binding with the portion in formula (1) excluding $R^0$, $R^1$, $R^2$, $R^3$ or $R^4$.]

In formula (3), R°, $R^1$ and $R^7$ each independently represent a hydrogen atom or a C1 to C8 alkyl group. Examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group.

$R^{2f}$ represents a hydrogen atom, a halogen atom, a C1 to C8 alkyl group or a formyl group. Examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group.

For use as R°, $R^1$, $R^{2f}$ and $R^7$, a hydrogen atom, a methyl group, an ethyl group, a propyl group, an isopropyl group, a butyl group, an isobutyl group, a sec-butyl group and a tert-butyl group are preferred because they lead to superior optical characteristics after the introduction of the dihydroxy compound according to the present invention into the polymer according to the present invention. A hydrogen atom, a methyl group and an ethyl group are particularly preferred because of the ease of introduction.

Specific examples of dihydroxy compounds indicated by formula (3) include the structures presented in chemical formulae (3-1) to (3-11) below.

[Chem. 133]

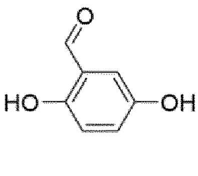
**(3-1)**
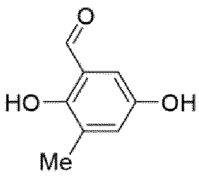
**(3-2)**
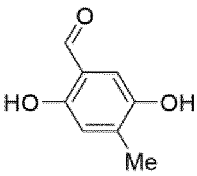
**(3-3)**
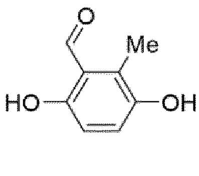
**(3-4)**

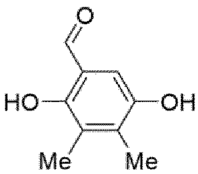
**(3-5)**
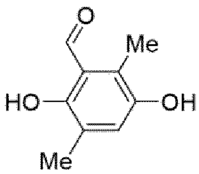
**(3-6)**
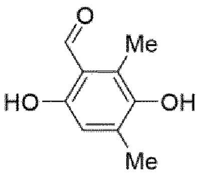
**(3-7)**
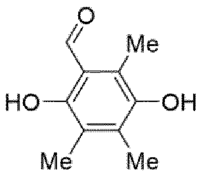
**(3-8)**

[Chem. 134]

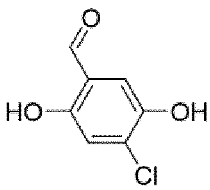
(3-10)
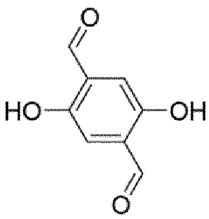
(3-11)

Of these (3-1) to (3-11), particularly preferred compounds are (3-1) to (3-4) and (3-10) to (3-11), with especially preferred ones being (3-1), (3-3) and (3-10) to (3-11).

The dihydroxy compound indicated by formula (3) can be easily prepared according to a method known or described in literature. Alternatively, if a commercially available compound is available, it may be used.

In formula (4'), $R^8$ represents a C1 to C8 alkyl group or a C1 to C6 haloalkyl group. Examples of C1 to C8 alkyl groups include a methyl group, an ethyl group, a propyl group, a butyl group, a pentyl group, a hexyl group, a heptyl group and an octyl group, and examples of C1 to C6 haloalkyl groups include a chloromethyl group and a 2,2,2-trifluoroethyl group.

For use as $R^8$, a methyl group, an ethyl group, a propyl group and a butyl group are preferred because they lead to superior optical characteristics after the introduction of the dihydroxy compound according to the present invention into the polymer according to the present invention. A methyl group and an ethyl group are particularly preferred because of the ease of introduction.

In formula (4'), Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system and annulated aromatic ring system optionally having a substituent.

In this context, examples of substituents in the monocyclic aromatic ring system, polycyclic aromatic ring system and annulated aromatic ring system include a C1 to C8 alkyl group, a halogen atom, a C1 to C4 alkoxy group and a C2

to C4 acyl group.

Examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group.

Examples of halogen atoms include a fluorine atom, a chlorine atom, a bromine atom and an iodine atom.

Examples of C1 to C4 alkoxy groups include a methoxy group, an ethoxy group, a propyloxy group, an isopropyloxy group, a butyloxy group, an isobutyloxy group, a sec-butyloxy group and a tert-butyloxy group.

Examples of C2 to C4 acyl groups include an acetyl group, a propionyl group and a butyryl group.

Examples of monocyclic aromatic ring systems at Ar whose ring-constituting atoms are atoms selected from the group consisting of a carbon atom, a nitrogen atom, an oxygen atom and a sulfur atom and that may have a substituent include benzene, pyridine, pyrazine, pyrimidine, pyridazine, furan, pyrrole, imidazole, thiophene, pyrazole, oxazole, isoxazole, thiazole and triazole.

Examples of polycyclic aromatic ring systems at Ar whose ring-constituting atoms are atoms selected from the group consisting of a carbon atom, a nitrogen atom, an oxygen atom and a sulfur atom and that may have a substituent include biphenyl, terphenyl, bipyridine, bithiophene and bifuran.

Examples of annulated aromatic ring systems at Ar whose ring-constituting atoms are atoms selected from the group consisting of a carbon atom, a nitrogen atom, an oxygen atom and a sulfur atom and that may have a substituent include naphthalene, anthracene, phenanthrene, quinoline, benzofuran, benzimidazole, benzothiophene, indole, indazole, benzoxazole, benzothiazole and benzotriazole.

For use as Ar, a monocyclic aromatic ring system whose ring-constituting atoms are atoms selected from the group consisting of a carbon atom, a nitrogen atom, an oxygen atom and a sulfur atom whose ring-constituting atoms are atoms selected from the group consisting of a carbon atom, a nitrogen atom, an oxygen atom and a sulfur atom and that may have a substituent is preferred because of easy availability. A benzene ring, a furan ring, a thiophene ring, a thiazole ring or an oxazole ring is particularly preferred because they lead to superior optical characteristics after the introduction of the dihydroxy compound according to the present invention into the polymer according to the present invention. A benzene ring or thiophene ring is especially preferred.

Preferred examples of Ars include the structures indicated by chemical formulae (Ar-1) to (Ar-7) below.

[Chem. 135]

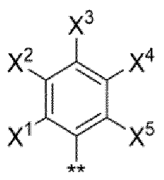
(Ar-1)

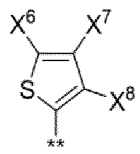
(Ar-2)

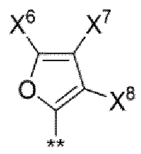
(Ar-3)

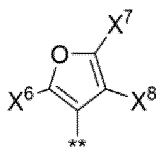
(Ar-4)

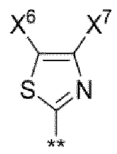
(Ar-5)

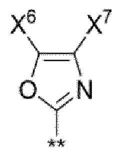
(Ar-6)

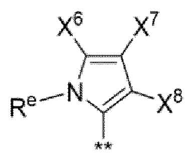
(Ar-7)

[In chemical formula (Ar-1) to (Ar-7) above, $X^1$, $X^2$, $X^3$, $X^4$, $X^5$, $X^6$, $X^7$ and $X^8$ each independently represent a hydrogen atom, a C1 to C8 alkyl group, a C1 to C6 alkoxy group, a halogen atom, a nitro group, a cyano group, a C1 to C6 alkylthio group or a C2 to C8 dialkylamino group.

$R^e$ represents a hydrogen atom or a C1 to C10 alkyl group.

** represents a site of binding with the other portion in chemical formula (1) above, excluding Ar.]

In formulae (Ar-1) to (Ar-7), $X^1$, $X^2$, $X^3$, $X^4$, $X^5$, $X^6$, $X^7$ and $X^8$ each independently represent a hydrogen atom, a C1 to C8 alkyl group, a C1 to C6 alkoxy group, a halogen atom, a nitro group, a cyano group, a C1 to C6 alkylthio group or a C2 to C8 dialkylamino group. Examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group, examples of C1 to C6 alkoxy groups include a methoxy group, an

ethoxy group, a propyloxy group, an isopropyloxy group, a butyloxy group, an isobutyloxy group, a sec-butyloxy group, a tert-butyloxy group, a pentyloxy group and a hexyloxy group, examples of halogen atoms include a fluorine atom, a chlorine atom, a bromine atom and an iodine atom, examples of C1 to C6 alkylthio groups include a methylthio group, an ethylthio group, a propylthio group, an isopropylthio group, a butylthio group, an isobutylthio group, a sec-butylthio group, a tert-butylthio group, a pentylthio group and a hexylthio group, and examples of C2 to C8 dialkylamino groups include a dimethylamino group, a diethylamino group, a dipropylamino group and a dibutylamino group.

For $X^1$, $X^2$, $X^3$, $X^4$, $X^5$, $X^6$, $X^7$ and $X^8$, it is preferred that each be a hydrogen atom, a C1 to C8 alkyl group, a C1 to C6 alkoxy group or a halogen atom because this leads to superior optical characteristics after the introduction of the dihydroxy compound according to the present invention into the polymer according to the present invention. It is particularly preferred that each be a hydrogen atom, a methyl group, a methoxy group or a halogen atom because of the ease of introduction.

Of the ketones indicated by formula (4'), the ketones indicated by formula (4) below are particularly preferred.

[Chem. 136]

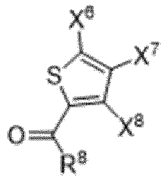

(4)

[In chemical formula (4) above, $R^8$ represents a C1 to C8 alkyl group or a C1 to C6 haloalkyl group.
$X^6$, $X^7$ and $X^8$ each independently represent a hydrogen atom, a C1 to C8 alkyl group, a C1 to C6 alkoxy group, a halogen atom, a nitro group, a cyano group, a C1 to C6 alkylthio group or a C2 to C8 dialkylamino group.]

In formula (4), $R^8$ represents a C1 to C8 alkyl group or a C1 to C6 haloalkyl group. Examples of C1 to C8 alkyl groups include a methyl group, an ethyl group, a propyl group, a butyl group, a pentyl group, a hexyl group, a heptyl group and an octyl group, and examples of C1 to C6 haloalkyl groups include a chloromethyl group and a 2,2,2-trifluoroethyl group.

For use as $R^8$, a methyl group, an ethyl group, a propyl group and a butyl group are preferred because they lead to superior optical characteristics after the introduction of the dihydroxy compound according to the present invention into the polymer according to the present invention. A methyl group and an ethyl group are particularly preferred because of the ease of introduction.

In formula (4), $X^6$, $X^7$ and $X^8$ each independently represent a hydrogen atom, a C1 to C8 alkyl group, a C1 to C6 alkoxy group, a halogen atom, a nitro group, a cyano group, a C1 to C6 alkylthio group or a C2 to C8 dialkylamino group. Examples of C1 to C8 alkyl groups include a methyl group, an ethyl group, a propyl group, an isopropyl group, a butyl group, an isobutyl group, a sec-butyl group, a tert-butyl group, a pentyl group, a hexyl group, a heptyl group and an octyl group, examples of C1 to C6 alkoxy groups include a methoxy group, an ethoxy group, a propyloxy group, an isopropyloxy group, a butyloxy group, an isobutyloxy group, a sec-butyloxy group, a tert-butyloxy group, a pentyloxy group and a hexyloxy group, examples of halogen atoms include a fluorine atom, a chlorine atom, a bromine atom and an iodine atom, examples of C1 to C6 alkylthio groups include a methylthio group, an ethylthio group, a propylthio group, an isopropylthio group, a butylthio group, an isobutylthio group, a sec-butylthio group, a tert-butylthio group, a pentylthio group and a hexylthio group, and examples of C2 to C8 dialkylamino groups include a dimethylamino group, a diethylamino group, a dipropylamino group and a dibutylamino group.

For $X^6$, $X^7$ and $X^8$, it is preferred that each be a hydrogen atom, a C1 to C8 alkyl group, a C1 to C6 alkoxy group or a halogen atom because this leads to superior optical characteristics after the introduction of the dihydroxy compound according to the present invention into the polymer according to the present invention. A hydrogen atom, a methyl group, a methoxy group or a halogen atom is particularly preferred because of the ease of introduction.

Specific examples of ketones indicated by formula (4) include the structures presented in chemical formulae (4-1-1) to (4-2-13) below.

[Chem. 137]

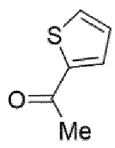 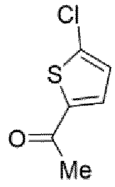 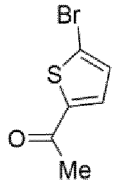 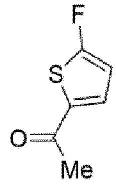 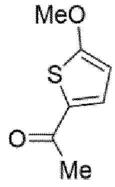 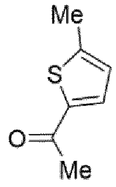

(4-1-1) (4-1-2) (4-1-3) (4-1-4) (4-1-5) (4-1-6)

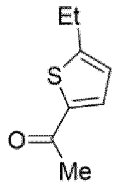 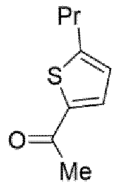 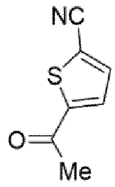 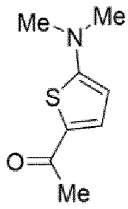 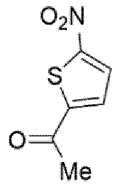 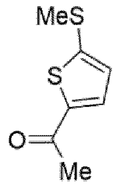

(4-1-7) (4-1-8) (4-1-9) (4-1-10) (4-1-11) (4-1-12)

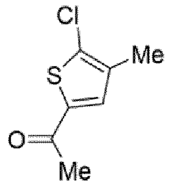

(4-1-13)

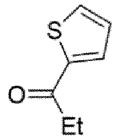 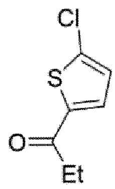 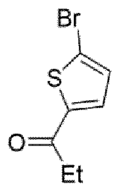 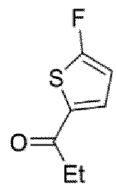 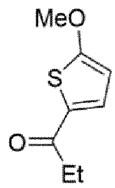 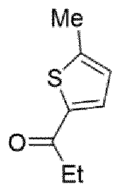

(4-2-1) (4-2-2) (4-2-3) (4-2-4) (4-2-5) (4-2-6)

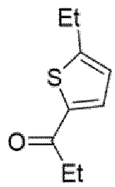 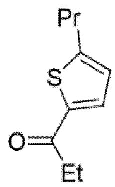 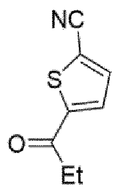 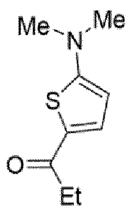 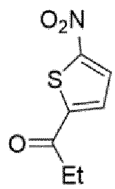 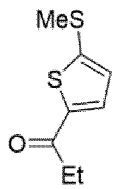

(4-2-7) (4-2-8) (4-2-9) (4-2-10) (4-2-11) (4-2-12)

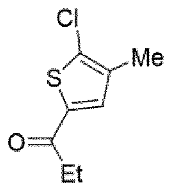

(4-2-13)

Of these (4-1-1) to (4-2-13), particularly preferred ketones are (4-1-1) to (4-1-13) because they lead to superior optical characteristics after the introduction of the dihydroxy compound according to the present invention into the polymer according to the present invention, with especially preferred ones being (4-1-1) to (4-1-6) and (4-1-13).

The ketone indicated by formula (4) can be easily prepared according to a method known or described in literature. Alternatively, if a commercially available ketone is available, it may be used.

The dihydroxy compound according to the present invention, indicated by formula (1'), can be synthesized by allowing a dihydroxy compound indicated by chemical formula (3) above and a ketone indicated by chemical formula (4') above to react together.

[Chem. 138]

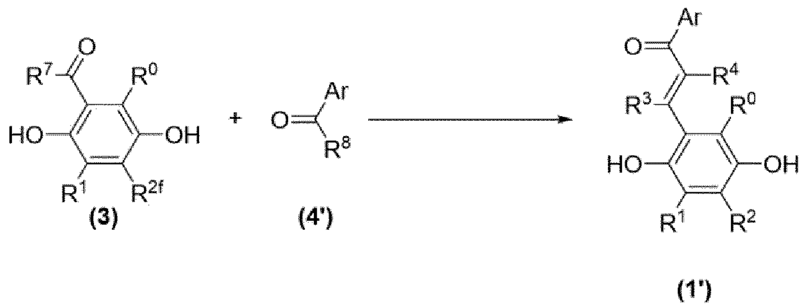

For the proportion between the dihydroxy compound indicated by formula (3) and the ketone indicated by formula (4') in the method for producing the dihydroxy compound indicated by formula (1'), it is preferred that the dihydroxy compound indicated by formula (3):the ketone indicated by formula (4') = 1:0.8 to 1:2.2 as a molar ratio.

In the method for producing the dihydroxy compound indicated by formula (1'), it is preferred to conduct the process with an added base to ensure that the reaction will proceed smoothly.

Examples of bases used include organic bases, inorganic bases, organometallic compounds, metal alkoxides, and metal amides.

Examples of organic bases include triethylamine, tributylamine, diisopropylethylamine, pyridine, 4-dimethylaminopyridine, piperidine, piperazine, pyrrolidine, morpholine, N-methylmorpholine, imidazole and N-methylimidazole.

Examples of inorganic bases include lithium hydroxide, sodium hydroxide, potassium hydroxide, calcium hydroxide, lithium carbonate, sodium carbonate, potassium carbonate, cesium carbonate, sodium hydrogencarbonate, potassium hydrogencarbonate, sodium hydride and potassium hydride.

Examples of organometallic compounds include normal butyllithium, sec-butyllithium, tert-butyllithium and phenyllithium.

Examples of metal alkoxides include sodium methoxide, sodium ethoxide, sodium tert-butoxide and potassium tert-butoxide.

Examples of metal amides include lithium amide, sodium amide, lithium diisopropylamide, lithium hexamethyldisilazide, sodium hexamethyldisilazide and potassium hexamethyldisilazide.

Among bases, inorganic bases are particularly preferred because they allow the reaction to proceed in a favorable manner and are inexpensive. Sodium hydroxide and potassium hydroxide are more preferred.

As for the amount of base used, it is preferred to use 3 to 10 molar equivalents per molar equivalent of formula (3). More preferably, 5 to 7 molar equivalents of base is used.

The base may be used in particle form or granule form or directly as a powdery solid depending on its properties. Solutions of them can also be used, and there is no specific restriction on their concentration.

In the method for producing the dihydroxy compound indicated by formula (1'), the process can be conducted without a solvent or in at least one solvent. The solvent is not particularly limited as long as it does not harm the reaction. Examples include the halogenated solvents of dichloromethane and chloroform, the ether solvents of dioxane, tetrahydrofuran (Hereinafter abbreviated as THF.), diisopropyl ether and cyclopentyl methyl ether, the aromatic solvents of benzene, toluene, xylene, chlorobenzene and dichlorobenzene, the amide solvents of N,N-dimethylformamide (Hereinafter abbreviated as DMF), N,N-dimethylacetamide and N-methyl-2-pyrrolidone (Hereinafter abbreviated as NMP.), alcohol solvents, such as methanol, ethanol, propyl alcohol, isopropyl alcohol and tert-butyl alcohol, and water. One such solvent may be used alone, or a mixture of two or more may be used. Of these, alcohol solvents are particularly preferred because they allow the reaction to proceed with a good yield, and ethanol and methanol are more preferred.

The reaction temperature in the method for producing the dihydroxy compound indicated by formula (1') is preferably a reaction temperature selected as appropriate from the range of -20°C to 100°C, particularly preferably a reaction temperature selected as appropriate from the range of 0°C to 70°C. The duration of reaction is determined as appropriate according to the progress of the reaction, and a duration of reaction selected as appropriate from the range of 1 hour to 24 hours is preferred.

In the method for producing the dihydroxy compound indicated by formula (1'), it is preferred to neutralize the reaction system using an acid after the completion of the reaction.

Examples of acids used include hydrochloric acid, sulfuric acid, acetic acid, chloroacetic acid and citric acid. Of these, acetic acid is particularly preferred.

For the amount of acid used, there is no specific restriction as long as it can neutralize the base used.

The dihydroxy compound obtained by the production method in formula (1') may be purified until suitable purity is achieved. Examples of purification methods include the methods of washing, column chromatography, reprecipitation,

trituration, adsorption treatment and recrystallization. Purification by washing, reprecipitation, recrystallization or trituration is preferred because of the small amount of organic solvent used, and purification by trituration is more preferred because of the simplicity of operations.

In trituration, at least one organic solvent can be used.

For the organic solvent used in trituration, there is no specific limitation as long as by-products can be removed therewith. Examples include the alcohol solvents of methanol, ethanol and isopropyl alcohol, the nitrile solvent of acetonitrile, the ester solvents of methyl acetate, ethyl acetate and butyl acetate, the hydrocarbon solvents of pentane, hexane, heptane and octane and the ketone solvents of acetone, methyl ethyl ketone and methyl isobutyl ketone. Methanol, ethanol, ethyl acetate or acetonitrile is preferred because they are inexpensive and allow for efficient removal of by-products, and a mixture of two or more such solvents may be used.

An example of a method for producing a polymer, in the context of the polymer according to the present invention, at least one end of which is a monocarboxylic acid ester is a production method comprising, when polymerizing a source composition containing a dihydroxy compound indicated by chemical formula (1') above, adding a monocarboxylic acid derivative compound represented by any of chemical formula (2-1) or (2-2) below.

[Chem. 139]

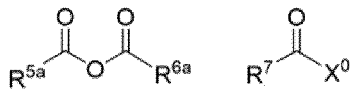

(2-1) (2-2)

[In chemical formula (2-1) or (2-2) above, $R^{5a}$, $R^{6a}$ and $R^7$ each independently represent a group selected from the group consisting of a substituted or unsubstituted C1 to C20 alkyl group, a substituted or unsubstituted C3 to C8 cycloalkyl group and a substituted or unsubstituted C3 to C12 aromatic group.
X° represents a leaving group.]

In chemical formula (2-1) or (2-2), examples of C1 to C20 alkyl groups at $R^{5a}$, $R^{6a}$ and $R^7$ include a methyl group, an ethyl group, a propyl group, an isopropyl group, a butyl group, an isobutyl group, a sec-butyl group, a tert-butyl group, a pentyl group, a hexyl group, a heptyl group, an octyl group, a nonyl group, a decyl group and an icosyl group. Of these, a methyl group, an ethyl group, a propyl group, an isopropyl group, a butyl group, an isobutyl group, a sec-butyl group and a tert-butyl group are particularly preferred, with a methyl group and an ethyl group being especially preferred because of the ease of introduction.

Examples of C3 to C8 cycloalkyl groups include a cyclopropyl group, a cyclobutyl group, a cyclopentyl group, a cyclohexyl group, a cycloheptyl group and a cyclooctyl group.

Examples of C3 to C12 aromatic groups include a phenyl group, a naphthyl group, a biphenylyl group and a pyridyl group.

In chemical formula (2-2), an example of a leaving group represented by X° is a halogen atom. For this halogen atom, examples include a fluorine atom, a chlorine atom, a bromine atom and an iodine atom.

The monocarboxylic acid derivative compound is represented by, for example, chemical formula (2-1) or (2-2). Examples of specific chemical formulae (2-1) include acetic anhydride, propionic anhydride, butyric anhydride, isobutyric anhydride, difluoroacetic anhydride, valeric anhydride, isovaleric anhydride, pivalic anhydride, trifluoroacetic anhydride, hexanoic anhydride, benzoic anhydride, 3-pyridinecarboxylic anhydride, cyclohexanecarboxylic anhydride, heptanoic anhydride, chloroacetic anhydride, chlorodifluoroacetic anhydride, 2-methylbenzoic anhydride, n-octanoic anhydride, 4-methoxybenzoic anhydride, nonanoic anhydride, dichloroacetic anhydride, trichloroacetic anhydride, pentafluoropropionic anhydride, decanoic anhydride, 2-methyl-6-nitrobenzoic anhydride, lauric anhydride, 3,4,5-trimethoxybenzoic anhydride and heptafluorobutyric anhydride. Preferred compounds are acetic anhydride, propionic anhydride, butyric anhydride, isobutyric anhydride, valeric anhydride, isovaleric anhydride, pivalic anhydride, trifluoroacetic anhydride, hexanoic anhydride, benzoic anhydride, cyclohexanecarboxylic anhydride, heptanoic anhydride, chloroacetic anhydride and 2-methylbenzoic anhydride, with particularly preferred ones being acetic anhydride, propionic anhydride, butyric anhydride, isobutyric anhydride, valeric anhydride, isovaleric anhydride, pivalic anhydride, hexanoic anhydride, benzoic anhydride, cyclohexanecarboxylic anhydride, heptanoic anhydride and 2-methylbenzoic anhydride. Examples of specific chemical formulae (2-2) include benzoyl fluoride, undecafluorohexanoyl fluoride, acetyl chloride, propyl chloride, cyclopropanecarbonyl chloride, butyryl chloride, isobutyryl chloride, chloroacetyl chloride, valeryl chloride, isovaleryl chloride, pivaloyl chloride, 2-chloropropionyl chloride, 3-chloropropionyl chloride, 2-furoyl chloride, cyclopentanecarbonyl chloride, hexanoyl chloride, 2,2-dimethylbutyryl chloride, 4-methylvaleryl chloride, 3,3-dimethylbutyryl chloride, benzoyl chloride, 4-chlorobutyryl chloride, 2-thenoyl chloride, cyclohexanecarbonyl chloride, dichloroacetyl chloride, tetrahydro-2H-pyran-4-carbonyl chloride, heptanoyl chloride, p-toluoyl chloride, o-toluoyl chloride, m-toluoyl chloride, 3-chloropivaloyl chloride, 5-chlorovaleryl chloride, bromoacetyl chloride, 3-fluorobenzoyl chloride, 2-fluorobenzoyl chloride, 4-fluorobenzoyl chloride, 3-methyl-2-thenoyl chloride, n-octanoyl chloride, 2-propylvaleryl chloride, 2-ethylhexanoyl chloride, 4-cyanobenzoyl chloride, 3,5-dimethylbenzoyl chloride, 4-methoxybenzoyl chloride, 3-methoxybenzoyl chloride, 2-methoxybenzoyl chloride, 2-bromopropionyl chloride, 3-bromopropionyl chloride, 6-chloronicotinoyl chloride, 5-chlorothiophene-2-carbonyl chloride, trichloroacetyl chloride, dodecanoyl chloride, 4-(trifluoromethyl)benzoyl chloride, acetyl bromide, propionyl bromide, isobutyryl bromide, valeryl bromide, benzoyl bromide, bromoacetyl bromide, 2-bromopropionyl bromide, 2-bromoisobutyryl bromide, 2-bromobutyryl bromide and acetyl iodide. Preferred compounds are acetyl chloride, propyl chloride, cyclopropanecarbonyl chloride, butyryl chloride, isobutyryl chloride, chloroacetyl chloride, valeryl chloride, isovaleryl chloride, pivaloyl chloride, 2-chloropropionyl chloride, 3-chloropropionyl chloride, 2-furoyl chloride, cyclopentanecarbonyl chloride, hexanoyl chloride, 2,2-dimethylbutyryl chloride, 4-methylvaleryl chloride, 3,3-dimethylbutyryl chloride, benzoyl chloride, 4-chlorobutyryl chloride, cyclohexanecarbonyl chloride, dichloroacetyl chloride, heptanoyl chloride, p-toluoyl chloride, o-toluoyl chloride, m-toluoyl chloride, 3-chloropivaloyl chloride, 5-chlorovaleryl chloride, bromoacetyl chloride, 3-fluorobenzoyl chloride, 2-fluorobenzoyl chloride, 4-fluorobenzoyl chloride, n-octanoyl chloride, 2-propylvaleryl chloride, 2-ethylhexanoyl chloride, 4-cyanobenzoyl chloride, 3,5-dimethylbenzoyl chloride, 4-methoxybenzoyl chloride, 3-methoxybenzoyl chloride, 2-methoxybenzoyl chloride, 2-bromopropionyl chloride, 3-bromopropionyl chloride, trichloroacetyl chloride, dodecanoyl chloride and 4-(trifluoromethyl)benzoyl chloride, with particularly preferred ones being acetyl chloride, propyl chloride, cyclopropanecarbonyl chloride, butyryl chloride, isobutyryl chloride, valeryl chloride, isovaleryl chloride, pivaloyl chloride, cyclopentanecarbonyl chloride, hexanoyl chloride, 2,2-dimethylbutyryl chloride, 4-methylvaleryl chloride, 3,3-dimethylbutyryl chloride, benzoyl chloride, cyclohexanecarbonyl chloride, heptanoyl chloride, p-toluoyl chloride, o-toluoyl chloride, m-toluoyl chloride, n-octanoyl chloride, 2-propylvaleryl chloride, 2-ethylhexanoyl chloride, 3,5-dimethylbenzoyl chloride and dodecanoyl chloride.

The polymer according to the present invention can also be made into a resin composition containing 70% to 99.99% by weight of the polymer according to the present invention and 0.01% to 30% by weight of a thermal reorientation accelerator (hereinafter also expressed as the resin composition according to the present invention). This resin composition is also an aspect of the present invention.

The resin composition according to the present invention contains a thermal reorientation accelerator. An optical film obtained using the resin composition according to the present invention allows for thermal reorientation treatment for a polymer, even on a general-purpose resin support substrate having a low temperature tolerance, by virtue of molecular mobility improved by the thermal reorientation accelerator.

For the thermal reorientation accelerator contained in the resin composition according to the present invention, it is preferred, for reduced separation and exudation of the thermal reorientation accelerator while in a high-temperature environment or reduced evaporation of the thermal reorientation accelerator while in a high-temperature environment, that its molecular weight be from 100 to 20000. It is particularly preferred that the molecular weight be from 300 to 20000.

The blending percentages of the polymer according to the present invention and the thermal reorientation accelerator in the resin composition according to the present invention are from 70% to 99.99% by weight for the polymer according to the present invention and from 0.01% to 30% by weight for the thermal reorientation accelerator. For reduced separation and exudation of the thermal reorientation accelerator while in a high-temperature environment, it is more preferred that the percentages be from 85% to 99.9% by weight for the polymer according to the present invention and from 0.1% to 15% by weight for the thermal reorientation accelerator. From the viewpoint of efficiency in the acceleration of thermal reorientation, it is particularly preferred that the percentages be from 85% to 99.0% by weight for the polymer according to the present invention and from 1.0% to 15% by weight for the thermal reorientation accelerator. In the present invention, when the percentage of the thermal reorientation accelerator is less than 0.01% by weight, the acceleration of thermal reorientability becomes difficult, and when the percentage of the thermal reorientation accelerator is greater than 30% by weight, the separation and exudation of the thermal reorientation accelerator are likely to occur.

Examples of thermal reorientation accelerators contained in the resin composition according to the present invention include plasticizers, antioxidants and photostabilizers.

For the plasticizers, examples include carboxylates, phosphates and polymeric plasticizers.

Specific examples of carboxylates include phthalates, trimellitates, pyromellitates, citrates, oleates, ricinoleates, sebacates, stearates, adipates and epoxidized esters. Of these, phthalates and adipates are particularly preferred because of their easy availability.

Examples of phthalates include ones including a structure indicated by formula (7) below and having a molecular weight of 100 to 20000.

[Chem. 140]

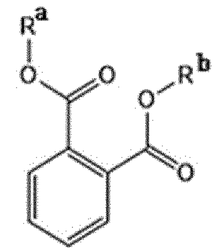

(7)

(In formula (7), $R^a$ and $R^b$ each independently represent one type selected from the group consisting of a substituted or unsubstituted C1 to C20 alkyl group, aromatic group, heterocyclic group, polycyclic aromatic group and annulated aromatic group, with these optionally having a linkage such as an -O-group, a -(C=O)O- group, an -O(C=O)-group, an -O(C=O)-O- group, a -C(=O)-NH- group, an -NH-(C=O)- group, a -CH=CH- group or a -C=C- group. It is preferred that each of $R^a$ and $R^b$ independently be a C2 to C20 alkyl group because in that case separation and exudation are rare, and improving effects on the molecular orientation of the polymer are expected.)

Examples of specific phthalates include dimethyl phthalate, diethyl phthalate, dipropyl phthalate, diisopropyl phthalate, dibutyl phthalate, diisobutyl phthalate, diamyl phthalate, dihexyl phthalate, diheptyl phthalate, di-n-octyl phthalate, dinonyl phthalate, diisononyl phthalate, di-n-decyl phthalate, diisodecyl phthalate, diundecyl phthalate, ditridecyl phthalate, dicyclohexyl phthalate, diphenyl phthalate, di-2-ethylhexyl phthalate, benzyl butyl phthalate and di-2-ethylhexyl isophthalate.

Examples of trimellitates include ones including a structure indicated by formula (8) below and having a molecular weight of 100 to 20000.

[Chem. 141]

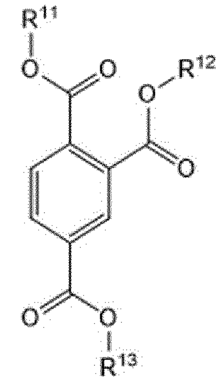

(8)

(In formula (8), $R^{11}$, $R^{12}$ and $R^{13}$ each independently represent one type selected from the group consisting of a substituted or unsubstituted C1 to C20 alkyl group, aromatic group, heterocyclic group, polycyclic aromatic group and annulated aromatic group, with these optionally having a linkage such as an -O- group, -a (C=O)O- group, an -O(C=O)-group, an -O(C=O)-O- group, a - C(=O)-NH- group, an -NH-(C=O)- group, a -CH=CH- group or a -C=C- group.)

Specific examples of trimellitates include trimethyl trimellitate, triethyl trimellitate, tributyl trimellitate, tris(2-ethylhexyl) trimellitate, tri-n-octyl trimellitate, triisooctyl trimellitate, trinonyl trimellitate, triisononyl trimellitate, tri-n-decyl trimellitate, triisodecyl trimellitate, triundecyl trimellitate, tridodecyl trimellitate, tri(tridecyl) trimellitate and tritetradecyl trimellitate.

Examples of pyromellitates include ones including a structure indicated by formula (9) below and having a molecular weight of 100 to 20000.

[Chem. 142]

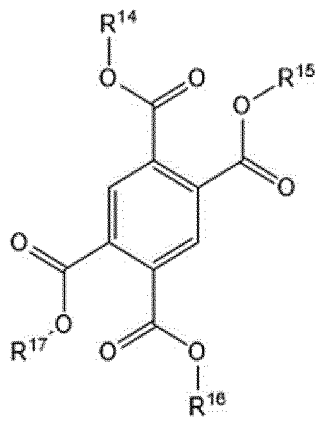

(9)

(In formula (9), $R^{14}$, $R^{15}$, $R^{16}$ and $R^{17}$ each independently represent one type selected from the group consisting of a substituted or unsubstituted C1 to C20 alkyl group, aromatic group, heterocyclic group, polycyclic aromatic group and annulated aromatic group, with these optionally having a linkage such as an -O- group, a -(C=O)O- group, an -O(C=O)- group, an -O(C=O)-O- group, a - C(=O)-NH- group, an -NH-(C=O)- group, a -CH=CH- group or a -C=C- group.)

Specific examples of pyromellitates include tetramethyl pyromellitate, tetraethyl pyromellitate, tetrapropyl pyromellitate, tetrabutyl pyromellitate, pyromellitic acid 2-ethylhexyl esters, tetra(2-ethylhexyl) pyromellitate, tetra-n-octyl pyromellitate, tetraisooctyl pyromellitate, tetranonyl pyromellitate, tetraisononyl pyromellitate, tetra-n-decyl pyromellitate, tetraisodecyl pyromellitate, tetraundecyl pyromellitate, tetradodecyl pyromellitate, tetratridecyl pyromellitate and tetra(tetradecyl) pyromellitate.

Examples of citrates include ones including a structure indicated by formula (10) below and having a molecular weight of 100 to 20000.

[Chem. 143]

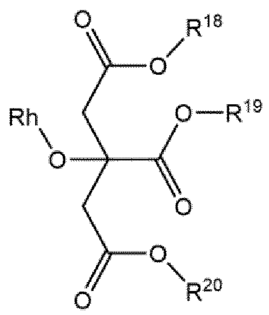

(10)

(In formula (10), $R^{18}$, $R^{19}$ and $R^{20}$ each independently represent one type selected from the group consisting of a substituted or unsubstituted C1 to C20 alkyl group, aromatic ring, heterocyclic group, polycyclic aromatic group or annulated aromatic group, with these optionally having a linkage such as an -O-group, a -(C=O)O- group, an -O(C=O)- group, an -O(C=O)-O- group, a -C(=O)-NH- group, an -NH-(C=O)- group, a -CH=CH- group or a -C=C- group. Rh represents one type selected from the group consisting of a hydrogen atom or a substituted or unsubstituted C1 to C20 acyl group, C1 to C20 alkyl group, aromatic group, heterocyclic group, polycyclic aromatic group and annulated aromatic group, with these optionally having a linkage such as an -O- group, a - (C=O)O- group, an -O(C=O)- group, an -O(C=O)-O- group, a -C(=O)-NH- group, an -NH-(C=O)- group, a -CH=CH- group or a -C=C- group.)

Specific examples of citrates include trimethyl citrate, triethyl citrate, tripropyl citrate, tributyl citrate, tripentyl citrate, trihexyl citrate, trimethyl acetylcitrate, triethyl acetylcitrate, tripropyl acetylcitrate, tributyl acetylcitrate, tripentyl acetylcitrate, trihexyl acetylcitrate and trihexyl butyryl citrate (trihexyl o-butyrylcitrate).

Examples of oleates include ones including a structure indicated by formula (11) below and having a molecular weight of 100 to 20000.

[Chem. 144]

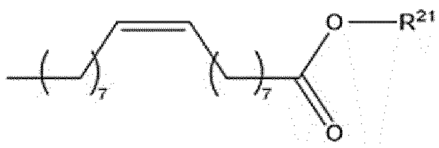

(11)

(In formula (11), $R^{21}$ represents one type selected from the group consisting of a substituted or unsubstituted C1 to C20 alkyl group, aromatic ring, heterocycle, polycyclic aromatic ring system or annulated aromatic ring system, with these optionally having a linkage such as an -O- group, a -(C=O)O- group, an -O(C=O)- group, an -O(C=O)-O- group, a -C(=O)-NH- group, an -NH-(C=O)-group, a -CH=CH- group or a -C=C- group.)

Specific examples of oleates include methyl oleate, ethyl oleate, propyl oleate, butyl oleate, hexyl oleate, heptyl oleate, n-octyl-oleate, nonyl oleate and n-decyl-oleate.

Examples of ricinoleates include ones including a structure indicated by formula (12) below and having a molecular weight of 100 to 20000.

[Chem. 145]

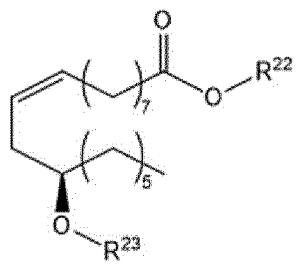

(12)

(In formula (12), $R^{22}$ represents one type selected from the group consisting of a substituted or unsubstituted C1 to C20 alkyl group, aromatic ring, heterocycle, polycyclic aromatic ring system or annulated aromatic ring system, with these optionally having a linkage such as an -O- group, a -(C=O)O- group, an -O(C=O)- group, an -O(C=O)-O- group, a -C(=O)-NH- group, an -NH-(C=O)-group, a -CH=CH- group or a -C≡C- group. $R^{23}$ represents one type selected from the group consisting of a hydrogen atom or a substituted or unsubstituted C1 to C20 acetyl group, C1 to C20 alkyl group, aromatic ring, heterocycle, polycyclic aromatic ring system or annulated aromatic ring system, with these optionally having a linkage such as an -O- group, a -(C=O)O- group, an -O(C=O)-group, an -O(C=O)-O- group, a -C(=O)-NH-group, an -NH-(C=O)- group, a - CH=CH- group or a -C=C- group.)

Specific examples of ricinoleates include methyl ricinoleate, ethyl ricinoleate, propyl ricinoleate, butyl ricinoleate, pentyl ricinoleate, hexyl ricinoleate, methyl acetylricinoleate, ethyl acetylricinoleate and propyl acetylricinoleate.

Example of sebacates include ones including a structure indicated by formula (13) below and having a molecular weight of 100 to 20000.

[Chem. 146]

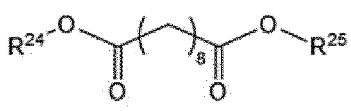

(13)

(In formula (13), $R^{24}$ and $R^{25}$ each independently represent one type selected from the group consisting of a substituted or unsubstituted C1 to C20 alkyl group, aromatic ring, heterocycle, polycyclic aromatic ring system or annulated aromatic ring system, with these optionally having a linkage such as an -O- group, a -(C=O)O- group, an -O(C=O)- group, an -O(C=O)-O- group, a - C(=O)-NH- group, an -NH-(C=O)- group, a -CH=CH- group or a -C=C- group.)

Specific examples of sebacates include dimethyl sebacate, diethyl sebacate, dipropyl sebacate, dibutyl sebacate, di(2-ethylhexyl) sebacate, di-n-octyl sebacate, diisooctyl sebacate, dinonyl sebacate, diisononyl sebacate, di-n-decyl sebacate, diisodecyl sebacate, diundecyl sebacate, didodecyl sebacate, ditridecyl sebacate and ditetradecyl sebacate.

Examples of stearates include ones including a structure indicated by formula (14) below and having a molecular

weight of 100 to 20000.

Chem. 147]

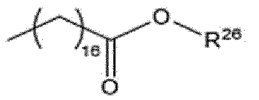

(14)

(In formula (14), $R^{26}$ represents one type selected from the group consisting of a substituted or unsubstituted C1 to C40 alkyl group, aromatic ring, heterocycle, polycyclic aromatic ring system or annulated aromatic ring system, with these optionally having a linkage such as an -O- group, a -(C=O)O- group, an -O(C=O)- group, an -O(C=O)-O- group, a -C(=O)-NH- group, an -NH-(C=O)-group, a -CH=CH- group or a -C=C- group.)

Specific examples of stearates include methyl stearate, ethyl stearate, propyl stearate, butyl stearate, pentyl stearate, hexyl stearate, heptyl stearate, n-octyl-stearate, nonyl stearate, decyl stearate, dodecyl stearate, phenyl stearate, glycidyl stearate, methyl dichlorostearate, monostearin and tristearin.

Examples of adipates include ones including a structure indicated by formula (15) below and having a molecular weight of 100 to 20000.

[Chem. 148]

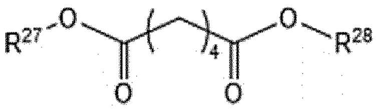

(15)

(In formula (15), $R^{27}$ and $R^{28}$ each independently represent one type selected from the group consisting of a substituted or unsubstituted C1 to C20 alkyl group, aromatic ring, heterocycle, polycyclic aromatic ring system or annulated aromatic ring system, with these optionally having a linkage such as an -O- group, a -(C=O)O- group, an -O(C=O)- group, an -O(C=O)-O- group, a - C(=O)-NH- group, an -NH-(C=O)- group, a -CH=CH- group or a -C=C- group. It is preferred that each of $R^{27}$ and $R^{28}$ independently be a C2 to C20 alkyl group because in that case separation and exudation are rare, and improving effects on the molecular orientation of the polymer are expected.)

Specific examples of adipates include dimethyl adipate, diethyl adipate, dipropyl adipate, dibutyl adipate, diisobutyl adipate, bis[2-(2-butoxyethoxy)ethyl] adipate, di(2-ethylhexyl) adipate, di-n-octyl adipate, diisooctyl adipate, dinonyl adipate, diisononyl adipate, di-n-decyl adipate, diisodecyl adipate, diundecyl adipate, didodecyl adipate, ditridecyl adipate and ditetradecyl adipate.

For epoxidized esters, there is no specific restriction as long as they have a molecular weight of 100 or more and one or more epoxy groups and one or more ester linkages. Examples include di 2-ethylhexyl 4,5-epoxycyclohexane-1,2-dicarboxylate, di(9,10-epoxystearyl) 4,5-epoxycyclohexane-1,2-dicarboxylate, epoxidized soybean oil, epoxidized linseed oil, epoxidized fatty acid isobutyl esters and epoxidized fatty acid 2-ethylhexyl esters.

The carboxylates only need to have a molecular weight of 100 to 20000 and are not limited to those indicated by formulae (7) to (15). Examples also include dimethyl isophthalate, diethyl isophthalate, dipropyl isophthalate, dibutyl isophthalate, bis(2-ethylhexyl) isophthalate, di-n-decyl isophthalate, diisodecyl isophthalate, diundecyl isophthalate, didodecyl isophthalate, ditridecyl isophthalate, ditetradecyl isophthalate, dimethyl terephthalate, diethyl terephthalate, dipropyl terephthalate, dibutyl terephthalate, bis(2-ethylhexyl) terephthalate, di-n-decyl terephthalate, diisodecyl terephthalate, diundecyl terephthalate, didodecyl terephthalate, ditridecyl terephthalate, ditetradecyl terephthalate, diisodecyl 4-cyclohexene-1,2-dicarboxylate, bis(2-ethylhexyl) 4-cyclohexene-1,2-dicarboxylate, dimethyl succinate, diethyl succinate, dipropyl succinate, dibutyl succinate, bis(2-ethylhexyl) succinate, di-n-decyl succinate, diisodecyl succinate, diundecyl succinate, didodecyl succinate, ditridecyl succinate, ditetradecyl succinate, dimethyl maleate, diethyl maleate, dipropyl maleate, dibutyl maleate, bis(2-ethylhexyl) maleate, di-n-decyl maleate, diisodecyl maleate, diundecyl maleate, didodecyl maleate, ditridecyl maleate, ditetradecyl maleate, dimethyl fumarate, diethyl fumarate, dipropyl fumarate, dibutyl fumarate, bis(2-ethylhexyl) fumarate, di-n-decyl fumarate, diisodecyl fumarate, diundecyl fumarate, didodecyl fumarate, ditridecyl fumarate, ditetradecyl fumarate, dimethyl suberate, diethyl suberate, dipropyl suberate, dibutyl suberate, bis(2-ethylhexyl) suberate, di-n-decyl suberate, diisodecyl suberate, diundecyl suberate, didodecyl suberate, ditridecyl suberate, ditetradecyl suberate, dimethyl azelate, diethyl azelate, dipropyl azelate, dibutyl azelate, bis(2-ethylhexyl) azelate, di-n-decyl azelate, diisodecyl azelate, diundecyl azelate, didodecyl azelate, ditridecyl azelate, ditetradecyl azelate, dimethyl dodecanoate, diethyl dodecanoate, dipropyl dodecanoate, dibutyl dodecanoate, bis(2-ethylhexyl) dodecanoate, di-n-decyl dodecanoate, diisodecyl dodecanoate, diundecyl dodecanoate, didodecyl dodecanoate, ditridecyl dodecanoate, ditetradecyl dodecanoate, dimethyl tetradecanoate, diethyl tetradecanoate, dipropyl tetradecanoate, dibutyl tetradecanoate, bis(2-ethylhexyl) tetradecanoate, di-n-decyl tetradecanoate, diisodecyl tetradecanoate, diundecyl tetradecanoate, didodecyl tetradecanoate, ditridecyl tetradecanoate, ditetradecyl tetradecanoate, dimethyl hexadecanoate, diethyl hexadecanoate, dipropyl hexadecanoate, dibutyl hexadecanoate, bis(2-ethylhexyl) hexadecanoate, di-n-decyl hexadecanoate, diisodecyl hexadecanoate, diundecyl hexadecanoate, didodecyl hexadecanoate, ditridecyl hexadecanoate, ditetradecyl hexadecanoate, dimethyl octadecanoate, diethyl octadecanoate, dipropyl octadecanoate, dibutyl octadecanoate, bis(2-ethylhexyl) octadecanoate, di-n-decyl octadecanoate, diisodecyl octadecanoate, diundecyl octadecanoate, didodecyl octadecanoate, ditridecyl octadecanoate, ditetradecyl octadecanoate, dimethyl icosadecanoate, diethyl icosadecanoate, dipropyl icosadecanoate, dibutyl icosadecanoate, bis(2-ethylhexyl) icosadecanoate, di-n-decyl icosadecanoate, diisodecyl icosadecanoate, diundecyl icosadecanoate, didodecyl icosadecanoate, ditridecyl icosadecanoate and ditetradecyl icosadecanoate.

Examples of phosphates include the compounds indicated by formula (16) below.

[Chem. 149]

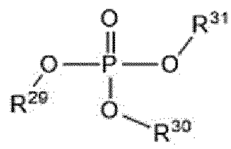

(16)

(In formula (16), $R^{29}$, $R^{30}$ and $R^{31}$ each independently represent one type selected from the group consisting of a substituted or unsubstituted C1 to C20 alkyl group, aromatic ring, heterocycle, polycyclic aromatic ring system or annulated aromatic ring system, with these optionally having a linkage such as an -O- group, a -(C=O)O- group, an -O(C=O)- group, an -O(C=O)-O- group, a - C(=O)-NH- group, an -NH-(C=O)- group, a -CH=CH- group or a -C=C- group. It is preferred that each of $R^{29}$ to $R^{31}$ independently be a C3 to C20 alkyl group because in that case separation and exudation are rare, and improving effects on the molecular orientation of the polymer are expected.)

Specific examples of phosphates include trimethyl phosphate, triethyl phosphate, tripropyl phosphate, tributyl phosphate, triamyl phosphate, trihexyl phosphate, triheptyl phosphate, tri-n-octyl phosphate, trinonyl phosphate, tri-n-decyl phosphate, tris(2-ethylhexyl) phosphate, tris(2-butoxyethyl) phosphate, tris(2-chloroethyl) phosphate, tris(1,3-dichloro-2-propyl) phosphate, 2-ethylhexyl diphenyl phosphate, triphenyl phosphate, trixylenyl phosphate, cresyl diphenyl phosphate, tris(2-ethylhexyl) phosphate and tricresyl phosphate.

Specific examples of polymeric plasticizers include polyester plasticizers and ether plasticizers.

Polyester plasticizers are polymers including a constituent unit indicated by formula (17) below, with examples including ones having a molecular weight of 100 to 20000.

[Chem. 150]

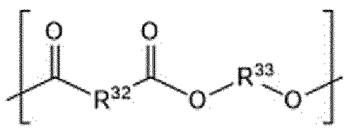

(17)

(In formula (17), $R^{32}$ and $R^{33}$ each independently represent one type selected from the group consisting of a substituted or unsubstituted C1 to C20 alkyl group, aromatic ring, heterocycle, polycyclic aromatic ring system or annulated aromatic ring system, with these optionally having a linkage such as an -O- group, a -(C=O)O- group, an -O(C=O)- group, an -O(C=O)-O- group, a - C(=O)-NH- group, an -NH-(C=O)- group, a -CH=CH- group or a -C=C- group.)

Specific examples of polyester plasticizers include ADK CIZER PN-160, PN-9302, PN-150, PN-170, PN-7230, PN-1010, PN-1020, P-200, PN-650, PN-7650, PN-1030, PN-1430, HPN-3130, PN-400, P-5040, PN-7250, PN-250, PN-7220, PN-7550, PN-446, PN-310, P-300, PN-280 and PN-5090 (all are trade names; manufactured by ADEKA Corporation), D620, D623, D643, D645, D633, D620N, D623N, D643D, D640A and D671N (all are trade names; manufactured by Mitsubishi Chemical Corporation) and POLYCIZER W-230-H, W-1430-EL, W-2050 and W-2310 (all are trade names; manufactured by DIC Corporation).

Examples of ether plasticizers include ones including a constituent unit indicated by formula (18) below and having a molecular weight of 100 to 20000.

[Chem. 151]

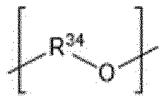

(18)

(In formula (18), $R^{34}$ represents one type selected from the group consisting of a substituted or unsubstituted C1 to C20 alkylene chain, aromatic ring, heterocycle, polycyclic aromatic ring system or annulated aromatic ring system, with these optionally having a linkage such as an -O- group, a -(C=O)O-group, an -O(C=O)- group, an -O(C=O)-O- group, a -C(=O)-NH- group, an -NH-(C=O)- group, a -CH=CH- group or a -C=C- group.)

Specific examples of ether plasticizers include ADK CIZER RS-107, RS-700, RS-735, RS-966 and RS-1000, (all are trade names; manufactured by ADEKA Corporation), MONOCIZER W-260 and W-262 (both are trade names; manufactured by DIC Corporation), diethylene glycol, triethylene glycol, tetraethylene glycol, pentaethylene glycol, hexaethylene glycol, heptaethylene glycol, octaethylene glycol, nonaethylene glycol, decaethylene glycol, dodecaethylene glycol, polyethylene glycol 200, polyethylene glycol 400, polyethylene glycol 300, polyethylene glycol 600, polyethylene glycol 1000, polyethylene glycol 2000, polyethylene glycol 4000, polyethylene glycol 6000, polyethylene glycol 11000 and polyethylene glycol 20000.

Examples of antioxidants include phenolic antioxidants, amine antioxidants, phosphorus antioxidants, sulfur antioxidants, lactone antioxidants, hydroxylamine antioxidants and vitamin-E antioxidants.

Examples of phenolic antioxidants include ones including a structure indicated by formula (19) below and having a molecular weight of 100 to 20000.

[Chem. 152]

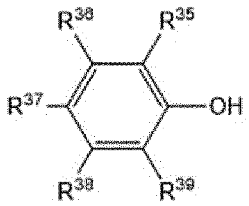

(19)

(In formula (19), at least one of $R^{35}$ or $R^{39}$ represents one type selected from the group consisting of a substituted or unsubstituted C3 to C20 secondary alkyl group, C4 to C20 tertiary alkyl group, C6 to C20 thioether group, alicyclic hydrocarbon, aromatic ring, polycyclic aromatic ring system or annulated aromatic ring system, with these optionally having a linkage such as -O- group, - (C=O)O- group, -O(C=O)- group, -O(C=O)-O- group, -C(=O)-NH- group, -NH-(C=O)- group, -CH=CH- group or -C=C- group, and the other represents one type selected from the group consisting of a hydrogen atom or a substituted or unsubstituted C1 to C20 alkyl group, C1 to C20 alkoxy group, C1 to C20 thioether group, alicyclic hydrocarbon, aromatic ring, polycyclic aromatic ring system or annulated aromatic ring system, with these optionally having a linkage such as an -O- group, a -(C=O)O- group, an -O(C=O)- group, an -O(C=O)-O- group, a - C(=O)-NH- group, an -NH-(C=O)- group, a -CH=CH- group or a -C=C- group. Their structures are not particularly limited, but tert-butyl groups are preferred because of easy availability. $R^{36}$ to $R^{38}$ each independently represent one type selected from the group consisting of a hydrogen atom or a substituted or unsubstituted C1 to C20 alkyl group, C1 to C20 alkoxy group, aromatic ring, heterocycle, polycyclic aromatic ring system or annulated aromatic ring system, with these optionally having a linkage such as an -O- group, a -(C=O)O- group, an -O(C=O)- group, an -O(C=O)-O- group, a -C(=O)-NH- group, an -NH-(C=O)-group, a -CH=CH- group or a -C≡C- group.)

Specific examples of phenolic antioxidants include Irganox 245 (bis[3-(3-tert-butyl-4-hydroxy-5-methylphenyl)propionic acid][ethylenebis(oxyethylene)]), Irganox 1010 (pentaerythritol tetrakis[3-(3,5-di-tert-butyl-4-hydroxyphenyl)propionate]), Irganox 1035 (2,2'-thiodiethyl bis[3-(3,5-di-tert-butyl-4-hydroxyphenyl)propionate]), Irganox 1076 (octadecyl 3-(3,5-di-tert-butyl-4-hydroxyphenyl)propionate), Irganox 1098 (N,N'-(hexane-1,6-diyl)bis[3-(3,5-di-tert-butyl-4-hydroxyphenyl)propanamide]), Irganox 1135 (octyl-3,5-di-tert-butyl-4-hydroxy-hydrocinnamate), Irganox 1330 (2,4,6-tris(3',5'-di-tert-butyl-4'-hydroxybenzyl)mesitylene), Irganox 1520 (2,4-bis(octylthiomethyl)-6-methylphenol), Irganox 259 (1,6-hexanediol bis[3-(3,5-di-tert-butyl-4-hydroxyphenyl)propionate]), Irganox 3114 (1,3,5-tris(3,5-di-tert-butyl-4-hydroxybenzyl)-1,3,5-triazine-2,4,6(1H,3H,5H)-trione) and Irganox 565 (4-[[4,6-bis(octylthio)-1,3,5-triazin-2-yl]amino]-2,6-di-tert-butylphenol) (all are trade names; manufactured by BASF Japan Ltd.), tetrakis[3-(3',5'-di-tert-butyl-4'-hydroxyphenyl)propionic acid] and 2,4,8,10-tetraoxaspiro[5,5]undecane-3,9-diylbis(2-methylpropane-2,1 -diyl) bis[3-[3-(tert-butyl)-4-hydroxy-5-methylphenyl]propanoate].

The phenolic antioxidants only need to have a molecular weight of 100 to 20000 and are not limited to those

indicated by formula (15). Examples also include galvinoxyl free radical, 3,3',5,5'-tetra-tert-butyl-4,4'-stilbenequinone and 4-(hexyloxy)-2,3,6-trimethylphenol.

Examples of amine antioxidants include ones including a structure indicated by formula (20) below and having a molecular weight of 100 to 20000.

[Chem. 153]

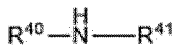

(20)

(In formula (20), $R^{40}$ represents a ring system selected from the group consisting of an aromatic ring, a polycyclic aromatic ring system or an annulated aromatic ring system whose ring-constituting atoms are atoms selected from the group consisting of a carbon atom, a nitrogen atom, an oxygen atom and a sulfur atom, with the aromatic ring, polycyclic aromatic ring system and annulated aromatic ring system optionally having a substituent. $R^{41}$ represents one type selected from the group consisting of a substituted or unsubstituted C1 to C20 alkyl group, aromatic ring, heterocycle, polycyclic aromatic ring system or annulated aromatic ring system, with these optionally having a linkage such as an -O- group, a -(C=O)O- group, an -O(C=O)- group, an -O(C=O)-O- group, a - C(=O)-NH- group, an -NH-(C=O)- group, a -CH=CH- group or a -C=C- group. It is preferred that $R^{41}$ be C9 to C20 because in that case separation and exudation are rare, and improving effects on the molecular orientation of the polymer are expected.)

Specific examples of amine antioxidants indicated by formula (20) include 6-ethoxy-2,2,4-trimethyl-1,2-dihydroquinoline, N-phenyl-1-naphthylamine, N,N'-di-sec-butyl-1,4-phenylenediamine, 4-isopropylaminodiphenylamine, N,N'-diphenyl-1,4-phenylenediamine, N-(1,3-dimethylbutyl)-N'-phenyl N,N'-phenylenediamine, N,N'-di-2-naphthyl-1,4-phenylenediamine and 4,4'-bis($\alpha$,$\alpha$-dimethylbenzyl)diphenylamine.

The amine antioxidants only need to have a molecular weight of 100 to 20000 and are not limited to those indicated by formula (20). Examples also include 6-ethoxy-2,2,4-trimethyl-1,2-dihydroquinoline and poly(2,2,4-trimethyl-1,2-dihydroquinoline).

Examples of phosphorus antioxidants include ones including a structure indicated by formula (21) below and having a molecular weight of 100 to 20000.

[Chem. 154]

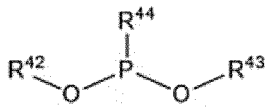

(21)

(In formula (21), $R^{42}$ and $R^{43}$ each independently represent one type selected from the group consisting of a substituted or unsubstituted C1 to C20 alkyl group, aromatic ring, heterocycle, polycyclic aromatic ring system or annulated aromatic ring system, with these optionally having a linkage such as an -O- group, a -(C=O)O- group, an -O(C=O)- group, an -O(C=O)-O- group, a - C(=O)-NH- group, an -NH-(C=O)- group, a -CH=CH- group or a -C=C- group. $R^{44}$ represents one type selected from the group consisting of a substituted or unsubstituted C1 to C20 alkyl group, C1 to C20 alkoxy group, aromatic ring, heterocycle, polycyclic aromatic ring system or annulated aromatic ring system, with these optionally having a linkage such as an -O- group, a -(C=O)O- group, an -O(C=O)- group, an -O(C=O)-O- group, a -C(=O)-NH- group, an -NH-(C=O)-group, a -CH=CH- group or a -C≡C- group. It is preferred that each of $R^{42}$, $R^{43}$ and $R^{44}$ independently be C3 to C20 because in that case separation and exudation are rare, and improving effects on the molecular orientation of the polymer are expected.)

Specific examples of phosphorus antioxidants include trimethyl phosphite, triethyl phosphite, tripropyl phosphite, tributyl phosphite, triphenyl phosphite, trihexyl phosphite, tri-o-cresyl phosphite, tri-m-cresyl phosphite, tri-p-cresyl phosphite, tris(2-ethylhexyl) phosphite, trioctyl phosphite, triisodecyl phosphite, tris(1,1,1,3,3,3-hexafluoro-2-propyl) phosphite, 3,9-bis(2,4-di-tert-butylphenoxy)-2,4,8,10-tetraoxa-3,9-diphosphaspiro[5.5]undecane, tris(2,4-di-tert-butylphenyl) phosphite and trioleyl phosphite.

Examples of sulfur antioxidants include ones including a structure indicated by formula (22) below and having a molecular weight of 100 to 20000.

[Chem. 155]

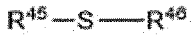

(22)

(In formula (22), $R^{45}$ represents one type selected from the group consisting of a substituted or unsubstituted C1 to C20 alkyl group, heterocycle, aromatic ring, polycyclic aromatic ring system or annulated aromatic ring system, with these optionally having a linkage such as an -O- group, a -(C=O)O- group, an -O(C=O)- group, an -O(C=O)-O- group, a -C(=O)-NH- group, an -NH-(C=O)-group, a -CH=CH- group or a -C≡C- group. $R^{46}$ represents one type selected from the group consisting of a hydrogen atom or a substituted or unsubstituted C1 to C20 alkyl group, aromatic ring, heterocycle, polycyclic aromatic ring system or annulated aromatic ring system, with these optionally having a linkage such as an -O- group, a -(C=O)O- group, an -O(C=O)- group, an -O(C=O)-O- group, a - C(=O)-NH- group, an -NH-(C=O)- group, a -CH=CH- group or a -C=C- group. It is preferred that each of $R^{45}$ and $R^{46}$ independently be C6 to C20 because in that case separation and exudation are rare, and improving effects on the molecular orientation of the polymer are expected.)

Specific examples of sulfur antioxidants include didodecyl 3,3'-thiodipropionate, dioctadecyl 3,3'-thiodipropionate and pentaerythritol tetrakis[3-(dodecylthio)propionate].

Examples of photostabilizers include hindered-amine photostabilizers.

Examples of hindered-amine photostabilizers include ones having a constituent unit indicated by formula (23) below and having a molecular weight of 100 to 20000.

[Chem. 156]

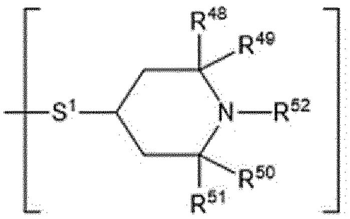

(23)

(In formula (23), $S^1$ represents one type in the group consisting of -O-, -C(=O)- and -NR$^{47}$-. In this context, $R^{47}$ represents one type in the group consisting of a hydrogen atom or a substituted or unsubstituted C1 to C5 alkyl group. $R^{48}$, $R^{49}$, $R^{50}$ and $R^{51}$ each independently represent one type selected from the group consisting of a substituted or unsubstituted C1 to C20 alkyl group, heterocycle, aromatic ring, polycyclic aromatic ring system or annulated aromatic ring system, with these optionally having a linkage such as an -O- group, a - (C=O)O- group, an -O(C=O)- group, an -O(C=O)-O- group, a -C(=O)-NH- group, an -NH-(C=O)- group, a -CH=CH- group or a -C≡C- group. Of these, a methyl group is particularly preferred because of easy availability. $R^{52}$ represents one type selected from the group consisting of a hydrogen atom or a substituted or unsubstituted C1 to C20 alkyl group, C1 to C20 alkoxy group, aromatic ring, heterocycle, polycyclic aromatic ring system or annulated aromatic ring system, with these optionally having a linkage such as an -O- group, a -(C=O)O- group, an -O(C=O)- group, an -O(C=O)-O- group, a -C(=O)-NH- group, an -NH-(C=O)-group, a -CH=CH- group or a -C≡C- group.)

Specific examples of hindered-amine photostabilizers include 2,2,6,6-tetramethyl-4-piperidyl methacrylate, 1,2,2,6,6-pentamethyl-4-piperidyl methacrylate, N,N'-bis(2,2,6,6-tetramethylpiperidin-4-yl)hexane-1,6-diamine, N,N'-bis(2,2,6,6-tetramethyl-4-piperidinyl)isophthalamide, bis(2,2,6,6-tetramethyl-4-piperidyl) sebacate, bis(2,2,6,6-tetramethyl-4-piperidyl-1-oxyl) sebacate, bis(1,2,2,6,6-pentamethyl-4-piperidyl) sebacate and bis(1,2,2,6,6-pentamethyl-4-piperidyl) butyl(3,5-di-tert-butyl-4-hydroxybenzyl)malonate.

Of these thermal reorientation accelerators, plasticizers are particularly preferred because of their easy availability, good compatibility with the polymer and superb thermal reorientation acceleration capability even after incorporation into the polymer.

The resin composition according to the present invention may contain at least one or more types of surfactants for reduced unevenness in the thickness of thin films obtained therewith. Examples of surfactants that can be contained include alkyl carboxylates, alkyl phosphates, alkyl sulfonates, fluoroalkyl carboxylates, fluoroalkyl phosphates, fluoroalkyl sulfonates, polyoxyethylene derivatives, fluoroalkyl ethylene oxide derivatives, polyethylene glycol derivatives, alkyl ammonium salts and fluoroalkyl ammonium salts, with fluorine-containing surfactants being particularly preferred.

The resin composition according to the present invention may contain, for example, other polymers, polymer electrolytes, conductive complexes, pigments, dyes, antistatic agents, anti-blocking agents and lubricants within the gist of the invention.

The resin composition according to the present invention can be obtained by blending a polymer having a photoreactive reverse wavelength dispersion unit that exhibits both functions of photoreactivity and reverse wavelength dispersion of birefringence and a thermal reorientation accelerator (hereinafter referred to as the polymer, etc.).

As for the method for blending, methods such as melt blending and solution blending can be used. Melt blending is a method in which the resin composition is produced by melting the polymer, etc., by heating them and kneading the molten materials. Solution blending is a method in which the polymer, etc., are blended through dissolution in a solvent.

As the solvent used in solution blending, solvents such as halogenated solvents, e.g., 1,1,1,3,3,3-hexafluoroisopropanol, methylene chloride and chloroform; aromatic solvents, e.g., toluene and xylene; ketone solvents, e.g., cyclopentanone, acetone, methyl ethyl ketone and methyl isobutyl ketone; alcohol solvents, e.g., methanol, ethanol and propanol; ether solvents, e.g., dioxane and tetrahydrofuran; and dimethylformamide and N-methylpyrrolidone can be used. It is possible to dissolve the polymer, etc., in solvents and then blending the resulting solutions, and it is also possible to knead powders or pellets, for example, of the polymers and then dissolving the resulting mixture in a solvent. It is possible to put the resulting blended polymer solution into a poor solvent to cause the resin composition to separate out, and it is also possible to use the blended polymer solution directly to produce an optical film.

The polymer according to the present invention and the resin composition according to the present invention can be used as optical films. An optical film comprising the polymer according to the present invention or the resin composition according to the present invention (hereinafter also indicated as "the optical film according to the present invention") is also an aspect of the present invention.

The optical film according to the present invention can meet formula (I) below.

$$Re\ (450) \leq Re\ (550)\ ...\ (I)$$

(In formula (I), Re (450) represents an in-plane retardation value measured at a wavelength of 450 nm, and Re (550) represents an in-plane retardation value measured at a wavelength of 550 nm.)

Meeting formula (I) above is synonymous with meeting formula (II) below in other words.

$$Re\ (450)/Re\ (550) \leq 1\ ...\ (II)$$

(In formula (II), Re (450) represents an in-plane retardation value measured at a wavelength of 450 nm, and Re (550) represents an in-plane retardation value measured at a wavelength of 550 nm.)

The optical film according to the present invention can be used with a thickness that suits its purpose. Preferably, the thickness is from 1 to 20 μm, more preferably 10 μm or less.

For an optical film, in the context of the optical film according to the present invention, made from a polymer according to the present invention at least one end of which has a monocarboxylic acid ester, the yellowness index (YI) at a film thickness of 5 μm is preferably 5% or less, more preferably 3% or less.

The yellowness index (YI) refers to the degree to which the hue deviates from the clear or white of a polymer in the direction toward yellow and is expressed as a positive value. Given the application as an optical film, lower yellowness indices (YI) are preferred.

When the resin composition according to the present invention is used as an optical film, the retardation Re indicated by mathematical expression (X) below as measured using a 589-nm light source increases at high temperatures through light irradiation treatment and heating and firing treatment at 190°C. The retardation Re is preferably from 5 to 400, more preferably from 5 to 300, particularly preferably from 5 to 150.

$$Re = (ny - nx) \times d \quad (X)$$

(In mathematical expression (X), nx indicates the refractive index in the direction along the in-plane fast axis (the direction with the smallest refractive index), ny indicates the refractive index in the direction along the in-plane slow axis (the direction with the largest refractive index), and d indicates the thickness of the film (nm).)

When the resin composition according to the present invention is used as an optical film, the retardation Re indicated by mathematical expression (X) below as measured using a 589-nm light source increases at high temperatures through light irradiation treatment and heating and firing treatment at 150°C. The retardation Re is preferably from 35 to 400, more preferably from 35 to 300, particularly preferably from 35 to 150.

$$Re = (ny - nx) \times d \quad (X)$$

(In mathematical expression (X), nx indicates the refractive index in the direction along the in-plane fast axis (the direction with the smallest refractive index), ny indicates the refractive index in the direction along the in-plane slow axis (the direction with the largest refractive index), and d indicates the thickness of the film (nm).)

The increase in retardation Re at high temperatures, more specifically, means that the retardation Re improves to 1.3 times or greater compared with that prior to the inclusion of the thermal reorientation accelerating material. The

inventors believe that this is because the molecular mobility of the polymer is improved by the thermal reorientation accelerator.

For the method for producing the optical film according to the present invention, there is no specific restriction. Examples include methods such as melt film formation and solution casting.

Specific melt film formation processes include melt extrusion using a T-die, calendering, heat-pressing, coextrusion, comelting, multilayer extrusion and film inflation. The process is not particularly limited.

Solution casting is a method in which a solution in which a polymer has been dissolved in a solvent (Hereinafter referred to as "the casting dope.") is cast onto a support substrate, followed by solvent removal by heating to give a film. Methods used during this process to cast the casting dope onto the support substrate are spin coating, T-die casting, doctor blading, bar coater casting, roller coater casting and lip coater casting. In particular, industrially, T-die casting, in which the casting dope is continuously extruded from a die onto a belt-shaped or drum-shaped support substrate, is the most common. Examples of support substrates used include the glass substrate of a quartz glass substrate, the metal substrates of a stainless-steel substrate and a ferrotype substrate and a film of polyethylene terephthalate.

The optical film according to the present invention may contain at least one or more types of surfactants for reduced unevenness in its thickness. Examples of surfactants include alkyl carboxylates, alkyl phosphates, alkyl sulfonates, fluoroalkyl carboxylates, fluoroalkyl phosphates, fluoroalkyl sulfonates, polyoxyethylene derivatives, fluoroalkyl ethylene oxide derivatives, polyethylene glycol derivatives, alkyl ammonium salts and fluoroalkyl ammonium salts, with the fluorine-containing surfactants of fluoroalkyl carboxylates, fluoroalkyl phosphates, fluoroalkyl sulfonates, fluoroalkyl ethylene oxide derivatives and fluoroalkyl ammonium salts being particularly preferred.

Among optical films according to the present invention, the film is suitable for use as a reverse wavelength dispersion film in particular, because it exhibits retardations with reverse wavelength dispersion properties.

After being made as a film by the method of melt film formation or solution casting, the optical film according to the present invention is preferably irradiated with any one type of ultraviolet radiation out of polarized ultraviolet radiation and obliquely incident ultraviolet radiation irradiation in order for it to exhibit retardations with reverse wavelength dispersion properties.

When ultraviolet radiation is used, the wavelength of the ultraviolet radiation is selected as appropriate from the range of 200 nm to 400 nm. The amount of irradiation energy is preferably from 10 $mJ/cm^2$ or more and 10000 $mJ/cm^2$ or less, particularly preferably 10 $mJ/cm^2$ or more and 1000 $mJ/cm^2$ or less.

The optical film according to the present invention is preferably subjected to heating treatment after the above ultraviolet irradiation in order for it to exhibit retardations with reverse wavelength dispersion more. An example of a temperature for the heating treatment is 50°C or above and 400°C or below.

Subjecting the optical film according to the present invention to irradiation with polarized ultraviolet radiation or obliquely incident ultraviolet radiation and then to heating treatment makes the optical film exhibit three-dimensional anisotropy in refractive index, allowing it to be used as a retardation film.

When the optical film according to the present invention is used as a retardation film, it may be used as a single film or may be used as a multilayer film in which other films have also been stacked. A multilayer film including this optical film is also an aspect of the present invention.

Examples of films stacked include a linear polarizing film, a PET film, a PEN film, a PVA film, a cellulose ester film, such as a TAC film, and a film made from a cycloolefin polymer.

The multilayer film can be used as a retardation film, a polarizing plate, a circular polarizing film or a liquid-crystal alignment film.

[Recapitulation]

[1] A main chain polymer comprising, in a polymer main chain thereof, a photoreactive reverse wavelength dispersion unit that exhibits both functions of photoreactivity and reverse wavelength dispersion of birefringence, wherein the photoreactive reverse wavelength dispersion unit has a structure represented by chemical formula (1) below.

[Chem. 157]

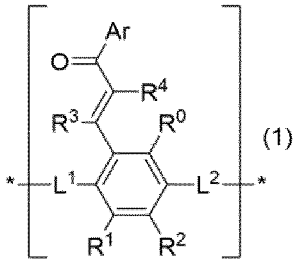

[In chemical formula (1) above, $L^1$ and $L^2$ may be the same or different from each other, each representing a carbonyl group, an ester linkage, an amide linkage, an ether linkage or a single bond.
* represents a site of binding with another structure in the main chain polymer.
Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.
$R^0$, $R^1$, $R^2$, $R^3$ and $R^4$ each independently represent a hydrogen atom, a halogen atom, a C1 to C8 alkyl group or a group represented by chemical formula (Z1) below.]

[Chem. 158]

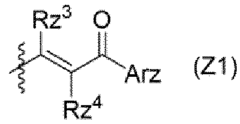

[In chemical formula (Z1) above, $Rz^3$ and $Rz^4$ each independently represent a hydrogen atom, a halogen atom or a C1 to C8 alkyl group.
Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.
A wavy-lined portion represents a site of binding with a portion in formula (1) excluding $R^0$, $R^1$, $R^2$, $R^3$ or $R^4$.]

[2] The main chain polymer according to [1] above, wherein in chemical formula (1), Ar is any of chemical formulae (Ar-1) to (Ar-7) below.

[Chem. 159]

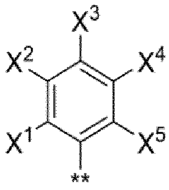 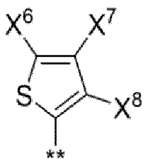 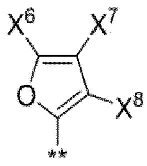 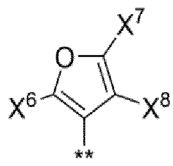 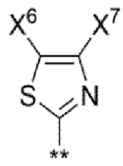 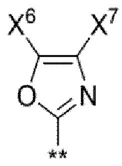

(Ar-1) (Ar-2) (Ar-3) (Ar-4) (Ar-5) (Ar-6)

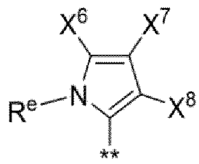

(Ar-7)

[In chemical formulae (Ar-1) to (Ar-7) above, $X^1$, $X^2$, $X^3$, $X^4$, $X^5$, $X^6$, $X^7$ and $X^8$ each independently represent a hydrogen atom or a C1 to C8 alkyl group, a C1 to C6 alkoxy group, a halogen atom, a nitro group, a cyano group, a C1 to C6 alkylthio group or a C2 to C8 dialkylamino group.
$R^e$ represents a hydrogen atom or a C1 to C10 alkyl group.
** represents a site of binding with the other portion in chemical formula (1), excluding Ar.]

[3] The main chain polymer according to [1] above, further comprising at least one type of structure selected from the group consisting of chemical formulae (2A), (2B), (2C) and (2D) below.

[Chem. 160]

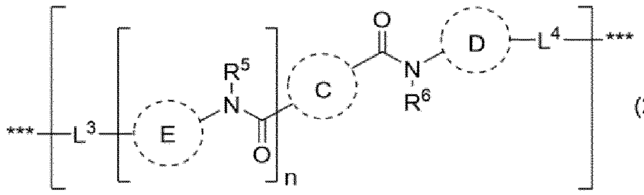 (2A)

[In chemical formula (2A) above, ring C, ring D and ring E each independently represent a ring system selected from the group consisting of a monocyclic aromatic ring system, a polycyclic aromatic ring system, an annulated aromatic ring system and an aliphatic hydrocarbon ring system whose ring-constituting atoms are atoms selected from the group consisting of a carbon atom, a nitrogen atom, an oxygen atom and a sulfur atom, with the monocyclic aromatic ring system, polycyclic aromatic ring system, annulated aromatic ring system and aliphatic hydrocarbon ring system optionally having a substituent.
$R^5$ and $R^6$ may be the same or different from each other, each representing a group selected from the group consisting of a hydrogen atom, a substituted or unsubstituted C1 to C20 alkyl group, a substituted or unsubstituted C3 to C8 cycloalkyl group and a substituted or unsubstituted C3 to C12 aromatic group.
n is 0 or 1.
$L^3$ and $L^4$ may be the same or different from each other, each representing a carbonyl group, an ester linkage, an amide linkage, an ether linkage or a single bond.
*** represents a site of binding with another structure in the main chain polymer.]

[Chem. 161]

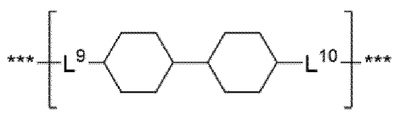 (2B)

[In chemical formula (2B) above, $L^9$ and $L^{10}$ may be the same or different from each other, each representing a carbonyl group, an ester linkage, an amide linkage, an ether linkage or a single bond.
*** represents a site of binding with another structure in the main chain polymer.]

[Chem. 162]

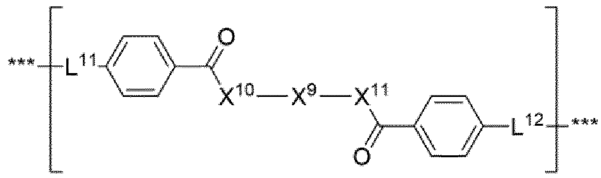

(2C)

[In chemical formula (2C) above, $X^9$ represents a C1 to C10 alkylene chain or a single bond.
$X^{10}$ represents -O- or -N($R^c$)-.
$X^{11}$ represents -O- or -N($R^d$)-.
$R^c$ and $R^d$ may be the same or different from each other, each representing a hydrogen atom or a C1 to C10 alkyl group.
$L^{11}$ and $L^{12}$ may be the same or different from each other, each representing a carbonyl group, an ester linkage, an amide linkage, an ether linkage or a single bond.
*** represents a site of binding with another structure in the main chain polymer.]

[Chem. 163]

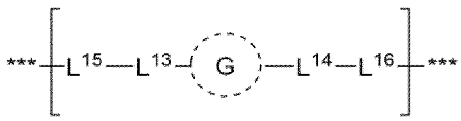

(2D)

[In chemical formula (2D) above, ring G represents a ring system selected from the group consisting of a monocyclic aromatic ring system, a polycyclic aromatic ring system, an annulated aromatic ring system, a spiro ring system and an aliphatic hydrocarbon ring system whose ring-constituting atoms are atoms selected from the group consisting of a carbon atom, a nitrogen atom, an oxygen atom and a sulfur atom, with the monocyclic aromatic ring system, polycyclic aromatic ring system, annulated aromatic ring system, spiro ring system and aliphatic hydrocarbon ring system optionally having a substituent.
$L^{13}$ and $L^{14}$ may be the same or different from each other, each representing a single bond or a C1 to C6 alkylene chain.
$L^{15}$ and $L^{16}$ may be the same or different from each other, each representing a carbonyl group, an ester linkage, an amide linkage, an ether linkage or a single bond.
*** represents a site of binding with another structure in the main chain polymer.]

[4] The main chain polymer according to any one of [1] to [3] above, wherein at least one end of the main chain polymer is a monocarboxylic acid ester represented by chemical formula (2') below.

[Chem. 164]

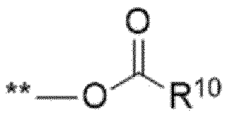

(2')

[In chemical formula (2') above, $R^{10}$ represents a group selected from the group consisting of a substituted or unsubstituted C1 to C20 alkyl group, a substituted or unsubstituted C3 to C8 cycloalkyl group and a substituted or unsubstituted C3 to C12 aromatic group.
** represents a site of binding with another structure in the main chain polymer.]

[5] A resin composition comprising 70% to 99.99% by weight of the main chain polymer according to any one of [1]

to [3] above and 0.01% to 30% by weight of a thermal reorientation accelerator.

[6] A resin composition comprising 70% to 99.99% by weight of the main chain polymer according to [4] above and 0.01% to 30% by weight of a thermal reorientation accelerator.

[7] A method for producing the main chain polymer according to any first of [1] to [3] above, the method comprising polymerizing a source composition containing a dihydroxy compound represented by chemical formula (1') below.

[Chem. 165]

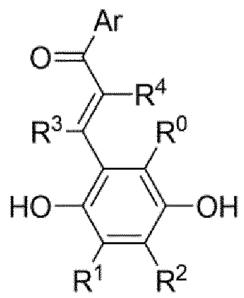

(1')

[In chemical formula (1') above, $R^0$, $R^1$, $R^2$, $R^3$ and $R^4$ each independently represent a hydrogen atom, a halogen atom, a C1 to C8 alkyl group or a group represented by chemical formula (Z1) below. Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.]

[Chem. 166]

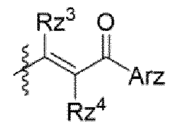

(Z1)

[In chemical formula (Z1) above, $Rz^3$ and $Rz^4$ each independently represent a hydrogen atom, a halogen atom or a C1 to C8 alkyl group.

Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.

A wavy-lined portion represents a site of binding with a portion in formula (1) excluding $R^0$, $R^1$, $R^2$, $R^3$ or $R^4$.]

[8] A method for producing the main chain polymer according to [4] above, the method comprising, when polymerizing a source composition containing a dihydroxy compound represented by chemical formula (1'), further adding a monocarboxylic acid derivative compound represented by any of chemical formula (2-1) or (2-2) below.

[Chem. 167]

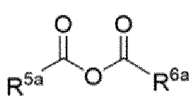

**(2-1)**

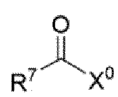

**(2-2)**

[In chemical formula (2-1) or (2-2) above, $R^{5a}$, $R^{6a}$ and $R^7$ each independently represent a group selected from the

group consisting of a substituted or unsubstituted C1 to C20 alkyl group, a substituted or unsubstituted C3 to C8 cycloalkyl group and a substituted or unsubstituted C3 to C12 aromatic group.]

[9] An optical film comprising the main chain polymer according to any one of [1] to [3] above or the resin composition according to [5] above.

[10] An optical film comprising the main chain polymer according to [4] above or the resin composition according to [6] above.

[11] The optical film according to [9] or [10] above, wherein retardations (Re) meet formula (I) below.

$$Re\ (450) \leq Re\ (550)\ ... \qquad (I)$$

(In formula (I), Re (450) represents an in-plane retardation value measured at a wavelength of 450 nm, and Re (550) represents an in-plane retardation value measured at a wavelength of 550 nm.)

[12] The optical film according to [10] above, wherein a yellowness index (YI) at a film thickness of 5 $\mu$m is 5% or less

[13] A method for producing the optical film according to any one of [9] to [12] above, the method comprising irradiation with any one type of ultraviolet radiation out of polarized ultraviolet radiation and obliquely incident ultraviolet radiation irradiation.

[14] The production method according to [13] above, further comprising a heat treatment step.

[15] A multilayer film comprising the optical film according to any one of [9] to [12] above.

[16] A dihydroxy compound represented by chemical formula (1') below.

[Chem. 168]

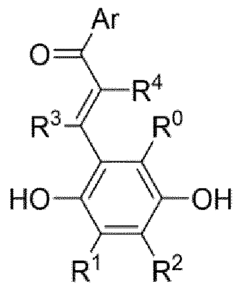

(1')

[In chemical formula (1') above, $R^0$, $R^1$, $R^2$, $R^3$ and $R^4$ each independently represent a hydrogen atom, a halogen atom or a C1 to C8 alkyl group or a group represented by chemical formula (Z1) below. Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.]

[Chem. 169]

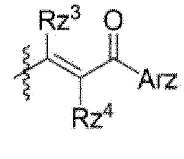

(Z1)

[In chemical formula (Z1) above, $Rz^3$ and $Rz^4$ each independently represent a hydrogen atom, a halogen atom or a C1 to C8 alkyl group.

Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.

A wavy-lined portion represents a site of binding with a portion in formula (1) excluding $R^0$, $R^1$, $R^2$, $R^3$ or $R^4$.]

[17] A method for producing a dihydroxy compound, the method comprising allowing a dihydroxy compound indicated by chemical formula (3) below and a ketone indicated by chemical formula (4') below to react together to give a dihydroxy compound represented by chemical formula (1') below.

[Chem. 170]

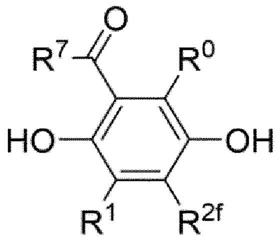

(3)

[In chemical formula (3) above, $R^0$, $R^1$ and $R^7$ each independently represent a hydrogen atom, a halogen atom or a C1 to C8 alkyl group.
$R^{2f}$ represents a hydrogen atom, a halogen atom, a C1 to C8 alkyl group or a formyl group.]

[Chem. 171]

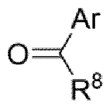

(4')

[In chemical formula (4') above, $R^8$ represents a C1 to C8 alkyl group or a C1 to C6 haloalkyl group.
Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system and annulated aromatic ring system optionally having a substituent.]

[0578] [Chem. 172]

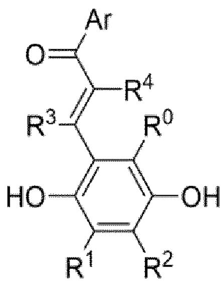

(1')

[In chemical formula (1') above, $R^0$, $R^1$, $R^2$, $R^3$ and $R^4$ each independently represent a hydrogen atom, a halogen atom or a C1 to C8 alkyl group or a group represented by chemical formula (Z1) below. Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.]

[0580] [Chem. 173]

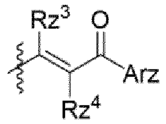

(Z1)

[In formula (Z1) above, $Rz^3$ and $Rz^4$ each independently represent a hydrogen atom, a halogen atom or a C1 to C8 alkyl group.

Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent. A wavy-lined portion represents a site of binding with a portion in formula (1) excluding $R^0$, $R^1$, $R^2$, $R^3$ or $R^4$.]

EXAMPLES

The present invention will now be described in further detail with examples, but the present invention is not to be construed as being limited to these.

Compound (2"-2-1-DC) in the Examples was synthesized according to the method described in Macromolecules, 2018, vol. 51 (23), pp. 9430-9441.

Compound (6'-1-DH) in the Examples was synthesized according to the method described in Japanese Patent No. 5325733.

<Measurement of Nuclear Magnetic Resonance Spectra>

Using a nuclear magnetic resonance spectrometer (manufactured by JEOL Ltd.; trade name: ECZ 400S), the $^1$H-NMR spectrum was measured.

<Measurement of the Thickness of Films>

The measurement of the thickness of films in the Examples was performed using a spectroscopic ellipsometer (manufactured by the J.A. Woollam Company; trade name: RC2-U).

<Evaluation of the Yellowness Index (YI)>

The yellowness indices (YI) in the Examples were determined by attaching a 7-mm diameter beam attachment to a spectral haze meter (manufactured by Nippon Denshoku Industries Co., Ltd.; trade name: SH7000; light source, D65) and conducting measurements according to ASTM E313-05.

The appearance of the films in the Examples was visually inspected. The film appearance was evaluated, with films that exhibited no visible color and were acceptable graded as "A," films that exhibited a visible but only faint color and were acceptable graded as "B" and films that exhibited an intense visible color and were unacceptable graded as "C."

<Polarized Ultraviolet Irradiation>

The polarized ultraviolet irradiation in the Examples was performed using a high-power mercury light source (manufactured by Asahi Spectra Co., Ltd.; trade name: REX-250) equipped with a band-pass filter (248 nm, 313 nm or 365 nm). Only P-polarized light was extracted using a polarizing beam splitter for the corresponding wavelength, and irradiation was conducted.

<Heating Treatment>

The heating treatment for films in the Examples was performed using an anaerobic temperature chamber (manufactured by ESPEC CORP.; trade name: IPHH-202).

<Measurement of Retardation Characteristics (Re)>

For the measurement of retardation characteristics (Re) in the Examples, measurement was performed using light with a wavelength of 589 nm, employing a tilted-sample automatic birefringence tester (manufactured by AXOMETRICS, Inc.; trade name: AxoScan).

$$Re = (ny - nx) \times d \ldots \qquad (III)$$

(In formula (III), nx indicates the refractive index in the direction along the in-film-plane fast axis (the direction with the smallest refractive index), ny indicates the refractive index in the direction along the in-film-plane slow axis (the direction with the largest refractive index), and d indicates the thickness of the film (nm).)

<Measurement of Wavelength Dispersion Characteristics (Re (450)/Re (550))>

The wavelength dispersion characteristics (Re (450)/Re (550)) in the Examples were calculated as a ratio of the in-plane retardation with light having a wavelength of 450 nm, Re (450), and the in-plane retardation with light having a wavelength of 550 nm, Re (550), as measured using a tilted-sample automatic birefringence tester (manufactured by AXOMETRICS, Inc.; trade name: AxoScan).

[Dihydroxy Compound Example 1]

[Chem. 174]

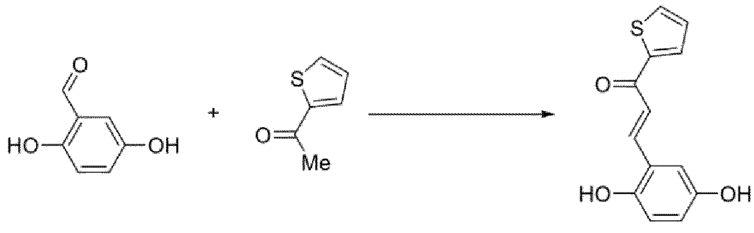

(3-1) (4-1-1) (1'-2-1)

2,5-dihydroxybenzaldehyde (5.00 g, 36.2 mmol) and 2-acetylthiophene (4.57 g, 36.2 mmol) were dissolved in ethanol (36 mL), and then an aqueous solution (7.4 mL) of potassium hydroxide (11.0 g, 195 mmol) was added dropwise with cooling on ice. After the reaction system was stirred at room temperature for 3 hours, acetic acid (13.0 mL, 228 mmol) was added with cooling on ice. The resulting reaction mixture was added to water (100 mL), followed by vigorous stirring with added dichloromethane (30 mL). The solid that formed was collected by filtration and washed with dichloromethane (50 mL) and distilled water (50 mL). The resulting solid was vacuum-dried at 60°C for 5 hours, giving 5.01 g (percent yield: 56%) of compound (1'-2-1) as a yellow solid. $^1$H-NMR (400 MHz, DMSO-$d_6$) $\delta$ 10.0-8.42 (br, 2H), 8.17 (m, 1H), 7.99 (m, 1H), 7.94 (d, J = 15.6 Hz, 1H), 7.62 (d, J = 15.6 Hz, 1H), 7.25 (m, 1H), 7.17 (s, 1H), 6.63-6.76 (m, 2H).

[Dihydroxy Compound Example 2]

[Chem. 175]

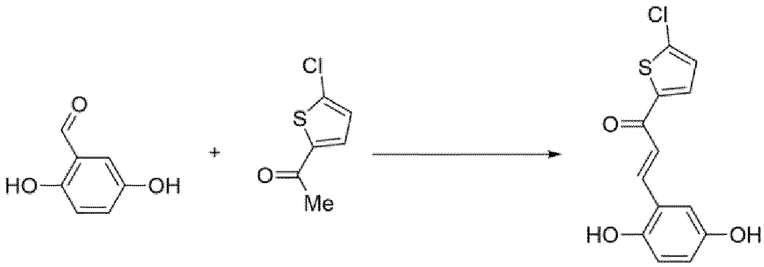

(3-1) (4-1-2) (1'-2-2)

2,5-dihydroxybenzaldehyde (58.1 g, 421 mmol) and 2-acetyl-5-chlorothiophene (67.5 g, 421 mmol) were dissolved in methanol (216 mL), and then a 48% aqueous solution of sodium hydroxide (126 mL, 2.25 mol) was added dropwise with cooling on ice. After the reaction system was stirred at 50°C for 3 hours, acetic acid (168 mL, 2.93 mol) was added with cooling on ice. Water (270 mL), toluene (95 mL) and isopropyl alcohol (13.5 mL) were added to the resulting reaction mixture, and then water (54 mL) was further added. The solid that formed was collected by filtration and washed with water (50 mL) and toluene (270 mL). The resulting solid was suspended in an acetonitrile (235 mL)-methanol (59 mL) solvent mixture, then the system was warmed in an oil bath at 70°C, and stirring was continued for one and a half hours thereafter. The system was cooled again in an ice bath, and the solid that formed in the system was collected by filtration using a Büchner funnel. Subsequently, the solid was washed by passing acetonitrile (270 mL) from the upper portion of the Büchner funnel and then vacuum-dried, giving 81.4 g (percent yield: 69%) of compound (1'-2-2) as yellow microcrystals. $^1$H-NMR (400 MHz, DMSO-$d_6$) δ 9.88-8.62 (br, 2H), 8.12 (d, J = 4.1 Hz, 1H), 7.95 (d, J = 15.8 Hz, 1H), 7.60 (d, J = 15.8 Hz, 1H), 7.31 (d, J = 4.1 Hz, 1H), 7.19 (s, 1H), 6.75-6.68 (m, 2H).

[Dihydroxy Compound Example 3]

[Chem. 176]

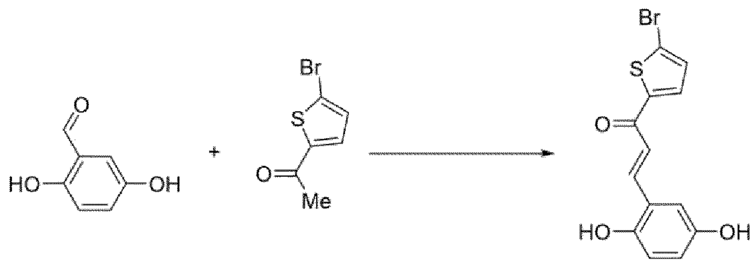

(3-1) (4-1-3) (1'-2-3)

2,5-dihydroxybenzaldehyde (10.0 g, 72.4 mmol) and 2-acetyl-5-bromothiophene (14.8 g, 72.4 mmol) were dissolved in ethanol (72 mL), and then an aqueous (14.8 mL) solution of potassium hydroxide (21.9 g, 391 mmol) was added dropwise with cooling on ice. After the reaction system was stirred at room temperature for 3 hours, acetic acid (26.1 mL, 456 mmol) was added with cooling on ice. The resulting reaction mixture was added to water (200 mL), followed by vigorous stirring with added dichloromethane (60 mL). The solid that formed was collected by filtration and washed with dichloromethane (100 mL) and distilled water (100 mL). The resulting solid was vacuum-dried at 60°C for 5 hours, giving 6.08 g (percent yield: 26%) of compound (1'-2-3) as a yellow solid. $^1$H-NMR (400 MHz, DMSO-$d_6$) δ 9.45 (br, 1H), 8.83 (br, 1H), 7.99 (d, J = 4.6 Hz, 1H), 7.85 (d, J = 15.8 Hz, 1H), 7.55 (d, J = 15.8 Hz, 1H), 7.15 (m, 1H), 6.69 (m, 2H), 6.49 (d, J = 4.6 Hz, 1H).

[Dihydroxy Compound Example 4]

[Chem. 177]

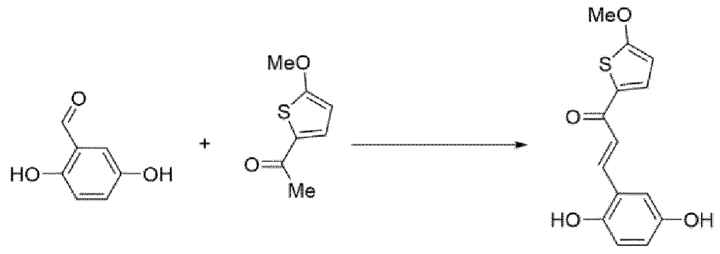

(3-1) (4-1-5) (1'-2-5)

2,5-dihydroxybenzaldehyde (2.00 g, 14.5 mmol) and 2-acetyl-5-methoxythiophene (2.26 g, 14.5 mmol) were dissolved in ethanol (14.4 mL), and then an aqueous (2.8 mL) solution of potassium hydroxide (4.39 g, 78.2 mmol) was added dropwise with cooling on ice. After the reaction system was stirred at room temperature for 3 hours, acetic acid (5.22 mL, 91.2 mmol) was added with cooling on ice. The resulting reaction mixture was added to water (50 mL), followed by vigorous stirring. The solid that formed was collected by filtration and washed with dichloromethane (50 mL) and distilled water (100 mL). The resulting solid was vacuum-dried at 60°C for 5 hours, giving 1.05 g (percent yield: 26%) of compound (1'-2-5) as a yellow solid. $^1$H-NMR (400 MHz, DMSO-$d_6$) $\delta$ 9.46 (br, 1H), 8.85 (br, 1H), 7.99 (d, J = 4.6 Hz, 1H), 7.85 (d, J = 15.8 Hz, 1H), 7.55 (d, J = 15.8 Hz, 1H), 7.15 (m, 1H), 6.70-6.68 (m, 2H), 6.49 (d, J = 4.6 Hz, 1H), 3.94 (s, 3H).

[Dihydroxy Compound Example 5]

[Chem. 178]

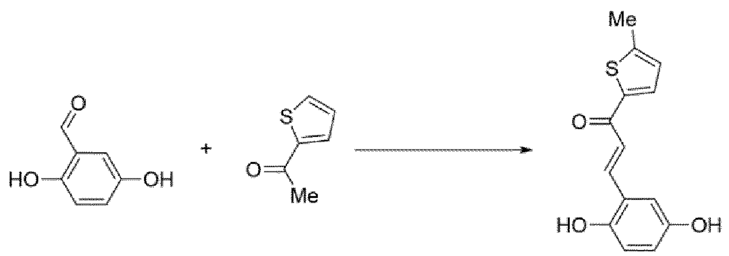

(3-1) (4-1-6) (1'-2-6)

2,5-dihydroxybenzaldehyde (2.00 g, 14.5 mmol) and 2-acetyl-5-methylthiophene (2.03 g, 14.5 mmol) were dissolved in ethanol (14.4 mL), and then an aqueous (2.8 mL) solution of potassium hydroxide (4.39 g, 78.2 mmol) was added dropwise with cooling on ice. After the reaction system was stirred at room temperature for 3 hours, acetic acid (5.22 mL, 91.2 mmol) was added with cooling on ice. The resulting reaction mixture was added to water (50 mL), followed by vigorous stirring. The solid that formed was collected by filtration and washed with dichloromethane (50 mL) and distilled water (100 mL). The resulting solid was vacuum-dried at 60°C for 5 hours, giving 1.66 g (percent yield: 44%) of compound (1'-2-6) as a yellow solid. $^1$H-NMR (400 MHz, DMSO-$d_6$) $\delta$ 9.92-8.58 (br, 2H), 7.99 (d, J = 3.9 Hz, 1H), 7.89 (d, J = 15.8 Hz, 1H), 7.56 (d, J = 15.8 Hz, 1H), 7.14 (m, 1H), 6.96 (d, J = 3.9 Hz, 1H), 6.73-6.67 (m, 2H), 2.49 (s, 3H).

[Dihydroxy Compound Example 6]

[Chem. 179]

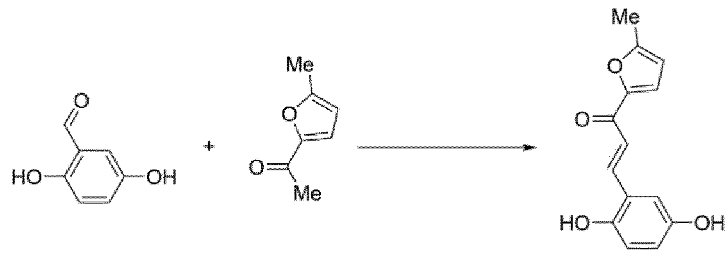

(3-1) (1'-2-6-Furan)

2,5-dihydroxybenzaldehyde (1.00 g, 7.24 mmol) and 2-acetyl-5-methylfuran (899 mg, 7.24 mmol) were dissolved in ethanol (7.2 mL), and then an aqueous (1.4 mL) solution of potassium hydroxide (2.19 g, 39.1 mmol) was added dropwise with cooling on ice. After the reaction system was stirred at room temperature for 3 hours, acetic acid (2.61 mL, 45.6 mmol) was added with cooling on ice. The resulting reaction mixture was added to water (50 mL), followed by vigorous stirring. The solid that formed was collected by filtration and washed with dichloromethane (50 mL) and distilled water (100 mL). The resulting solid was vacuum-dried at 60°C for 5 hours, giving 1.07 g (percent yield: 61%) of compound (1'-2-6-Furan) as a yellow solid. $^1$H-NMR (400 MHz, DMSO-$d_6$) $\delta$ 9.53 (br, 1H), 8.87 (br, 1H), 7.89 (d, J = 16.0 Hz, 1H), 7.57 (d, J = 3.5 Hz, 1H), 7.42 (d, J = 16.0 Hz, 1H), 7.10 (m, 1H), 6.72-6.66 (m, 2H), 6.37 (d, J = 3.5 Hz, 1H), 2.36 (s, 3H).

[Dihydroxy Compound Example 7]

[Chem. 180]

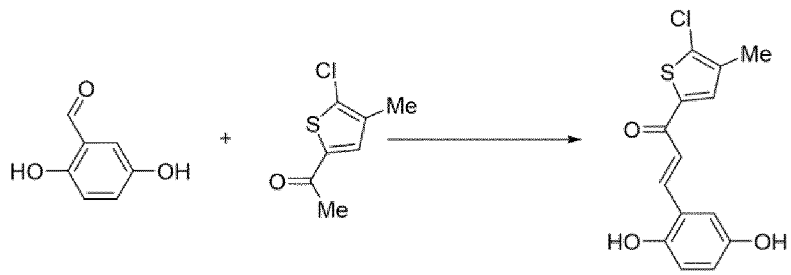

(3-1) (4-1-13) (1'-2-7)

2,5-dihydroxybenzaldehyde (4.49 g, 32.3 mmol) and 2-acetyl-4-methyl-5-chlorothiophene (5.65 g, 32.3 mmol) were dissolved in methanol (16 mL), and then a 48% aqueous solution of sodium hydroxide (10 mL, 180 mmol) was added dropwise with cooling on ice. After the reaction system was stirred at room temperature for 4 hours, acetic acid (17 mL, 297 mmol) was added with cooling on ice. Water (30 mL) was added to the resulting reaction mixture, followed by vigorous stirring. The solid that formed was collected by filtration and washed with hexane (100 mL) and distilled water (100 mL). The resulting solid was vacuum-dried at 50°C for 5 hours, giving 7.51 g (percent yield: 79%) of compound (1'-2-7) as an orange solid. $^1$H-NMR (400 MHz, DMSO-de) $\delta$ 9.60 (br, 1H), 9.02 (br, 1H), 8.16 (s, 1H), 7.98 (d, J = 15.6 Hz, 1H), 7.62 (d, J = 15.6 Hz, 1H), 7.23 (m, 1H), 6.79-6.74 (m, 2H), 2.23 (s, 3H).

[Dihydroxy Compound Example 8]

[Chem. 181]

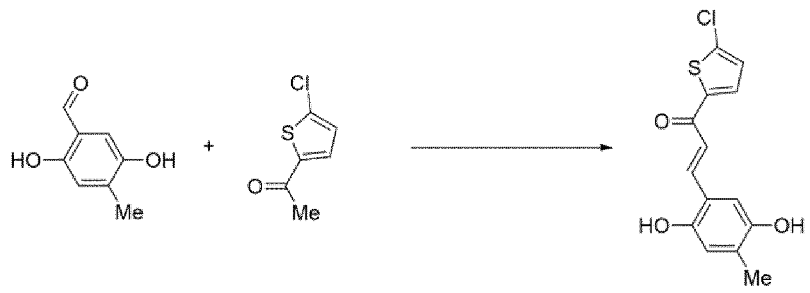

(3-9) (4-1-2) (1'-2-10)

2,5-dihydroxy-4-methylbenzaldehyde (1.84 g, 12.1 mmol) and 2-acetyl-5-chlorothiophene (2.50 g, 15.5 mmol) were dissolved in methanol (16 mL), and then a 48% aqueous solution of sodium hydroxide (3.7 mL, 66.1 mmol) was added dropwise with cooling on ice. After the reaction system was stirred at room temperature for 6.5 hours, acetic acid (4.8 mL, 83.7 mmol) was added with cooling on ice. Dichloromethane (10 mL) and water (34 mL) were added to the resulting reaction mixture, followed by vigorous stirring. The solid that formed was collected by filtration and washed with distilled water (50 mL) and a solution mixture of dichloromethane:methanol (10 mL; ratio by volume: dichloromethane/methanol = 5/1). The resulting solid was vacuum-dried at 50°C for 3 hours, giving 1.67 g (percent yield: 47%) of compound (1'-2-10) as a yellow solid. $^1$H-NMR (400 MHz, DMSO-$d_6$) $\delta$ 9.62 (br, 1H), 8.78 (br, 1H), 8.05 (d, J = 4.2 Hz, 1H), 7.96 (d, J = 15.6 Hz, 1H), 7.51 (d, J = 15.6 Hz, 1H), 7.34 (d, J = 4.2 Hz, 1H), 7.12 (s, 1H), 6.66 (s, 1H), 2.10 (s, 3H).

[Dihydroxy Compound Example 9]

[Chem. 182]

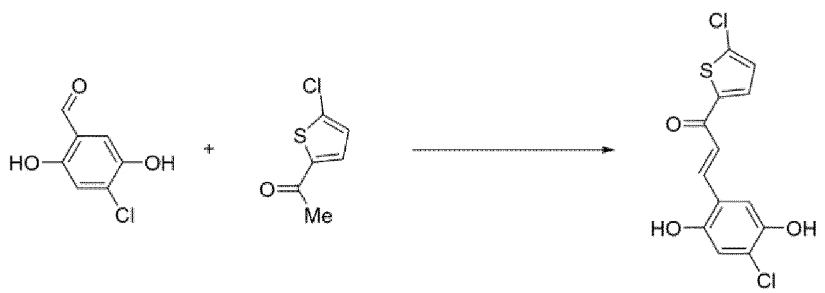

(3-10) (4-1-2) (1'-2-11)

4-chloro-2,5-dihydroxybenzaldehyde (2.59 g, 15.0 mmol) and 2-acetyl-5-chlorothiophene (2.14 g, 15.0 mmol) were dissolved in methanol (15 mL), and then a 48% aqueous solution of sodium hydroxide (4.5 mL, 80.4 mmol) was added dropwise with cooling on ice. After the reaction system was stirred at room temperature for 4 hours, acetic acid (6.0 mL, 105 mmol) was added with cooling on ice. Water (15 mL) and toluene (3.4 mL) were added to the resulting reaction mixture, followed by vigorous stirring. The solid that formed was collected by filtration and washed with distilled water (50 mL) and a solution mixture of dichloromethane:methanol (10 mL, ratio by volume: dichloromethane/methanol = 50/1). The resulting solid was vacuum-dried at 50°C for 3 hours, giving 1.85 g (percent yield: 39%) of compound (1'-2-11) as a yellow solid. $^1$H-NMR (400 MHz, DMSO-$d_6$) $\delta$ 9.95 (br, 1H), 9.61 (br, 1H), 8.11 (d, J = 4.2 Hz, 1H), 7.91 (d, J = 15.7 Hz, 1H), 7.63 (d, J = 15.7 Hz, 1H), 7.37 (s, 1H), 7.36 (d, J = 4.2 Hz, 1H), 6.90 (s, 1H).

[Dihydroxy Compound Example 10]

[Chem. 183]

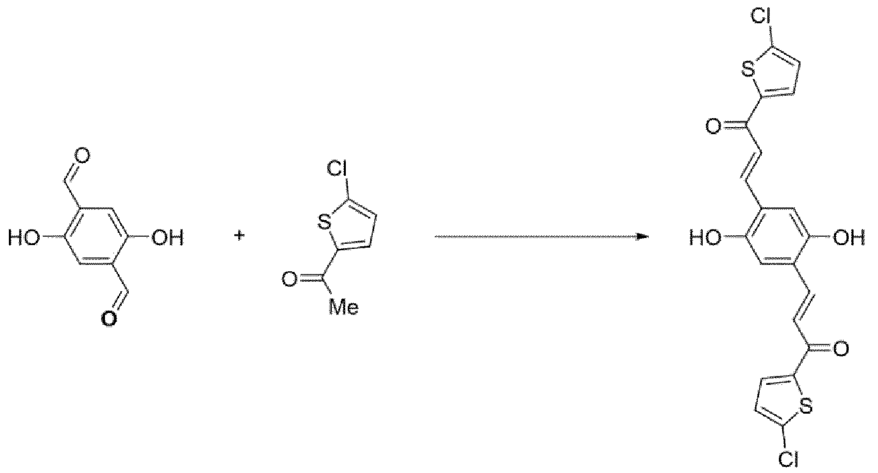

(3-11) (4-1-2) (1'-7-2)

2,5-dihydroxyterephthalaldehyde (1.29 g, 7.74 mmol) and 2-acetyl-5-chlorothiophene (2.49 g, 15.5 mmol) were dissolved in methanol (12 mL), and then a 48% aqueous solution of sodium hydroxide (1.8 mL, 32.1 mmol) was added dropwise with cooling on ice. After the reaction system was stirred at room temperature for 2.5 hours, acetic acid (3.1 mL, 54.1 mmol) was added with cooling on ice. Dichloromethane (6.5 mL) and water (22 mL) were added to the resulting reaction mixture, followed by vigorous stirring. The solid that formed was collected by filtration and washed with distilled water (50 mL) and methanol (6.0 mL). The resulting solid was vacuum-dried at 50°C for 3 hours, giving 2.0 g (percent yield: 57%) of compound (1'-7-2) as a red solid. $^1$H-NMR (400 MHz, DMSO-$d_6$) δ 9.87 (br, 2H), 8.11 (d, J = 4.2 Hz, 2H), 7.96 (d, J = 15.6 Hz, 2H), 7.66 (d, J = 15.6 Hz, 2H), 7.37 (d, J = 4.2 Hz, 2H), 7.30 (s, 2H).

[Dihydroxy Compound Example 11]

[Chem. 184]

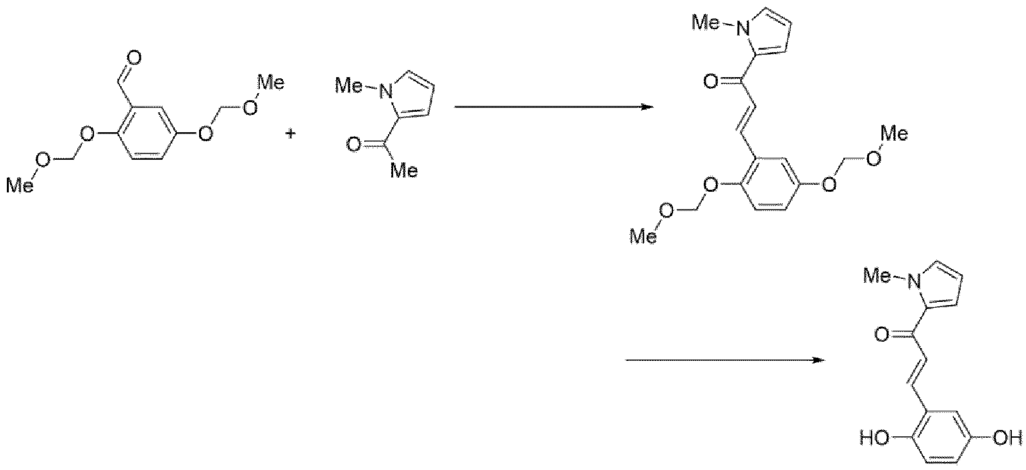

(1'-6-2)

2,5-bis(methoxymethoxy)benzaldehyde (4.53 g, 20.0 mmol) and 2-acetyl-1-methylpyrrole (2.35 mL, 20.0 mmol) were dissolved in methanol (10 mL), and then a 48% aqueous solution of sodium hydroxide (6.1 mL, 110 mmol) was added dropwise with cooling on ice. After the reaction system was stirred at room temperature for 2 hours, the temperature was increased to 50°C, and 2 hours of stirring was performed. After the end of the reaction, methanol (90 mL) and a 10% aqueous solution of hydrochloric acid (50 mL) were added with cooling on ice, and the resulting mixture was stirred at 50°C for 1 hour. The reaction solution was concentrated under reduced pressure, and extraction with ethyl acetate (100 mL × 3) was performed, followed by the washing of the combined organic layer with saturated saline solution (50 mL × 3) and drying with anhydrous sodium sulfate. The solvent was distilled away under reduced pressure, and the resulting crude product was washed with ethyl acetate (2.0 mL) and cold methanol (1.0 mL). The resulting solid was vacuum-dried at 50°C for 3 hours, giving compound (1'-6-2) as a yellow solid (actual yield: 2.11 g; percent yield: 43%). 1H-NMR (400 MHz, DMSO-d6) δ 9.43 (brs, 1H), 8.86 (brs, 1H), 7.85 (d, J = 15.8 Hz, 1H), 7.50 (d, J = 15.8 Hz, 1H), 7.31 (dd, J = 4.1, 1.8 Hz, 1H), 7.18 (t, J = 1.8 Hz, 1H), 7.13 (d, J = 2.7 Hz, 1H), 6.73 (d, J = 8.7 Hz, 1H), 6.69 (dd, J = 8.7, 2.7 Hz, 1H), 6.18 (dd, J = 4.1, 2.5 Hz, 1H), 3.94 (s, 3H).

[Dihydroxy Compound Example 12]

[Chem. 185]

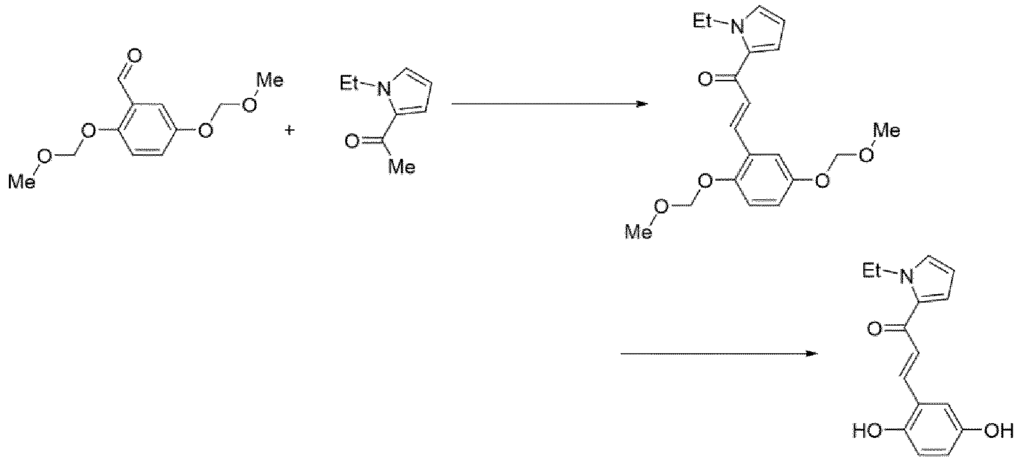

(1'-6-3)

2,5-bis(methoxymethoxy)benzaldehyde (4.53 g, 20.0 mmol) and 2-acetyl-1-ethylpyrrole (2.72 mL, 20.0 mmol) were dissolved in methanol (10 mL), and then a 48% aqueous solution of sodium hydroxide (6.1 mL, 110 mmol) was added dropwise with cooling on ice. After the reaction system was stirred at room temperature for 2 hours, the temperature was increased to 50°C, and 2 hours of stirring was performed. After the end of the reaction, methanol (90 mL) and a 10% aqueous solution of hydrochloric acid (50 mL) were added with cooling on ice, and the resulting mixture was stirred at 50°C for 1 hour. The reaction solution was concentrated under reduced pressure, and extraction with ethyl acetate (100 mL × 3) was performed, followed by the washing of the combined organic layer with saturated saline solution (50 mL × 2) and drying with anhydrous sodium sulfate. The solvent was distilled away under reduced pressure, and the resulting crude product was washed with a solution mixture of hexane:ethyl acetate (2.0 mL; ratio by volume: hexane/ethyl acetate = 1/1) and cold methanol (1.0 mL). The resulting solid was vacuum-dried at 50°C for 3 hours, giving compound (1'-6-3) as a yellow solid (actual yield: 2.02 g; percent yield: 39%). 1H-NMR (400 MHz, DMSO-d6) δ 9.43 (brs, 1H), 8.86 (brs, 1H), 7.85 (d, J = 15.7 Hz, 1H), 7.51 (d, J = 15.7 Hz, 1H), 7.33 (dd, J = 4.1, 1.7 Hz, 1H), 7.25 (dd, J = 2.4, 1.7 Hz, 1H), 7.13 (d, J = 2.8 Hz, 1H), 6.73 (d, J = 8.6 Hz, 1H), 6.69 (dd, J = 8.6, 2.8 Hz, 1H), 6.19 (dd, J = 4.1, 2.4 Hz, 1H), 4.40 (q, J = 7.1 Hz, 2H), 1.29 (t, J = 7.1 Hz, 3H).

[Synthesis Example 1]

[Chem. 186]

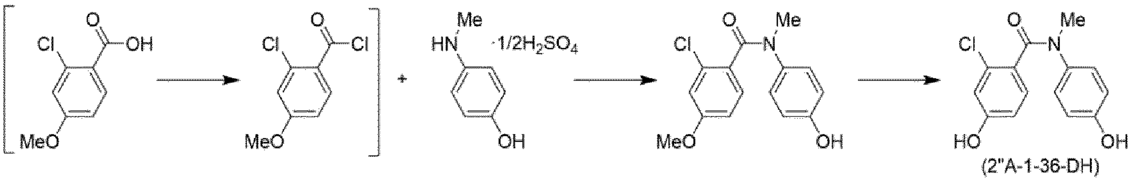

(2"A-1-36-DH)

In an argon atmosphere, thionyl chloride (15.5 mL) and DMF (250 μL) were added to a toluene (25 mL) solution of 2-chloro-4-methoxybenzoic acid (5.00 g, 26.8 mmol). After 1 hour of stirring at 100°C, an excess of thionyl chloride was distilled away under reduced pressure, and THF (45 mL) was added to the resulting residue. This solution was used entirely in the next reaction.

A THF (45 mL)-water (45 mL) solution of 4-(methylamino)phenol sulfate (4.61 g, 13.4 mmol) was cooled to 0°C, sodium hydrogencarbonate (5.63 g, 67.0 mmol) was added with stirring, and then the THF solution of an acid chloride prepared in advance was added dropwise over 5 minutes. This mixture was allowed to warm to room temperature and vigorously stirred for one and a half hours, followed by the addition of concentrated hydrochloric acid (10 mL) and concentration under reduced pressure. The solid that formed in the system was collected by filtration and then washed with water (200 mL) to give a crude solid. This solid was further purified through reprecipitation (hexane: 50 mL; ethyl acetate: 5 mL; ethanol : 3 mL), giving a white solid of 2-chloro-4'-hydroxy-4-methoxy-N-methylbenzanilide (actual yield: 6.42 g; percent yield: 82%). $^1$H-NMR (400 MHz, DMSO-$d_6$): δ 9.50 (brs, 1H), 7.16 (d, J = 8.6 Hz, 1H), 6.99 (d, J = 8.7 Hz, 2H), 6.85 (d, J = 2.4 Hz, 1H), 6.73 (dd, J = 8.6, 2.4 Hz, 1H), 6.56 (d, J = 8.7 Hz, 2H), 3.69 (s, 3H), 3.28 (s, 3H).

In an argon atmosphere, a dichloromethane (170 mL) suspension of 2-chloro-4'-hydroxy-4-methoxy-N-methylbenzanilide (5.00 g, 17.1 mmol) was cooled to 0°C, and then a 1 M dichloromethane solution of boron tribromide (68.4 mL, 68.4 mmol) was added dropwise over 15 minutes. After the reaction system was allowed to warm to room temperature, 13 hours of stirring was performed, and the system was cooled to 0°C again. Ice (400 g) was added, followed by stirring at room temperature until the ice melted. Dichloromethane was distilled away through the concentration of the mixture under reduced pressure, and then extraction with ethyl acetate (200 mL × 3) was performed. The resulting organic layer was dried with anhydrous sodium sulfate and then concentrated under reduced pressure. The resulting residue was purified by silica gel chromatography (hexane:ethyl acetate = 5:95) and then dried under reduced pressure (120°C, a vacuum pump), giving a white solid of compound (2"A-1-36-DH) (actual yield: 3.67 g; percent yield: 77%). $^1$H-NMR (400 MHz, DMSO-$d_6$): δ 10.4-9.15 (br, 2H), 7.02 (m, 1H), 6.95 (d, J = 8.5 Hz, 2H), 6.61 (m, 1H), 6.56 (d, J = 8.5 Hz, 2H), 6.53 (m, 1H), 3.27 (s, 3H).

[Synthesis Example 2]

[Chem. 187]

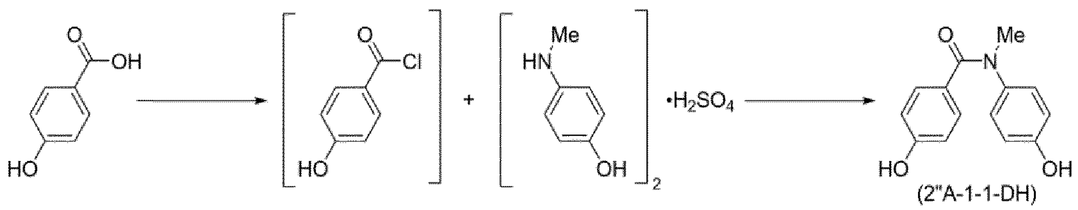

(2"A-1-1-DH)

A mixture of 4-hydroxybenzoic acid (40.1 g, 290 mmol), thionyl chloride (276 g, 2.32 mol) and N,N-dimethylformamide (21.2 mg, 290 μmol) was stirred for 1 hour with heating under reflux, and then an excess of thionyl chloride was distilled away to give 4-hydroxybenzoyl chloride. The resulting 4-hydroxybenzoyl chloride was used entirely in the next reaction after being made into a THF (350 mL) solution.

In a nitrogen atmosphere, a mixture of p-methylaminophenol sulfate (50.0 g, 290 mmol), water (350 mL) and THF (350 mL) was stirred with cooling on ice, and sodium hydrogencarbonate (122 g, 1.45 mol) was added over 5 minutes at the same time. Subsequently, the THF solution of 4-hydroxybenzoyl chloride prepared in the foregoing was added dropwise over 5 minutes. After the dropwise addition, the resulting mixture was stirred for 15 hours while being allowed to gradually warm to room temperature, and then THF was distilled away under reduced pressure. The solid that formed was collected by filtration and washed with water (1 L) and then washed with 2 M hydrochloric acid (600

mL) and acetonitrile (200 mL), giving compound (2"A-1-1-DH) as a white solid (actual yield: 44.3 g; percent yield: 62.7%). $^1$H-NMR (400 MHz, DMSO-$d_6$): δ 9.97-9.22 (br, 2H), 7.03 (d, J = 8.7 Hz, 2H), 6.86 (d, J = 8.7 Hz, 2H), 6.58 (d, J = 8.7 Hz, 2H), 6.50 (d, J = 8.7 Hz, 2H), 3.20 (s, 3H).

[Synthesis Example 3]

[Chem. 188]

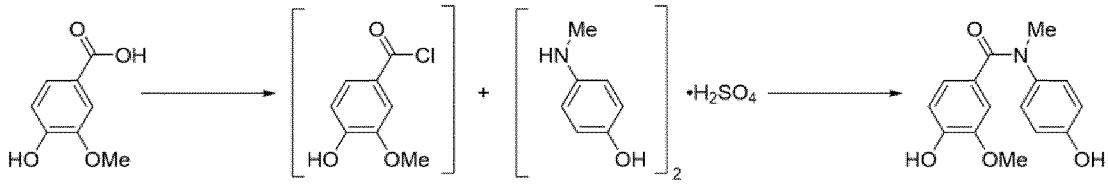


(2"A-1-6-DH)

A mixture of vanillic acid (5.0 g, 29.7 mmol), thionyl chloride (28.3 g, 238 mol) and N,N-dimethylformamide (2.17 mg, 29.7 μmol) was stirred for 1 hour with heating under reflux, and then thionyl chloride was distilled away to give vanillyl chloride. The resulting vanillyl chloride was used entirely in the next reaction after being made into a THF (35 mL) solution.

In a nitrogen atmosphere, a mixture of p-methylaminophenol sulfate (5.12 g, 29.7 mmol), water (35 mL) and THF (35 mL) was stirred with cooling on ice, and sodium hydrogencarbonate (12.5 g, 149 mol) was added over 5 minutes at the same time. Subsequently, the THF solution of vanillyl chloride prepared in the foregoing was added dropwise over 5 minutes. After the dropwise addition, the resulting mixture was stirred for 15 hours while being allowed to gradually warm to room temperature, and then THF was distilled away under reduced pressure. The resulting mixture was subjected to extraction with ethyl acetate (50 mL × 3), and the organic layer was washed with 2 M hydrochloric acid (200 mL) and water (200 mL), followed by drying with anhydrous sodium sulfate. The resulting organic layer was concentrated under reduced pressure, giving compound (2"A-1-6-DH) as a white solid (actual yield: 4.90 g; percent yield: 60%). $^1$H-NMR (400 MHz, DMSO-$d_6$): δ 9.60-9.09 (br, 2H), 6.90-6.84 (m, 2H), 6.70-6.62 (m, 2H), 6.60-6.54 (m, 2H), 6.52 (m, 1H), 3.51 (s, 3H), 3.22 (s, 3H).

[Synthesis Example 4]

[Chem. 189]

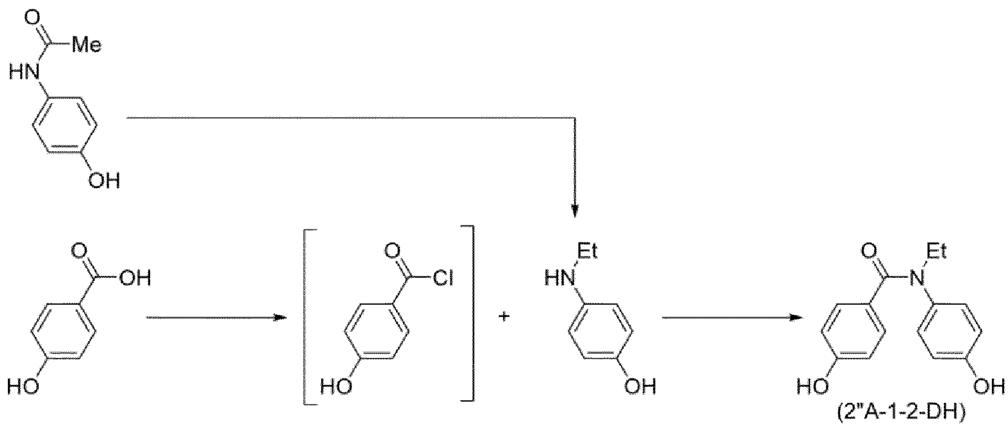

(2"A-1-2-DH)

In a nitrogen atmosphere, 4'-hydroxyacetanilide (3.85 g, 25.5 mmol) was cooled on ice, and then a 1 mol/L THF solution of borane-THF complex (100 mL, 100 mmol) was slowly added. The reaction system was allowed to warm to room temperature and then heated and stirred at 60°C for 24 hours. After the end of the reaction, the system was cooled in an ice bath, and methanol (21 mL) was added over 5 minutes. Water (100 mL) was added to the resulting

reaction mixture, then THF was distilled away using an evaporator, and extraction with dichloromethane (100 mL × 3) was performed. The resulting organic layer was dried with sodium sulfate and then concentrated under reduced pressure. The resulting composition was purified by silica gel column chromatography (ethyl acetate/hexane = 61/39 Vol%), giving 4-ethylaminophenol as a brown solid (actual yield: 2.07 g; percent yield: 59%). $^1$H-NMR (400 MHz, $CDCl_3$): δ 6.69 (d, J = 8.9 Hz, 2H), 6.53 (d, J = 8.9 Hz, 2H), 4.60-3.20 (br, 2H), 3.09 (q, J = 7.2 Hz, 2H), 1.22 (t, J = 7.2 Hz, 3H).

A mixture of 4-hydroxybenzoic acid (2.01 g, 29.7 mmol), thionyl chloride (13.9 g, 117 mmol) and N,N-dimethylformamide (1.07 mg, 14.6 μmol) was stirred for 1 hour with heating under reflux, and then thionyl chloride was distilled away to give 4-hydroxybenzoyl chloride. The resulting 4-hydroxybenzoyl chloride was used entirely in the next reaction after being made into a THF (13 mL) solution.

In a nitrogen atmosphere, a mixture of 4-ethylaminophenol (2.00 g, 14.6 mmol), water (13 mL) and THF (13 mL) was stirred with cooling on ice, and sodium hydrogencarbonate (6.12 g, 72.9 mmol) was added over 5 minutes at the same time. Subsequently, the THF solution of 4-hydroxybenzoyl chloride prepared in the foregoing was added dropwise over 5 minutes. After the dropwise addition, the resulting mixture was stirred for 15 hours while being allowed to gradually warm to room temperature, and then THF was distilled away under reduced pressure. The resulting mixture was subjected to extraction with ethyl acetate (50 mL × 3), and the organic layer was washed with 2 N hydrochloric acid (100 mL × 2) and water (100 mL), followed by drying with anhydrous sodium sulfate. The resulting organic layer was concentrated under reduced pressure, giving compound (2"A-1-2-DH) as a white solid (actual yield: 1.99 g; percent yield: 53%). $^1$H-NMR (400 MHz, DMSO-$d_6$): δ 8.85-10.12(2H), 7.02 (d, J = 8.7 Hz, 2H), 6.83 (d, J = 8.7 Hz, 2H), 6.59 (d, J = 8.7 Hz, 2H), 6.49 (d, J = 8.7 Hz, 2H), 3.70 (q, J = 7.2 Hz, 2H), 1.01 (t, J = 7.2 Hz, 3H).

[Synthesis Example 5]

[Chem. 190]

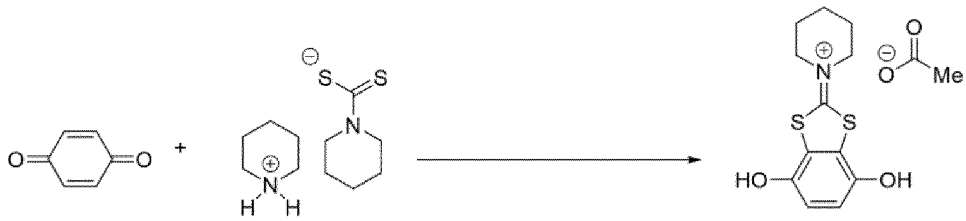

(6C)

With stirring, an NMP (32.4 mL)-acetic acid (40.5 mL) solution of 1,4-benzoquinone (16.2 g, 150 mmol) was gradually added to an NMP (40.5 mL) solution of piperidinium pentamethylenedithiocarbamate (18.5 g, 75 mmol) at 15°C. The reaction system was warmed to 50°C and stirred for 2 hours. After it was confirmed that a solid formed in the reaction system, the system was cooled to room temperature, acetone (100 mL) was added. The solid in the system was collected by filtration, washed with an acetone (50 mL)-acetic acid (50 mL) solvent mixture and then dried under reduced pressure, giving 10.2 g (percent yield: 20.7%) of compound (6C) as a yellow solid. $^1$H-NMR (400 MHz, CDsOD): δ 6.83 (s, 2H), 3.99-3.97 (m, 4H), 1.98-1.92 (m, 7H), 1.84-1.82 (m, 2H).

[Synthesis Example 6]

[Chem. 191]

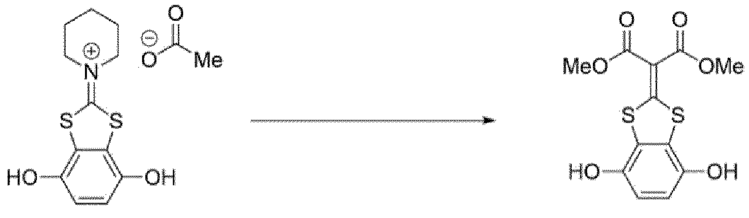

(6C) (6'-2-DH)

In a nitrogen atmosphere, dimethyl malonate (1.5 g, 11.3 mol) was added, at room temperature, to a DMF (15 mL) solution of compound (6C) (3.7 g, 11.3 mmol), obtained in Synthesis Example 5. The reaction system was heated to 100°C with stirring, and stirring was continued for 6 hours in that state. Subsequently, the reaction system was cooled to room temperature, and then water (100 mL) was added. The mixture was subjected to extraction with ethyl acetate (100 mL × 2 times), and the combined organic layer was washed with 1 N hydrochloric acid (100 mL) and saturated saline solution (50 mL). The organic layer was dried with sodium sulfate and then dried under reduced pressure to give a partially purified product. This partially purified product was purified by silica gel column chromatography (methanol:dichloromethane = 3:97 to 4:96), giving 950 mg (percent yield: 4%) of compound (6'-2-DH) as a light brown solid. $^1$H-NMR (400 MHz, DMSO-d6): δ 10.09 (s, 2H), 6.70 (s, 2H), 3.77 (s, 6H).

[Synthesis Example 7]

[Chem. 192]

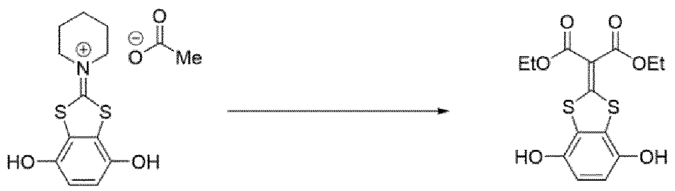

(6C) (6'-3-DH)

In a nitrogen atmosphere, diethyl malonate (1.2 g, 7.40 mmol) was added, at room temperature, to a DMF (24 mL) solution of compound (6C) (2.4 g, 7.40 mmol), obtained in Synthesis Example 5. The reaction system was heated to 100°C with stirring, and stirring was continued for 6 hours in that state. Subsequently, the reaction system was cooled to room temperature, and then water (100 mL) was added. The mixture was subjected to extraction with ethyl acetate (100 mL × 2 times), and the combined organic layer was washed with 1 N hydrochloric acid (100 mL) and saturated saline solution (50 mL). The organic layer was dried with sodium sulfate and then dried under reduced pressure to give a partially purified product. This partially purified product was purified by silica gel column chromatography (methanol:dichloromethane = 3:97 to 4:96), giving 470 mg (percent yield: 18%) of compound (6'-3-DH) as a light brown solid. $^1$H-NMR (400 MHz, $CDCl_3$). δ 10.08 (s, 2H), 6.69 (s, 2H), 4.22 (q, J = 7.2 Hz, 4H), 1.27 (t, J = 7.2 Hz, 6H).

[Synthesis Example 8]

[Chem. 193]

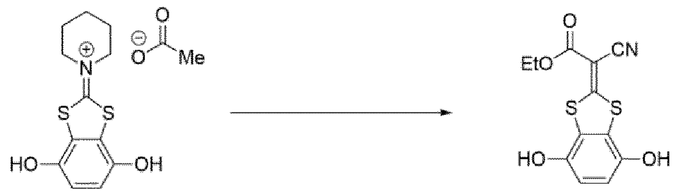

(6C) (6'-5-DH)

In a nitrogen atmosphere, ethyl cyanoacetate (1.5 g, 13.2 mmol) was added, at room temperature, to a DMF (30 mL) solution of compound (6C) (4.33 g, 13.2 mmol), obtained in Synthesis Example 5. The reaction system was heated to 100°C with stirring, and stirring was continued for 6 hours in that state. Subsequently, the reaction system was cooled to room temperature, and then water (100 mL) was added. The mixture was subjected to extraction with ethyl acetate (100 mL × 2 times), and the combined organic layer was washed with 1 N hydrochloric acid (100 mL) and saturated saline solution (50 mL). The organic layer was dried with sodium sulfate and then dried under reduced pressure to give a partially purified product. This partially purified product was purified by silica gel column chromatography (methanol:dichloromethane = 3:97 to 4:96), giving 950 mg (percent yield: 24%) of compound (6'-5-DH) as a light brown solid. 1H-NMR (400 MHz, DMSO-d6): δ 10.39 (s, 1H), 10.34 (s, 1H), 6.78 (s, 2H), 4.25 (q, J = 7.2 Hz, 2H), 1.28 (t, J = 7.2 Hz, 3H).

[Synthesis Example 9]

[Chem. 194]

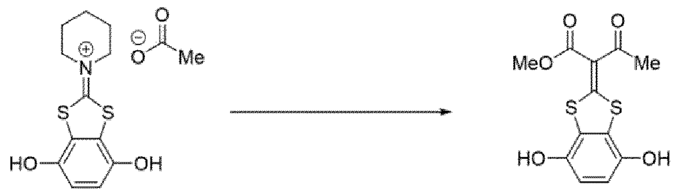

(6C) (6'-7-DH)

In a nitrogen atmosphere, methyl acetoacetate (1.5 g, 12.9 mmol) was added, at room temperature, to a DMF (15 mL) solution of compound (6C) (4.22 g, 12.9 mmol), obtained in Synthesis Example 5. The reaction system was heated to 100°C with stirring, and stirring was continued for 6 hours in that state. Subsequently, the reaction system was cooled to room temperature, and then water (100 mL) was added. The mixture was subjected to extraction with ethyl acetate (100 mL × 2 times), and the combined organic layer was washed with 1 N hydrochloric acid (100 mL) and saturated saline solution (50 mL). The organic layer was dried with sodium sulfate and then dried under reduced pressure to give a partially purified product. This partially purified product was purified by silica gel column chromatography (methanol:dichloromethane = 3:97 to 4:96), giving 940 mg (percent yield: 25%) of compound (6'-7-DH) as a light brown solid. 1H-NMR (400 MHz, DMSO-d6): δ 10.14 (s, 1H), 10.12 (s, 1H), 6.74 (s, 2H), 3.85 (s, 3H), 2.50 (s, 3H).

[Synthesis Example 10]

[Chem. 195]

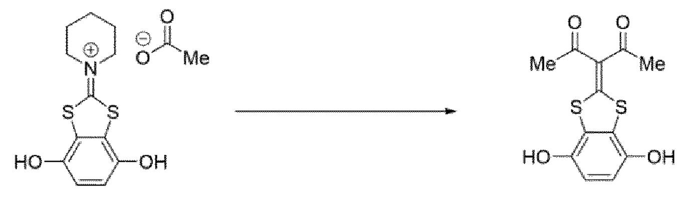

(6C) (6'-6-DH)

In a nitrogen atmosphere, acetylacetone (1.5 g, 14.9 mmol) was added, at room temperature, to a DMF (30 mL) solution of compound (6C) (4.89 g, 14.9 mmol), obtained in Synthesis Example 5. The reaction system was heated to 100°C with stirring, and stirring was continued for 8 hours in that state. Subsequently, the reaction system was cooled to room temperature, and then water (100 mL) was added. The mixture was subjected to extraction with ethyl acetate (100 mL × 2 times), and the combined organic layer was washed with 1 N hydrochloric acid (100 mL) and saturated saline solution (50 mL). The organic layer was dried with sodium sulfate and then dried under reduced pressure to give a partially purified product. This partially purified product was purified by silica gel column chromatography (methanol:dichloromethane = 3:97 to 4:96), giving 940 mg (percent yield: 22%) of compound (6'-6-DH) as a light brown solid. 1H-NMR (400 MHz, DMSO-d6): δ 10.11 (s, 2H), 6.74 (s, 2H), 2.61 (s, 6H).

[Synthesis Example 11]

[Chem. 196]

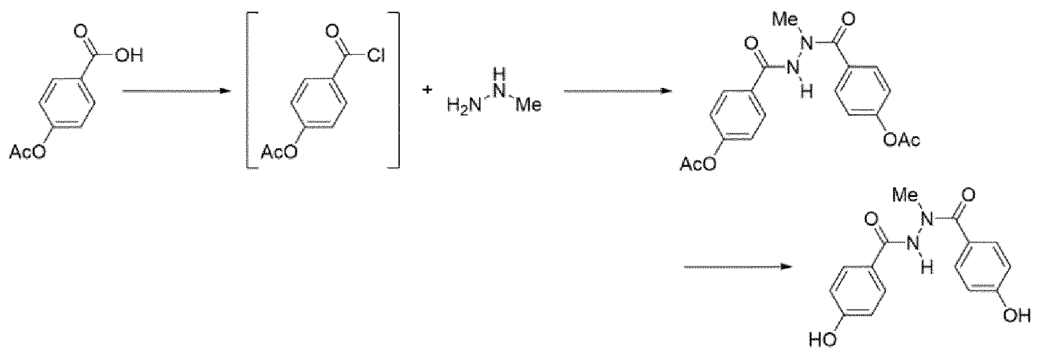

(2"C-2-2)

In an argon atmosphere, a mixture of 4-acetoxybenzoic acid (7.56 g, 42.0 mmol), thionyl chloride (24.0 mL, 331 mmol) and N,N-dimethylformamide (catalytic amount) was boiled under reflux for 3 hours. Volatile components were distilled away, and the residue was azeotropically distilled with toluene (3 × 20 mL) to give 4-acetoxybenzoyl chloride. The resulting 4-acetoxybenzoyl chloride was used entirely in the next reaction after being made into a tetrahydrofuran (50 mL) solution.

In an argon atmosphere, methyl hydrazine (1.05 mL, 19.8 mmol) and triethylamine (8.40 mL, 60.3 mmol) were dissolved in tetrahydrofuran (40 mL). With cooling on ice, the tetrahydrofuran solution of 4-acetoxybenzoyl chloride prepared in advance was slowly added. The resulting mixture was allowed to warm to room temperature and stirred overnight. The solvent was distilled away under reduced pressure, 1 M hydrochloric acid (100 mL) was added, and extraction with ethyl acetate (2 × 100 mL) was performed. The combined organic layer was washed sequentially with a saturated aqueous solution of sodium hydrogencarbonate (100 mL) and saturated saline solution (100 mL) and dried with anhydrous sodium sulfate, followed by concentration under reduced pressure. The residue was suspended in methanol (75 mL), and the resulting suspension was stirred at room temperature for 1 hour with an added aqueous (25 mL) solution of sodium hydroxide (4.13 g, 103 mmol). The solvent was distilled away under reduced pressure, 2 M hydrochloric acid (75 mL) was added, and extraction with ethyl acetate (2 × 100 mL) was performed. The combined organic layer was washed sequentially with water (100 mL), a saturated aqueous solution of sodium hydrogencarbonate (2 × 100 mL) and saturated saline solution (100 mL), followed by drying with anhydrous sodium sulfate. The precipitated solid was collected by filtration and washed with ethyl acetate and water, giving a white solid of compound (2"C-2-2).

The organic layer in the filtrate was isolated and concentrated under reduced pressure, the residue was purified by silica gel column chromatography, and the resulting solid was washed in diethyl ether (50 mL) for the removal of slurry, giving a white solid of compound (2"C-2-2). The combined actual yield was 3.02 g, and the percent yield was 53%. $^1$H-NMR (400 MHz, DMSO-$d_6$): δ 10.76 (brs, 1H), 10.12 (brs, 1H), 9.81 (brs, 1H), 7.66-7.46 (brm, 2H), 7.46-7.36 (m, 2H), 6.82-6.73 (m, 2H), 6.73-6.60 (brm, 2H), 3.15 (s, 3H).

[Synthesis Example 12]

[Chem. 197]

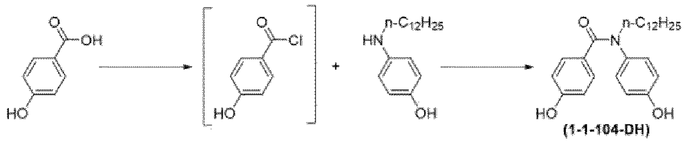

(1-1-104-DH)

In an argon atmosphere, a mixture of 4-hydroxybenzoic acid (2.49 g, 18.0 mmol), toluene (18 mL), thionyl chloride (10.5 mL, 145 mmol) and N,N-dimethylformamide (catalytic amount) was boiled under reflux for 1 hour. Volatile components were distilled away, and the residue was azeotropically distilled with toluene (3 × 15 mL) to give 4-hydroxybenzoyl chloride. The resulting 4-hydroxybenzoyl chloride was used entirely in the next reaction after being made into a tetrahydrofuran (18 mL) solution.

In an argon atmosphere, 4-(dodecylamino)phenol (4.16 g, 15.0 mmol) and sodium hydrogencarbonate (2.52 g, 30.0 mmol) were suspended in a solvent mixture of tetrahydrofuran (17 mL)/water (17 mL). With cooling on ice, the tetrahydrofuran solution of 4-hydroxybenzoyl chloride prepared in advance was slowly added. The resulting mixture was allowed to warm to room temperature and stirred for 4 hours. The solvent was distilled away under reduced pressure, water (50 mL) was added, and extraction with ethyl acetate (3 × 50 mL) was performed. The combined organic layer was washed with a saturated aqueous solution of sodium hydrogencarbonate (80 mL), 2 M hydrochloric acid (80 mL) and saturated saline solution (80 mL) and dried with anhydrous sodium sulfate, followed by concentration under reduced pressure. The residue was dissolved in methanol (50 mL), and the resulting solution was stirred at room temperature for 1 hour with an added aqueous (10 mL) solution of sodium hydroxide (1.21 g, 30.3 mmol) and then concentrated under reduced pressure. Water (35 mL) was added to the residue, the pH was adjusted to 1 with concentrated hydrochloric acid, and extraction with ethyl acetate (3 × 50 mL) was performed. The combined organic layer was washed with a saturated aqueous solution of sodium hydrogencarbonate (100 mL) and saturated saline solution (100 mL) and dried with anhydrous sodium sulfate, followed by concentration under reduced pressure. The residue was purified by silica gel column chromatography, and then the resulting solid was washed in a solvent mixture of toluene (15 mL)/hexane (15 mL) for the removal of slurry, giving a grayish white solid (actual yield: 4.74 g; percent yield: 80%) of compound (1-1-104-DH). 1H-NMR (400 MHz, DMSO-$d_6$): δ 9.69 (brs, 1H), 9.47 (brs, 1H), 7.10-7.00 (m, 2H), 6.92-6.80 (m, 2H), 6.68-6.58 (m, 2H), 6.58-6.48 (m, 2H), 3.69 (t, J = 7.5 Hz, 2H), 1.53-1.36 (m, 2H), 1.34-1.12 (m, 18H), 0.85 (t, J = 6.8 Hz, 3H).

[Synthesis Example 13]

[Chem. 198]

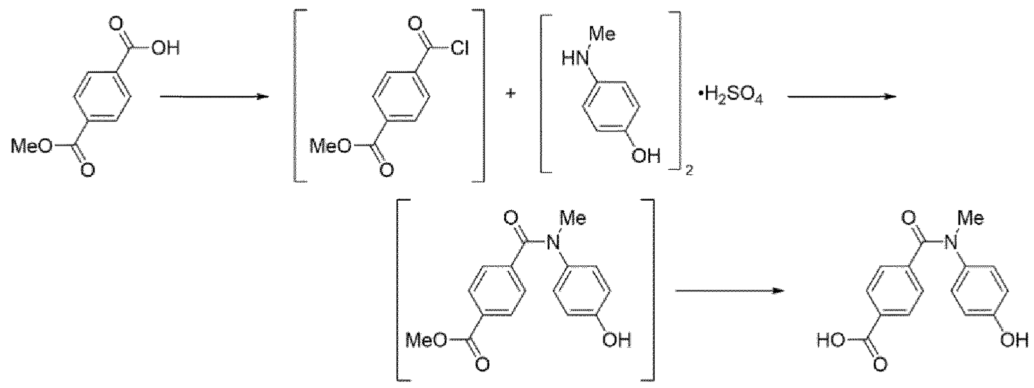

(2"A-1-1-CH)

A mixture of monomethyl terephthalate (9.19 g, 50.0 mmol), thionyl chloride (59.53 g, 0.50 mol) and toluene (50 mL) was stirred for 1 hour with heating under reflux, and then thionyl chloride was distilled away to give methyl 4-(chloroformyl)benzoate. The resulting methyl 4-(chloroformyl)benzoate was used entirely in the next reaction after being made into a THF (50 mL) solution.

A solution mixture of p-methylaminophenol sulfate (8.44 g, 24.5 mmol), sodium hydrogencarbonate (12.60 g, 0.15 mol), THF (50 mL) and water (50 mL) was cooled to 0°C, and the THF (50 mL) solution of methyl 4-(chloroformyl)benzoate prepared in advance was added dropwise over 15 minutes. Then the resulting mixture was allowed to warm to room temperature and vigorously stirred for 12 hours. After the end of the reaction, the solvent was concentrated under reduced pressure, and the solid that formed in the system was collected by filtration and then washed with a saturated aqueous solution of sodium carbonate (50 mL) and water (50 mL), giving a white crude solid (actual crude yield: 13.53 g; percent crude yield: 97%).

Methanol (50 mL) was added to the resulting crude solid, and the resulting mixture was stirred at room temperature for 4 hours with an added 48% aqueous solution of sodium hydroxide (6.0 mL, 0.11 mol). After the end of the reaction, water (50 mL) was added, and undissolved residue was isolated by filtration. Concentrated hydrochloric acid was added to the filtrate to make pH = 2, then the solid that formed was collected by filtration, and reprecipitation from ethyl acetate/hexane was performed, giving a white solid of compound (2"A-1-1-CH) (actual yield: 11.67 g; total percent yield: 83%). $^1$H-NMR (400 MHz, (DMSO-$d_6$: δ 13.01 (brs, 1H), 9.53 (s, 1H), 7.74 (d, J = 7.3 Hz, 2H), 7.32 (d, J = 7.3 Hz, 2H), 6.96 (d, J = 7.8 Hz, 2H), 6.60 (d, J = 7.8 Hz, 2H), 3.31 (s, 3H).

[Synthesis Example 14]

[Chem. 199]

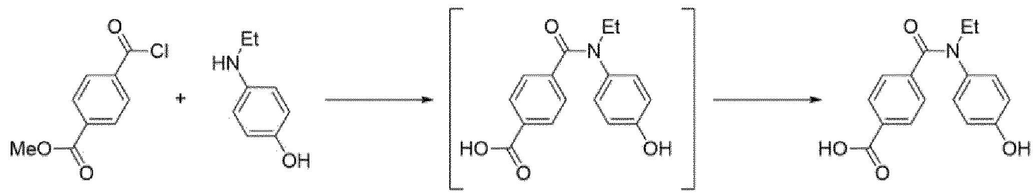

(2"A-1-2-CH)

A solution mixture of 4-(ethylamino)phenol (4.84 g, 25.0 mmol), sodium hydrogencarbonate (4.21 g, 50.1 mmol), THF (30 mL) and water (60 mL) was cooled to 0°C, and a THF (30 mL) solution of methyl 4-(chloroformyl)benzoate (5.24 g, 25.1 mmol) was added dropwise over 15 minutes. Then the resulting mixture was allowed to warm to room temperature and vigorously stirred for 12 hours. After the end of the reaction, the solvent was concentrated under reduced pressure, and the solid that formed in the system was collected by filtration and then washed with a saturated aqueous solution of sodium carbonate (50 mL) and water (50 mL), giving a brown crude solid (actual crude yield, 7.93 g; actual percent yield, 89%).

The resulting crude solid was cooled to 0°C with added methanol (80 mL) and water (80 mL), and then a 48% aqueous solution of sodium hydroxide (4.0 mL, 72.3 mmol) was added. Thereafter, the reaction system was allowed to warm to room temperature and heated under reflux for 4 hours. After the end of the reaction, the system was allowed

to cool to room temperature and then concentrated under reduced pressure. Concentrated hydrochloric acid was added to the residue to make pH = 2, and then the solid that formed was collected by filtration and dried under reduced pressure, giving a brown solid (actual yield: 6.93 g; total percent yield, 97%) of compound (2"A-1-2-CH). 1 H-NMR (400 MHz, DMSO-$d_6$): δ 12.99 (brs, 1H), 9.52 (s, 1H), 7.73 (d, J = 7.8 Hz, 2H), 7.31 (d, J = 7.8 Hz, 2H), 6.92 (d, J = 8.2 Hz, 2H), 6.60 (d, J = 8.2 Hz, 2H), 3.80 (q, J = 7.0 Hz, 2H), 1.09 (t, J = 7.0 Hz, 3H).

[Synthesis Example 15]

[Chem. 200]

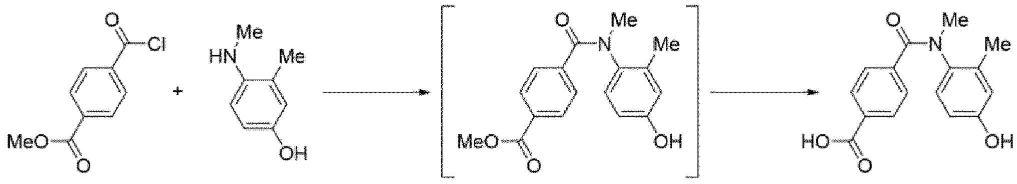

(2"A-1-46-CH)

A solution mixture of 3-methyl-4-(methylamino)phenol (2.06 g, 15.0 mmol), sodium hydrogencarbonate (2.52 g, 30.0 mol), THF (30 mL) and water (15 mL) was cooled to 0°C, and a THF (15 mL) solution of methyl 4-(chloroformyl)benzoate was added dropwise over 15 minutes. Then the resulting mixture was allowed to warm to room temperature and vigorously stirred for 12 hours. After the end of the reaction, the solvent was concentrated under reduced pressure, and extraction with ethyl acetate (50 mL × 3) was performed. The combined organic phase was washed with a saturated aqueous solution of sodium carbonate (50 mL) and saturated saline solution (50 mL), then dehydrated with magnesium sulfate and concentrated under reduced pressure, giving a white crude solid (actual crude yield: 3.76 g; percent crude yield: 84%).

Methanol (40 mL) and water (40 mL) were added to the resulting crude solid. A 48% aqueous solution of sodium hydroxide (3.2 mL, 57.9 mmol) was added, and the resulting mixture was stirred at room temperature for 12 hours and with heating under reflux for 1 hour. After the end of the reaction, the mixture was concentrated under reduced pressure. Concentrated hydrochloric acid was added to the residue to make pH = 2, then the solid that formed was collected by filtration, and reprecipitation from ethyl acetate/hexane was performed, giving a white solid (actual yield: 3.49 g; total percent yield: 82%) of compound (2"A-1-46-CH). $^1$H-NMR (400 MHz, DMSO-$d_6$): δ 13.05 (brs, 1H), 9.45 (s, 1H), 7.73 (d, J = 8.5 Hz, 2H), 7.31 (d, J = 8.5 Hz, 2H), 6.99 (d, J = 8.4 Hz, 1H), 6.49 (d, J = 2.8 Hz, 1H), 6.46 (dd, J = 8.4, 2.8 Hz, 1H), 3.21 (s, 3H), 2.05 (s, 3H).

[Synthesis Example 16]

[Chem. 201]

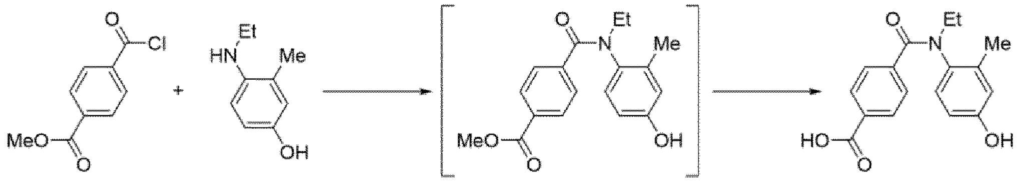

(2"A-1-47-CH)

A solution mixture of 4-(ethylamino)-3-methylphenol (3.02 g, 20.0 mmol), sodium hydrogencarbonate (3.36 g, 40.0 mol), THF (40 mL) and water (20 mL) was cooled to 0°C, and a THF (20 mL) solution of methyl 4-(chloroformyl)benzoate was added dropwise over 15 minutes. Then the resulting mixture was allowed to warm to room temperature and vigorously stirred for 12 hours. After the end of the reaction, the solvent was concentrated under reduced pressure, and extraction with ethyl acetate (50 mL × 3) was performed. The combined organic phase was washed with a saturated aqueous solution of sodium carbonate (50 mL) and saturated saline solution (50 mL), then dehydrated with magnesium sulfate and concentrated under reduced pressure, giving a brown crude solid (actual crude yield: 23.5 g).

Methanol (50 mL) was added to the resulting crude solid, and the resulting mixture was stirred at room temperature for 12 hours with an added 48% aqueous solution of sodium hydroxide (2.5 mL, 45.2 mmol). After the end of the reaction, water (50 mL) was added, and the resulting mixture was concentrated under reduced pressure. Concentrated hydrochloric acid was added to the filtrate to make pH = 2, and then the solid that formed was collected by filtration, giving a white solid (actual yield: 3.65 g; total percent yield: 61%) of compound (2"A-1-47-CH). $^1$H-NMR (400 MHz, DMSO-$d_6$): δ 13.04 (brs, 1H), 9.45 (s, 1H), 7.72 (d, J = 8.5 Hz, 2H), 7.30 (d, J = 8.5 Hz, 2H), 6.95 (d, J = 8.4 Hz, 1H), 6.49 (d, J = 7.8 Hz, 1H), 6.48 (dd, J = 7.8, 2.7 Hz, 1H), 3.99 (dq, J = 14.0, 7.0 Hz, 1H), 3.42 (dq, J = 14.0, 7.0 Hz, 1H), 2.03 (s, 3H).

[Synthesis Example 17]

[Chem. 202]

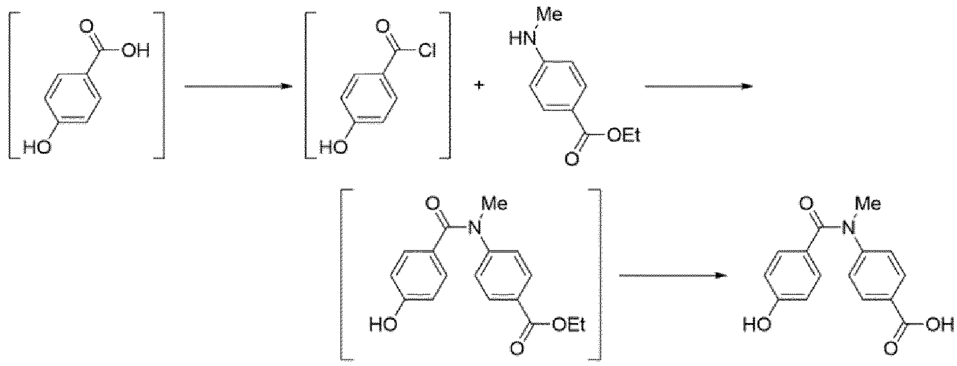

(2"A-1-1-HC)

A mixture of 4-hydroxybenzoic acid (3.63 g, 26.3 mmol), thionyl chloride (24.60 g, 0.207 mol) and N,N-dimethylformamide (3.0 mg) was stirred for 1 hour with heating under reflux, and then thionyl chloride was distilled away to give 4-hydroxybenzoyl chloride. The resulting 4-hydroxybenzoyl chloride was used entirely in the next reaction after being made into a THF (50 mL) solution.

In a nitrogen atmosphere, a mixture of ethyl 4-(methylamino)benzoate (4.13 g, 25.0 mmol), water (50 mL) and THF (50 mL) was stirred with cooling on ice, and sodium hydrogencarbonate (4.20 g, 50.0 mmol) at the same time. Subsequently, the THF solution of 4-hydroxybenzoyl chloride prepared in advance was added dropwise over 5 minutes. After the dropwise addition, the resulting mixture was stirred for 12 hours while being allowed to gradually warm to room temperature, and then THF was distilled away under reduced pressure. The solid that formed was collected by filtration, washed with a saturated aqueous solution of sodium hydrogencarbonate (50 mL) and water (50 mL) and then dried under reduced pressure, giving a white crude solid (actual crude yield: 7.12 g).

Methanol (85 mL) was added to the resulting crude solid, and the resulting mixture was stirred at room temperature for 12 hours with an added 48% aqueous solution of sodium hydroxide (2.8 mL, 50.0 mmol). After the end of the reaction, water (50 mL) was added, and undissolved residue was isolated by filtration. Concentrated hydrochloric acid was added to the resulting filtrate to make pH = 2, and then the solid that formed was collected by filtration, giving a white solid (actual yield: 5.40 g; total percent yield: 80%) of compound (2"A-1-1-HC). $^1$H-NMR (400 MHz, DMSO-$d_6$): δ 12.94 (brs, 1H), 9.86 (s, 1H), 7.81 (d, J = 8.6 Hz, 2H), 7.21 (d, J = 8.6 Hz, 2H), 7.11 (d, J = 8.7 Hz, 2H), 6.58 (d, J = 8.7 Hz, 2H), 3.38 (s, 3H).

[Synthesis Example 18]

[Chem. 203]

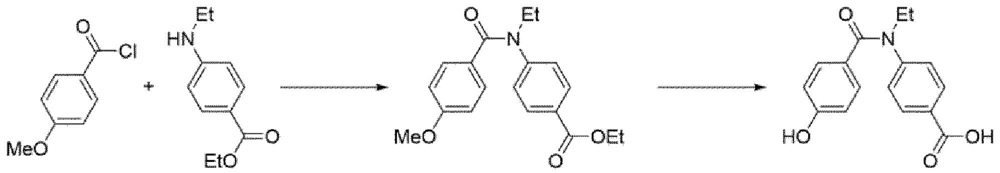

(2"A-1-2-HC)

A solution mixture of ethyl 4-(ethylamino)benzoate (13.6 g, 70.4 mmol), sodium hydrogencarbonate (11.8 g, 0.14 mol), THF (85 mL) and water (170 mL) was cooled to 0°C, and a THF (85 mL) solution of 4-methoxybenzoyl chloride (12.0 g, 70.3 mmol) was added dropwise over 30 minutes. Then the resulting mixture was allowed to warm to room temperature and vigorously stirred for 12 hours. After the end of the reaction, the solvent was concentrated under reduced pressure, and extraction with ethyl acetate (50 mL × 3) was performed. The combined organic phase was washed with a saturated aqueous solution of sodium carbonate (50 mL) and saturated saline solution (50 mL), then dehydrated with magnesium sulfate and concentrated under reduced pressure, giving a brown crude solid (actual crude yield: 23.5 g).

A dichloromethane (30 mL) solution of the resulting crude solid was cooled to 0°C, and then a dichloromethane (40 mL) solution of boron tribromide (19.6 mL, 0.21 mmol) was added dropwise over 30 minutes. After the reaction system was allowed to warm to room temperature, 12 hours of stirring was performed, and the system was cooled to 0°C again. 2 M hydrochloric acid (100 mL) was gradually added, and then the solvent was distilled away through concentration under reduced pressure. Thereafter, the solid that formed was collected by filtration and washed with acetonitrile (50 mL). The resulting solid was dried under reduced pressure. Separately, extraction from the filtrate with ethyl acetate (100 mL × 3) was performed. The combined organic layer was dried with anhydrous magnesium sulfate, followed by concentration under reduced pressure.

The resulting residue was washed with cold acetonitrile (50 mL) and then dried under reduced pressure, giving a white solid (actual yield: 9.86 g; percent yield, 49%) of compound (2"A-1-2-HC). 1H-NMR (400 MHz, DMSO-$d_6$): δ 12.96 (brs, 1H), 9.84 (s, 1H), 7.81 (d, J = 8.7 Hz, 2H), 7.18 (d, J = 8.7 Hz, 2H), 7.10 (d, J = 8.7 Hz, 2H), 6.56 (d, J = 8.7 Hz, 2H), 3.89 (q, J = 7.0 Hz, 2H), 1.09 (t, J = 7.0 Hz, 3H).

[Synthesis Example 19]

[Chem. 204]

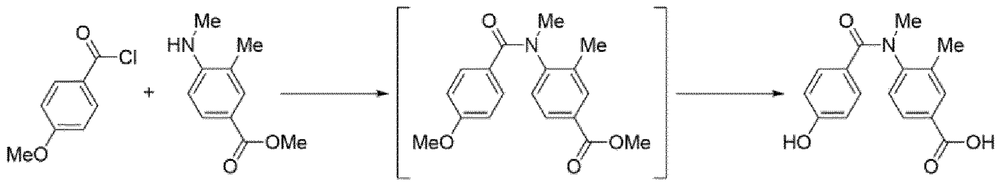

(2"A-1-46-HC)

A solution mixture of methyl 3-methyl-4-(methylamino)benzoate (2.15 g, 12.0 mmol), triethylamine (3.03 mL, 23.9 mmol) and dichloromethane (40 mL) was cooled to 0°C, and a dichloromethane (15 mL) solution of 4-methoxybenzoyl chloride (2.17 g, 12.6 mmol) was added dropwise over 15 minutes. Then the resulting mixture was allowed to warm to room temperature and stirred for 8 hours. After the end of the reaction, water (20 mL) was added, and extraction with chloroform (50 mL × 3) was performed. The combined organic layer was dehydrated with magnesium sulfate and concentrated under reduced pressure, giving a white crude solid (actual crude yield: 3.02 g).

A dichloromethane (30 mL) solution of the resulting crude solid was cooled to 0°C, and then a dichloromethane (3.50 mL, 6.4 mmol) solution of boron tribromide was added dropwise over 15 minutes. After the reaction system was allowed to warm to room temperature, 12 hours of stirring was performed, and the system was cooled to 0°C again. 2 M hydrochloric acid (10 mL) was gradually added, and then the solvent was distilled away through concentration under reduced pressure. Thereafter, the solid that formed was collected by filtration and washed with acetonitrile (50 mL). The resulting solid was dried under reduced pressure, giving a white solid (actual yield: 2.34 g; total percent yield: 69%) of compound (2"A-1-46-HC). 1H-NMR (400 MHz, DMSO-$d_6$): δ 12.98 (brs, 1H), 9.81 (s, 1H), 7.77 (s, 1H), 7.69 (d, J = 7.1 Hz, 1H), 7.26 (d, J = 7.1 Hz, 1H), 7.67 (d, J = 7.3 Hz, 2H), 6.54 (d, J = 7.3 Hz, 2H), 3.22 (s, 3H), 2.17 (s, 3H).

[Example 1]

[Chem. 205]

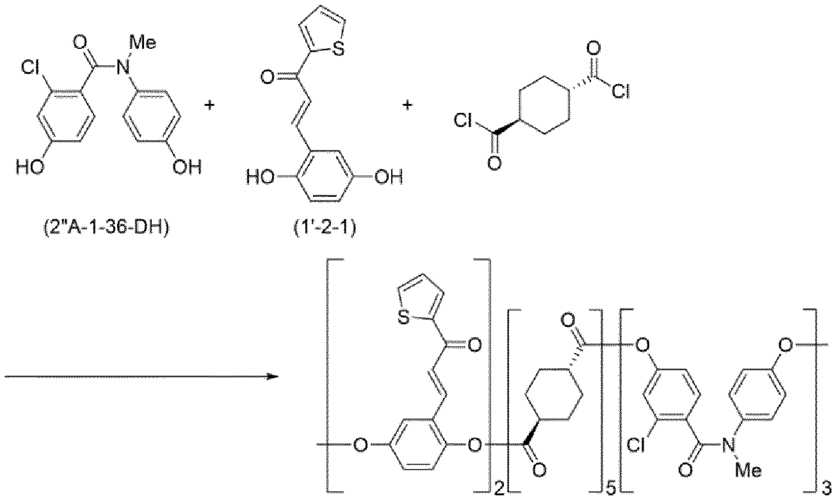

(2"A-1-36-DH) (1'-2-1)

Polymer 1

In a nitrogen stream, compound (2"A-1-36-DH) (333 mg, 1.20 mmol), obtained in Synthesis Example 1, and compound (1 '-2-1) (197 mg, 0.800 mmol), obtained in Dihydroxy Compound Example 1, as diol monomers were added to a reaction vessel. Then an aqueous (19.2 mL) solution of sodium hydroxide (160 mg, 4.00 mmol) and a 2 wt% aqueous solution of tetrabutylammonium bromide (0.8 mL) were added, and stirring was initiated. Trans-1,4-cyclohexanedicarboxylic acid dichloride (418 mg, 2.00 mmol) as a dicarboxylic acid dichloride monomer, furthermore, was dissolved in chloroform (20 mL), followed by the addition of this solution to the reaction system. After the reaction system was stirred for 3 hours, the mixture in the system was added to methanol (300 mL). The solid that formed was collected by filtration, washed with methanol (100 mL) and water (100 mL) and then vacuum-dried, giving 583 mg (percent yield: 73%) of polymer 1.

[Example 1-1]

6.0% by mass of polymer 1 was dissolved in 94.0% by mass of 1,1,1,3,3,3-hexafluoroisopropyl alcohol. After this solution was cast onto a quartz glass substrate, spin coating was performed, and the resulting coating was dried in an oven at 60°C for 30 minutes to give a thin film. The resulting thin film (thickness, 1 μm) was irradiated with 500 $mJ/cm^2$ of polarized ultraviolet light at 248 nm, followed by heating at 150°C. Retardations and wavelength dispersion properties are presented in Table 1.

[Example 2]

[Chem. 206]

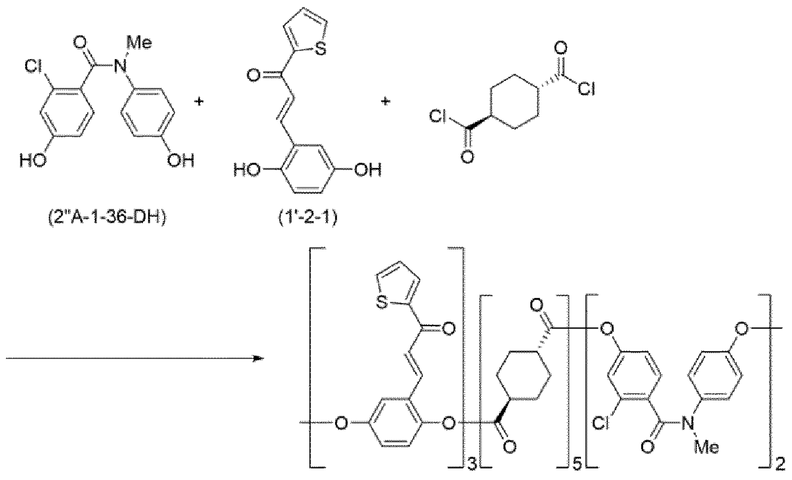

(2"A-1-36-DH)

(1'-2-1)

Polymer 2

407 mg (percent yield: 52%) of polymer 2 was obtained by the same method as in Example 1, except that compound (2"A-1-36-DH) (222 mg, 0.800 mmol), obtained in Synthesis Example 1, and compound (1 ’-2-1) (296 mg, 1.20 mmol), obtained in Dihydroxy Compound Example 1, were used as diol monomers.

[Example 2-1]

A thin film was obtained by the same method as in Example 1-1, except that polymer 2 was used instead of polymer 1. The resulting thin film (thickness, 1 μm) was irradiated with 100 mJ/cm$^2$ of polarized ultraviolet light at 248 nm, followed by heating at 150°C. Retardations and wavelength dispersion properties are presented in Table 1.

[Example 2-2]

A thin film was obtained by the same method as in Example 1-1, except that polymer 2 was used instead of polymer 1. The resulting thin film (thickness, 1 μm) was irradiated with 500 mJ/cm$^2$ of polarized ultraviolet light at 248 nm, followed by heating at 150°C. Retardations and wavelength dispersion properties are presented in Table 1.

[Example 2-3]

A thin film was obtained by the same method as in Example 1-1, except that polymer 2 was used instead of polymer 1. The resulting thin film (thickness, 1 μm) was irradiated with 100 mJ/cm$^2$ of polarized ultraviolet light at 248 nm, followed by heating at 250°C. Retardations and wavelength dispersion properties are presented in Table 1.

[Example 2-4]

A thin film was obtained by the same method as in Example 1-1, except that polymer 2 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 1.

[Example 3]

[Chem. 207]

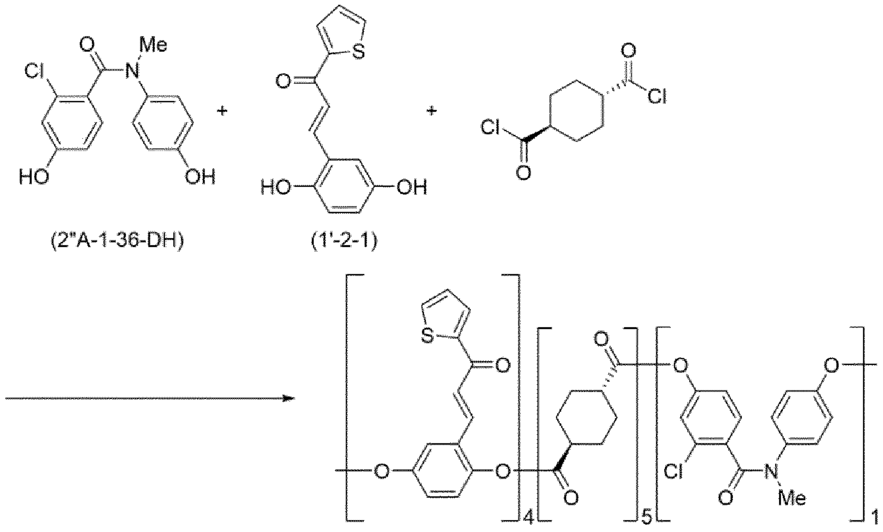

(2"A-1-36-DH) (1'-2-1)

Polymer 3

527 mg (percent yield: 68%) of polymer 3 was obtained by the same method as in Example 1, except that compound (2"A-1-36-DH) (111 mg, 0.400 mmol), obtained in Synthesis Example 1, and compound (1'-2-1) (394 mg, 1.60 mmol), obtained in Dihydroxy Compound Example 1, were used as diol monomers.

[Example 3-1]

A thin film was obtained by the same method as in Example 1-1, except that polymer 3 was used instead of polymer 1. The resulting thin film (thickness, 1 μm) was irradiated with 500 $mJ/cm^2$ of polarized ultraviolet light at 248 nm, followed by heating at 150°C. Retardations and wavelength dispersion properties are presented in Table 1.

[Example 3-2]

A thin film was obtained by the same method as in Example 1-1, except that polymer 3 was used instead of polymer 1. The resulting thin film (thickness, 1 μm) was irradiated with 500 $mJ/cm^2$ of polarized ultraviolet light at 248 nm, followed by heating at 250°C. Retardations and wavelength dispersion properties are presented in Table 1.

[Example 4]

[Chem. 208]

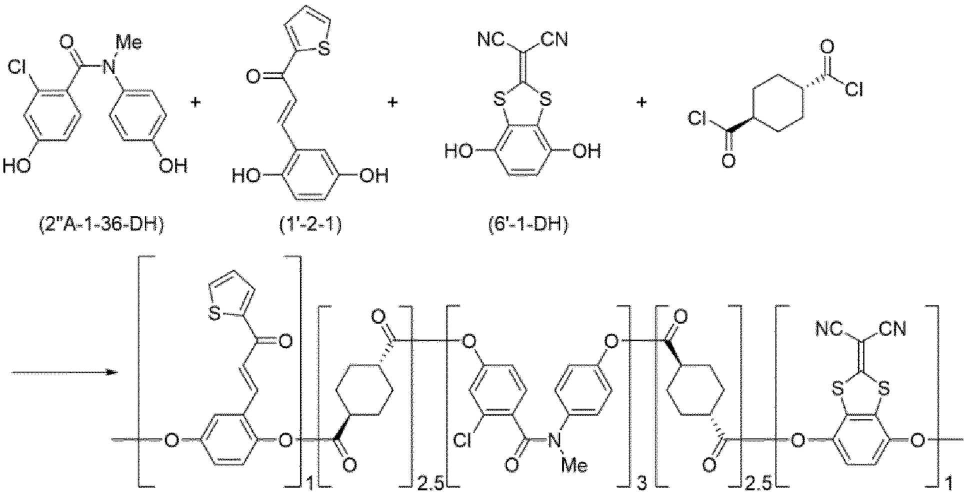

(2"A-1-36-DH) (1'-2-1) (6'-1-DH)

Polymer 4

191 mg (percent yield: 24%) of polymer 4 was obtained by the same method as in Example 1, except that compound (2"A-1-36-DH) (333 mg, 1.20 mmol), obtained in Synthesis Example 1, compound (1'-2-1) (98.4 mg, 0.400 mmol), obtained in Dihydroxy Compound Example 1, and compound (6'-1-DH) (99.2 mg, 0.400 mmol) were used as diol monomers.

[Example 4-1]

A thin film was obtained by the same method as in Example 1-1, except that polymer 4 was used instead of polymer 1. The resulting thin film (thickness, 1 μm) was irradiated with 500 mJ/cm$^2$ of polarized ultraviolet light at 248 nm, followed by heating at 150°C. Retardations and wavelength dispersion properties are presented in Table 1.

[Example 4-2]

A thin film was obtained by the same method as in Example 1-1, except that polymer 4 was used instead of polymer 1. The resulting thin film (thickness, 1 μm) was irradiated with 1000 mJ/cm$^2$ of polarized ultraviolet light at 248 nm, followed by heating at 150°C. Retardations and wavelength dispersion properties are presented in Table 1.

[Example 4-3]

A thin film was obtained by the same method as in Example 1-1, except that polymer 4 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 1.

[Example 4-4]

A thin film was obtained by the same method as in Example 1-1, except that polymer 4 was used instead of polymer 1. The resulting thin film (thickness, 1 μm) was irradiated with 1000 mJ/cm$^2$ of polarized ultraviolet light at 248 nm, followed by heating at 250°C. Retardations and wavelength dispersion properties are presented in Table 1.

[Example 5]

[Chem. 209]

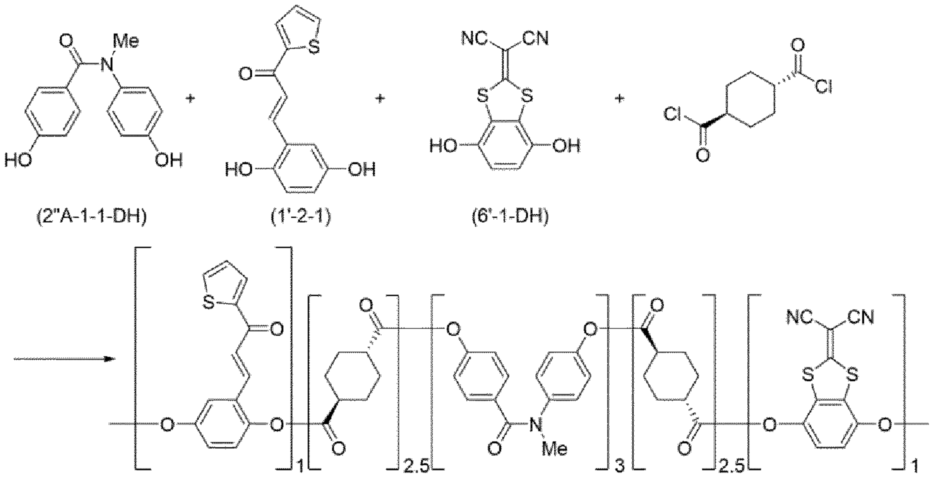

(2"A-1-1-DH) (1'-2-1) (6'-1-DH)

Polymer 5

191 mg (percent yield: 42%) of polymer 5 was obtained by the same method as in Example 1, except that compound (2"A-1-1-DH) (292 mg, 1.20 mmol), obtained in Synthesis Example 2, compound (1'-2-1) (98.4 mg, 0.400 mmol), obtained in Dihydroxy Compound Example 1, and compound (6'-1-DH) (99.2 mg, 0.400 mmol) were used as diol monomers.

[Example 5-1]

A thin film was obtained by the same method as in Example 1-1, except that polymer 5 was used instead of polymer 1. The resulting thin film (thickness, 1 μm) was irradiated with 500 $mJ/cm^2$ of polarized ultraviolet light at 248 nm, followed by heating at 150°C. Retardations and wavelength dispersion properties are presented in Table 1.

[Example 5-2]

A thin film was obtained by the same method as in Example 1-1, except that polymer 5 was used instead of polymer 1. The resulting thin film (thickness, 1 μm) was irradiated with 1000 $mJ/cm^2$ of polarized ultraviolet light at 248 nm, followed by heating at 150°C. Retardations and wavelength dispersion properties are presented in Table 1.

[Example 5-3]

A thin film was obtained by the same method as in Example 1-1, except that polymer 5 was used instead of polymer 1. The resulting thin film (thickness, 1 μm) was irradiated with 500 $mJ/cm^2$ of polarized ultraviolet light at 248 nm, followed by heating at 250°C. Retardations and wavelength dispersion properties are presented in Table 1.

[Example 5-4]

A thin film was obtained by the same method as in Example 1-1, except that polymer 5 was used instead of polymer 1. The resulting thin film (thickness, 1 μm) was irradiated with 1000 $mJ/cm^2$ of polarized ultraviolet light at 248 nm, followed by heating at 250°C. Retardations and wavelength dispersion properties are presented in Table 1.

[Example 6]

[Chem. 210]

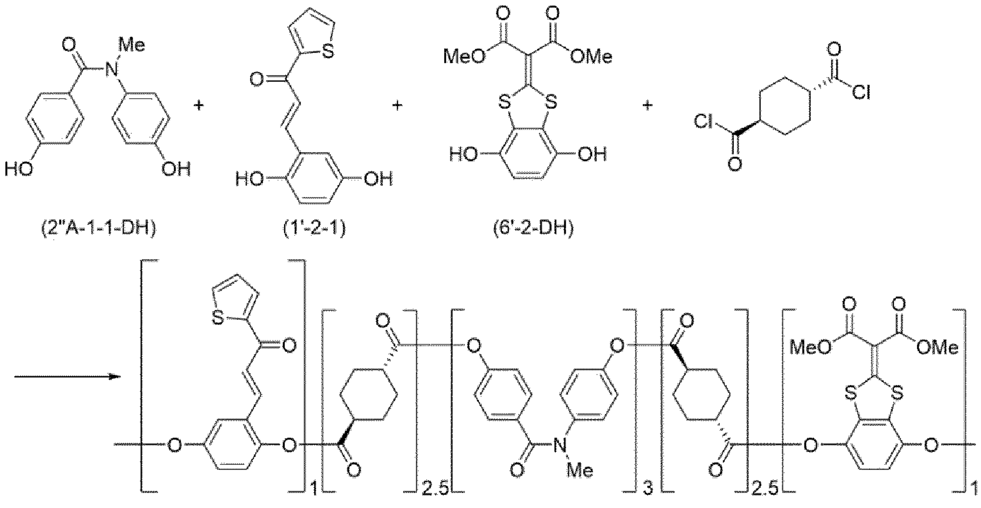

(2"A-1-1-DH) (1'-2-1) (6'-2-DH)

Polymer 6

562 mg (percent yield: 71%) of polymer 6 was obtained by the same method as in Example 1, except that compound (2"A-1-1-DH) (292 mg, 1.20 mmol), obtained in Synthesis Example 2, compound (1'-2-1) (98.4 mg, 0.400 mmol), obtained in Dihydroxy Compound Example 1, and compound (6'-2-DH) (126 mg, 0.400 mmol), obtained in Synthesis Example 6, were used as diol monomers.

[Example 6-1]

A thin film was obtained by the same method as in Example 1-1, except that polymer 6 was used instead of polymer 1. The resulting thin film (thickness, 1 μm) was irradiated with 500 mJ/cm$^2$ of polarized ultraviolet light at 248 nm, followed by heating at 150°C. Retardations and wavelength dispersion properties are presented in Table 1.

[Example 6-2]

A thin film was obtained by the same method as in Example 1-1, except that polymer 6 was used instead of polymer 1. The resulting thin film (thickness, 1 μm) was irradiated with 1000 mJ/cm$^2$ of polarized ultraviolet light at 248 nm, followed by heating at 150°C. Retardations and wavelength dispersion properties are presented in Table 1.

[Example 6-3]

A thin film was obtained by the same method as in Example 1-1, except that polymer 6 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 1.

[Example 6-4]

A thin film was obtained by the same method as in Example 1-1, except that polymer 6 was used instead of polymer 1. The resulting thin film (thickness, 1 μm) was irradiated with 1000 mJ/cm$^2$ of polarized ultraviolet light at 248 nm, followed by heating at 250°C. Retardations and wavelength dispersion properties are presented in Table 1.

[Example 7]

[Chem. 211]

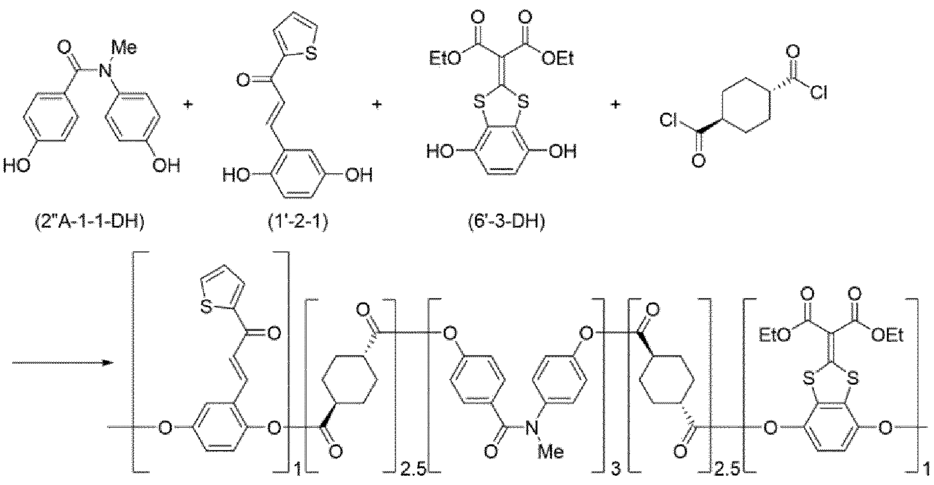

(2"A-1-1-DH) (1'-2-1) (6'-3-DH)

Polymer 7

457 mg (percent yield: 57%) of polymer 7 was obtained by the same method as in Example 1, except that compound (2"A-1-1-DH) (292 mg, 1.20 mmol), obtained in Synthesis Example 2, compound (1'-2-1) (98.4 mg, 0.400 mmol), obtained in Dihydroxy Compound Example 1, and compound (6'-3-DH) (137 mg, 0.400 mmol), obtained in Synthesis Example 7, were used as diol monomers.

[Example 7-1]

A thin film was obtained by the same method as in Example 1-1, except that polymer 7 was used instead of polymer 1. The resulting thin film (thickness, 1 μm) was irradiated with 500 $mJ/cm^2$ of polarized ultraviolet light at 248 nm, followed by heating at 150°C. Retardations and wavelength dispersion properties are presented in Table 1.

[Example 7-2]

A thin film was obtained by the same method as in Example 1-1, except that polymer 7 was used instead of polymer 1. The resulting thin film (thickness, 1 μm) was irradiated with 1000 $mJ/cm^2$ of polarized ultraviolet light at 248 nm, followed by heating at 150°C. Retardations and wavelength dispersion properties are presented in Table 1.

[Example 7-3]

A thin film was obtained by the same method as in Example 1-1, except that polymer 7 was used instead of polymer 1. The resulting thin film (thickness, 1 μm) was irradiated with 500 $mJ/cm^2$ of polarized ultraviolet light at 248 nm, followed by heating at 250°C. Retardations and wavelength dispersion properties are presented in Table 1.

[Example 7-4]

A thin film was obtained by the same method as in Example 1-1, except that polymer 7 was used instead of polymer 1. The resulting thin film (thickness, 1 μm) was irradiated with 1000 $mJ/cm^2$ of polarized ultraviolet light at 248 nm, followed by heating at 250°C. Retardations and wavelength dispersion properties are presented in Table 1.

[Example 8]

[Chem. 212]

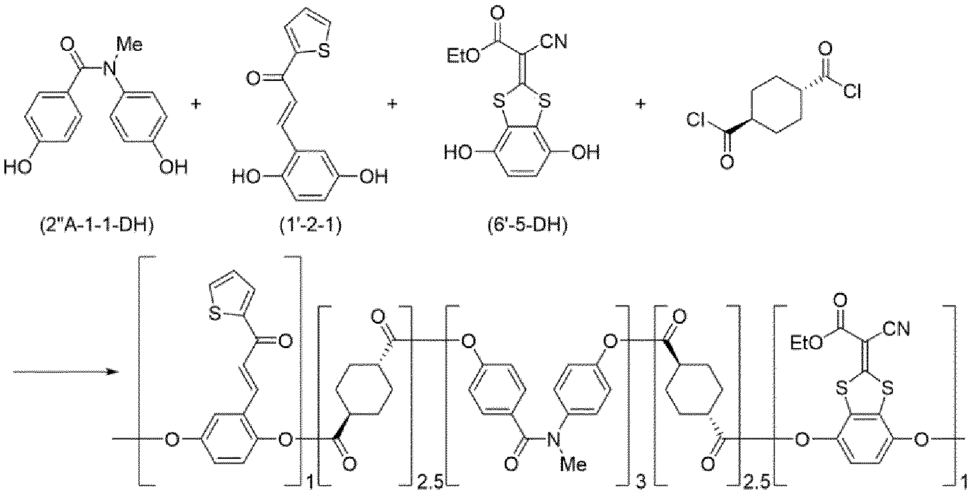

(2"A-1-1-DH) (1'-2-1) (6'-5-DH)

Polymer 8

340 mg (percent yield: 44%) of polymer 8 was obtained by the same method as in Example 1, except that compound (2"A-1-1-DH) (292 mg, 1.20 mmol), obtained in Synthesis Example 2, compound (1'-2-1) (98.4 mg, 0.400 mmol), obtained in Dihydroxy Compound Example 1, and compound (6'-5-DH) (118 mg, 0.400 mmol), obtained in Synthesis Example 8, were used as diol monomers.

[Example 8-1]

A thin film was obtained by the same method as in Example 1-1, except that polymer 8 was used instead of polymer 1. The resulting thin film (thickness, 1 μm) was irradiated with 500 mJ/cm$^2$ of polarized ultraviolet light at 248 nm, followed by heating at 150°C. Retardations and wavelength dispersion properties are presented in Table 1.

[Example 8-2]

A thin film was obtained by the same method as in Example 1-1, except that polymer 8 was used instead of polymer 1. The resulting thin film (thickness, 1 μm) was irradiated with 1000 mJ/cm$^2$ of polarized ultraviolet light at 248 nm, followed by heating at 150°C. Retardations and wavelength dispersion properties are presented in Table 1.

[Example 8-3]

A thin film was obtained by the same method as in Example 1-1, except that polymer 8 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 1.

[Example 8-4]

A thin film was obtained by the same method as in Example 1-1, except that polymer 8 was used instead of polymer 1. The resulting thin film (thickness, 1 μm) was irradiated with 1000 mJ/cm$^2$ of polarized ultraviolet light at 248 nm, followed by heating at 250°C. Retardations and wavelength dispersion properties are presented in Table 1.

[Example 9]

[Chem. 213]

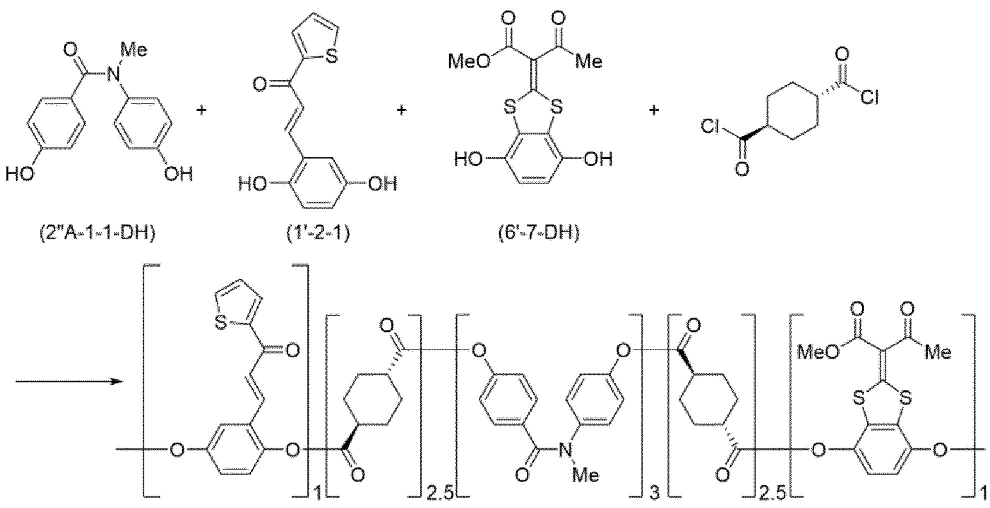

(2"A-1-1-DH) (1'-2-1) (6'-7-DH)

Polymer 9

531 mg (percent yield: 68%) of polymer 9 was obtained by the same method as in Example 1, except that compound (2"A-1-1-DH) (292 mg, 1.20 mmol), obtained in Synthesis Example 2, (1'-2-1) (98.4 mg, 0.400 mmol), obtained in Dihydroxy Compound Example 1, and compound (6'-7-DH) (119 mg, 0.400 mmol), obtained in Synthesis Example 9, were used as diol monomers.

[Example 9-1]

A thin film was obtained by the same method as in Example 1-1, except that polymer 9 was used instead of polymer 1. The resulting thin film (thickness, 1 μm) was irradiated with 500 $mJ/cm^2$ of polarized ultraviolet light at 248 nm, followed by heating at 150°C. Retardations and wavelength dispersion properties are presented in Table 1.

[Example 9-2]

A thin film was obtained by the same method as in Example 1-1, except that polymer 9 was used instead of polymer 1. The resulting thin film (thickness, 1 μm) was irradiated with 1000 $mJ/cm^2$ of polarized ultraviolet light at 248 nm, followed by heating at 150°C. Retardations and wavelength dispersion properties are presented in Table 1.

[Example 9-3]

A thin film was obtained by the same method as in Example 1-1, except that polymer 9 was used instead of polymer 1. The resulting thin film (thickness, 1 μm) was irradiated with 500 $mJ/cm^2$ of polarized ultraviolet light at 248 nm, followed by heating at 250°C. Retardations and wavelength dispersion properties are presented in Table 1.

[Example 9-4]

A thin film was obtained by the same method as in Example 1-1, except that polymer 9 was used instead of polymer 1. The resulting thin film (thickness, 1 μm) was irradiated with 1000 $mJ/cm^2$ of polarized ultraviolet light at 248 nm, followed by heating at 250°C. Retardations and wavelength dispersion properties are presented in Table 1.

[Example 10]

[Chem. 214]

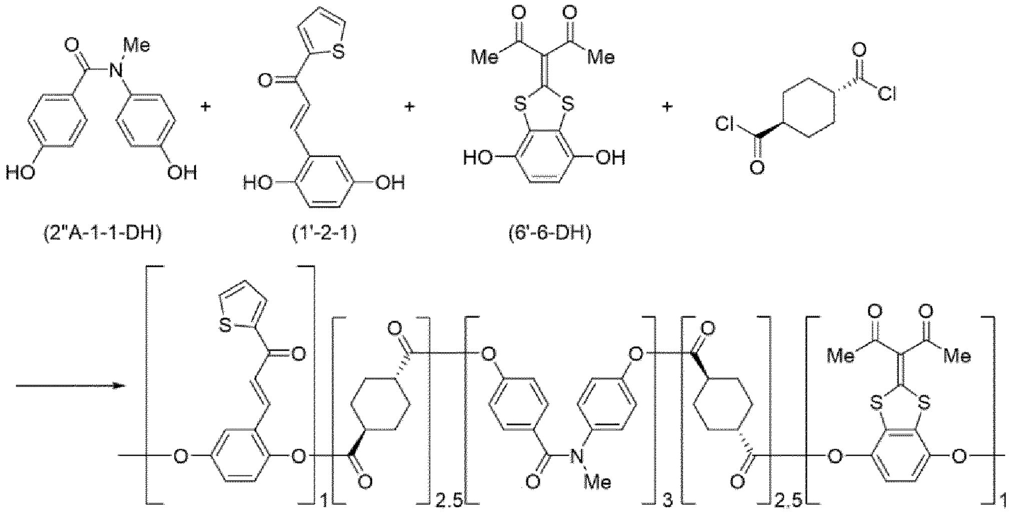

(2"A-1-1-DH) (1'-2-1) (6'-6-DH)

Polymer 10

658 mg (percent yield: 85%) of polymer 10 was obtained by the same method as in Example 1, except that compound (2"A-1-1-DH) (292 mg, 1.20 mmol), obtained in Synthesis Example 2, compound (1'-2-1) (98.4 mg, 0.400 mmol), obtained in Dihydroxy Compound Example 1, and compound (6'-6-DH) (113 mg, 0.400 mmol), obtained in Synthesis Example 10, were used as diol monomers.

[Example 10-1]

A thin film was obtained by the same method as in Example 1-1, except that polymer 10 was used instead of polymer 1. The resulting thin film (thickness, 1 μm) was irradiated with 1000 $mJ/cm^2$ of polarized ultraviolet light at 248 nm, followed by heating at 150°C. Retardations and wavelength dispersion properties are presented in Table 1.

[Example 10-2]

A thin film was obtained by the same method as in Example 1-1, except that polymer 10 was used instead of polymer 1. The resulting thin film (thickness, 1 μm) was irradiated with 500 $mJ/cm^2$ of polarized ultraviolet light at 248 nm, followed by heating at 250°C. Retardations and wavelength dispersion properties are presented in Table 1.

[Example 10-3]

A thin film was obtained by the same method as in Example 1-1, except that polymer 10 was used instead of polymer 1. The resulting thin film (thickness, 1 μm) was irradiated with 1000 $mJ/cm^2$ of polarized ultraviolet light at 248 nm, followed by heating at 250°C. Retardations and wavelength dispersion properties are presented in Table 1.

[Example 11]

[Chem. 215]

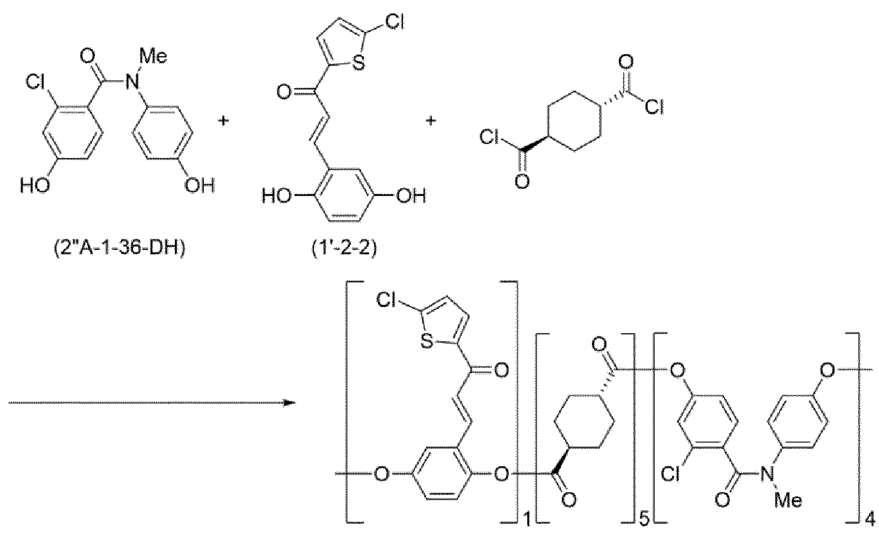

(2"A-1-36-DH) (1'-2-2)

Polymer 11

354 mg (percent yield: 43%) of polymer 11 was obtained by the same method as in Example 1, except that compound (2"A-1-36-DH) (444 mg, 1.60 mmol), obtained in Synthesis Example 1, and compound (1 '-2-2) (112 mg, 0.400 mmol), obtained in Dihydroxy Compound Example 2, were used as diol monomers.

[Example 11-1]

A thin film was obtained by the same method as in Example 1-1, except that polymer 11 was used instead of polymer 1. The resulting thin film (thickness, 1 μm) was irradiated with 100 mJ/cm$^2$ of polarized ultraviolet light at 248 nm, followed by heating at 250°C. Retardations and wavelength dispersion properties are presented in Table 1.

[Example 11-2]

A thin film was obtained by the same method as in Example 1-1, except that polymer 11 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 1.

[Example 12]

[Chem. 216]

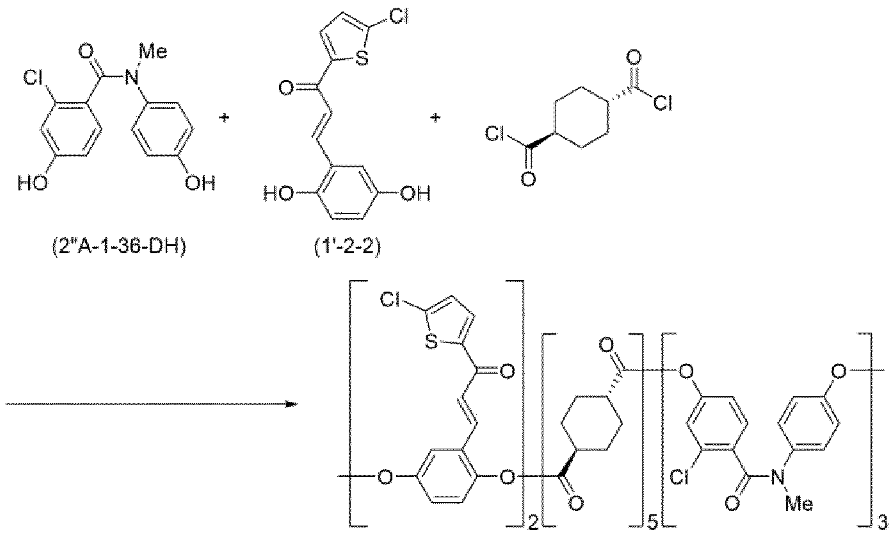

Polymer 12

565 mg (percent yield: 68%) of polymer 12 was obtained by the same method as in Example 1, except that compound (2"A-1-36-DH) (333 mg, 1.60 mmol), obtained in Synthesis Example 1, and compound (1 '-2-2) (224 mg, 0.800 mmol), obtained in Dihydroxy Compound Example 2, were used as diol monomers.

[Example 12-1]

A thin film was obtained by the same method as in Example 1-1, except that polymer 12 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 1.

[Example 12-2]

A thin film was obtained by the same method as in Example 1-1, except that polymer 12 was used instead of polymer 1. The resulting thin film (thickness, 1 μm) was irradiated with 1000 mJ/cm$^2$ of polarized ultraviolet light at 248 nm, followed by heating at 250°C. Retardations and wavelength dispersion properties are presented in Table 1.

[Example 12-3]

A thin film was obtained by the same method as in Example 1-1, except that polymer 12 was used instead of polymer 1. The resulting thin film (thickness, 1 μm) was irradiated with 170 mJ/cm$^2$ of polarized ultraviolet light at 313 nm, followed by heating at 150°C. Retardations and wavelength dispersion properties are presented in Table 1.

[Example 12-4]

A thin film was obtained by the same method as in Example 1-1, except that polymer 12 was used instead of polymer 1. The resulting thin film (thickness, 1 μm) was irradiated with 170 mJ/cm$^2$ of polarized ultraviolet light at 313 nm, followed by heating at 250°C. Retardations and wavelength dispersion properties are presented in Table 1.

[Example 12-5]

A thin film was obtained by the same method as in Example 1-1, except that polymer 12 was used instead of polymer 1. The resulting thin film (thickness, 1 μm) was irradiated with 340 mJ/cm$^2$ of polarized ultraviolet light at 313 nm, followed by heating at 250°C. Retardations and wavelength dispersion properties are presented in Table 1.

[Example 12-6]

A thin film was obtained by the same method as in Example 1-1, except that polymer 12 was used instead of polymer 1. The resulting thin film (thickness, 1 μm) was irradiated with 700 mJ/cm$^2$ of polarized ultraviolet light at 365 nm, followed by heating at 250°C. Retardations and wavelength dispersion properties are presented in Table 1.

[Example 12-7]

A thin film was obtained by the same method as in Example 1-1, except that polymer 12 was used instead of polymer 1. The resulting thin film (thickness, 1 μm) was irradiated with 1400 mJ/cm$^2$ of polarized ultraviolet light at 365 nm, followed by heating at 250°C. Retardations and wavelength dispersion properties are presented in Table 1.

[Example 13]

[Chem. 217]

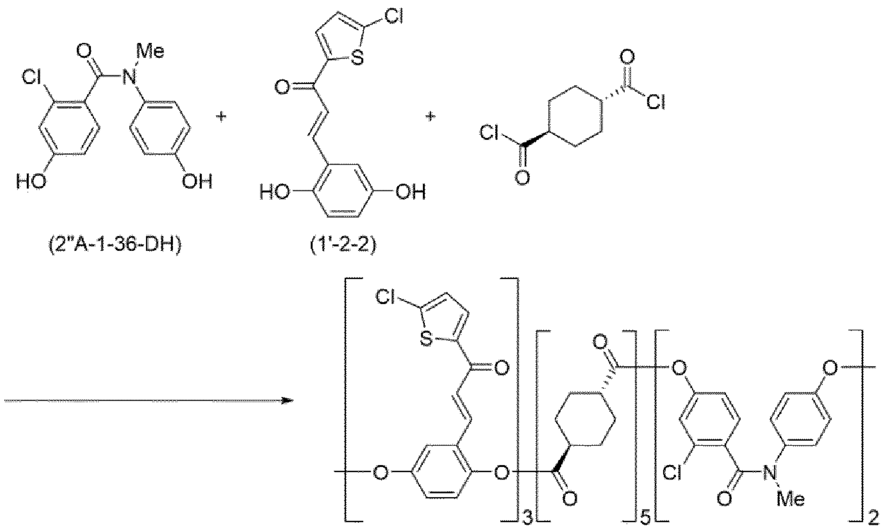

(2"A-1-36-DH)

(1'-2-2)

Polymer 13

463 mg (percent yield: 56%) of polymer 13 was obtained by the same method as in Example 1, except that compound (2"A-1-36-DH) (222 mg, 0.800 mmol), obtained in Synthesis Example 1, and compound (1 '-2-2) (337 mg, 1.20 mmol), obtained in Dihydroxy Compound Example 2, were used as diol monomers.

[Example 13-1]

A thin film was obtained by the same method as in Example 1-1, except that polymer 13 was used instead of polymer 1. The resulting thin film (thickness, 1 μm) was irradiated with 50 mJ/cm$^2$ of polarized ultraviolet light at 248 nm, followed by heating at 150°C. Retardations and wavelength dispersion properties are presented in Table 1.

[Example 13-2]

A thin film was obtained by the same method as in Example 1-1, except that polymer 13 was used instead of polymer 1. The resulting thin film (thickness, 1 μm) was irradiated with 50 mJ/cm$^2$ of polarized ultraviolet light at 248 nm, followed by heating at 250°C. Retardations and wavelength dispersion properties are presented in Table 1.

[Example 13-3]

A thin film was obtained by the same method as in Example 1-1, except that polymer 13 was used instead of polymer 1. The resulting thin film (thickness, 1 μm) was irradiated with 500 mJ/cm$^2$ of polarized ultraviolet light at 248 nm, followed by heating at 150°C. Retardations and wavelength dispersion properties are presented in Table 1.

[Example 13-4]

A thin film was obtained by the same method as in Example 1-1, except that polymer 13 was used instead of polymer 1. The resulting thin film (thickness, 1 μm) was irradiated with 1000 mJ/cm$^2$ of polarized ultraviolet light at 248 nm, followed by heating at 150°C. Retardations and wavelength dispersion properties are presented in Table 1.

[Example 13-5]

A thin film was obtained by the same method as in Example 1-1, except that polymer 13 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 1.

[Example 13-6]

A thin film was obtained by the same method as in Example 1-1, except that polymer 13 was used instead of polymer 1. The resulting thin film (thickness, 1 μm) was irradiated with 1000 mJ/cm$^2$ of polarized ultraviolet light at 248 nm, followed by heating at 250°C. Retardations and wavelength dispersion properties are presented in Table 1.

[Example 13-7]

A thin film was obtained by the same method as in Example 1-1, except that polymer 13 was used instead of polymer 1. The resulting thin film (thickness, 1 μm) was irradiated with 100 mJ/cm$^2$ of polarized ultraviolet light at 365 nm, followed by heating at 150°C. Retardations and wavelength dispersion properties are presented in Table 1.

[Example 13-8]

A thin film was obtained by the same method as in Example 1-1, except that polymer 13 was used instead of polymer 1. The resulting thin film (thickness, 1 μm) was irradiated with 500 mJ/cm$^2$ of polarized ultraviolet light at 365 nm, followed by heating at 150°C. Retardations and wavelength dispersion properties are presented in Table 1.

[Example 13-9]

A thin film was obtained by the same method as in Example 1-1, except that polymer 13 was used instead of polymer 1. The resulting thin film (thickness, 1 μm) was irradiated with 100 mJ/cm$^2$ of polarized ultraviolet light at 365 nm, followed by heating at 250°C. Retardations and wavelength dispersion properties are presented in Table 1.

[Example 13-10]

A thin film was obtained by the same method as in Example 1-1, except that polymer 13 was used instead of polymer 1. The resulting thin film (thickness, 1 μm) was irradiated with 500 mJ/cm$^2$ of polarized ultraviolet light at 365 nm, followed by heating at 250°C. Retardations and wavelength dispersion properties are presented in Table 1.

[Example 14]

[Chem. 218]

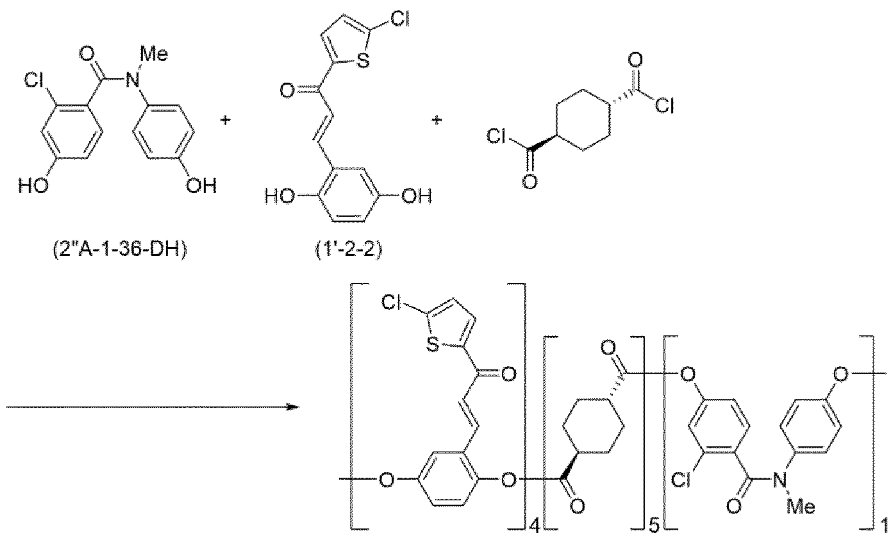

(2"A-1-36-DH) (1'-2-2)

Polymer 14

595 mg (percent yield: 72%) of polymer 14 was obtained by the same method as in Example 1, except that compound (2"A-1-36-DH) (111 mg, 0.400 mmol), obtained in Synthesis Example 1, and compound (1 '-2-2) (449 mg, 1.60 mmol), obtained in Dihydroxy Compound Example 2, were used as diol monomers.

[Example 14-1]

A thin film was obtained by the same method as in Example 1-1, except that polymer 14 was used instead of polymer 1. The resulting thin film (thickness, 1 μm) was irradiated with 500 mJ/cm$^2$ of polarized ultraviolet light at 248 nm, followed by heating at 150°C. Retardations and wavelength dispersion properties are presented in Table 2.

[Example 14-2]

A thin film was obtained by the same method as in Example 1-1, except that polymer 14 was used instead of polymer 1. The resulting thin film (thickness, 1 μm) was irradiated with 1000 mJ/cm$^2$ of polarized ultraviolet light at 248 nm, followed by heating at 150°C. Retardations and wavelength dispersion properties are presented in Table 2.

[Example 14-3]

A thin film was obtained by the same method as in Example 1-1, except that polymer 14 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 2.

[Example 14-4]

A thin film was obtained by the same method as in Example 1-1, except that polymer 14 was used instead of polymer 1. The resulting thin film (thickness, 1 μm) was irradiated with 1000 mJ/cm$^2$ of polarized ultraviolet light at 248 nm, followed by heating at 250°C. Retardations and wavelength dispersion properties are presented in Table 2.

[Example 15]

[Chem. 219]

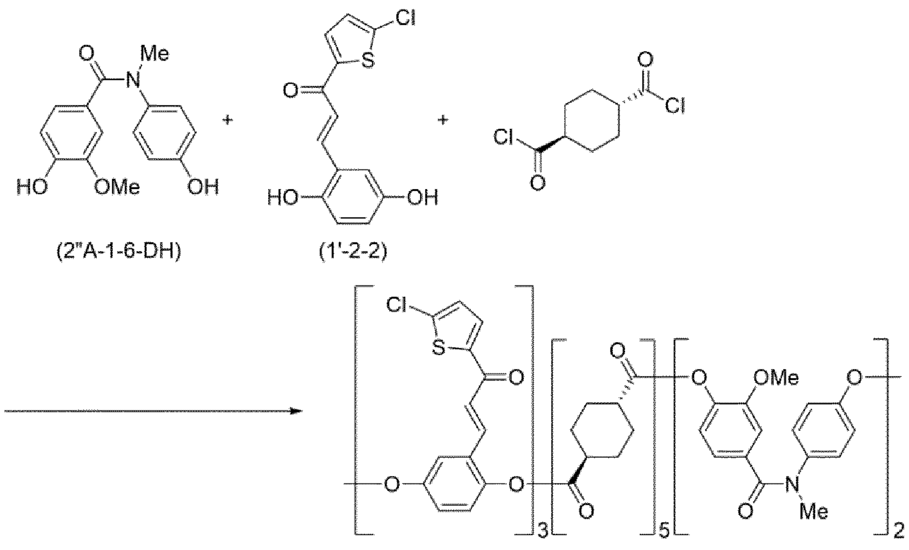

(2"A-1-6-DH) (1'-2-2)

Polymer 15

343 mg (percent yield: 41 %) of polymer 15 was obtained by the same method as in Example 1, except that compound (2"A-1-6-DH) (219 mg, 0.800 mmol), obtained in Synthesis Example 3, and compound (1'-2-2) (337 mg, 1.20 mmol), obtained in Dihydroxy Compound Example 2, were used as diol monomers.

[Example 15-1]

A thin film was obtained by the same method as in Example 1-1, except that polymer 15 was used instead of polymer 1. The resulting thin film (thickness, 1 μm) was irradiated with 100 mJ/cm$^2$ of polarized ultraviolet light at 365 nm, followed by heating at 150°C. Retardations and wavelength dispersion properties are presented in Table 2.

[Example 15-2]

A thin film was obtained by the same method as in Example 1-1, except that polymer 15 was used instead of polymer 1. The resulting thin film (thickness, 1 μm) was irradiated with 500 mJ/cm$^2$ of polarized ultraviolet light at 365 nm, followed by heating at 150°C. Retardations and wavelength dispersion properties are presented in Table 2.

[Example 15-3]

A thin film was obtained by the same method as in Example 1-1, except that polymer 15 was used instead of polymer 1. The resulting thin film (thickness, 1 μm) was irradiated with 100 mJ/cm$^2$ of polarized ultraviolet light at 365 nm, followed by heating at 250°C. Retardations and wavelength dispersion properties are presented in Table 2.

[Example 15-4]

A thin film was obtained by the same method as in Example 1-1, except that polymer 15 was used instead of polymer 1. The resulting thin film (thickness, 1 μm) was irradiated with 500 mJ/cm$^2$ of polarized ultraviolet light at 365 nm, followed by heating at 250°C. Retardations and wavelength dispersion properties are presented in Table 2.

[Example 16]

[Chem. 220]

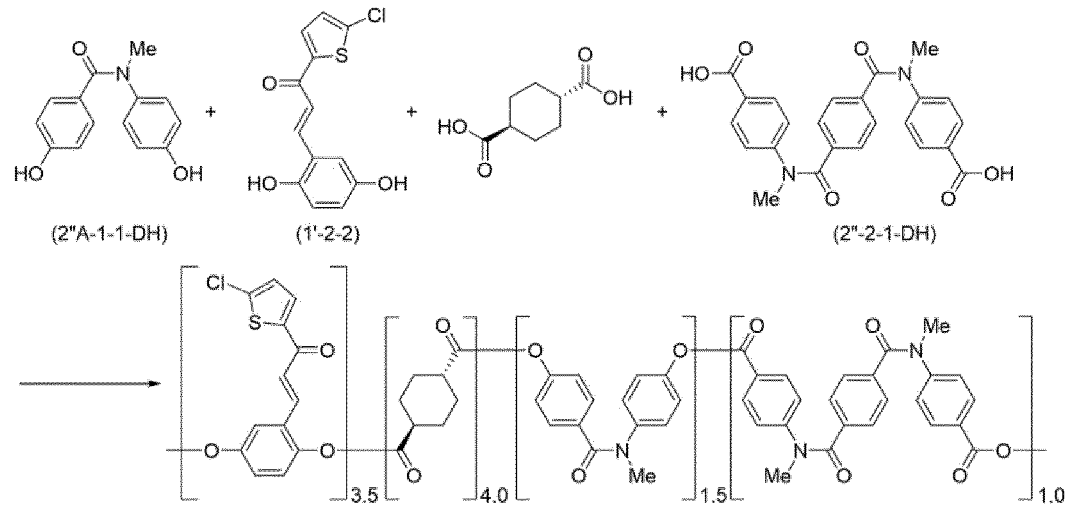

(2"A-1-1-DH) (1'-2-2) (2"-2-1-DH)

Polymer 16

In a nitrogen stream, compound (2"A-1-1-DH) (212 mg, 0.871 mmol), obtained in Synthesis Example 2, and compound (1 '-2-2) (571 mg, 2.03 mmol), obtained in Dihydroxy Compound Example 2, as diol monomers and trans-1,4-cyclohexanedicarboxylic acid (400 mg, 2.32 mmol) and compound (2"-2-1-DC) (251 mg, 0.581 mmol) as dicarboxylic acid monomers were added to a reaction vessel. Then 1-ethyl-3-(3-dimethylaminopropyl)carbodiimide hydrochloride (1.23 g, 6.39 mmol) as a condensing agent, NMP (11.2 mL) as a solvent and 4-dimethylaminopyridine (78.1 mg, 0.639 mmol) as a catalyst were added, and stirring was initiated. After the reaction system was stirred for 3 hours, the mixture in the system was added to methanol (300 mL). The solid that formed was collected by filtration, washed with methanol (100 mL) and water (100 mL) and then vacuum-dried, giving 455 mg (percent yield: 34%) of polymer 16.

[Example 16-1]

A thin film was obtained by the same method as in Example 1-1, except that polymer 16 was used instead of polymer 1. The resulting thin film (thickness, 1 μm) was irradiated with 100 mJ/cm$^2$ of polarized ultraviolet light at 365 nm, followed by heating at 150°C. Retardations and wavelength dispersion properties are presented in Table 2.

[Example 16-2]

A thin film was obtained by the same method as in Example 1-1, except that polymer 16 was used instead of polymer 1. The resulting thin film (thickness, 1 μm) was irradiated with 500 mJ/cm$^2$ of polarized ultraviolet light at 365 nm, followed by heating at 150°C. Retardations and wavelength dispersion properties are presented in Table 2.

[Example 16-3]

A thin film was obtained by the same method as in Example 1-1, except that polymer 16 was used instead of polymer 1. The resulting thin film (thickness, 1 μm) was irradiated with 100 mJ/cm$^2$ of polarized ultraviolet light at 365 nm, followed by heating at 250°C. Retardations and wavelength dispersion properties are presented in Table 2.

[Example 16-4]

A thin film was obtained by the same method as in Example 1-1, except that polymer 16 was used instead of polymer 1. The resulting thin film (thickness, 1 μm) was irradiated with 500 mJ/cm$^2$ of polarized ultraviolet light at 365 nm, followed by heating at 250°C. Retardations and wavelength dispersion properties are presented in Table 2.

[Example 17]

[0818] [Chem. 221]

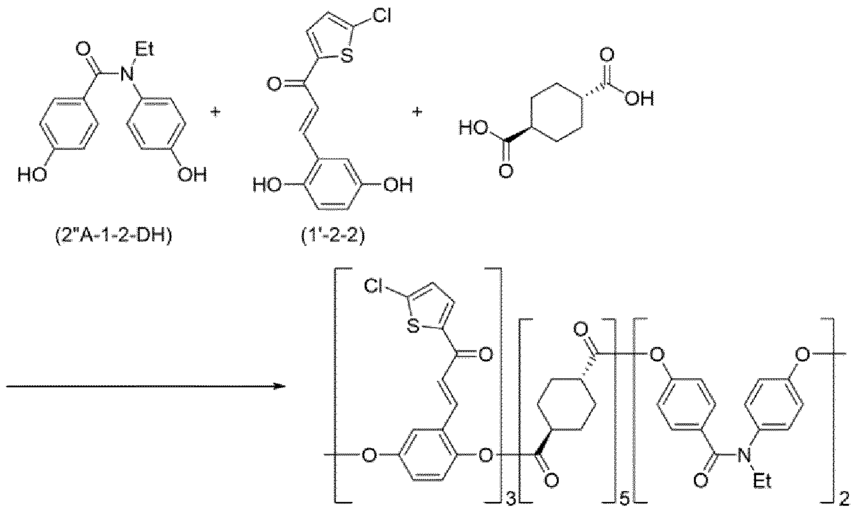

(2"A-1-2-DH) (1'-2-2)

Polymer 17

511 mg (percent yield: 43%) of polymer 17 was obtained by the same method as in Example 16, except that compound (2"A-1-2-DH) (299 mg, 1.16 mmol), obtained in Synthesis Example 4, and compound (1'-2-2) (489 mg, 1.74 mmol), obtained in Dihydroxy Compound Example 2, were used as diol monomers and that trans-1,4-cyclohexanedicarboxylic acid (500 mg, 2.90 mmol) was used as a dicarboxylic acid monomer.

[Example 17-1]

A thin film was obtained by the same method as in Example 1-1, except that polymer 17 was used instead of polymer 1. The resulting thin film (thickness, 1 μm) was irradiated with 100 mJ/cm$^2$ of polarized ultraviolet light at 365 nm, followed by heating at 150°C. Retardations and wavelength dispersion properties are presented in Table 2.

[Example 17-2]

A thin film was obtained by the same method as in Example 1-1, except that polymer 17 was used instead of polymer 1. The resulting thin film (thickness, 1 μm) was irradiated with 500 mJ/cm$^2$ of polarized ultraviolet light at 365 nm, followed by heating at 150°C. Retardations and wavelength dispersion properties are presented in Table 2.

[Example 17-3]

A thin film was obtained by the same method as in Example 1-1, except that polymer 17 was used instead of polymer 1. The resulting thin film (thickness, 1 μm) was irradiated with 100 mJ/cm$^2$ of polarized ultraviolet light at 365 nm, followed by heating at 250°C. Retardations and wavelength dispersion properties are presented in Table 2.

[Example 17-4]

A thin film was obtained by the same method as in Example 1-1, except that polymer 17 was used instead of polymer 1. The resulting thin film (thickness, 1 μm) was irradiated with 500 mJ/cm$^2$ of polarized ultraviolet light at 365 nm, followed by heating at 250°C. Retardations and wavelength dispersion properties are presented in Table 2.

[Example 18]

[0825] [Chem. 222]

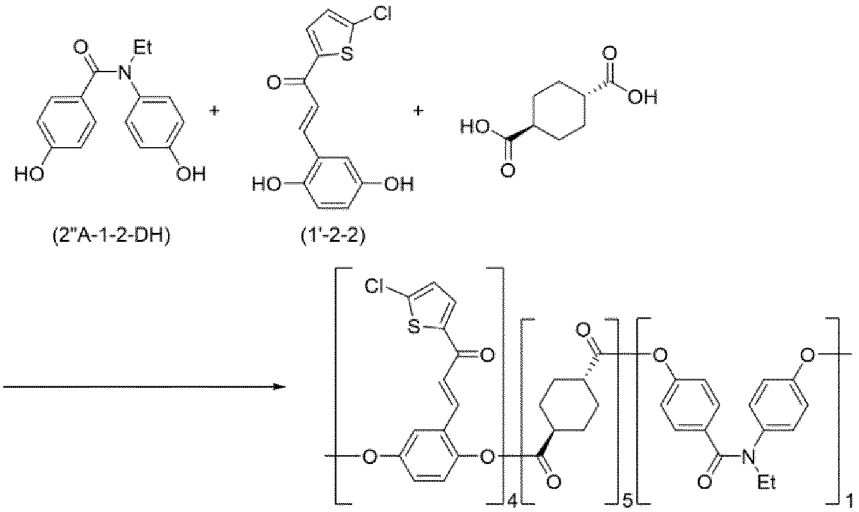

(2"A-1-2-DH) (1'-2-2)

Polymer 18

987 mg (percent yield: 83%) of polymer 18 was obtained by the same method as in Example 16, except that compound (2"A-1-2-DH) (149 mg, 0.581 mmol), obtained in Synthesis Example 4, and compound (1'-2-2) (652 mg, 2.32 mmol), obtained in Dihydroxy Compound Example 2, were used as diol monomers, that trans-1 ,4-cyclohexanedicarboxylic acid (500 mg, 2.90 mmol) was used as a dicarboxylic acid monomer and that NMP (5.2 mL) was used as a solvent.

[Example 18-1]

A thin film was obtained by the same method as in Example 1-1, except that polymer 18 was used instead of polymer 1. The resulting thin film (thickness, 1 μm) was irradiated with 100 mJ/cm$^2$ of polarized ultraviolet light at 365 nm, followed by heating at 250°C. Retardations and wavelength dispersion properties are presented in Table 2.

[Example 18-2]

A thin film was obtained by the same method as in Example 1-1, except that polymer 18 was used instead of polymer 1. The resulting thin film (thickness, 1 μm) was irradiated with 500 mJ/cm$^2$ of polarized ultraviolet light at 365 nm, followed by heating at 250°C. Retardations and wavelength dispersion properties are presented in Table 2.

[Example 19]

[Chem. 223]

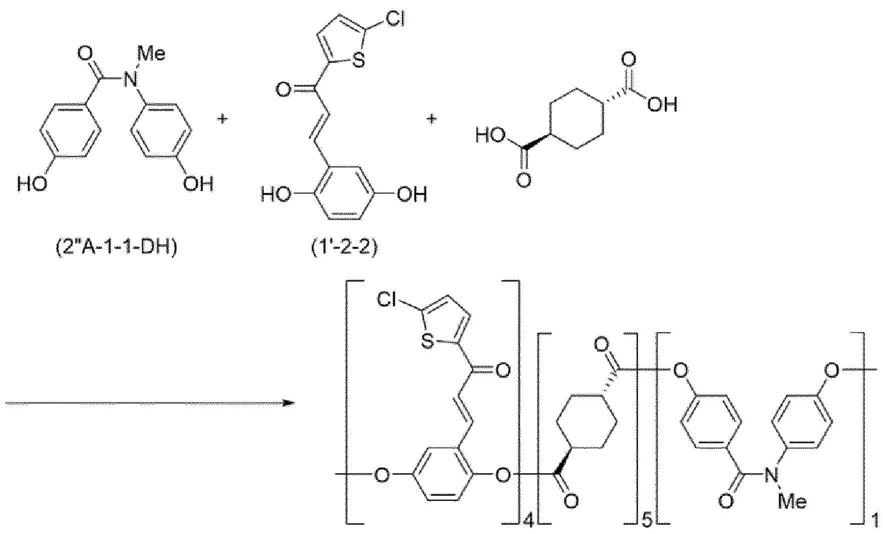

(2"A-1-1-DH) (1'-2-2)

Polymer 19

414 mg (percent yield: 35%) of polymer 19 was obtained by the same method as in Example 16, except that compound (2"A-1-1-DH) (141 mg, 581 mmol), obtained in Synthesis Example 2, and compound (1'-2-2) (652 mg, 2.32 mmol), obtained in Dihydroxy Compound Example 2, were used as diol monomers, that trans-1 ,4-cyclohexanedicarboxylic acid (500 mg, 2.90 mmol) was used as a dicarboxylic acid monomer and that NMP (29 mL) was used as a solvent.

[Example 19-1]

A thin film was obtained by the same method as in Example 1-1, except that polymer 19 was used instead of polymer 1. The resulting thin film (thickness, 1 μm) was irradiated with 50 mJ/cm2 of polarized ultraviolet light at 365 nm, followed by heating at 150°C. Retardations and wavelength dispersion properties are presented in Table 2.

[Example 19-2]

A thin film was obtained by the same method as in Example 1-1, except that polymer 19 was used instead of polymer 1. The resulting thin film (thickness, 1 μm) was irradiated with 100 $mJ/cm^2$ of polarized ultraviolet light at 365 nm, followed by heating at 150°C. Retardations and wavelength dispersion properties are presented in Table 2.

[Example 19-3]

A thin film was obtained by the same method as in Example 1-1, except that polymer 19 was used instead of polymer 1. The resulting thin film (thickness, 1 μm) was irradiated with 50 $mJ/cm^2$ of polarized ultraviolet light at 365 nm, followed by heating at 250°C. Retardations and wavelength dispersion properties are presented in Table 2.

[Example 19-4]

A thin film was obtained by the same method as in Example 1-1, except that polymer 19 was used instead of polymer 1. The resulting thin film (thickness, 1 μm) was irradiated with 100 $mJ/cm^2$ of polarized ultraviolet light at 365 nm, followed by heating at 250°C. Retardations and wavelength dispersion properties are presented in Table 2.

[Example 20]

[Chem. 224]

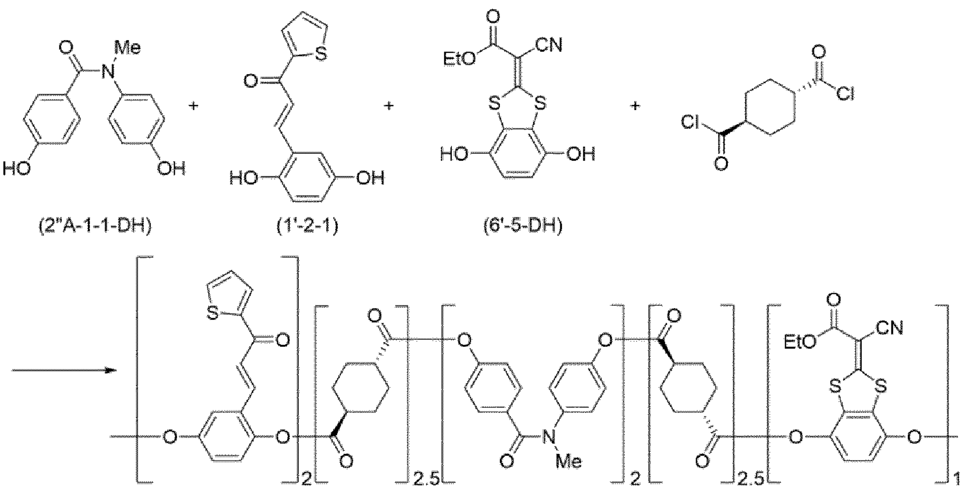


Polymer 20

510 mg (percent yield: 65%) of polymer 20 was obtained by the same method as in Example 1, except that compound (2"A-1-1-DH) (195 mg, 0.800 mmol), obtained in Synthesis Example 2, compound (1'-2-1) (197 mg, 0.800 mmol), obtained in Dihydroxy Compound Example 1, and compound (6'-5-DH) (118 mg, 0.400 mmol), obtained in Synthesis Example 8, were used as diol monomers.

[Example 20-1]

A thin film was obtained by the same method as in Example 1-1, except that polymer 20 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 2.

[Example 20-2]

A thin film was obtained by the same method as in Example 1-1, except that polymer 20 was used instead of polymer 1. The resulting thin film (thickness, 1 μm) was irradiated with 1000 mJ/cm$^2$ of polarized ultraviolet light at 248 nm, followed by heating at 250°C. Retardations and wavelength dispersion properties are presented in Table 2.

[Example 21]

[Chem. 225]

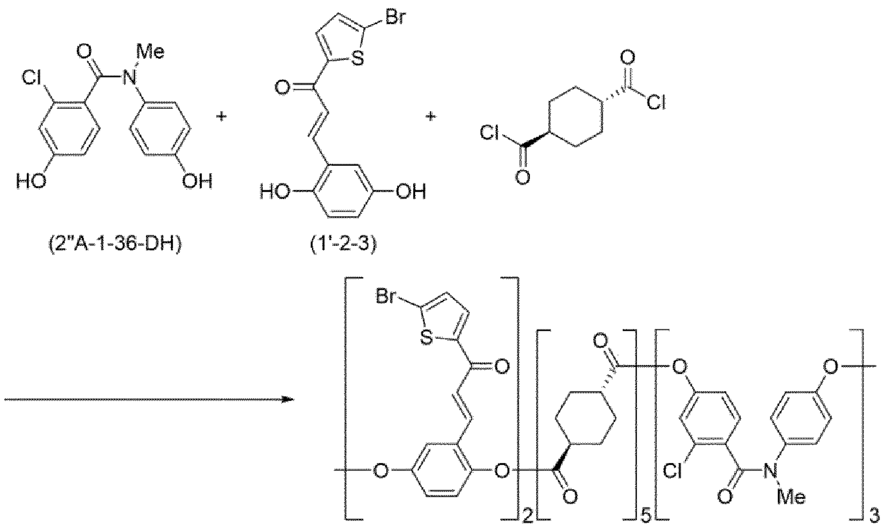

(2"A-1-36-DH) (1'-2-3)

Polymer 21

588 mg (percent yield: 68%) of polymer 21 was obtained by the same method as in Example 1, except that compound (2"A-1-36-DH) (333 mg, 1.20 mmol), obtained in Synthesis Example 1, and compound (1 '-2-3) (260 mg, 0.800 mmol), obtained in Dihydroxy Compound Example 3, were used as diol monomers.

[Example 21-1]

A thin film was obtained by the same method as in Example 1-1, except that polymer 21 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 2.

[Example 22]

[Chem. 226]

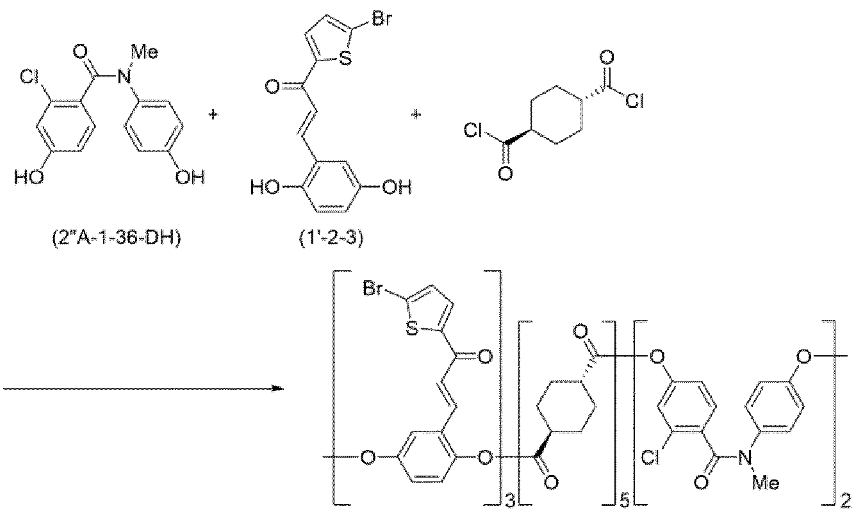

(2"A-1-36-DH) (1'-2-3)

Polymer 22

621 mg (percent yield: 70%) of polymer 22 was obtained by the same method as in Example 1, except that compound (2"A-1-36-DH) (222 mg, 0.800 mmol), obtained in Synthesis Example 1, and compound (1 '-2-3) (390 mg, 1.20 mmol), obtained in Dihydroxy Compound Example 3, were used as diol monomers.

[Example 22-1]

A thin film was obtained by the same method as in Example 1-1, except that polymer 22 was used instead of polymer 1. The resulting thin film (thickness, 1 μm) was irradiated with 500 mJ/cm$^2$ of polarized ultraviolet light at 248 nm, followed by heating at 150°C. Retardations and wavelength dispersion properties are presented in Table 2.

[Example 22-2]

A thin film was obtained by the same method as in Example 1-1, except that polymer 22 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 2.

[Example 22-3]

A thin film was obtained by the same method as in Example 1-1, except that polymer 22 was used instead of polymer 1. The resulting thin film (thickness, 1 μm) was irradiated with 100 mJ/cm$^2$ of polarized ultraviolet light at 365 nm, followed by heating at 150°C. Retardations and wavelength dispersion properties are presented in Table 2.

[Example 22-4]

A thin film was obtained by the same method as in Example 1-1, except that polymer 22 was used instead of polymer 1. The resulting thin film (thickness, 1 μm) was irradiated with 500 mJ/cm$^2$ of polarized ultraviolet light at 365 nm, followed by heating at 150°C. Retardations and wavelength dispersion properties are presented in Table 2.

[Example 22-5]

A thin film was obtained by the same method as in Example 1-1, except that polymer 22 was used instead of polymer 1. The resulting thin film (thickness, 1 μm) was irradiated with 100 mJ/cm$^2$ of polarized ultraviolet light at 365 nm, followed by heating at 250°C. Retardations and wavelength dispersion properties are presented in Table 2.

[Example 22-6]

A thin film was obtained by the same method as in Example 1-1, except that polymer 22 was used instead of

polymer 1. The resulting thin film (thickness, 1 μm) was irradiated with 500 mJ/cm$^2$ of polarized ultraviolet light at 365 nm, followed by heating at 250°C. Retardations and wavelength dispersion properties are presented in Table 2.

[Example 23]

[Chem. 227]

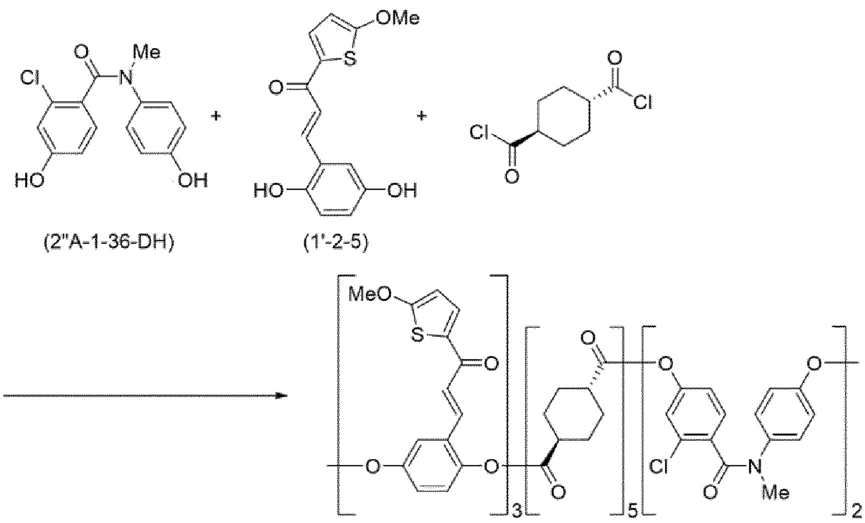

(2"A-1-36-DH) (1'-2-5)

Polymer 23

523 mg (percent yield: 63%) of polymer 23 was obtained by the same method as in Example 1, except that compound (2"A-1-36-DH) (222 mg, 0.800 mmol), obtained in Synthesis Example 1, and compound (1 ’-2-5) (331 mg, 1.20 mmol), obtained in Dihydroxy Compound Example 4, were used as diol monomers.

[Example 23-1]

A thin film was obtained by the same method as in Example 1-1, except that polymer 23 was used instead of polymer 1. The resulting thin film (thickness, 1 μm) was irradiated with 500 mJ/cm$^2$ of polarized ultraviolet light at 248 nm, followed by heating at 150°C. Retardations and wavelength dispersion properties are presented in Table 2.

[Example 23-2]

A thin film was obtained by the same method as in Example 1-1, except that polymer 23 was used instead of polymer 1. The resulting thin film (thickness, 1 μm) was irradiated with 1000 mJ/cm$^2$ of polarized ultraviolet light at 248 nm, followed by heating at 150°C. Retardations and wavelength dispersion properties are presented in Table 2.

[Example 23-3]

A thin film was obtained by the same method as in Example 1-1, except that polymer 23 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 2.

[Example 23-4]

A thin film was obtained by the same method as in Example 1-1, except that polymer 23 was used instead of polymer 1. The resulting thin film (thickness, 1 μm) was irradiated with 1000 mJ/cm$^2$ of polarized ultraviolet light at 248

nm, followed by heating at 250°C. Retardations and wavelength dispersion properties are presented in Table 2.

[Example 24]

[Chem. 228]

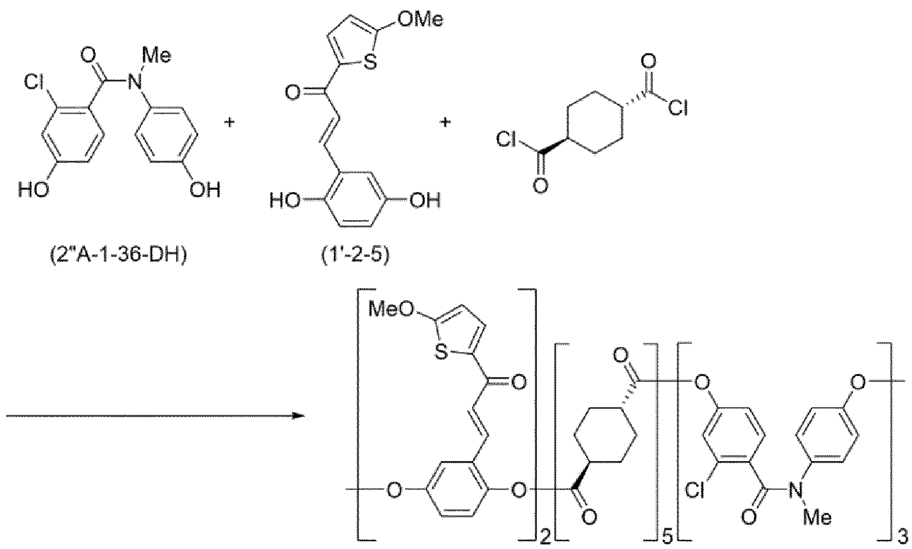

Polymer 24

690 mg (percent yield: 84%) of polymer 24 was obtained by the same method as in Example 1, except that compound (2"A-1-36-DH) (333 mg, 1.20 mmol), obtained in Synthesis Example 1, and compound (1'-2-5) (221 mg, 0.800 mmol), obtained in Dihydroxy Compound Example 4, were used as diol monomers.

[Example 24-1]

A thin film was obtained by the same method as in Example 1-1, except that polymer 24 was used instead of polymer 1. The resulting thin film (thickness, 1 μm) was irradiated with 500 mJ/cm$^2$ of polarized ultraviolet light at 248 nm, followed by heating at 150°C. Retardations and wavelength dispersion properties are presented in Table 2.

[Example 24-2]

A thin film was obtained by the same method as in Example 1-1, except that polymer 24 was used instead of polymer 1. The resulting thin film (thickness, 1 μm) was irradiated with 1000 mJ/cm$^2$ of polarized ultraviolet light at 248 nm, followed by heating at 150°C. Retardations and wavelength dispersion properties are presented in Table 2.

[Example 24-3]

A thin film was obtained by the same method as in Example 1-1, except that polymer 24 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 2.

[Example 24-4]

A thin film was obtained by the same method as in Example 1-1, except that polymer 24 was used instead of polymer 1. The resulting thin film (thickness, 1 μm) was irradiated with 1000 mJ/cm$^2$ of polarized ultraviolet light at 248 nm, followed by heating at 250°C. Retardations and wavelength dispersion properties are presented in Table 2.

[Example 25]

[Chem. 229]

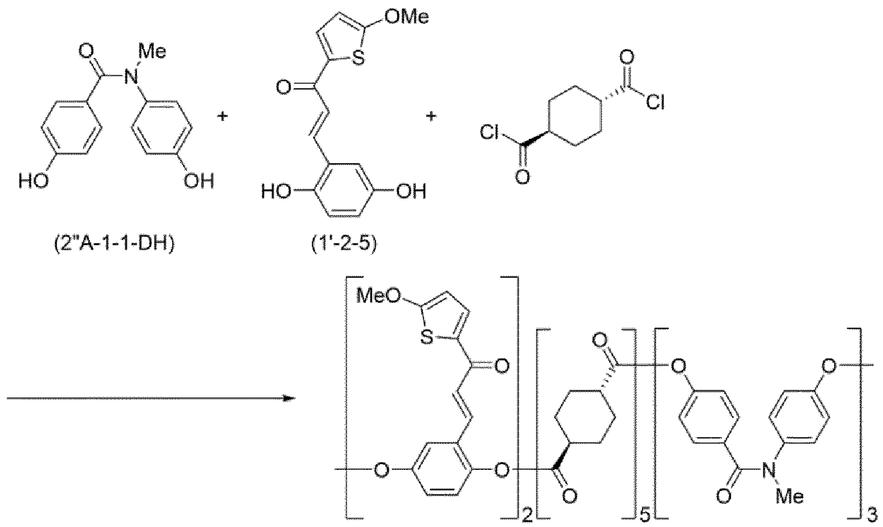

(2"A-1-1-DH) (1'-2-5)

Polymer 25

512 mg (percent yield: 65%) of polymer 25 was obtained by the same method as in Example 1, except that compound (2"A-1-1-DH) (292 mg, 1.20 mmol), obtained in Synthesis Example 2, and compound (1 ’-2-5) (221 mg, 0.800 mmol), obtained in Dihydroxy Compound Example 4, were used as diol monomers.

[Example 25-1]

A thin film was obtained by the same method as in Example 1-1, except that polymer 25 was used instead of polymer 1. The resulting thin film (thickness, 1 μm) was irradiated with 500 mJ/cm$^2$ of polarized ultraviolet light at 248 nm, followed by heating at 150°C. Retardations and wavelength dispersion properties are presented in Table 2.

[Example 25-2]

A thin film was obtained by the same method as in Example 1-1, except that polymer 25 was used instead of polymer 1. The resulting thin film (thickness, 1 μm) was irradiated with 1000 mJ/cm$^2$ of polarized ultraviolet light at 248 nm, followed by heating at 150°C. Retardations and wavelength dispersion properties are presented in Table 2.

[Example 25-3]

A thin film was obtained by the same method as in Example 1-1, except that polymer 25 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 2.

[Example 25-4]

A thin film was obtained by the same method as in Example 1-1, except that polymer 25 was used instead of polymer 1. The resulting thin film (thickness, 1 μm) was irradiated with 1000 mJ/cm$^2$ of polarized ultraviolet light at 248 nm, followed by heating at 250°C. Retardations and wavelength dispersion properties are presented in Table 2.

[Example 26]

[Chem. 230]

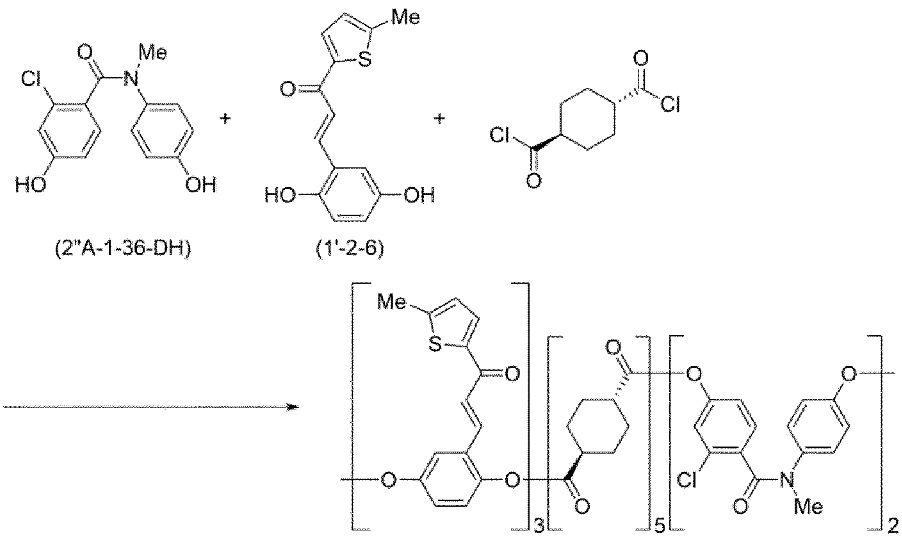

(2"A-1-36-DH) (1'-2-6)

Polymer 26

523 mg (percent yield: 65%) of polymer 26 was obtained by the same method as in Example 1, except that compound (2"A-1-36-DH) (222 mg, 0.800 mmol), obtained in Synthesis Example 1, and compound (1'-2-6) (312 mg, 1.20 mmol), obtained in Dihydroxy Compound Example 5, were used as diol monomers.

[Example 26-1]

A thin film was obtained by the same method as in Example 1-1, except that polymer 26 was used instead of polymer 1. The resulting thin film (thickness, 1 μm) was irradiated with 500 mJ/cm$^2$ of polarized ultraviolet light at 248 nm, followed by heating at 250°C. Retardations and wavelength dispersion properties are presented in Table 2.

[Example 26-2]

A thin film was obtained by the same method as in Example 1-1, except that polymer 26 was used instead of polymer 1. The resulting thin film (thickness, 1 μm) was irradiated with 1000 mJ/cm$^2$ of polarized ultraviolet light at 248 nm, followed by heating at 250°C. Retardations and wavelength dispersion properties are presented in Table 2.

[Example 27]

[Chem. 231]

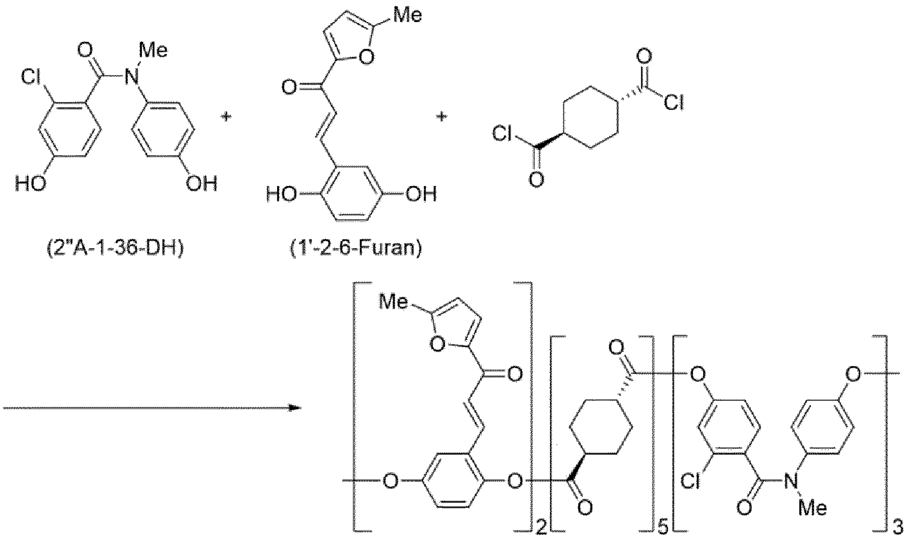

(2"A-1-36-DH) (1'-2-6-Furan)

Polymer 27

445 mg (percent yield: 56%) of polymer 27 was obtained by the same method as in Example 1, except that compound (2"A-1-36-DH) (333 mg, 1.20 mmol), obtained in Synthesis Example 1, and compound (1’-2-6-Furan) (195 mg, 0.800 mmol), obtained in Dihydroxy Compound Example 6, were used as diol monomers.

[Example 27-1]

A thin film was obtained by the same method as in Example 1-1, except that polymer 27 was used instead of polymer 1. The resulting thin film (thickness, 1 μm) was irradiated with 500 mJ/cm$^2$ of polarized ultraviolet light at 248 nm, followed by heating at 150°C. Retardations and wavelength dispersion properties are presented in Table 2.

[Example 27-2]

A thin film was obtained by the same method as in Example 1-1, except that polymer 27 was used instead of polymer 1. The resulting thin film (thickness, 1 μm) was irradiated with 1000 mJ/cm$^2$ of polarized ultraviolet light at 248 nm, followed by heating at 150°C. Retardations and wavelength dispersion properties are presented in Table 2.

[Example 28]

[Chem. 232]

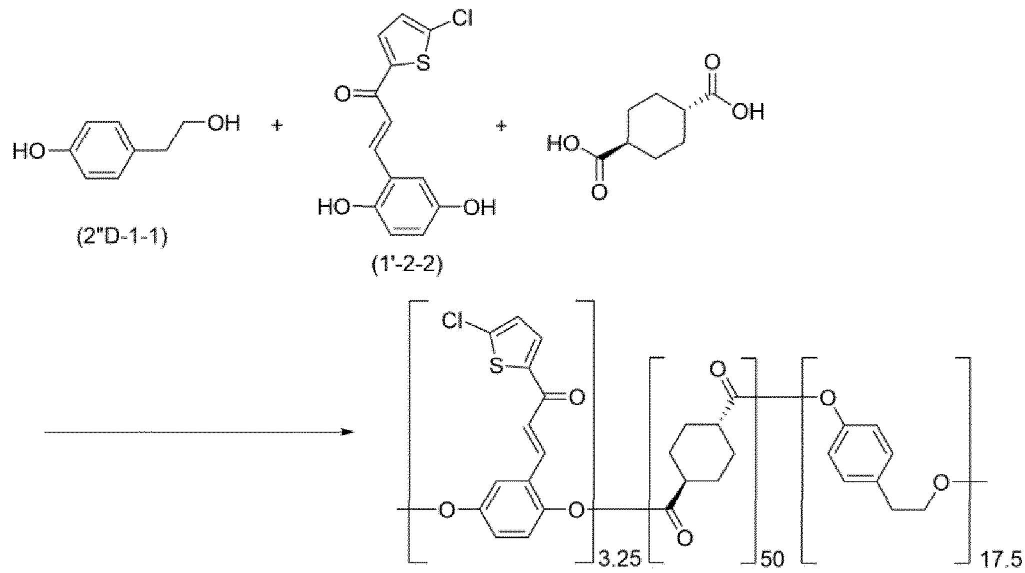

(2"D-1-1)

(1'-2-2)

Polymer 28

In a nitrogen stream, compound (2"D-1-1) (281 mg, 2.03 mmol) and compound (1'-2-2) (1.06 g, 3.78 mmol), obtained in Dihydroxy Compound Example 2, as diol monomers and trans-1,4-cyclohexanedicarboxylic acid (400 mg, 2.32 mmol) as a dicarboxylic acid monomer were added to a reaction vessel. Then pyridine (7.9 mL) as a solvent was added, and stirring was initiated with 30°C warming. As a condensing agent, furthermore, diisopropylcarbodiimide (1.61 g, 12.8 mmol) was added. After the reaction system was stirred for 3 hours, the mixture in the system was added to methanol (300 mL). The solid that formed was collected by filtration, washed with methanol (500 mL) and water (300 mL) and then vacuum-dried, giving 1.56 g (percent yield: 73%) of polymer 28.

[Example 28-1]

A thin film was obtained by the same method as in Example 1-1, except that polymer 28 was used instead of polymer 1. The resulting thin film (thickness, 1 μm) was irradiated with 100 $mJ/cm^2$ of polarized ultraviolet light at 365 nm, followed by heating at 150°C. Retardations and wavelength dispersion properties are presented in Table 3.

[Example 28-2]

A thin film was obtained by the same method as in Example 1-1, except that polymer 28 was used instead of polymer 1. The resulting thin film (thickness, 1 μm) was irradiated with 100 $mJ/cm^2$ of polarized ultraviolet light at 365 nm, followed by heating at 190°C. Retardations and wavelength dispersion properties are presented in Table 3.

[Example 29]

[Chem. 233]

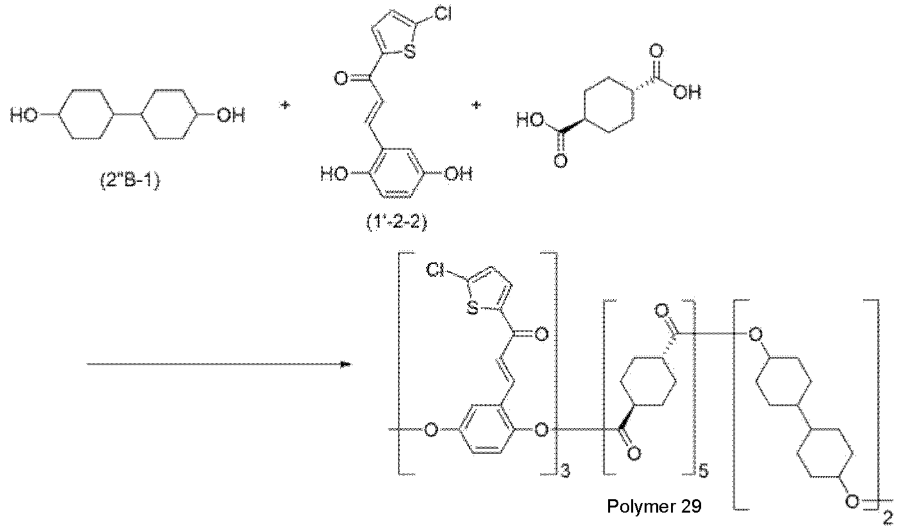

(2"B-1)

(1'-2-2)

Polymer 29

606 mg (percent yield: 54%) of polymer 29 was obtained by the same method as in Example 28, except that compound (2"B-1) (230 mg, 1.16 mmol) and compound (1'-2-2) (489 mg, 1.74 mmol), obtained in Dihydroxy Compound Example 2, were used as diol monomers, that trans-1,4-cyclohexanedicarboxylic acid (500 mg, 2.90 mmol) was used as a dicarboxylic acid monomer, that diisopropylcarbodiimide (806 mg, 6.39 mmol) was used as a condensing agent and that pyridine (4.2 mL) was used as a solvent.

[Example 29-1]

A thin film was obtained by the same method as in Example 1-1, except that polymer 29 was used instead of polymer 1. The resulting thin film (thickness, 1 μm) was irradiated with 100 mJ/cm$^2$ of polarized ultraviolet light at 365 nm, followed by heating at 150°C. Retardations and wavelength dispersion properties are presented in Table 3.

[Example 29-2]

A thin film was obtained by the same method as in Example 1-1, except that polymer 29 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 3.

[Example 30]

[Chem. 234]

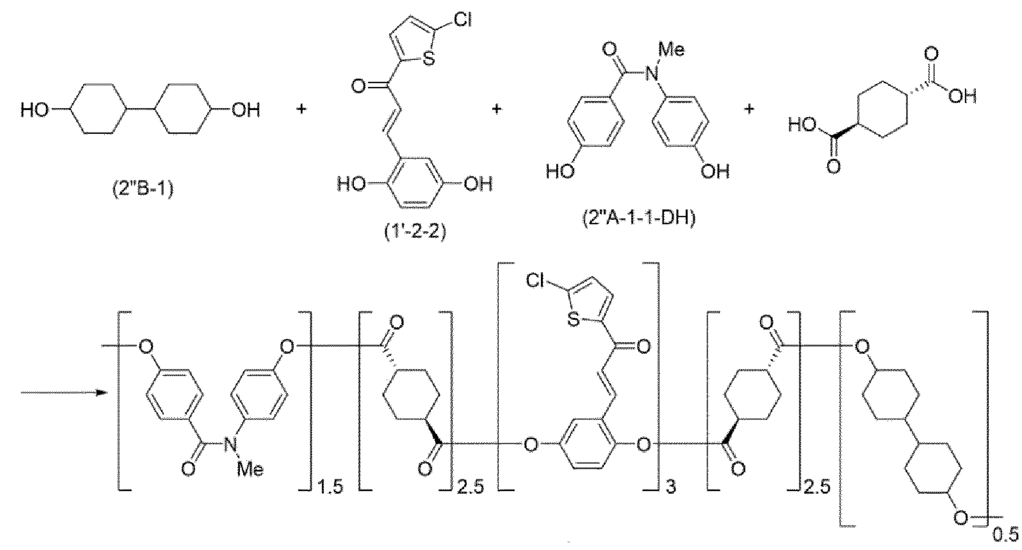

(2"B-1)

(1'-2-2)

(2"A-1-1-DH)

Polymer 30

837 mg (percent yield: 73%) of polymer 30 was obtained by the same method as in Example 28, except that compound (2"B-1) (57.6 mg, 0.290 mmol), compound (1'-2-2) (489 mg, 1.74 mmol), obtained in Dihydroxy Compound Example 2, and compound (2"A-1-1-DH) (212 mg, 0.871 mmol), obtained in Synthesis Example 2, were used as diol monomers, that trans-1,4-cyclohexanedicarboxylic acid (500 mg, 2.90 mmol) was used as a dicarboxylic acid monomer, that diisopropylcarbodiimide (806 mg, 6.39 mmol) was used as a condensing agent and that pyridine (4.3 mL) was used as a solvent.

[Example 30-1]

A thin film was obtained by the same method as in Example 1-1, except that polymer 30 was used instead of polymer 1. The resulting thin film (thickness, 1 μm) was irradiated with 100 mJ/cm$^2$ of polarized ultraviolet light at 365 nm, followed by heating at 150°C. Retardations and wavelength dispersion properties are presented in Table 3.

[Example 30-2]

A thin film was obtained by the same method as in Example 1-1, except that polymer 30 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 3.

[Example 31]

[Chem. 235]

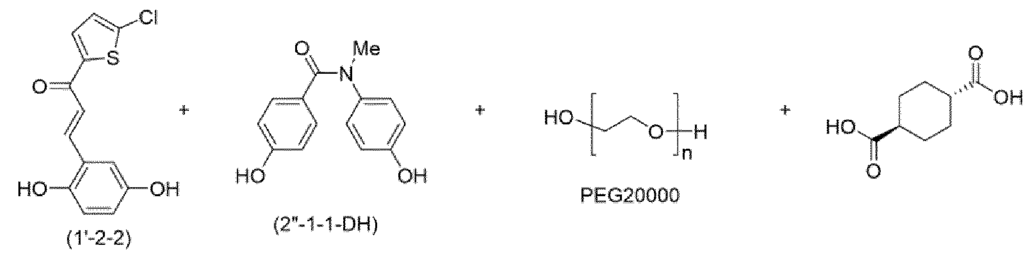

(1'-2-2)

(2"-1-1-DH)

PEG20000

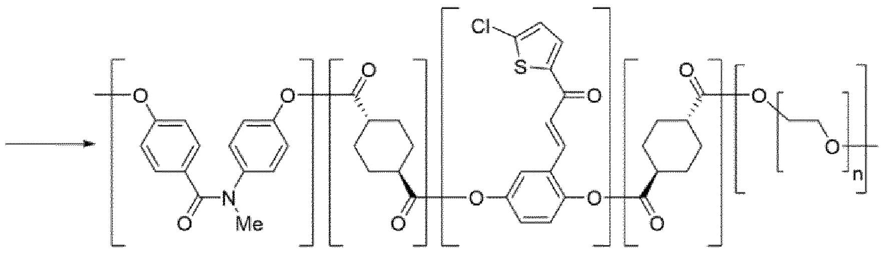

Polymer 31

4.53 g (percent yield: 58%) of polymer 31 was obtained by the same method as in Example 28, except that compound (2"-1-1-DH) (1.44 g, 5.92 mmol), obtained in Synthesis Example 2, compound (1'-2-2) (3.22 g, 11.5 mmol), obtained in Dihydroxy Compound Example 2, and PEG 20000 (801 mg) were used as diol monomers, that trans-1,4-cyclohexanedicarboxylic acid (3.00g, 17.4 mmol) was used as a dicarboxylic acid monomer, that diisopropylcarbodiimide (4.84 g, 38.3 mmol) was used as a condensing agent and that pyridine (29.6 mL) was used as a solvent.

[Example 31-1]

A thin film was obtained by the same method as in Example 1-1, except that polymer 31 was used instead of polymer 1. The resulting thin film (thickness, 1 μm) was irradiated with 100 mJ/$cm^2$ of polarized ultraviolet light at 365 nm, followed by heating at 150°C. Retardations and wavelength dispersion properties are presented in Table 3.

[Example 31-2]

A thin film was obtained by the same method as in Example 1-1, except that polymer 31 was used instead of polymer 1. The resulting thin film (thickness, 1 μm) was irradiated with 100 mJ/$cm^2$ of polarized ultraviolet light at 365 nm, followed by heating at 190°C. Retardations and wavelength dispersion properties are presented in Table 3.

[Example 32]

[Chem. 236]

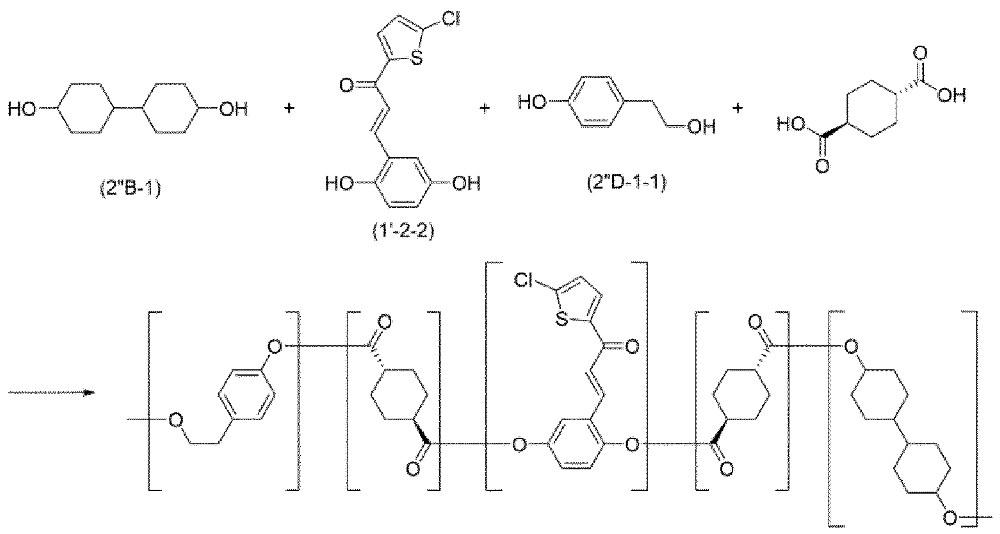

(2"B-1) (1'-2-2) (2"D-1-1)

Polymer 32

1.36 g (percent yield: 65%) of polymer 32 was obtained by the same method as in Example 28, except that compound (2"B-1) (230 mg, 1.16 mmol), compound (2"D-1-1) (225 mg, 1.63 mmol) and compound (1'-2-2) (848 mg, 3.02 mmol), obtained in Dihydroxy Compound Example 2, were used as diol monomers, that trans-1,4-cyclohexanedicarboxylic acid (1.00 g, 5.81 mmol) was used as a dicarboxylic acid monomer, that diisopropylcarbodiimide (1.61 g, 12.8 mmol) was used as a condensing agent and that pyridine (16.2 mL) was used as a solvent.

[Example 32-1]

A thin film was obtained by the same method as in Example 1-1, except that polymer 32 was used instead of polymer 1. The resulting thin film (thickness, 1 μm) was irradiated with 100 $mJ/cm^2$ of polarized ultraviolet light at 365 nm, followed by heating at 150°C. Retardations and wavelength dispersion properties are presented in Table 3.

[Example 32-2]

A thin film was obtained by the same method as in Example 1-1, except that polymer 32 was used instead of polymer 1. The resulting thin film (thickness, 1 μm) was irradiated with 100 $mJ/cm^2$ of polarized ultraviolet light at 365 nm, followed by heating at 190°C. Retardations and wavelength dispersion properties are presented in Table 3.

[Example 33]

[Chem. 237]

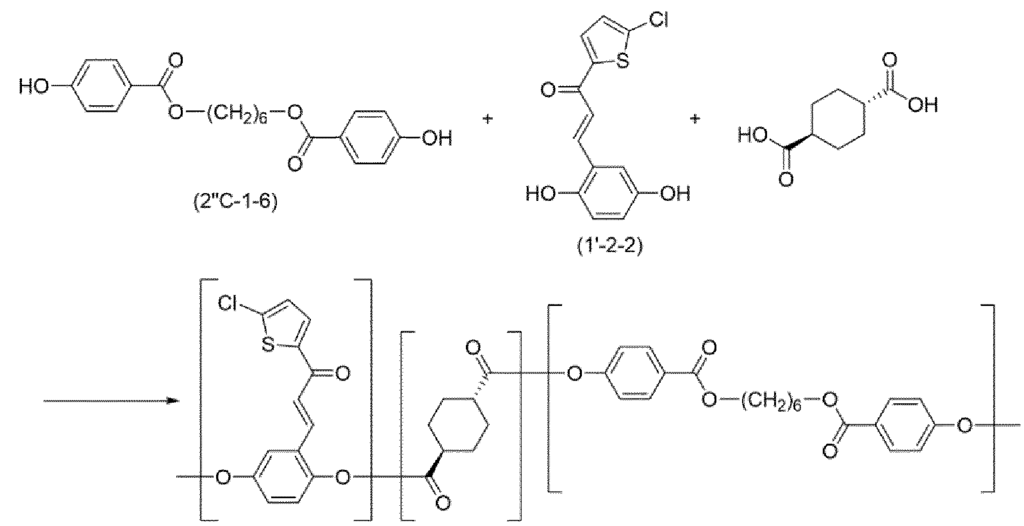

(2"C-1-6)

(1'-2-2)

Polymer 33

2.02g (percent yield: 78%) of polymer 33 was obtained by the same method as in Example 28, except that compound (2"C-1-6) (833 mg, 2.32 mmol) and compound (1'-2-2) (978 mg, 3.49 mmol), obtained in Dihydroxy Compound Example 2, were used as diol monomers, that trans-1,4-cyclohexanedicarboxylic acid (1.00 mg, 5.81 mmol) was used as a dicarboxylic acid monomer, that diisopropylcarbodiimide (1.61 g, 12.8 mmol) was used as a condensing agent and that pyridine (9.8 mL) was used as a solvent.

[Example 33-1]

A thin film was obtained by the same method as in Example 1-1, except that polymer 33 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 3.

[Example 34]

[Chem. 238]

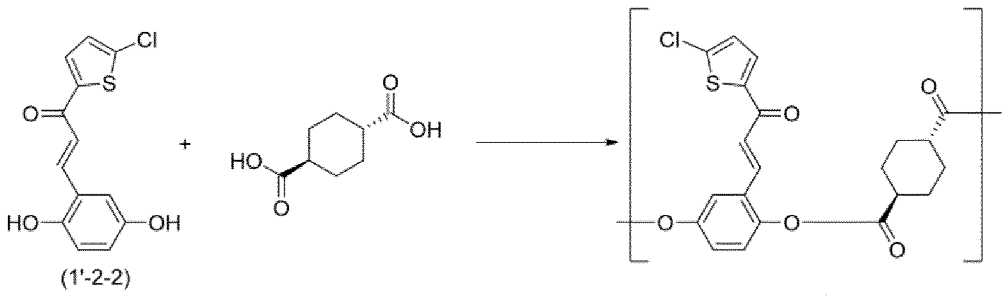

(1'-2-2)

Polymer 34

1.77 g (percent yield: 85%) of polymer 34 was obtained by the same method as in Example 28, except that compound (1'-2-2) (1.40 g, 5.00 mmol), obtained in Dihydroxy Compound Example 2, was used as a diol monomer, that trans-1,4-cyclohexanedicarboxylic acid (861 mg, 5.00 mmol) was used as a dicarboxylic acid monomer, that diisopropylcarbodiimide (1.39 g, 11.0 mmol) was used as a condensing agent and that pyridine (2.0 mL) and NMP (1.9 mL)

were used as solvents.

[Example 34-1]

A thin film was obtained by the same method as in Example 1-1, except that polymer 34 was used instead of polymer 1. The resulting thin film (thickness, 1 μm) was irradiated with 100 mJ/cm$^2$ of polarized ultraviolet light at 365 nm, followed by heating at 150°C. Retardations and wavelength dispersion properties are presented in Table 3.

[Example 35]

[Chem. 239]

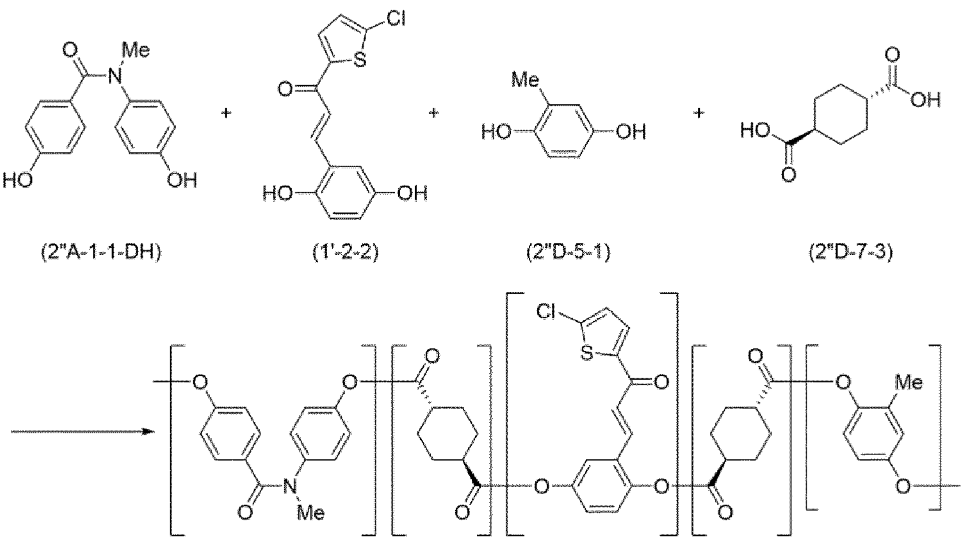

(2"A-1-1-DH) (1'-2-2) (2"D-5-1) (2"D-7-3)

Polymer 35

884 mg (percent yield: 76%) of polymer 35 was obtained by the same method as in Example 28, except that compound (2"A-1-1-DH) (212 mg, 0.871 mmol), obtained in Synthesis Example 2, compound (1'-2-2) (489 mg, 1.74 mmol), obtained in Dihydroxy Compound Example 2, and methylhydroquinone (2"D-5-1) (36.0 mg, 0.290 mmol) were used as diol monomers, that trans-1,4-cyclohexanedicarboxylic acid (2"D-7-3) (500 mg, 2.90 mmol) was used as a dicarboxylic acid monomer, that diisopropylcarbodiimide (806 mg, 6.39 mmol) was used as a condensing agent and that pyridine (4.2 mL) was used as a solvent.

[Example 35-1]

A thin film was obtained by the same method as in Example 1-1, except that polymer 35 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 4.

[Example 36]

[Chem. 240]

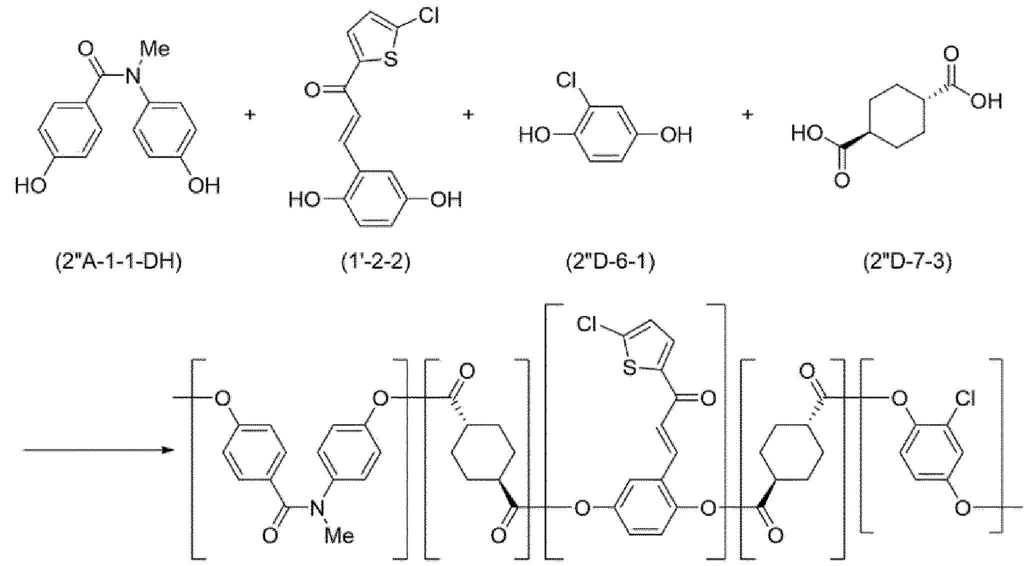

(2"A-1-1-DH) (1'-2-2) (2"D-6-1) (2"D-7-3)

Polymer 36

889 mg (percent yield: 78%) of polymer 36 was obtained by the same method as in Example 28, except that compound (2"A-1-1-DH) (212 mg, 0.871 mmol), obtained in Synthesis Example 2, compound (1'-2-2) (489 mg, 1.74 mmol), obtained in Dihydroxy Compound Example 2, and chlorohydroquinone (2"D-6-1) (42.0 mg, 0.290 mmol) were used as diol monomers, that trans-1,4-cyclohexanedicarboxylic acid (2"D-7-3) (500 mg, 2.90 mmol) was used as a dicarboxylic acid monomer, that diisopropylcarbodiimide (806 mg, 6.39 mmol) was used as a condensing agent and that pyridine (4.2 mL) was used as a solvent.

[Example 36-1]

A thin film was obtained by the same method as in Example 1-1, except that polymer 36 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 4.

[Example 37]

[Chem. 241]

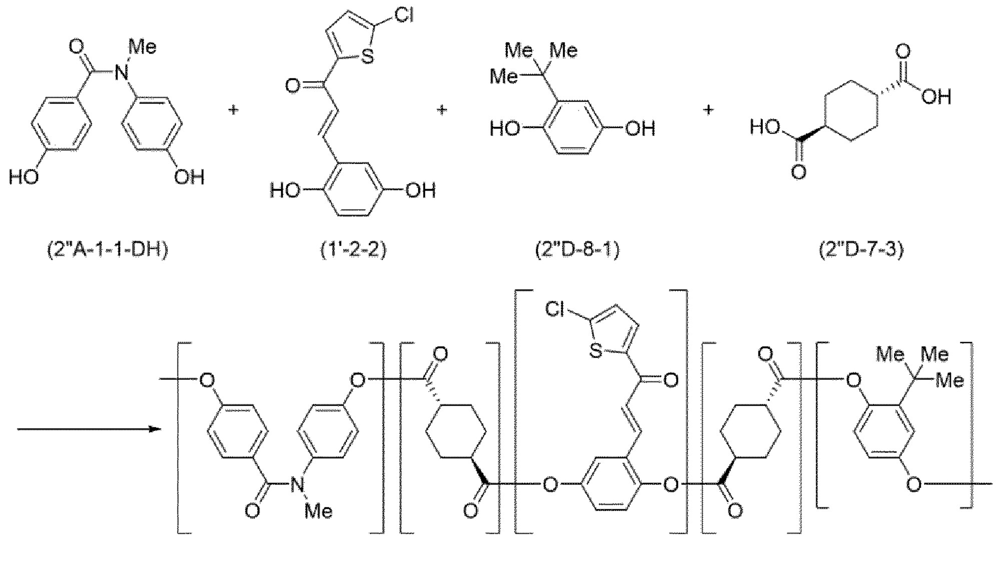

(2"A-1-1-DH) (1'-2-2) (2"D-8-1) (2"D-7-3)

Polymer 37

872 mg (percent yield: 76%) of polymer 37 was obtained by the same method as in Example 28, except that compound (2"A-1-1-DH) (212 mg, 0.871 mmol), obtained in Synthesis Example 2, compound (1'-2-2) (489 mg, 1.74 mmol), obtained in Dihydroxy Compound Example 2, and tert-butylhydroquinone (2"D-8-1) (48.3 mg, 0.290 mmol) were used as diol monomers, that trans-1,4-cyclohexanedicarboxylic acid (2"D-7-3) (500 mg, 2.90 mmol) was used as a dicarboxylic acid monomer, that diisopropylcarbodiimide (806 mg, 6.39 mmol) was used as a condensing agent and that pyridine (4.3 mL) was used as a solvent.

[Example 37-1]

A thin film was obtained by the same method as in Example 1-1, except that polymer 37 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 4.

[Example 38]

[Chem. 242]

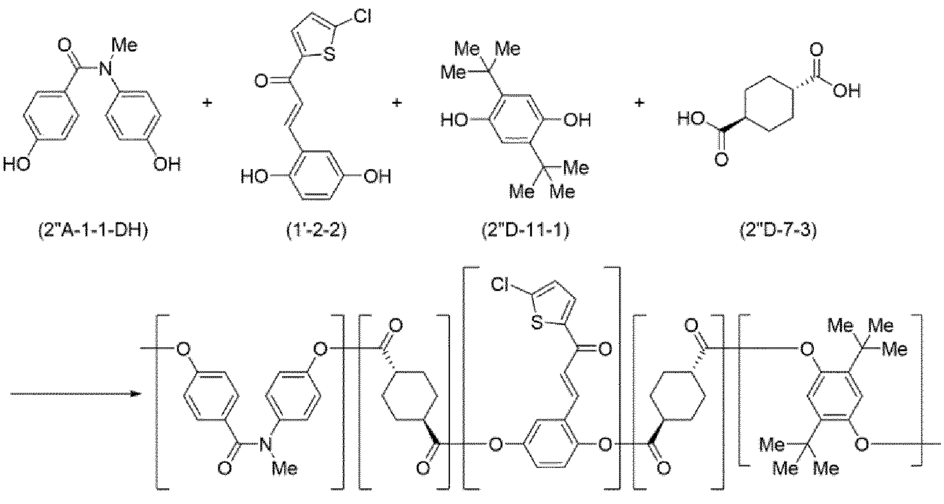

(2"A-1-1-DH) (1'-2-2) (2"D-11-1) (2"D-7-3)

Polymer 38

876 mg (percent yield: 76%) of polymer 38 was obtained by the same method as in Example 28, except that compound (2"A-1-1-DH) (212 mg, 0.871 mmol), obtained in Synthesis Example 2, compound (1'-2-2) (489 mg, 1.74 mmol), obtained in Dihydroxy Compound Example 2, and 2,5-di tert-butylhydroquinone (2"D-11-1) (64.6 mg, 0.290 mmol) were used as diol monomers, that trans-1,4-cyclohexanedicarboxylic acid (2"D-7-3) (500 mg, 2.90 mmol) was used as a dicarboxylic acid monomer, that diisopropylcarbodiimide (806 mg, 6.39 mmol) was used as a condensing agent and that pyridine (4.3 mL) was used as a solvent.

[Example 38-1]

A thin film was obtained by the same method as in Example 1-1, except that polymer 38 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 4.

[Example 39]

[Chem. 243]

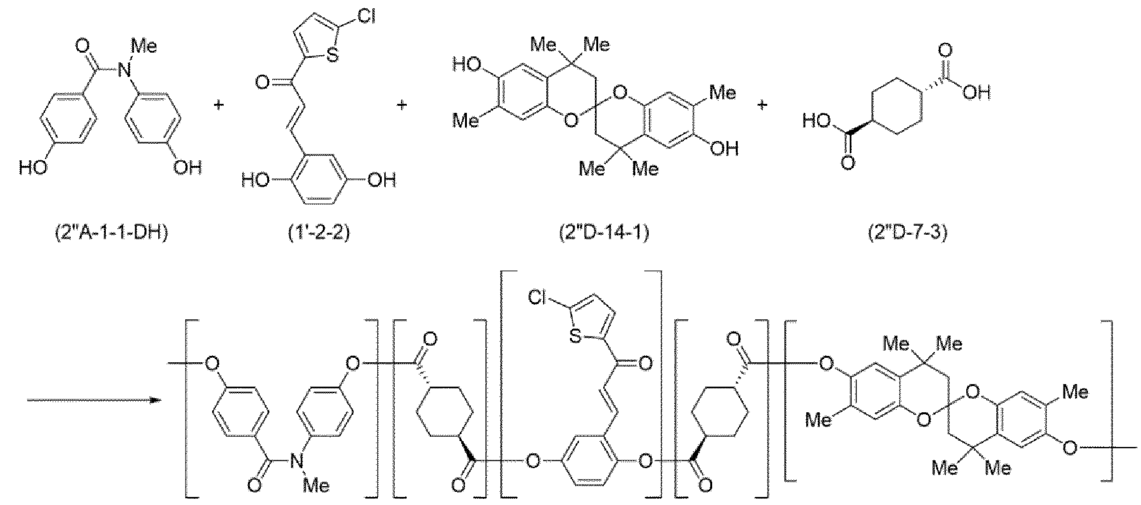

(2"A-1-1-DH) (1'-2-2) (2"D-14-1) (2"D-7-3)

Polymer 39

841 mg (percent yield: 64%) of polymer 39 was obtained by the same method as in Example 28, except that compound (2"A-1-1-DH) (212 mg, 0.871 mmol), obtained in Synthesis Example 2, compound (1'-2-2) (489 mg, 1.74 mmol), obtained in Dihydroxy Compound Example 2, and 6,6'-dihydroxy-4,4,4',4',7,7'-hexamethyl-2,2'-spirobicromane (2"D-14-1) (107 mg, 0.290 mmol) were used as diol monomers, that trans-1,4-cyclohexanedicarboxylic acid (2"D-7-3) (500 mg, 2.90 mmol) was used as a dicarboxylic acid monomer, that diisopropylcarbodiimide (806 mg, 6.39 mmol) was used as a condensing agent and that pyridine (4.5 mL) was used as a solvent.

[Example 39-1]

A thin film was obtained by the same method as in Example 1-1, except that polymer 39 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 4.

[Example 40]

[Chem. 244]

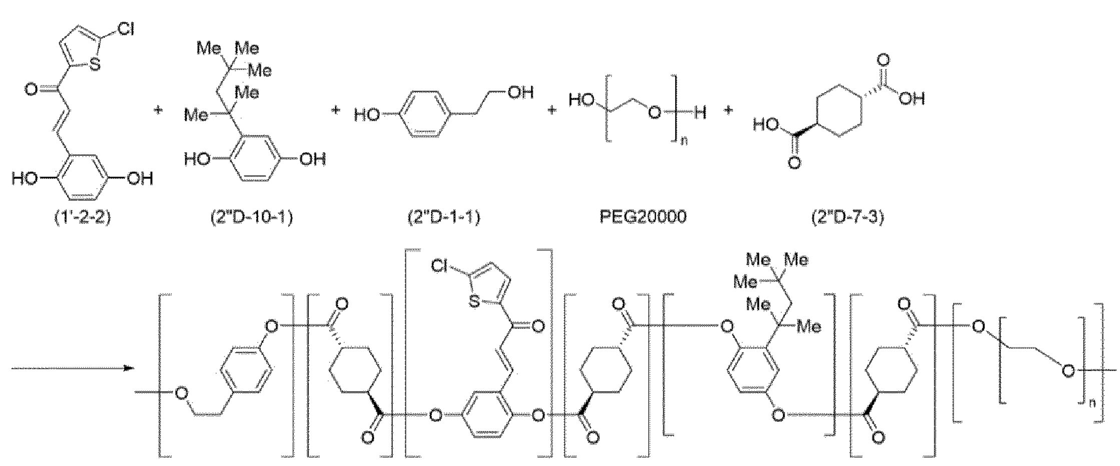

(1'-2-2) (2"D-10-1) (2"D-1-1) PEG20000 (2"D-7-3)

Polymer 40

1.33 g (percent yield: 59%) of polymer 40 was obtained by the same method as in Example 28, except that compound (1 '-2-2) (847 mg, 3.02 mmol), obtained in Dihydroxy Compound Example 2, compound (2"D-1-1) (192 mg, 1.39 mmol), 2-(1,1,3,3-tetramethylbutyl)hydroquinone (2"D-10-1) (310 mg, 1.39 mmol) and PEG 20000 (116 mg, 5.81 μmol) were used as diol monomers, that trans-1,4-cyclohexanedicarboxylic acid (2"D-7-3) (1.00 g, 5.81 mmol) was used as a dicarboxylic acid monomer, that diisopropylcarbodiimide (1.61 g, 12.8 mmol) was used as a condensing agent and that pyridine (8.4 mL) was used as a solvent.

[Example 40-1]

A thin film was obtained by the same method as in Example 1-1, except that polymer 40 was used instead of polymer 1. The resulting thin film (thickness, 1 μm) was irradiated with 100 mJ/cm$^2$ of polarized ultraviolet light at 365 nm, followed by heating at 150°C. Retardations and wavelength dispersion properties are presented in Table 4.

[Example 41]

[Chem. 245]

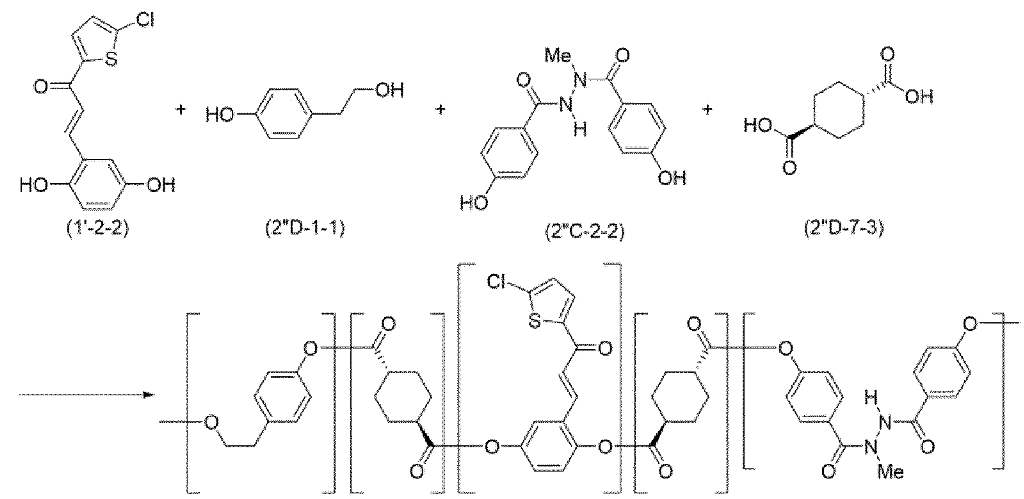

(1'-2-2) (2"D-1-1) (2"C-2-2) (2"D-7-3)

Polymer 41

1.71 g (percent yield: 78%) of polymer 41 was obtained by the same method as in Example 28, except that compound (1 '-2-2) (1.04 g, 3.72 mmol), obtained in Dihydroxy Compound Example 2, compound (2"D-1-1) (225 mg, 1.63 mmol) and compound (2"C-2-2) (133 mg, 465 μmol), obtained in Synthesis Example 11, were used as diol monomers, that trans-1,4-cyclohexanedicarboxylic acid (2"D-7-3) (1.00 g, 5.81 mmol) was used as a dicarboxylic acid monomer, that diisopropylcarbodiimide (1.61 g, 12.8 mmol) was used as a condensing agent and that pyridine (8.1 mL) was used as a solvent.

[Example 41-1]

A thin film was obtained by the same method as in Example 1-1, except that polymer 41 was used instead of polymer 1. The resulting thin film (thickness, 1 μm) was irradiated with 100 mJ/cm$^2$ of polarized ultraviolet light at 365 nm, followed by heating at 150°C. Retardations and wavelength dispersion properties are presented in Table 4.

[Example 42]

[Chem. 246]

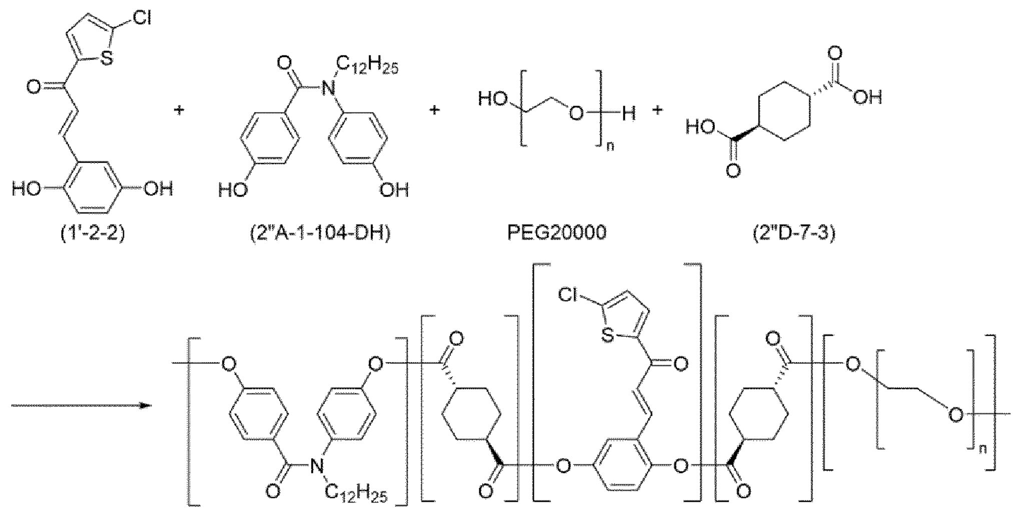

(1'-2-2) (2"A-1-104-DH) PEG20000 (2"D-7-3)

Polymer 42

1.80 g (percent yield: 63%) of polymer 42 was obtained by the same method as in Example 28, except that compound (1 '-2-2) (1.24 g, 4.43 mmol), obtained in Dihydroxy Compound Example 2, compound (2"A-1-104-DH) (541 mg, 1.36 mmol), obtained in Synthesis Example 12, and PEG 20000 (263 mg, 13.1 μmol) were used as diol monomers, that trans-1,4-cyclohexanedicarboxylic acid (2"D-7-3) (1.00 g, 5.81 mmol) was used as a dicarboxylic acid monomer, that diisopropylcarbodiimide (1.61 g, 12.8 mmol) was used as a condensing agent and that pyridine (10.8 mL) was used as a solvent.

[Example 42-1]

A thin film was obtained by the same method as in Example 1-1, except that polymer 42 was used instead of polymer 1. The resulting thin film (thickness, 1 μm) was irradiated with 100 mJ/cm$^2$ of polarized ultraviolet light at 365 nm, followed by heating at 150°C. Retardations and wavelength dispersion properties are presented in Table 4.

[Example 43]

[Chem. 247]

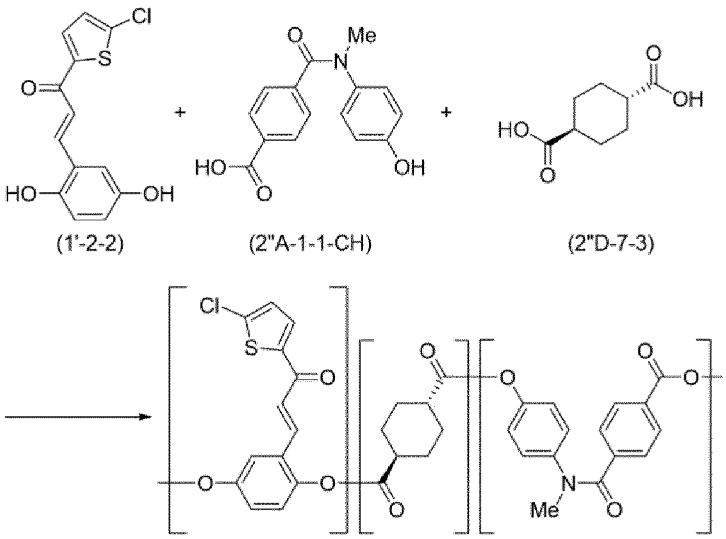

(1'-2-2) (2"A-1-1-CH) (2"D-7-3)

Polymer 43

1.47 g (percent yield: 61%) of polymer 43 was obtained by the same method as in Example 28, except that compound (1 '-2-2) (1.06 g, 3.78 mmol), obtained in Dihydroxy Compound Example 2, was used as a diol monomer, that compound (2"A-1-1-CH) (879 mg, 3.24 mmol), obtained in Synthesis Example 13, was used as a hydroxycarboxylic acid monomer, that trans-1,4-cyclohexanedicarboxylic acid (2"D-7-3) (651 mg, 3.78 mmol) was used as a dicarboxylic acid monomer, that diisopropylcarbodiimide (1.50 g, 11.9 mmol) was used as a condensing agent and that pyridine (9.1 mL) was used as a solvent.

[Example 43-1]

A thin film was obtained by the same method as in Example 1-1, except that polymer 43 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 4.

[Example 44]

[Chem. 248]

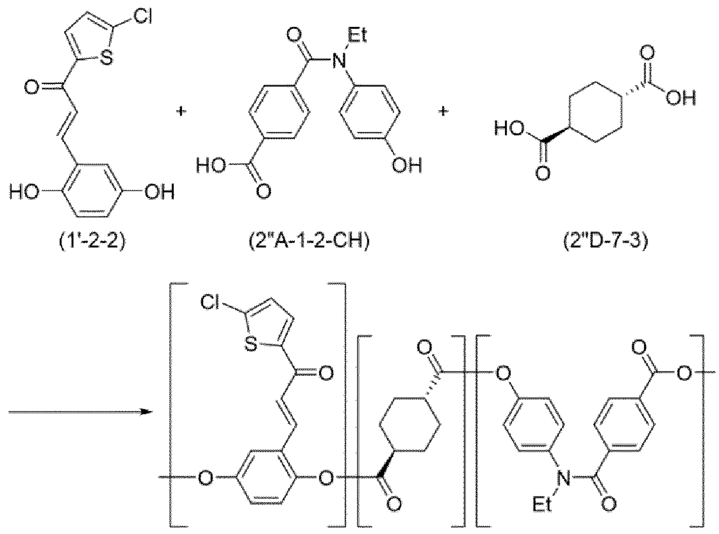

(1'-2-2) (2"A-1-2-CH) (2"D-7-3)

Polymer 44

1.42 g (percent yield: 58%) of polymer 44 was obtained by the same method as in Example 28, except that compound (1'-2-2) (1.06 mg, 3.78 mmol), obtained in Dihydroxy Compound Example 2, was used as a diol monomer, that compound (2"A-1-2-CH) (925 mg, 3.24 mmol), obtained in Synthesis Example 14, was used as a hydroxycarboxylic acid monomer, that trans-1,4-cyclohexanedicarboxylic acid (2"D-7-3) (651 mg, 3.78 mmol) was used as a dicarboxylic acid monomer, that diisopropylcarbodiimide (1.50 g, 11.9 mmol) was used as a condensing agent and that pyridine (9.2 mL) was used as a solvent.

[Example 44-1]

A thin film was obtained by the same method as in Example 1-1, except that polymer 44 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 4.

[Example 45]

[Chem. 249]

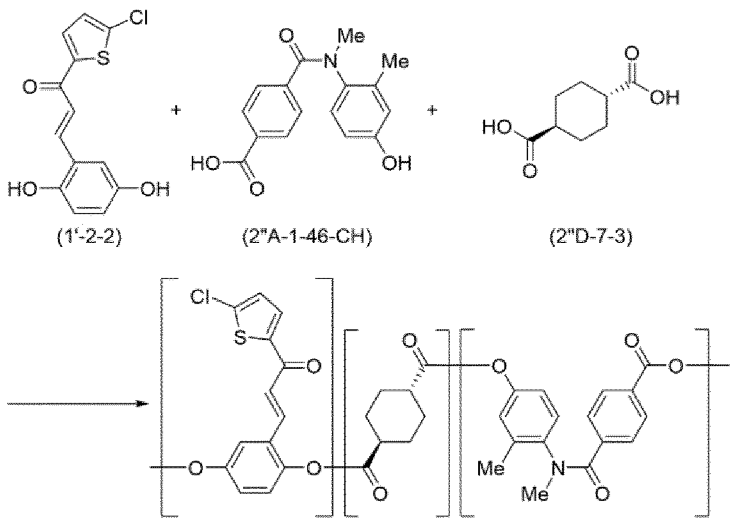
(1'-2-2) (2"A-1-46-CH) (2"D-7-3)

Polymer 45

1.02 g (percent yield: 62%) of polymer 45 was obtained by the same method as in Example 28, except that compound (1 '-2-2) (708 mg, 2.52 mmol), obtained in Dihydroxy Compound Example 2, was used as a diol monomer, that compound (2"A-1-46-CH) (617 mg, 2.16 mmol), obtained in Synthesis Example 15, was used as a hydroxycarboxylic acid monomer, that trans-1,4-cyclohexanedicarboxylic acid (2"D-7-3) (434 mg, 2.52 mmol) was used as a dicarboxylic acid monomer, that diisopropylcarbodiimide (1.00 g, 7.92 mmol) was used as a condensing agent and that pyridine (6.1 mL) was used as a solvent.

[Example 45-1]

A thin film was obtained by the same method as in Example 1-1, except that polymer 45 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 4.

[Example 46]

[Chem. 250]

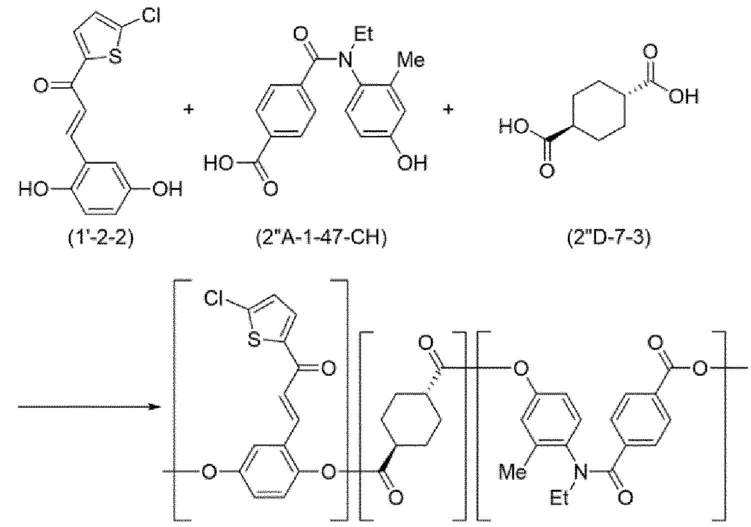

(1'-2-2) (2"A-1-47-CH) (2"D-7-3)

Polymer 46

1.28 g (percent yield: 77%) of polymer 46 was obtained by the same method as in Example 28, except that compound (1 '-2-2) (708 mg, 2.52 mmol), obtained in Dihydroxy Compound Example 2, was used as a diol monomer, that compound (2"A-1-47-CH) (647 mg, 2.16 mmol), obtained in Synthesis Example 16, was used as a hydroxycarboxylic acid monomer, that trans-1,4-cyclohexanedicarboxylic acid (2"D-7-3) (434 mg, 2.52 mmol) was used as a dicarboxylic acid monomer, that diisopropylcarbodiimide (1.00 g, 7.92 mmol) was used as a condensing agent and that pyridine (6.3 mL) was used as a solvent.

[Example 46-1]

A thin film was obtained by the same method as in Example 1-1, except that polymer 46 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 4.

[Example 46-2]

A thin film was obtained by the same method as in Example 1-1, except that polymer 46 was used instead of polymer 1. The resulting thin film (thickness, 1 μm) was irradiated with 100 mJ/cm$^2$ of polarized ultraviolet light at 365 nm, followed by heating at 250°C. Retardations and wavelength dispersion properties are presented in Table 4.

[Example 47]

[Chem. 251]

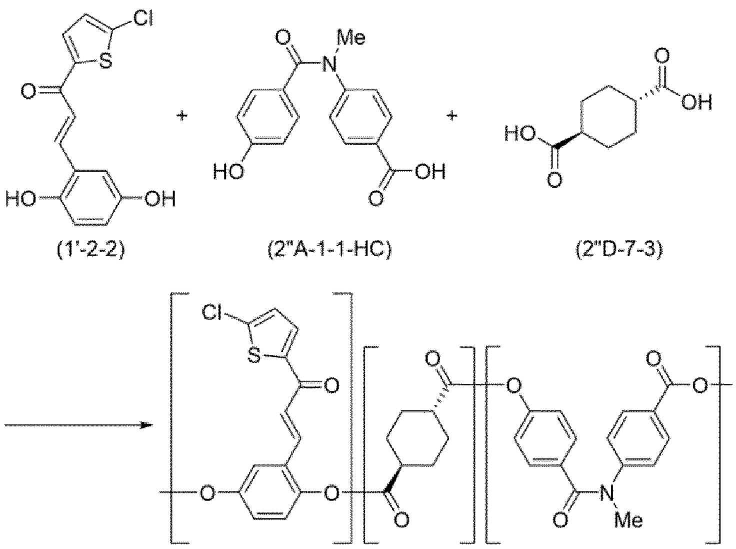

(1'-2-2) (2"A-1-1-HC) (2"D-7-3)

Polymer 47

979 mg (percent yield: 61%) of polymer 47 was obtained by the same method as in Example 28, except that compound (1'-2-2) (708 mg, 2.52 mmol), obtained in Dihydroxy Compound Example 2, was used as a diol monomer, that compound (2"A-1-1-HC) (586 mg, 2.16 mmol), obtained in Synthesis Example 17, was used as a hydroxycarboxylic acid monomer, that trans-1,4-cyclohexanedicarboxylic acid (2"D-7-3) (434 mg, 2.52 mmol) was used as a dicarboxylic acid monomer, that diisopropylcarbodiimide (1.00 g, 7.92 mmol) was used as a condensing agent and that pyridine (6.0 mL) was used as a solvent.

[Example 47-1]

A thin film was obtained by the same method as in Example 1-1, except that polymer 47 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 4.

[Example 48]

[Chem. 252]

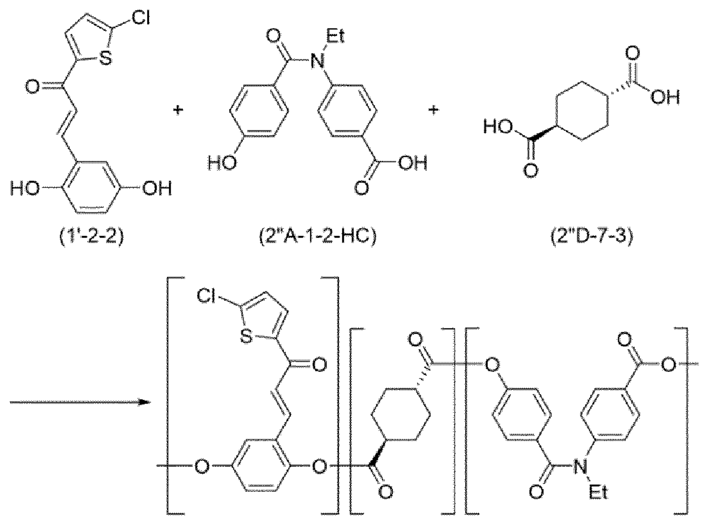

Polymer 48

1.03 g (percent yield: 63%) of polymer 48 was obtained by the same method as in Example 28, except that compound (1 '-2-2) (708 mg, 2.52 mmol), obtained in Dihydroxy Compound Example 2, was used as a diol monomer, that compound (2"A-1-2-HC) (616 mg, 2.16 mmol), obtained in Synthesis Example 18, was used as a hydroxycarboxylic acid monomer, that trans-1,4-cyclohexanedicarboxylic acid (2"D-7-3) (434 mg, 2.52 mmol) was used as a dicarboxylic acid monomer, that diisopropylcarbodiimide (1.00 g, 7.92 mmol) was used as a condensing agent and that pyridine (6.2 mL) was used as a solvent.

[Example 48-1]

A thin film was obtained by the same method as in Example 1-1, except that polymer 48 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 4.

[Example 48-2]

A thin film was obtained by the same method as in Example 1-1, except that polymer 48 was used instead of polymer 1. The resulting thin film (thickness, 1 μm) was irradiated with 100 mJ/cm$^2$ of polarized ultraviolet light at 365 nm, followed by heating at 250°C. Retardations and wavelength dispersion properties are presented in Table 4.

[Example 49]

[Chem. 253]

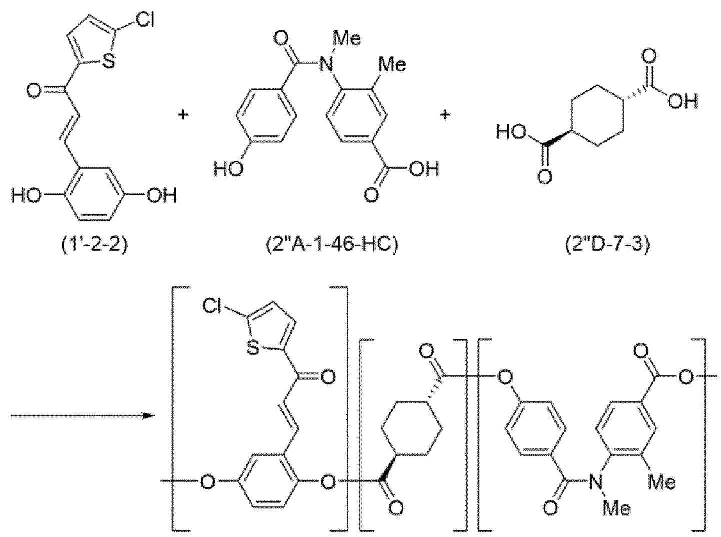

(1'-2-2) (2"A-1-46-HC) (2"D-7-3)

Polymer 49

1.07 g (percent yield: 66%) of polymer 49 was obtained by the same method as in Example 28, except that compound (1'-2-2) (708 mg, 2.52 mmol), obtained in Dihydroxy Compound Example 2, was used as a diol monomer, that compound (2"A-1-46-HC) (617 mg, 2.16 mmol), obtained in Synthesis Example 19, was used as a hydroxycarboxylic acid monomer, that trans-1,4-cyclohexanedicarboxylic acid (2"D-7-3) (434 mg, 2.52 mmol) was used as a dicarboxylic acid monomer, that diisopropylcarbodiimide (1.00 g, 7.92 mmol) was used as a condensing agent and that pyridine (6.1 mL) was used as a solvent.

[Example 49-1]

A thin film was obtained by the same method as in Example 1-1, except that polymer 49 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 4.

[Example 49-2]

A thin film was obtained by the same method as in Example 1-1, except that polymer 49 was used instead of polymer 1. The resulting thin film (thickness, 1 μm) was irradiated with 100 mJ/cm$^2$ of polarized ultraviolet light at 365 nm, followed by heating at 250°C. Retardations and wavelength dispersion properties are presented in Table 4.

[Example 50]

[Chem. 254]

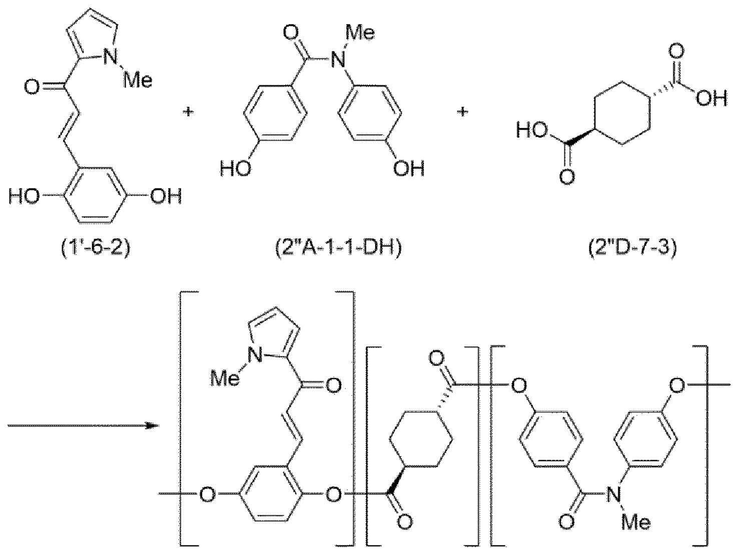

(1'-6-2) (2"A-1-1-DH) (2"D-7-3)

Polymer 50

738 mg (percent yield: 67%) of polymer 50 was obtained by the same method as in Example 28, except that compound (1'-6-2) (424 mg, 1.74 mmol), obtained in Dihydroxy Compound Example 11, and compound (2"A-1-1-DH) (283 mg, 1.16 mmol), obtained in Synthesis Example 2, were used as diol monomers, that trans-1,4-cyclohexanedicarboxylic acid (2"D-7-3) (500 mg, 2.90 mmol) was used as a dicarboxylic acid monomer, that diisopropylcarbodiimide (806 mg, 6.39 mmol) was used as a condensing agent and that pyridine (4.1 mL) was used as a solvent.

[Example 50-1]

A thin film was obtained by the same method as in Example 1-1, except that polymer 50 was used instead of polymer 1. The resulting thin film (thickness, 1 μm) was irradiated with 100 $mJ/cm^2$ of polarized ultraviolet light at 365 nm, followed by heating at 190°C. Retardations and wavelength dispersion properties are presented in Table 4.

[Example 51]

[Chem. 255]

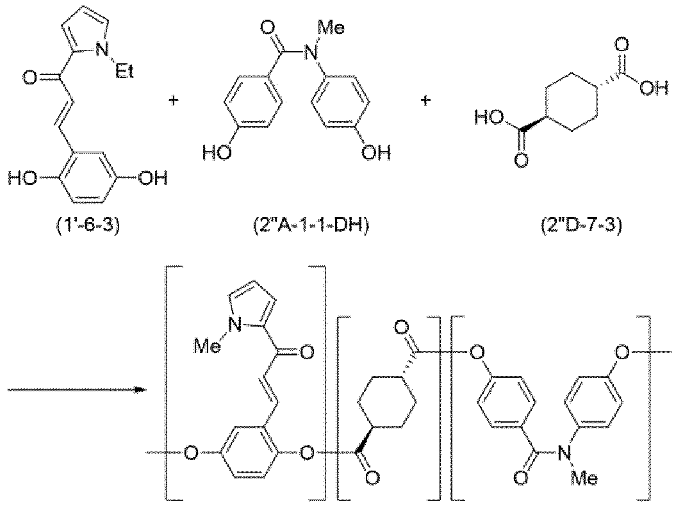

(1'-6-3) (2"A-1-1-DH) (2"D-7-3)

Polymer 51

657 mg (percent yield: 58%) of polymer 51 was obtained by the same method as in Example 28, except that compound (1 '-6-3) (448 mg, 1.74 mmol), obtained in Dihydroxy Compound Example 12, and compound (2"A-1-1-DH) (283 mg, 1.16 mmol), obtained in Synthesis Example 2, were used as diol monomers, that trans-1,4-cyclohexanedicarboxylic acid (2"D-7-3) (500 mg, 2.90 mmol) was used as a dicarboxylic acid monomer, that diisopropylcarbodiimide (806 mg, 6.39 mmol) was used as a condensing agent and that pyridine (4.1 mL) was used as a solvent.

[Example 51-1]

A thin film was obtained by the same method as in Example 1-1, except that polymer 51 was used instead of polymer 1. The resulting thin film (thickness, 1 μm) was irradiated with 100 mJ/cm$^2$ of polarized ultraviolet light at 365 nm, followed by heating at 150°C. Retardations and wavelength dispersion properties are presented in Table 4.

[Example 52]

[Chem. 256]

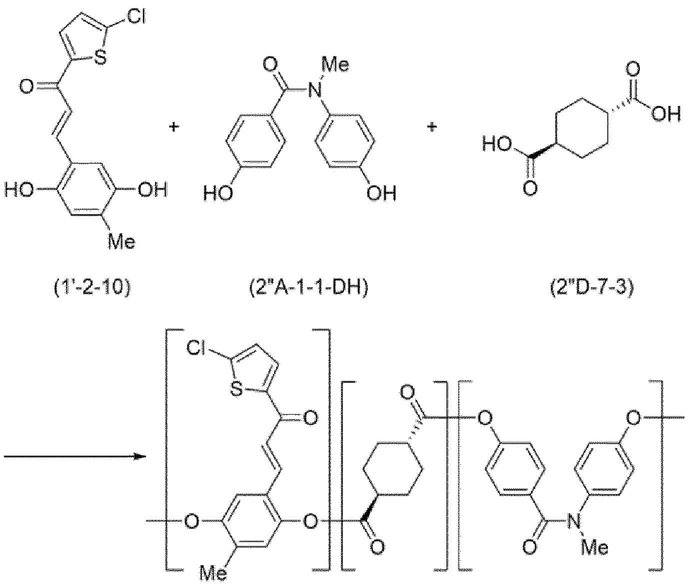

Polymer 52

966 mg (percent yield: 81%) of polymer 52 was obtained by the same method as in Example 28, except that compound (1'-2-10) (514 mg, 1.74 mmol), obtained in Dihydroxy Compound Example 8, and compound (2"A-1-1-DH) (283 mg, 1.16 mmol), obtained in Synthesis Example 2, were used as diol monomers, that trans-1,4-cyclohexanedicarboxylic acid (2"D-7-3) (500 mg, 2.90 mmol) was used as a dicarboxylic acid monomer, that diisopropylcarbodiimide (806 mg, 6.39 mmol) was used as a condensing agent and that pyridine (4.5 mL) was used as a solvent.

[Example 52-1]

A thin film was obtained by the same method as in Example 1-1, except that polymer 52 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 4.

[Example 53]

[Chem. 257]

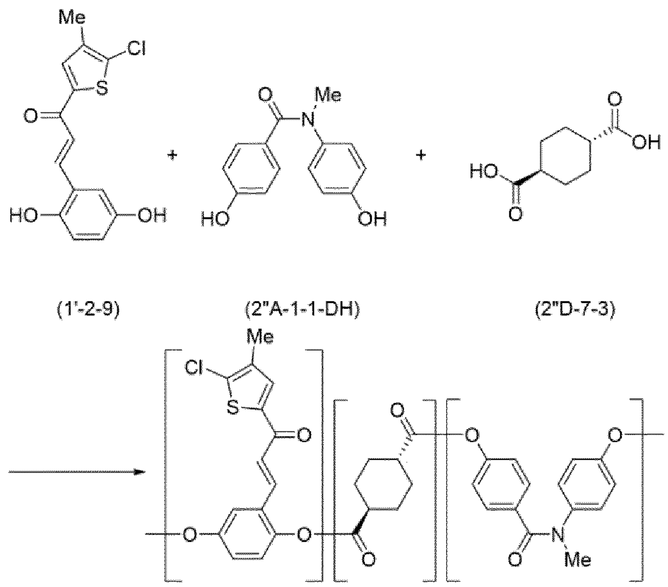

(1'-2-9) (2"A-1-1-DH) (2"D-7-3)

Polymer 53

884 mg (percent yield: 74%) of polymer 53 was obtained by the same method as in Example 28, except that compound (1 '-2-9) (514 mg, 1.74 mmol), obtained in Dihydroxy Compound Example 7, and compound (2"A-1-1-DH) (283 mg, 1.16 mmol), obtained in Synthesis Example 2, were used as diol monomers, that trans-1,4-cyclohexanedicarboxylic acid (2"D-7-3) (500 mg, 2.90 mmol) was used as a dicarboxylic acid monomer, that diisopropylcarbodiimide (806 mg, 6.39 mmol) was used as a condensing agent and that pyridine (4.5 mL) was used as a solvent.

[Example 53-1]

A thin film was obtained by the same method as in Example 1-1, except that polymer 53 was used instead of polymer 1. The resulting thin film (thickness, 1 μm) was irradiated with 100 mJ/cm$^2$ of polarized ultraviolet light at 365 nm, followed by heating at 190°C. Retardations and wavelength dispersion properties are presented in Table 4.

[Example 54]

[Chem. 258]

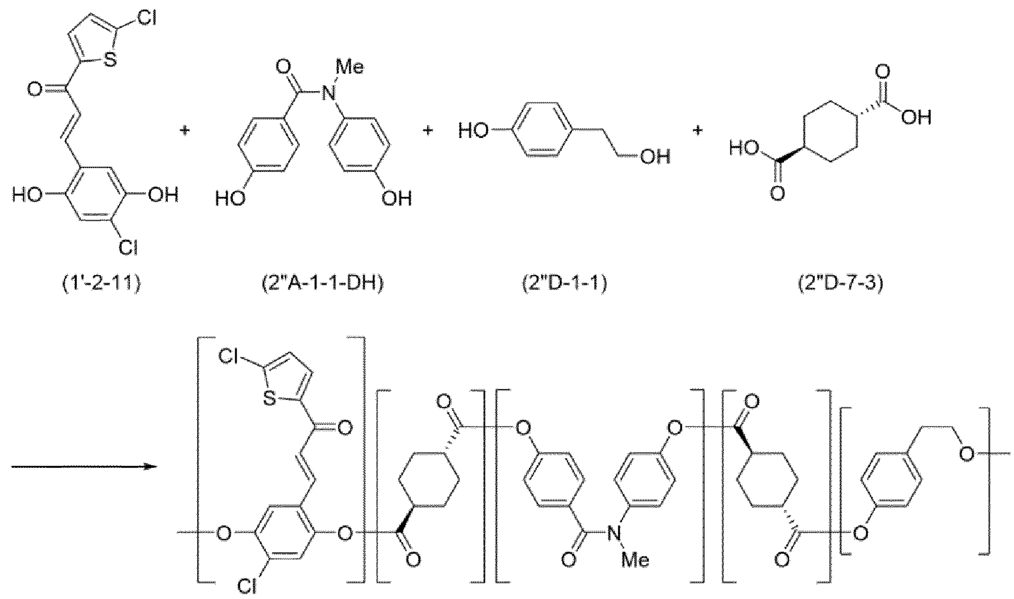

(1'-2-11) (2"A-1-1-DH) (2"D-1-1) (2"D-7-3)

Polymer 54

483 mg (percent yield: 55%) of polymer 54 was obtained by the same method as in Example 28, except that compound (1'-2-11) (157 mg, 499 μmol), obtained in Dihydroxy Compound Example 9, compound (2"A-1-1-DH) (243 mg, 999 μmol), obtained in Synthesis Example 2, and compound (2"D-1-1) (138 mg, 999 μmol) were used as diol monomers, that trans-1,4-cyclohexanedicarboxylic acid (2"D-7-3) (430 mg, 2.50 mmol) was used as a dicarboxylic acid monomer, that diisopropylcarbodiimide (693 mg, 5.49 mmol) was used as a condensing agent and that pyridine (3.2 mL) was used as a solvent.

[Example 55]

[Chem. 259]

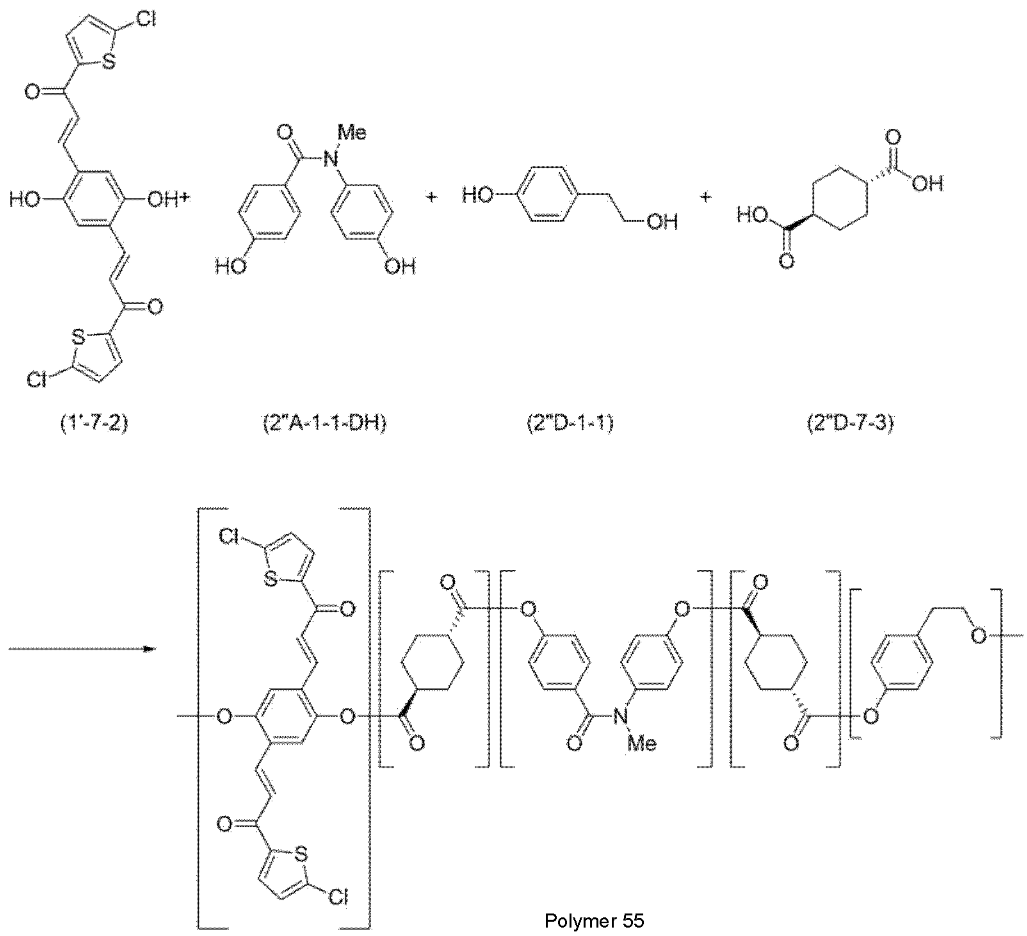

(1'-7-2) (2"A-1-1-DH) (2"D-1-1) (2"D-7-3)

Polymer 55

699 mg (percent yield: 74%) of polymer 55 was obtained by the same method as in Example 28, except that compound (1'-7-2) (225 mg, 499 μmol), obtained in Dihydroxy Compound Example 10, compound (2"A-1-1-DH) (243 mg, 999 μmol), obtained in Synthesis Example 2, and compound (2"D-1-1) (138 mg, 999 μmol) were used as diol monomers, that trans-1,4-cyclohexanedicarboxylic acid (2"D-7-3) (430 mg, 2.50 mmol) was used as a dicarboxylic acid monomer, that diisopropylcarbodiimide (693 mg, 5.49 mmol) was used as a condensing agent and that pyridine (3.5 mL) was used as a solvent.

[Example 56]

[Chem. 260]

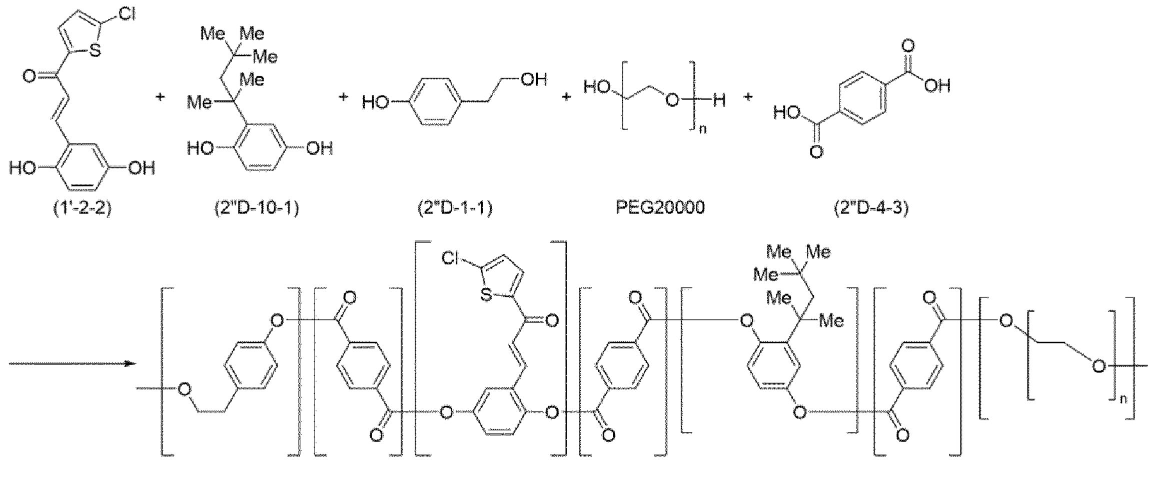

(1'-2-2) (2"D-10-1) (2"D-1-1) PEG20000 (2"D-4-3)

Polymer 56

1.21 g (percent yield: 54%) of polymer 56 was obtained by the same method as in Example 28, except that compound (1 '-2-2) (946 mg, 3.37 mmol), obtained in Dihydroxy Compound Example 2, compound (2"D-1-1) (233 mg, 1.68 mmol), 2-(1,1,3,3-tetramethylbutyl)hydroquinone (2"D-10-1) (214 mg, 963 mmol) and PEG 20000 (60.3 mg, 3.01 μmol) were used as diol monomers, that terephthalic acid (1.00 g, 1.68 mmol) was used as a dicarboxylic acid monomer, that diisopropylcarbodiimide (693 mg, 5.49 mmol) was used as a condensing agent and that pyridine (3.5 mL) was used as a solvent.

[Comparative Example 1]

[Chem. 261]

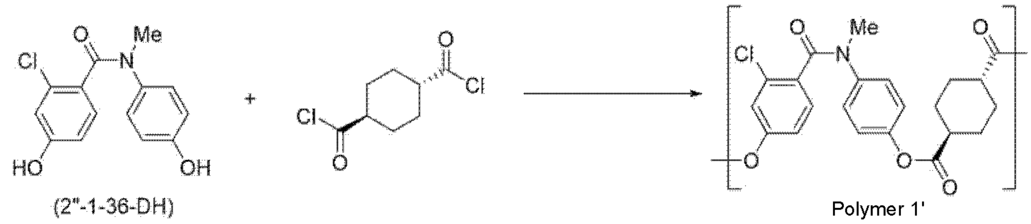

(2"-1-36-DH) Polymer 1'

Deionized water (20 mL) was taken into a 200-mL three-neck flask equipped with a dropping funnel, and compound (2"-1-36-DH) (1.11 g, 4.00 mmol), obtained in Synthesis Example 1, and sodium hydroxide (320 mg, 8.00 mmol) were added. After the substrate was dissolved through vigorous stirring (350 rpm), a 2% aqueous solution of tetrabutylammonium bromide (1.6 mL) as a catalyst was added, and the inside of the instrument was sufficiently purged with argon. A dichloromethane (20 mL) solution of trans-1,4-cyclohexanedicarboxylic acid dichloride was taken into the dropping funnel and added dropwise into the system quickly. After the end of dropwise addition, interfacial polymerization was performed through 3 hours of vigorous stirring at ordinary temperature. After the reaction, the solution was added dropwise to methanol (200 mL), and the precipitate was isolated by filtration, washed with water (100 mL) and methanol (100 mL) and then vacuum-dried, giving a white solid of polymer 1' weighing 1.04 g (percent yield: 63%).

6.0% by mass of polymer 1' was dissolved in 94.0% by mass of 1,1,1,3,3,3-hexafluoro-2-propanol. After this solution was cast onto a quartz glass substrate, spin coating was performed, and the resulting coating was dried in an oven at 60°C for 30 minutes to give a thin film (thickness, 1 μm). The resulting thin film was irradiated with 500 mJ/$cm^2$ of polarized ultraviolet light at 248 nm, followed by heating at 250°C. Retardations and wavelength dispersion properties are presented in Table 5.

[Table 1]

| Example | Polymer | Irradiation wavelength (nm) | Heat treatment temperature (°C) | Amount of light irradiation (mJ) | R(450)/R (550) | Re [10μm] (nm) |
|---|---|---|---|---|---|---|
| 1-1 | Polymer 1 | 248 | 150 | 500 | 0.99 | 313 |
| 2-1 | Polymer 2 | 248 | 150 | 100 | 0.86 | 34 |
| 2-2 | | 248 | 150 | 500 | 0.86 | 76 |
| 2-3 | | 248 | 250 | 100 | 0.95 | 86 |
| 2-4 | | 248 | 250 | 500 | 0.94 | 510 |
| 3-1 | Polymer 3 | 248 | 150 | 500 | 0.80 | 46 |
| 3-2 | | 248 | 250 | 500 | 0.89 | 211 |
| 4-1 | Polymer 4 | 248 | 150 | 500 | 0.86 | 85 |
| 4-2 | | 248 | 150 | 1000 | 0.86 | 180 |
| 4-3 | | 248 | 250 | 500 | 0.89 | 588 |
| 4-4 | | 248 | 250 | 1000 | 0.89 | 674 |
| 5-1 | Polymer 5 | 248 | 150 | 500 | 0.80 | 80 |
| 5-2 | | 248 | 150 | 1000 | 0.80 | 164 |
| 5-3 | | 248 | 250 | 500 | 0.84 | 370 |
| 5-4 | | 248 | 250 | 1000 | 0.84 | 579 |
| 6-1 | Polymer 6 | 248 | 150 | 500 | 0.98 | 128 |
| 6-2 | | 248 | 150 | 1000 | 0.98 | 178 |
| 6-3 | | 248 | 250 | 500 | 0.99 | 569 |
| 6-4 | | 248 | 250 | 1000 | 0.99 | 608 |
| 7-1 | Polymer 7 | 248 | 150 | 500 | 0.95 | 345 |
| 7-2 | | 248 | 150 | 1000 | 0.95 | 412 |
| 7-3 | | 248 | 250 | 500 | 0.96 | 723 |
| 7-4 | | 248 | 250 | 1000 | 0.96 | 690 |
| 8-1 | Polymer 8 | 248 | 150 | 500 | 0.89 | 336 |
| 8-2 | | 248 | 150 | 1000 | 0.89 | 521 |
| 8-3 | | 248 | 250 | 500 | 0.93 | 441 |
| 8-4 | | 248 | 250 | 1000 | 0.91 | 410 |
| 9-1 | Polymer 9 | 248 | 150 | 500 | 0.91 | 192 |
| 9-2 | | 248 | 150 | 1000 | 0.91 | 278 |
| 9-3 | | 248 | 250 | 500 | 0.93 | 898 |
| 9-4 | | 248 | 250 | 1000 | 0.92 | 945 |
| 10-1 | Polymer 10 | 248 | 150 | 1000 | 0.93 | 95 |
| 10-2 | | 248 | 250 | 500 | 0.94 | 390 |
| 10-3 | | 248 | 250 | 1000 | 0.94 | 420 |
| 11-1 | Polymer 11 | 248 | 250 | 100 | 1.00 | 723 |
| 11-2 | | 248 | 250 | 500 | 1.00 | 798 |

(continued)

| Example | Polymer | Irradiation wavelength (nm) | Heat treatment temperature (°C) | Amount of light irradiation (mJ) | R(450)/R (550) | Re [10μm] (nm) |
|---|---|---|---|---|---|---|
| 12-1 | Polymer 12 | 248 | 250 | 500 | 0.96 | 863 |
| 12-2 | | 248 | 250 | 1000 | 0.96 | 824 |
| 12-3 | | 313 | 150 | 170 | 1.00 | 145 |
| 12-4 | | 313 | 250 | 170 | 0.94 | 913 |
| 12-5 | | 313 | 250 | 340 | 0.94 | 1013 |
| 12-6 | | 365 | 250 | 700 | 0.95 | 668 |
| 12-7 | | 365 | 250 | 1400 | 0.96 | 457 |
| 13-1 | Polymer 13 | 248 | 150 | 50 | 0.87 | 392 |
| 13-2 | | 248 | 250 | 50 | 0.81 | 791 |
| 13-3 | | 248 | 150 | 500 | 0.87 | 199 |
| 13-4 | | 248 | 150 | 1000 | 0.89 | 205 |
| 13-5 | | 248 | 250 | 500 | 0.87 | 510 |
| 13-6 | | 248 | 250 | 1000 | 0.86 | 346 |
| 13-7 | | 365 | 150 | 100 | 0.87 | 330 |
| 13-8 | | 365 | 150 | 500 | 0.90 | 254 |
| 13-9 | | 365 | 250 | 100 | 0.86 | 702 |
| 13-10 | | 365 | 250 | 500 | 0.86 | 490 |

[Table 2]

| Example | Polymer | Irradiation wavelength (nm) | Heat treatment temperature (°C) | Amount of light irradiation (mJ) | R(450)/R (550) | Re [10μm] (nm) |
|---|---|---|---|---|---|---|
| 14-1 | Polymer 14 | 248 | 150 | 500 | 0.84 | 34 |
| 14-2 | | 248 | 150 | 1000 | 0.83 | 149 |
| 14-3 | | 248 | 250 | 500 | 0.86 | 37 |
| 14-4 | | 248 | 250 | 1000 | 0.84 | 114 |
| 15-1 | Polymer 15 | 365 | 150 | 100 | 0.97 | 384 |
| 15-2 | | 365 | 150 | 500 | 0.95 | 398 |
| 15-3 | | 365 | 250 | 100 | 0.90 | 147 |
| 15-4 | | 365 | 250 | 500 | 0.91 | 99 |
| 16-1 | Polymer 16 | 365 | 150 | 100 | 0.91 | 175 |
| 16-2 | | 365 | 150 | 500 | 0.92 | 195 |
| 16-3 | | 365 | 250 | 100 | 0.92 | 317 |
| 16-4 | | 365 | 250 | 500 | 0.84 | 331 |

(continued)

| Example | Polymer | Irradiation wavelength (nm) | Heat treatment temperature (°C) | Amount of light irradiation (mJ) | R(450)/R (550) | Re [10μm] (nm) |
|---|---|---|---|---|---|---|
| 17-1 | Polymer 17 | 365 | 150 | 100 | 0.92 | 168 |
| 17-2 | | 365 | 150 | 500 | 0.93 | 170 |
| 17-3 | | 365 | 250 | 100 | 0.88 | 142 |
| 17-4 | | 365 | 250 | 500 | 0.88 | 203 |
| 18-1 | Polymer 18 | 365 | 250 | 100 | 0.87 | 155 |
| 18-2 | | 365 | 250 | 500 | 0.86 | 149 |
| 19-1 | Polymer 19 | 365 | 150 | 50 | 0.90 | 339 |
| 19-2 | | 365 | 150 | 100 | 0.91 | 369 |
| 19-3 | | 365 | 250 | 50 | 0.86 | 364 |
| 19-4 | | 365 | 250 | 100 | 0.85 | 289 |
| 20-1 | Polymer 20 | 248 | 250 | 500 | 0.90 | 155 |
| 20-2 | | 248 | 250 | 1000 | 0.91 | 295 |
| 21-1 | Polymer 21 | 248 | 250 | 500 | 0.98 | 820 |
| 22-1 | Polymer 22 | 248 | 150 | 500 | 0.94 | 45 |
| 22-2 | | 248 | 250 | 500 | 0.93 | 333 |
| 22-3 | | 365 | 150 | 100 | 0.94 | 127 |
| 22-4 | | 365 | 150 | 500 | 0.94 | 95 |
| 22-5 | | 365 | 250 | 100 | 0.93 | 603 |
| 22-6 | | 365 | 250 | 500 | 0.94 | 308 |
| 23-1 | Polymer 23 | 248 | 150 | 500 | 0.77 | 88 |
| 23-2 | | 248 | 150 | 1000 | 0.78 | 89 |
| 23-3 | | 248 | 250 | 500 | 0.79 | 335 |
| 23-4 | | 248 | 250 | 1000 | 0.79 | 246 |
| 24-1 | Polymer 24 | 248 | 150 | 500 | 0.97 | 92 |
| 24-2 | | 248 | 150 | 1000 | 0.96 | 176 |
| 24-3 | | 248 | 250 | 500 | 0.95 | 811 |
| 24-4 | | 248 | 250 | 1000 | 0.92 | 1197 |
| 25-1 | Polymer 25 | 248 | 150 | 500 | 0.98 | 143 |
| 25-2 | | 248 | 150 | 1000 | 0.98 | 257 |
| 25-3 | | 248 | 250 | 500 | 0.92 | 978 |
| 25-4 | | 248 | 250 | 1000 | 0.92 | 1085 |
| 26-1 | Polymer 26 | 248 | 250 | 500 | 1.00 | 511 |
| 26-2 | | 248 | 250 | 1000 | 1.00 | 564 |
| 27-1 | Polymer 27 | 248 | 150 | 500 | 1.00 | 165 |
| 27-2 | | 248 | 150 | 1000 | 1.00 | 244 |

[Table 3]

| Example | Polymer | Irradiation wavelength (nm) | Heat treatment temperature (°C) | Amount of light irradiation (mJ) | R(450)/ R (550) | Re [10μm ] (nm) |
|---|---|---|---|---|---|---|
| 28-1 | Polymer 28 | 365 | 150 | 100 | 0.88 | 146 |
| 28-2 | | 365 | 190 | 100 | 0.86 | 25 |
| 29-1 | Polymer 29 | 365 | 150 | 100 | 0.88 | 165 |
| 29-2 | | 365 | 190 | 100 | 0.87 | 90 |
| 30-1 | Polymer 30 | 365 | 150 | 100 | 0.93 | 71 |
| 30-2 | | 365 | 190 | 100 | 0.93 | 305 |
| 31-1 | Polymer 31 | 365 | 150 | 100 | 0.95 | 310 |
| 31-2 | | 365 | 190 | 100 | 0.92 | 802 |
| 32-1 | Polymer 32 | 365 | 150 | 100 | 0.91 | 480 |
| 32-2 | | 365 | 190 | 100 | 0.91 | 164 |
| 33-1 | Polymer 33 | 365 | 190 | 100 | 0.95 | 200 |
| 34-1 | Polymer 34 | 365 | 150 | 100 | 0.82 | 26 |

[Table 4]

| Example | Polymer | Irradiation wavelength (nm) | Heat treatment temperature (°C) | Amount of light irradiation (mJ) | R(450)/ R (550) | Re [10μm ] (nm) |
|---|---|---|---|---|---|---|
| 35-1 | Polymer 35 | 365 | 190 | 100 | 0.94 | 177 |
| 36-1 | Polymer 36 | 365 | 190 | 100 | 0.95 | 411 |
| 37-1 | Polymer 37 | 365 | 190 | 100 | 0.95 | 296 |
| 38-1 | Polymer 38 | 365 | 190 | 100 | 0.94 | 183 |
| 39-1 | Polymer 39 | 365 | 190 | 100 | 0.94 | 205 |
| 40-1 | Polymer 40 | 365 | 150 | 100 | 0.88 | 570 |
| 41-1 | Polymer 41 | 365 | 150 | 100 | 0.92 | 236 |
| 42-1 | Polymer 42 | 365 | 150 | 100 | 0.86 | 308 |
| 43-1 | Polymer 43 | 365 | 190 | 100 | 0.96 | 316 |
| 44-1 | Polymer 44 | 365 | 190 | 100 | 0.93 | 188 |

(continued)

| Example | Polymer | Irradiation wavelength (nm) | Heat treatment temperature (°C) | Amount of light irradiation (mJ) | R(450)/ R (550) | Re [10μm ] (nm) |
|---|---|---|---|---|---|---|
| 45-1 | Polymer 45 | 365 | 190 | 100 | 0.95 | 274 |
| 46-1 | Polymer 46 | 365 | 190 | 100 | 1.00 | 443 |
| 46-2 | | 365 | 250 | 100 | 0.87 | 367 |
| 47-1 | Polymer 47 | 365 | 190 | 100 | 0.94 | 170 |
| 48-1 | Polymer 48 | 365 | 190 | 100 | 0.98 | 287 |
| 48-2 | | 365 | 250 | 100 | 0.92 | 206 |
| 49-1 | Polymer 49 | 365 | 190 | 100 | 0.95 | 260 |
| 49-2 | | 365 | 250 | 100 | 0.85 | 258 |
| 50-1 | Polymer 50 | 365 | 190 | 100 | 0.92 | 238 |
| 51-1 | Polymer 51 | 365 | 150 | 100 | 0.90 | 74 |
| 52-1 | Polymer 52 | 365 | 190 | 100 | 0.98 | 76 |
| 53-1 | Polymer 53 | 365 | 190 | 100 | 0.94 | 296 |

[Table 5]

| Comparative Example | Polymer | Irradiation wavelength (nm) | Heat treatment temperature (°C) | Amount of light irradiation (mJ) | R(450)/ R(550) | Re [10μm ] (nm) |
|---|---|---|---|---|---|---|
| 1 | Polymer 1' | 248 | 250 | 500 | 1.06 | 61 |

[In Table 1, Table 2, Table 3, Table 4 and Table 5, R (450)/R (550) represents the ratio between the in-plane retardation at 450 nm and the in-plane retardation at 550 nm.

Re [10 μm] indicates a retardation value equivalent to a film thickness of 10 μm.]

As shown in Table 1, Table 2, Table 3 and Table 4, the polymers in Examples 1 to 53 develop reverse wavelength dispersion retardations through irradiation with ultraviolet light and heating treatment.

The development of reverse wavelength dispersion retardations by the polymers according to the present invention, which had a photoreactive reverse wavelength dispersion unit A, becomes more conspicuous through comparison with Comparative Example 1, presented in Table 5.

[Dihydroxy Compound Example 14]

2,5-dihydroxybenzaldehyde (57.5 g, 416 mmol) and acetophenone (50.0 g, 416 mmol) were dissolved in methanol (200 mL), and then a 50% aqueous solution of sodium hydroxide (120 mL, 2.25 mol) was added dropwise with cooling on ice. After the reaction system was stirred at room temperature for 12 hours, acetic acid (167 mL, 2.91 mmol) was added with cooling on ice. The resulting reaction mixture was added to water (300 mL), followed by vigorous stirring with added toluene (30 mL), isopropyl alcohol (2 mL) and dichloromethane (100 mL). The solid that formed was collected by filtration and washed with dichloromethane (50 mL) and distilled water (50 mL). The resulting solid was vacuum-dried, giving 14.8 g (percent yield: 15%) of compound (1'-1-1) as a yellow solid. $^1$H-NMR (400 MHz, DMSO-$d_6$) δ 9.90-8.39 (br, 2H), 8.04 (d, J = 8.2 Hz, 2H), 7.94 (d, J = 15.4 Hz, 1H), 7.67 (d, J = 15.4 Hz, 1H), 7.61 (t, J = 7.4 Hz, 1H), 7.52 (t, J

= 7.4 Hz, 2H), 7.15 (s, 1H), 6.76-6.67 (m, 2H).

[Chem. 262]

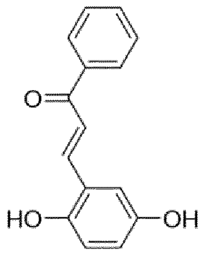

(1'-1-1)

[Synthesis Example 20-1]

In an argon atmosphere, a mixture of 4-acetoxybenzoic acid (7.56 g, 42.0 mmol), thionyl chloride (24.0 mL, 331 mmol) and N,N-dimethylformamide (catalytic amount) was boiled under reflux for 3 hours. Volatile components were distilled away, and the residue was azeotropically distilled with toluene (3 × 20 mL) to give 4-acetoxybenzoyl chloride. The resulting 4-acetoxybenzoyl chloride was used entirely in the next reaction after being made into a tetrahydrofuran (50 mL) solution.

In an argon atmosphere, 1,2-dimethylhydrazine dihydrochloride (2.66 g, 20.0 mmol) and N,N-diisopropylethylamine (17.0 mL, 100 mmol) were suspended in tetrahydrofuran (40 mL). With cooling on ice, the tetrahydrofuran solution of 4-acetoxybenzoyl chloride prepared in advance was slowly added. The resulting mixture was allowed to warm to room temperature and stirred overnight. The solvent was distilled away under reduced pressure, 1 M hydrochloric acid (100 mL) was added, and extraction with ethyl acetate (2 × 100 mL) was performed. The combined organic layer was washed sequentially with a saturated aqueous solution of sodium hydrogencarbonate (100 mL) and saturated saline solution (100 mL) and dried with anhydrous sodium sulfate, followed by concentration under reduced pressure. The residue was suspended in methanol (75 mL), and the resulting suspension was stirred at room temperature for 1 hour with an added aqueous (25 mL) solution of sodium hydroxide (4.16 g, 104 mmol). The solvent was distilled away under reduced pressure, 2 M hydrochloric acid (75 mL) was added, and extraction with ethyl acetate (2 × 100 mL) was performed. The combined organic layer was washed sequentially with a saturated aqueous solution of sodium hydrogencarbonate (2 × 100 mL) and saturated saline solution (100 mL), followed by drying with anhydrous sodium sulfate. The solvent was concentrated under reduced pressure, the residue was purified by silica gel column chromatography, and then the resulting solid was recrystallized from a solvent mixture of ethanol/hexane, giving a white solid (actual yield: 3.02g; percent yield: 50%) of compound (2"C-2-13). $^1$H-NMR (400 MHz, $(CD_3)_2SO$): δ 9.95 (brs, 2H), 7.58-6.87 (brm, 4H), 6.87-6.61 (brm, 4H), 3.13 (brs, 6H).

[Chem. 263]

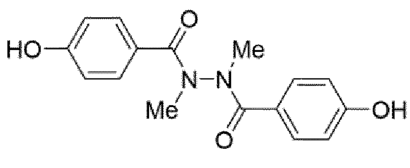

(2"C-2-13)

[Example 57]

In a nitrogen atmosphere, compound (1'-2-2) (4.0 g, 14.3 mmol), obtained in Dihydroxy Compound Example 2, compound (2"A-1-1-DH) (1.9 g, 7.7 mmol), obtained in Synthesis Example 2, trans-1,4-cyclohexanedicarboxylic acid (3.8 g, 21.9 mmol), pyridine (8.2 mL) and N-methyl-2-pyrrolidone (8.2 mL) were added to a reaction vessel. The reaction system was stirred at 40°C for 1 hour for dissolution, and then N,N'-diisopropylcarbodiimide (6.1 g, 48.2 mmol) as an ester-linkage-forming condensing agent was added dropwise to initiate polymerization. The reaction system was stirred at 40°C for 3 hours, followed by the addition of acetic anhydride (2.2 g, 21.9 mmol) as a monocarboxylic acid derivative compound. After the reaction system was stirred at 40°C for 30 minutes, the mixture in the system was added to methanol

(300 mL). The solid that formed was collected by filtration, washed with methanol (500 mL) and then vacuum-dried to give polymer 57 (actual yield: 6.4 g; percent yield: 72%).

[Chem. 264]

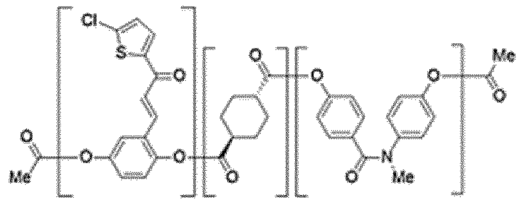

Polymer 57

13.0% by mass of polymer 57 was dissolved in 87.0% by mass of hexafluoro-2-propanol. This solution was cast onto a quartz substrate, spin coating was performed at 2000 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 58]

In a nitrogen atmosphere, compound (1'-2-2) (4.0 g, 14.3 mmol), obtained in Dihydroxy Compound Example 2, compound (2"A-1-1-DH) (1.9 g, 7.7 mmol), obtained in Synthesis Example 2, trans-1,4-cyclohexanedicarboxylic acid (3.8 g, 21.9 mmol), pyridine (8.2 mL) and N-methyl-2-pyrrolidone (8.2 mL) were added to a reaction vessel. The reaction system was stirred at 40°C for 1 hour for dissolution, and then N,N'-diisopropylcarbodiimide (6.1 g, 48.2 mmol) as an ester-linkage-forming condensing agent was added dropwise to initiate polymerization. The reaction system was stirred at 40°C for 3 hours, followed by the addition of cyclohexanecarbonyl chloride (3.2 g, 21.9 mmol) as a monocarboxylic acid derivative compound. After the reaction system was stirred at 40°C for 30 minutes, the mixture in the system was added to methanol (300 mL). The solid that formed was collected by filtration, washed with methanol (500 mL) and then vacuum-dried to give polymer 58 (actual yield: 6.6 g; percent yield: 75%).

[Chem. 265]

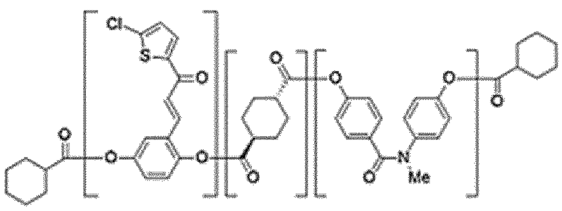

Polymer 58

13.0% by mass of polymer 58 was dissolved in 87.0% by mass of hexafluoro-2-propanol. This solution was cast onto a quartz substrate, spin coating was performed at 1600 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 5.

[Example 59]

In a nitrogen atmosphere, compound (1'-2-2) (4.0 g, 14.3 mmol), obtained in Dihydroxy Compound Example 2, compound (2"A-1-1-DH) (1.9 g, 7.7 mmol), obtained in Synthesis Example 2, trans-1,4-cyclohexanedicarboxylic acid (3.8 g, 21.9 mmol), pyridine (8.2 mL) and N-methyl-2-pyrrolidone (8.2 mL) were added to a reaction vessel. The reaction system was stirred at 40°C for 1 hour for dissolution, and then N,N'-diisopropylcarbodiimide (6.1 g, 48.2 mmol) as an ester-linkage-forming condensing agent was added dropwise to initiate polymerization. The reaction system was stirred at 40°C for 3 hours, followed by the addition of a solution in which chloroacetic anhydride (3.7 g, 21.9 mmol) as a monocarboxylic acid derivative compound had been dissolved in N-methyl-2-pyrrolidone (3.0 mL). After the reaction system was stirred at 40°C for 30 minutes, the mixture in the system was added to methanol (300 mL). The solid that formed was collected by filtration, washed with methanol (500 mL) and then vacuum-dried to give polymer 59 (actual yield: 6.5 g; percent yield: 73%).

[Chem. 266]

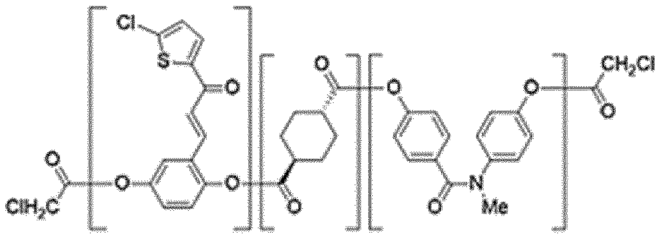

Polymer 59

13.0% by mass of polymer 59 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 $mJ/cm^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 60]

In a nitrogen atmosphere, compound (1'-2-2) (4.0 g, 14.3 mmol), obtained in Dihydroxy Compound Example 2, compound (2"A-1-1-DH) (1.9 g, 7.7 mmol), obtained in Synthesis Example 2, trans-1,4-cyclohexanedicarboxylic acid (3.8 g, 21.9 mmol), pyridine (8.2 mL) and N-methyl-2-pyrrolidone (8.2 mL) were added to a reaction vessel. The reaction system was stirred at 40°C for 1 hour for dissolution, and then N,N'-diisopropylcarbodiimide (6.1 g, 48.2 mmol) as an ester-linkage-forming condensing agent was added dropwise to initiate polymerization. The reaction system was stirred at 40°C for 3 hours, followed by the addition of p-toluoyl chloride (3.4 g, 21.9 mmol) as a monocarboxylic acid derivative compound. After the reaction system was stirred at 40°C for 30 minutes, the mixture in the system was added to methanol (300 mL). The solid that formed was collected by filtration, washed with methanol (500 mL) and then vacuum-dried to give polymer 60 (actual yield: 7.7 g; percent yield: 87%).

[Chem. 267]

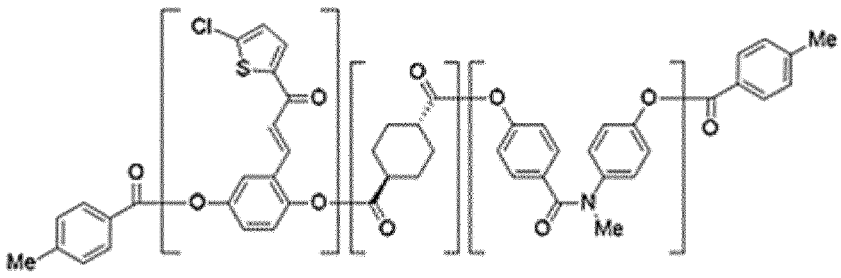

Polymer 60

13.0% by mass of polymer 60 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 $mJ/cm^2$ of polarized ultraviolet radiation light at 365 nm, then heating treatment was performed at 250°C for 10 minutes, and retardation characteristics and wavelength dispersion characteristics were evaluated. The results are presented in Table 6.

[Example 61]

In a nitrogen atmosphere, compound (1'-1-1) (1.0 g, 4.2 mmol), obtained in Dihydroxy Compound Example 14, compound (2"A-1-1-DH) (0.55 g, 2.2 mmol), obtained in Synthesis Example 2, trans-1,4-cyclohexanedicarboxylic acid (1.1 g, 6.4 mmol), pyridine (2.2 mL) and N-methyl-2-pyrrolidone (2.2 mL) were added to a reaction vessel. The reaction system was stirred at 40°C for 1 hour for dissolution, and then N,N'-diisopropylcarbodiimide (1.8 g, 14.1 mmol) as an ester-linkage-forming condensing agent was added dropwise to initiate polymerization. The reaction system was stirred at 40°C for 3 hours, followed by the addition of acetic anhydride (0.65 g, 6.4 mmol) as a monocarboxylic acid derivative compound. After the reaction system was stirred at 40°C for 30 minutes, the mixture in the system was added to methanol (100 mL). The solid that formed was collected by filtration, washed with methanol (500 mL) and then vacuum-dried to give polymer 61 (actual yield: 1.3 g; percent yield: 54%).

[Chem. 268]

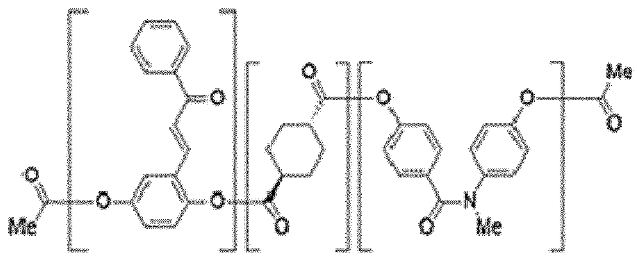

Polymer 61

13.0% by mass of polymer 61 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 $mJ/cm^2$ of polarized ultraviolet radiation light at 365 nm, then heating treatment was performed at 250°C for 10 minutes, and retardation characteristics and wavelength dispersion characteristics were evaluated. The results are presented in Table 6.

[Example 62]

In a nitrogen atmosphere, compound (1'-2-2) (2.5 g, 9.1 mmol), obtained in Dihydroxy Compound Example 2, 4,4'-bicyclohexanol (0.69 g, 3.5 mmol), 4-(2-hydroxyethyl)phenol (0.67 g, 4.9 mmol), trans-1,4-cyclohexanedicarboxylic acid (3.0 g, 17.4 mmol) and pyridine (16.2 mL) were added to a reaction vessel. The reaction system was stirred at 30°C for 1 hour for dissolution, and then N,N'-diisopropylcarbodiimide (4.8 g, 38.3 mmol) as an ester-linkage-forming condensing agent was added dropwise to initiate polymerization. The reaction system was stirred at 30°C for 3 hours, followed by the addition of acetic anhydride (1.8 g, 17.4 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 62 (actual yield: 4.3 g; percent yield: 69%).

[Chem. 269]

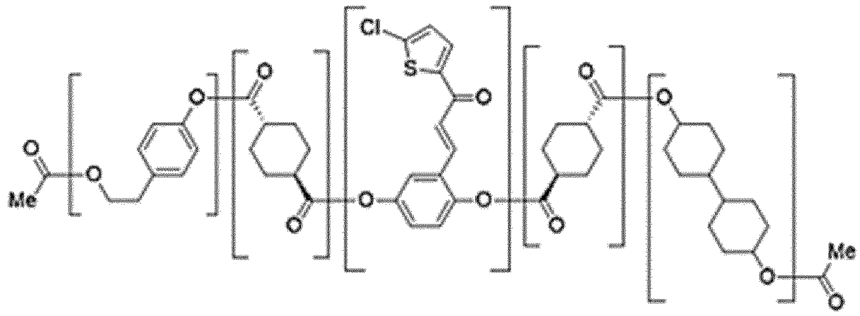

Polymer 62

13.0% by mass of polymer 62 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 $mJ/cm^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 63]

In a nitrogen atmosphere, compound (1'-2-2) (1.1 g, 4.1 mmol), obtained in Dihydroxy Compound Example 2, compound (2"A-1-1-DH) (7.1 g, 0.3 mmol), obtained in Synthesis Example 2, 4-(2-hydroxyethyl)phenol (0.20 g, 1.5 mmol), trans-1,4-cyclohexanedicarboxylic acid (1.0 g, 5.8 mmol) and pyridine (8.2 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.59 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 63 (actual yield: 1.7 g; percent yield: 80%).

[Chem. 270]

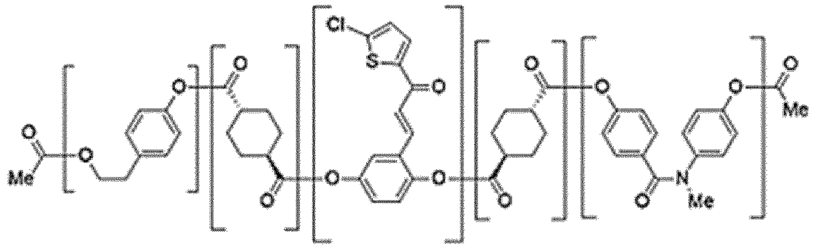

Polymer 63

10.0% by mass of polymer 63 was dissolved in 90.0% by mass of hexafluoro-2-propanol. This solution was cast onto a quartz substrate, spin coating was performed at 1000 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 64]

In a nitrogen atmosphere, compound (1'-2-2) (1.1 g, 4.1 mmol), obtained in Dihydroxy Compound Example 2, compound (2"A-1-1-DH) (0.07 g, 0.3 mmol), obtained in Synthesis Example 2, 4-(2-hydroxyethyl)phenol (0.20 g, 1.5 mmol), polyethylene glycol 20000 (manufactured by Tokyo Chemical Industry Co., Ltd., 0.11 g), trans-1,4-cyclohexanedicarboxylic acid (1.0 g, 5.8 mmol) and pyridine (8.7 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.59 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 64 (actual yield: 1.6 g; percent yield: 68%).

[Chem. 271]

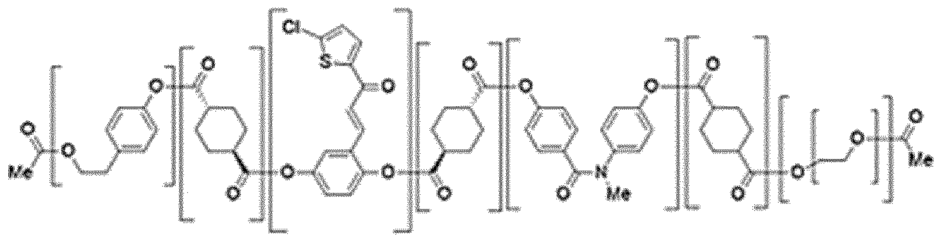

Polymer 64

10.0% by mass of polymer 64 was dissolved in 90.0% by mass of hexafluoro-2-propanol. This solution was cast onto a quartz substrate, spin coating was performed at 1000 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 65]

In a nitrogen atmosphere, compound (1'-2-2) (1.3 g, 4.7 mmol), obtained in Dihydroxy Compound Example 2, hexamethylenebis(4-hydroxybenzoate) (0.10 g, 0.3 mmol), 4-(2-hydroxyethyl)phenol (0.12 g, 0.9 mmol), trans-1,4-cyclohexanedicarboxylic acid (1.0 g, 5.8 mmol) and pyridine (8.7 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.59 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 65 (actual yield: 1.9 g; percent yield: 81%).

[Chem. 272]

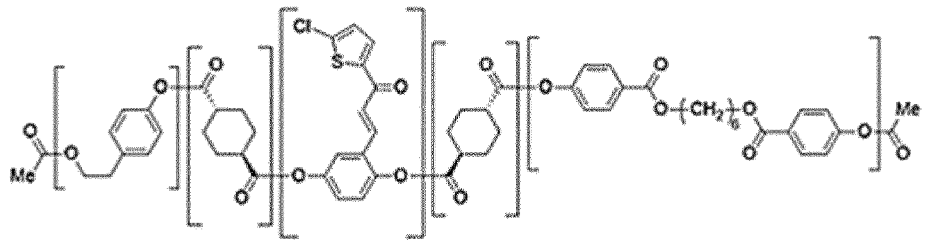

Polymer 65

10.0% by mass of polymer 65 was dissolved in 90.0% by mass of hexafluoro-2-propanol. This solution was cast onto a quartz substrate, spin coating was performed at 1000 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 66]

In a nitrogen atmosphere, compound (1'-2-2) (0.91 g, 3.3 mmol), obtained in Dihydroxy Compound Example 2, 4,4'-bicyclohexanol (0.18 g, 0.9 mmol), 4-(2-hydroxyethyl)phenol (0.23 g, 1.6 mmol), trans-1,4-cyclohexanedicarboxylic acid (1.0 g, 5.8 mmol) and pyridine (7.8 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of isobutyric anhydride (0.92 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 66 (actual yield: 1.4 g; percent yield: 66%).

[Chem. 273]

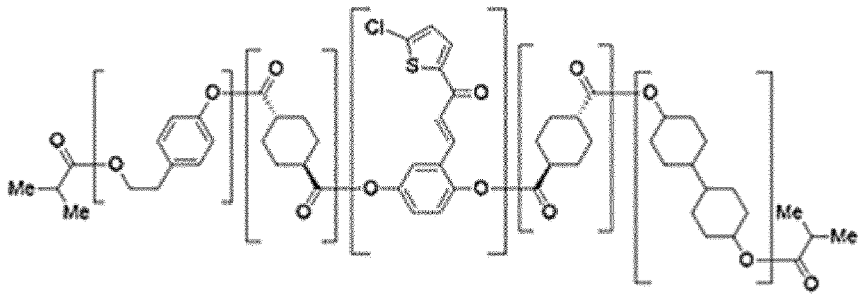

Polymer 66

13.0% by mass of polymer 66 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 67]

In a nitrogen atmosphere, compound (1'-2-2) (0.91 g, 3.3 mmol), obtained in Dihydroxy Compound Example 2, 4,4'-bicyclohexanol (0.18 g, 0.9 mmol), 4-(2-hydroxyethyl)phenol (0.23 g, 1.6 mmol), trans-1,4-cyclohexanedicarboxylic acid (1.0 g, 5.8 mmol) and pyridine (7.8 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of hexanoic anhydride (1.3 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 67 (actual yield: 1.4 g; percent yield: 65%).

[Chem. 274]

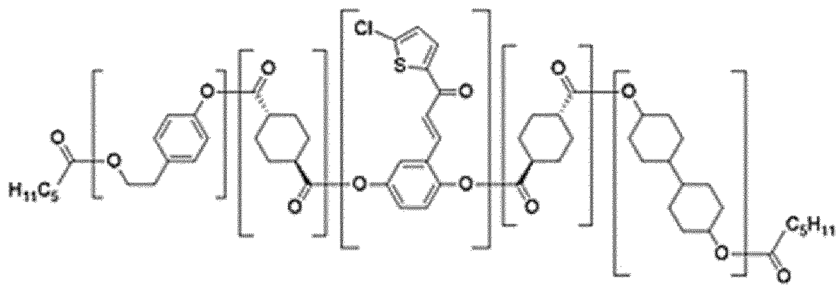

Polymer 67

13.0% by mass of polymer 67 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 68]

In a nitrogen atmosphere, compound (1'-2-2) (0.91 g, 3.3 mmol), obtained in Dihydroxy Compound Example 2, 4,4'-bicyclohexanol (0.18 g, 0.9 mmol), 4-(2-hydroxyethyl)phenol (0.23 g, 1.6 mmol), trans-1,4-cyclohexanedicarboxylic acid (1.0 g, 5.8 mmol) and pyridine (7.8 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of decanoic anhydride (1.9 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 68 (actual yield: 1.4 g; percent yield: 67%).

[Chem. 275]

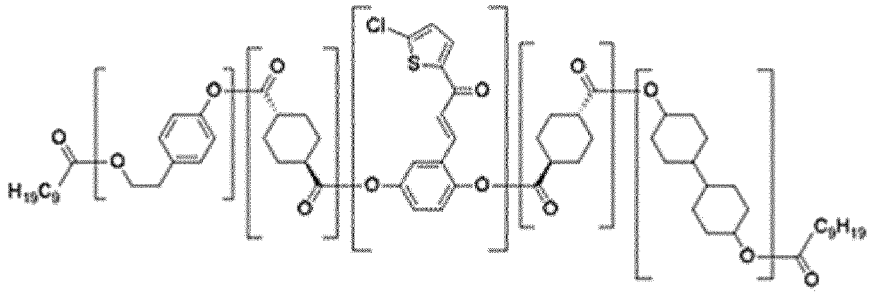

Polymer 68

13.0% by mass of polymer 68 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 69]

In a nitrogen atmosphere, compound (1'-2-2) (0.91 g, 3.3 mmol), obtained in Dihydroxy Compound Example 2, 4,4'-bicyclohexanol (0.18 g, 0.9 mmol), 4-(2-hydroxyethyl)phenol (0.23 g, 1.6 mmol), trans-1,4-cyclohexanedicarboxylic acid (1.0 g, 5.8 mmol) and pyridine (7.8 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of cyclohexanecarboxylic anhydride (1.4 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 69 (actual yield: 1.4 g; percent yield: 66%).

[Chem. 276]

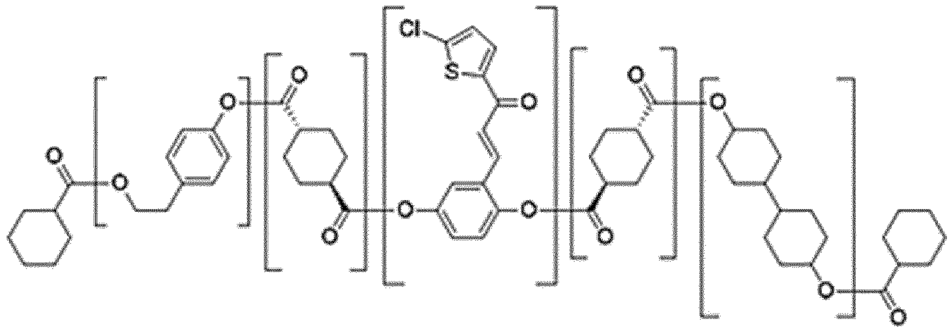

Polymer 69

13.0% by mass of polymer 69 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 70]

In a nitrogen atmosphere, compound (1'-2-2) (37.3 g, 133.0 mmol), obtained in Dihydroxy Compound Example 2, compound (2"A-1-1-DH) (9.9 g, 40.8 mmol), obtained in Synthesis Example 2, polyethylene glycol 20000 (manufactured by Tokyo Chemical Industry Co., Ltd., 7.9 g), trans-1,4-cyclohexanedicarboxylic acid (30.0 g, 174.2 mmol), pyridine (74.7 mL) and N-methyl-2-pyrrolidone (74.7 mL) were added to a reaction vessel. The reaction system was stirred at 40°C for 1 hour for dissolution, and then N,N'-diisopropylcarbodiimide (48.4 g, 383.3 mmol) as an ester-linkage-forming condensing agent was added dropwise to initiate polymerization. The reaction system was stirred at 40°C for 3 hours, followed by the addition of acetic anhydride (17.8 g, 174.2 mmol) as a monocarboxylic acid derivative compound. After the reaction system was stirred at 30°C for 30 minutes, the mixture in the system was added to methanol (1320 mL). The solid that formed was collected by filtration, washed with methanol (3000 mL) and then vacuum-dried to give polymer 70 (actual yield: 39.2 g; percent yield: 50%).

[Chem. 277]

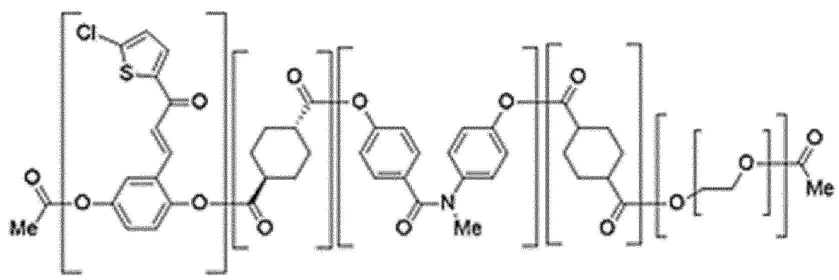

Polymer 70

13.0% by mass of polymer 70 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes,

and retardation characteristics and wavelength dispersion characteristics were evaluated. The results are presented in Table 6.

[Example 71]

In a nitrogen atmosphere, compound (1'-2-2) (0.91 g, 3.3 mmol), obtained in Dihydroxy Compound Example 2, 4-(2-hydroxyethyl)phenol (0.29 g, 2.1 mmol), hydroquinone (0.05 g, 0.5 mmol), trans-1,4-cyclohexanedicarboxylic acid (1.0 g, 5.8 mmol) and pyridine (7.5 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.59 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 71 (actual yield: 1.6 g; percent yield: 76%).

[Chem. 278]

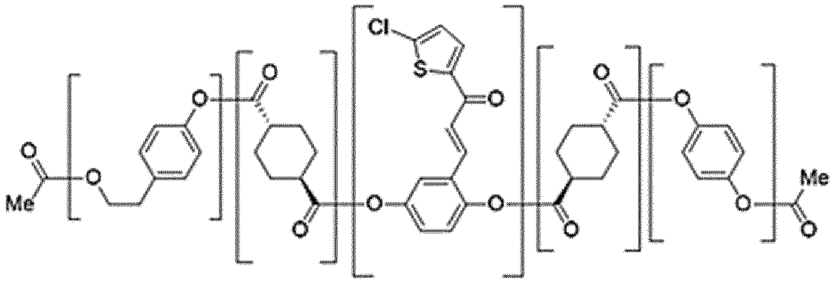

Polymer 71

13.0% by mass of polymer 71 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 $mJ/cm^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 72]

In a nitrogen atmosphere, compound (1'-2-2) (0.91 g, 3.3 mmol), obtained in Dihydroxy Compound Example 2, 4-(2-hydroxyethyl)phenol (0.29 g, 2.1 mmol), methylhydroquinone (0.06 g, 0.5 mmol), trans-1,4-cyclohexanedicarboxylic acid (1.0 g, 5.8 mmol) and pyridine (7.6 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.59 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 72 (actual yield: 1.5 g; percent yield: 71 %).

[Chem. 279]

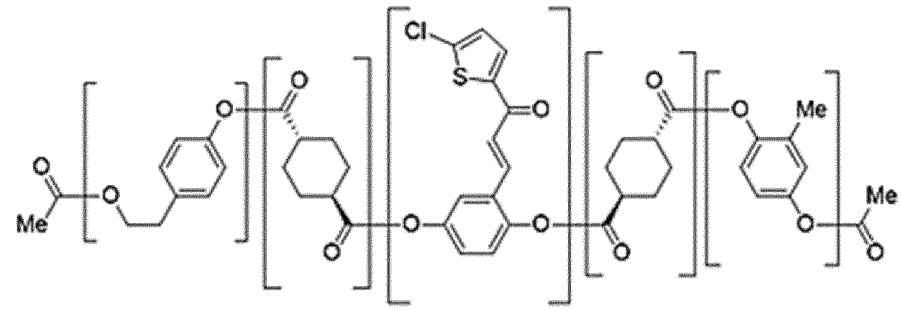

Polymer 72

13.0% by mass of polymer 72 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 73]

In a nitrogen atmosphere, compound (1'-2-2) (0.91 g, 3.3 mmol), obtained in Dihydroxy Compound Example 2, 4-(2-hydroxyethyl)phenol (0.29 g, 2.1 mmol), 2-tert-butylhydroquinone (0.08 g, 0.5 mmol), trans-1,4-cyclohexanedicarboxylic acid (1.0 g, 5.8 mmol) and pyridine (7.6 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.59 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 73 (actual yield: 1.5 g; percent yield: 70%).

[Chem. 280]

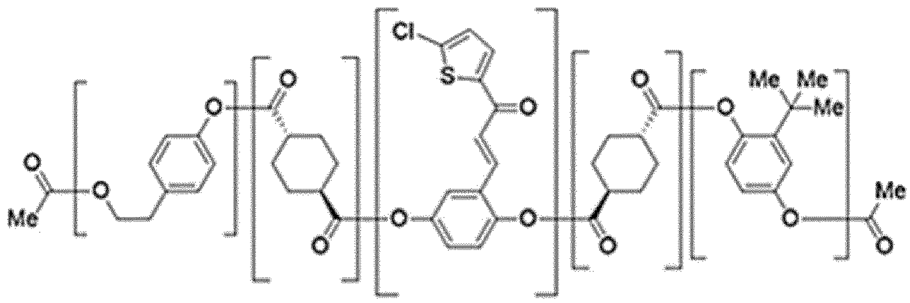

Polymer 73

13.0% by mass of polymer 73 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 74]

In a nitrogen atmosphere, compound (1'-2-2) (0.91 g, 3.3 mmol), obtained in Dihydroxy Compound Example 2, 4-(2-hydroxyethyl)phenol (0.22 g, 1.6 mmol), 2-(1,1,3,3-tetramethylbutyl)hydroquinone (0.21 g, 0.9 mmol), polyethylene glycol 20000 (manufactured by Tokyo Chemical Industry Co., Ltd., 0.1 g), trans-1,4-cyclohexanedicarboxylic acid (1.0 g, 5.8 mmol) and pyridine (8.4 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.59 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 74 (actual yield: 1.3 g; percent yield: 59%).

[Chem. 281]

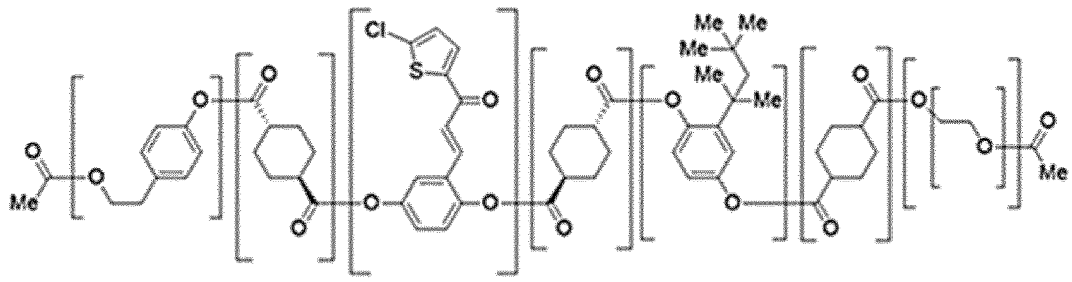

Polymer 74

13.0% by mass of polymer 74 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 $mJ/cm^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 75]

In a nitrogen atmosphere, compound (1'-2-2) (0.91 g, 3.3 mmol), obtained in Dihydroxy Compound Example 2, 4-(2-hydroxyethyl)phenol (0.22 g, 1.6 mmol), 2,5-di-tert-butylhydroquinone (0.21 g, 0.9 mmol), trans-1,4-cyclohexanedicarboxylic acid (1.0 g, 5.8 mmol) and pyridine (7.9 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.59 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 75 (actual yield: 1.5 g; percent yield: 68%).

[Chem. 282]

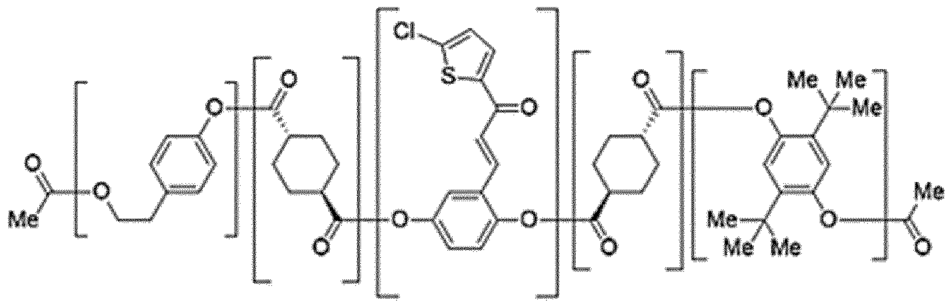

Polymer 75

13.0% by mass of polymer 75 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 $\mu$m). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 76]

In a nitrogen atmosphere, compound (1'-2-2) (0.91 g, 3.3 mmol), obtained in Dihydroxy Compound Example 2, 4-(2-hydroxyethyl)phenol (0.22 g, 1.6 mmol), 2,5-di-tert-pentylhydroquinone (0.23 g, 0.9 mmol), trans-1,4-cyclohexanedicarboxylic acid (1.0 g, 5.8 mmol) and pyridine (8.0 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.59 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 76 (actual yield: 1.4 g; percent yield: 66%).

[Chem. 283]

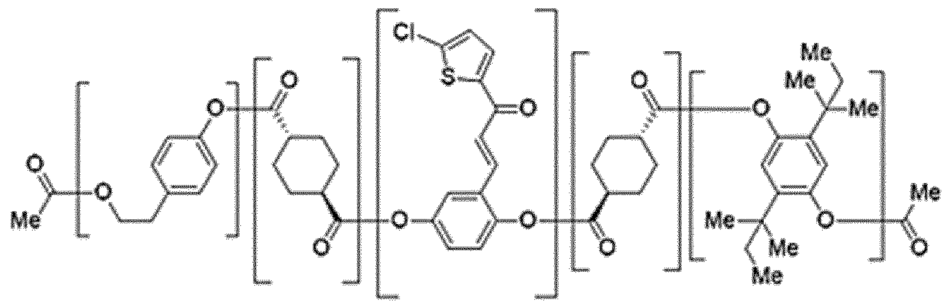

Polymer 76

13.0% by mass of polymer 76 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 $\mu$m). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 77]

In a nitrogen atmosphere, compound (1'-2-2) (0.91 g, 3.3 mmol), obtained in Dihydroxy Compound Example 2, 4-(2-hydroxyethyl)phenol (0.22 g, 1.6 mmol), 2,5-bis(1,1,3,3-tetramethylbutyl)hydroquinone (0.31 g, 0.9 mmol), trans-1,4-cyclohexanedicarboxylic acid (1.0 g, 5.8 mmol) and pyridine (8.3 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.59 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 77 (actual yield: 1.4 g; percent yield: 63%).

[Chem. 284]

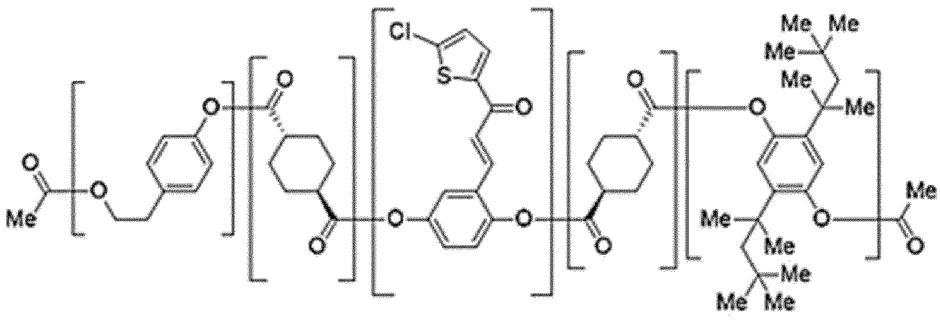

Polymer 77

13.0% by mass of polymer 77 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/$cm^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 78]

In a nitrogen atmosphere, compound (1'-2-2) (0.85 g, 3.0 mmol), obtained in Dihydroxy Compound Example 2, 4-(2-hydroxyethyl)phenol (0.19 g, 1.4 mmol), 2-(1,1,3,3-tetramethylbutyl)hydroquinone (0.31 g, 1.4 mmol), polyethylene glycol 20000 (manufactured by Tokyo Chemical Industry Co., Ltd., 0.1 g), trans-1,4-cyclohexanedicarboxylic acid (1.0 g, 5.8 mmol) and pyridine (8.4 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of decanoic anhydride (1.9 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 78 (actual yield: 1.2 g; percent yield: 53%).

[Chem. 285]

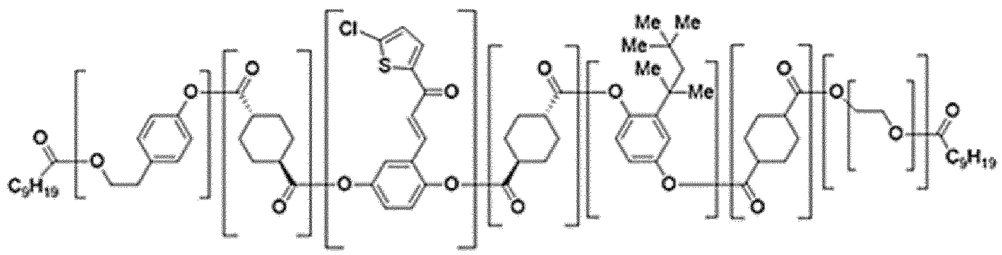

Polymer 78

13.0% by mass of polymer 78 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 79]

In a nitrogen atmosphere, compound (1'-2-2) (0.85 g, 3.0 mmol), obtained in Dihydroxy Compound Example 2, 4-(2-hydroxyethyl)phenol (0.06 g, 0.5 mmol), 2-tert-butylhydroquinone (0.39 g, 2.3 mmol), polyethylene glycol 20000 (manufactured by Tokyo Chemical Industry Co., Ltd., 0.1 g), trans-1,4-cyclohexanedicarboxylic acid (1.0 g, 5.8 mmol) and pyridine (5.7 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of decanoic anhydride (1.9 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 79 (actual yield: 1.2 g; percent yield: 56%).

[Chem. 286]

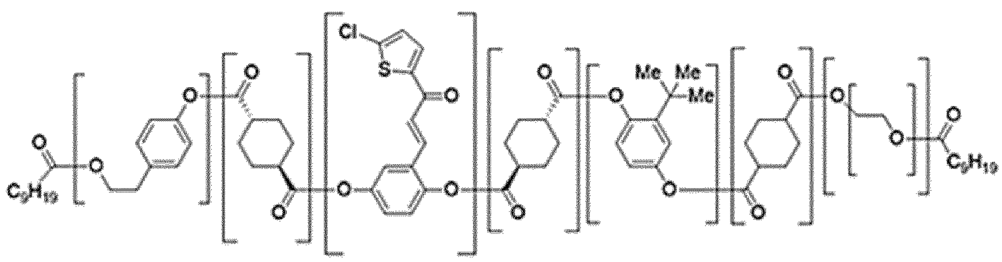

Polymer 79

13.0% by mass of polymer 79 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in

Table 6.

[Example 80]

In a nitrogen atmosphere, compound (1'-2-2) (1.0 g, 3.7 mmol), obtained in Dihydroxy Compound Example 2, 4-(2-hydroxyethyl)phenol (0.22 g, 1.6 mmol), methylhydroquinone (0.06 g, 0.5 mmol), trans-1,4-cyclohexanedicarboxylic acid (1.0 g, 5.8 mmol) and pyridine (7.8 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of hexanoic anhydride (1.2 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 80 (actual yield: 1.7 g; percent yield: 78%).

[Chem. 287]

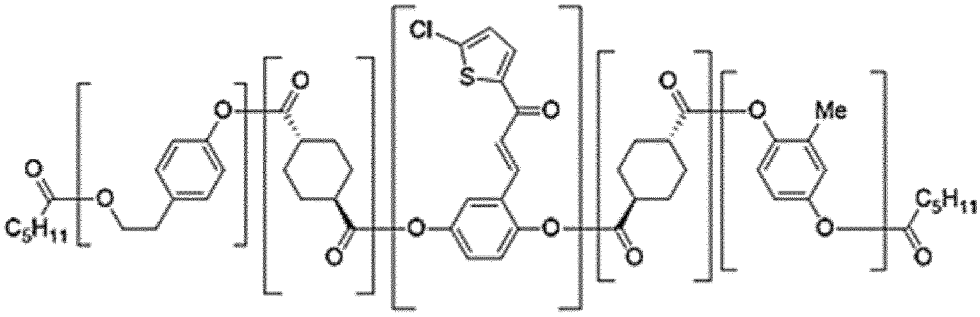

Polymer 80

13.0% by mass of polymer 80 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 81]

In a nitrogen atmosphere, compound (1'-2-2) (1.0 g, 3.7 mmol), obtained in Dihydroxy Compound Example 2, compound (2"C-2-2) (0.1 g, 0.5 mmol), obtained in Synthesis Example 11, 4-(2-hydroxyethyl)phenol (0.22 g, 1.6 mmol), trans-1,4-cyclohexanedicarboxylic acid (1.0 g, 5.8 mmol) and pyridine (8.1 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.6 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 81 (actual yield: 1.7 g; percent yield: 79%).

[Chem. 288]

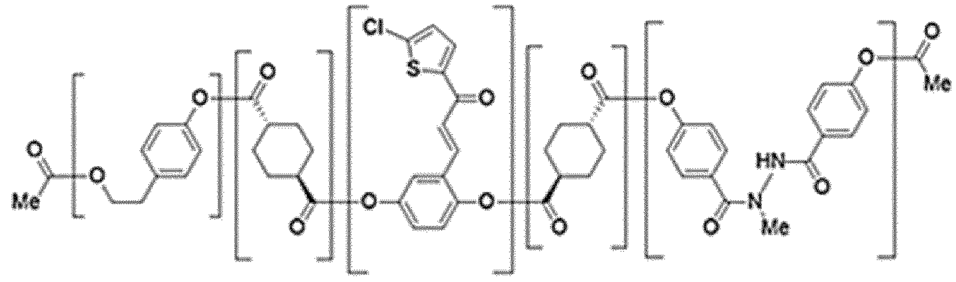

Polymer 81

13.0% by mass of polymer 81 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 82]

In a nitrogen atmosphere, compound (1'-2-2) (1.0 g, 3.7 mmol), obtained in Dihydroxy Compound Example 2, compound (2"C-2-13) (0.1 g, 0.5 mmol), obtained in Synthesis Example 20-1, 4-(2-hydroxyethyl)phenol (0.22 g, 1.6 mmol), trans-1,4-cyclohexanedicarboxylic acid (1.0 g, 5.8 mmol) and pyridine (8.2 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.6 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 82 (actual yield: 1.7 g; percent yield: 78%).

[Chem. 289]

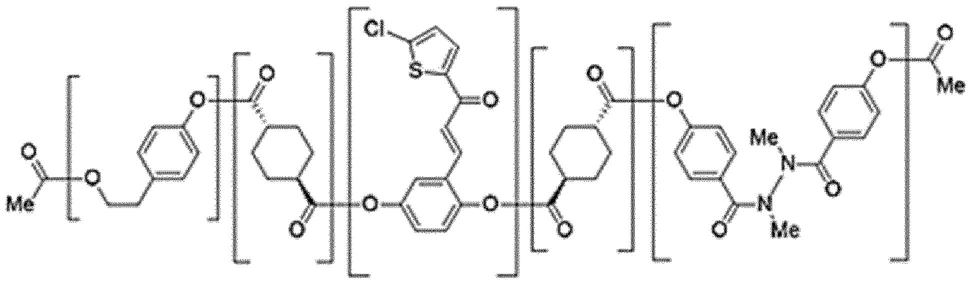

Polymer 82

13.0% by mass of polymer 82 was dissolved in 87.0% by mass of hexafluoro-2-propanol. This solution was cast onto a quartz substrate, spin coating was performed at 1800 rpm for 60 seconds, and the resulting coating was dried in an oven at 60°C for 30 minutes to give a film (thickness, 5 μm). The measurement of the yellowness index (YI) of the resulting film and visual evaluation of film appearance were conducted. The resulting film was irradiated with 100 mJ/cm$^2$ of polarized ultraviolet radiation light at 365 nm, then heating treatment was performed at 150°C for 10 minutes, and retardation characteristics and wavelength dispersion characteristics were evaluated. The results are presented in Table 6.

[Example 83]

In a nitrogen atmosphere, compound (1'-2-2) (0.91 g, 3.3 mmol), obtained in Dihydroxy Compound Example 2, 4-(2-hydroxyethyl)phenol (0.22 g, 1.6 mmol), 2-(1,1,3,3-tetramethylbutyl)hydroquinone (0.21 g, 0.9 mmol), trans-1,4-cyclohexanedicarboxylic acid (1.0 g, 5.8 mmol) and pyridine (7.9 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.6 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 83 (actual yield: 1.4 g; percent yield: 63%).

[Chem. 290]

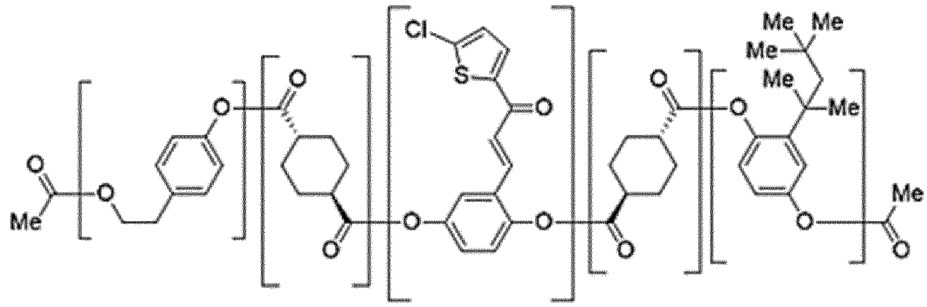

Polymer 83

[Example 84]

In a nitrogen atmosphere, compound (1'-2-2) (0.91 g, 3.3 mmol), obtained in Dihydroxy Compound Example 2, 1,4-cyclohexanedimethanol (0.13 g, 0.87 mmol), 4-(2-hydroxyethyl)phenol (0.23 g, 1.7 mmol), trans-1,4-cyclohexanedicarboxylic acid (1.0 g, 5.8 mmol) and pyridine (7.6 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.6 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 84 (actual yield: 1.6 g; percent yield: 77%).

[Chem. 291]

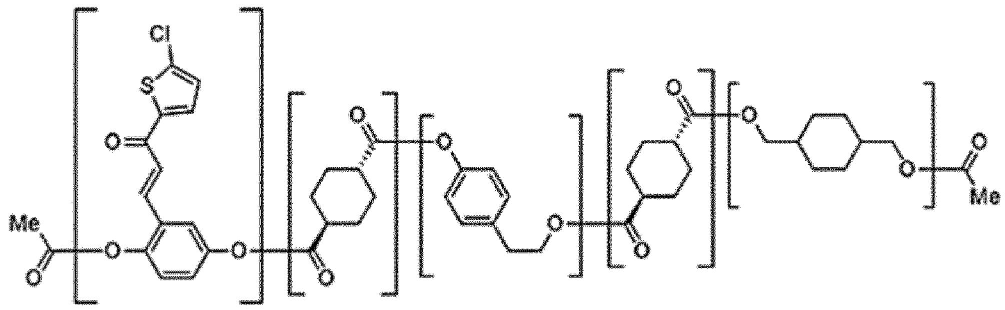

Polymer 84

6.0% by mass of polymer 84 was dissolved in 94.0% by mass of 1,1,1,3,3,3-hexafluoroisopropyl alcohol. This solution was cast onto a quartz glass substrate, then spin coating was performed, and the resulting coating was dried in an oven at 60°C for 30 minutes to give a thin film. The resulting thin film (thickness, 1 μm) was irradiated with 500 mJ/$cm^2$ of polarized ultraviolet light at 365 nm and then heated at 150°C, with the result that the film exhibited retardation

Re = 46.8 nm and wavelength dispersion Re (450)/Re (550) = 0.89.

[Example 85]

In a nitrogen atmosphere, compound (1'-2-2) (0.93 g, 3.3 mmol), obtained in Dihydroxy Compound Example 2, tricyclodecanedimethanol (0.13 g, 0.64 mmol), 4-(2-hydroxyethyl)phenol (0.26 g, 1.7 mmol), trans-1,4-cyclohexanedicarboxylic acid (1.0 g, 5.8 mmol) and pyridine (7.8 mL) were added to a reaction vessel. The reaction system was stirred at 30°C for 1 hour for dissolution, and then N,N'-diisopropylcarbodiimide (1.6 g, 12.8 mmol) as an ester-linkage-forming condensing agent was added dropwise to initiate polymerization. The reaction system was stirred at 30°C for 3 hours, followed by the addition of acetic anhydride (0.6 g, 5.8 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 85 (actual yield: 1.5 g; percent yield: 70%).

[Chem. 292]

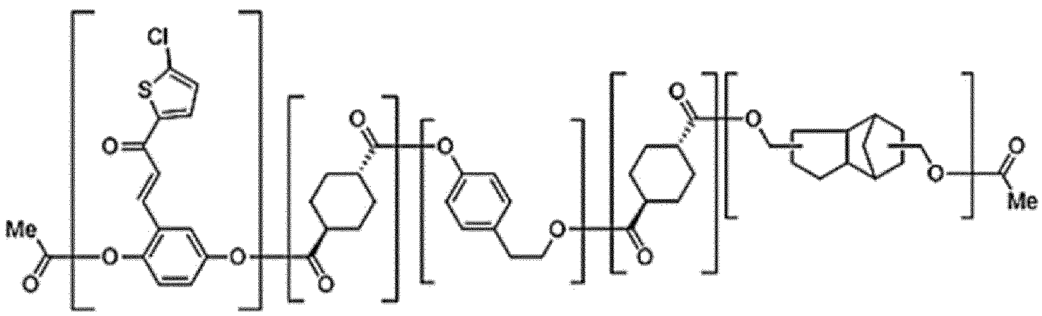

Polymer 85

6.0% by mass of polymer 85 was dissolved in 94.0% by mass of 1,1,1,3,3,3-hexafluoroisopropyl alcohol. This solution was cast onto a quartz glass substrate, then spin coating was performed, and the resulting coating was dried in an oven at 60°C for 30 minutes to give a thin film. The resulting thin film (thickness, 1 μm) was irradiated with 500 mJ/cm$^2$ of polarized ultraviolet light at 365 nm and then heated at 150°C, with the result that the film exhibited retardation Re = 39.9 nm and wavelength dispersion Re (450)/Re (550) = 0.88.

Table 6

| | Polymer | YI (%) | Film appearance | Re (nm) | R(450)/R(550) |
|---|---|---|---|---|---|
| Example 57 | Polymer 57 | 2.7 | A | 36 | 0.96 |
| Example 58 | Polymer 58 | 2.5 | A | 48 | 0.96 |
| Example 59 | Polymer 59 | 4.9 | B | 36 | 0.94 |
| Example 60 | Polymer 60 | 2.8 | A | 52 | 0.88 |
| Example 61 | Polymer 61 | 3.6 | B | 270 | 0.88 |
| Example 62 | Polymer 62 | 1.8 | A | 16 | 0.92 |
| Example 63 | Polymer 63 | 1.7 | A | 9 | 0.86 |
| Example 64 | Polymer 64 | 3.8 | B | 19 | 0.82 |
| Example 65 | Polymer 65 | 1.4 | A | 16 | 0.84 |
| Example 66 | Polymer 66 | 2.8 | A | 7 | 0.87 |
| Example 67 | Polymer 67 | 2.4 | A | 12 | 0.87 |
| Example 68 | Polymer 68 | 2.3 | A | 7 | 0.88 |
| Example 69 | Polymer 69 | 2.7 | A | 6 | 0.87 |
| Example 70 | Polymer 70 | 3.2 | B | 10 | 0.87 |

(continued)

| | Polymer | YI (%) | Film appearance | Re (nm) | R(450)/R(550) |
|---|---|---|---|---|---|
| Example 71 | Polymer 71 | 2.7 | A | 7 | 0.96 |
| Example 72 | Polymer 72 | 2.4 | A | 9 | 0.88 |
| Example 73 | Polymer 73 | 2.5 | A | 7 | 0.87 |
| Example 74 | Polymer 74 | 3.0 | B | 3 | 0.83 |
| Example 75 | Polymer 75 | 3.0 | B | 5 | 0.89 |
| Example 76 | Polymer 76 | 3.5 | B | 11 | 0.88 |
| Example 77 | Polymer 77 | 2.7 | A | 10 | 0.91 |
| Example 78 | Polymer 78 | 2.7 | A | 6 | 0.86 |
| Example 79 | Polymer 79 | 3.3 | B | 11 | 0.83 |
| Example 80 | Polymer 80 | 3.2 | B | 10 | 0.84 |
| Example 81 | Polymer 81 | 3.0 | B | 5 | 0.84 |
| Example 82 | Polymer 82 | 3.2 | B | 7 | 0.88 |

As shown in Table 6, films produced using the polymers in Examples 57 to 82 exhibit a low yellowness index (YI) and develop reverse wavelength dispersion retardations through irradiation with ultraviolet radiation light and heating treatment.
Polymers 57 to 82 exhibit a low yellowness index (YI).

[Polymerization Example 1]

[Chem. 293]

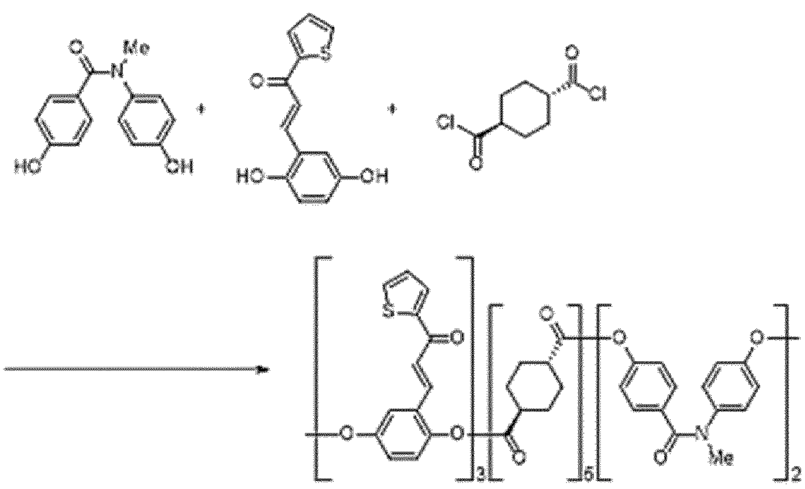

Polymer 86

In a nitrogen stream, compound (2"A-1-1-DH) (1.16 g, 4.78 mmol), obtained in Synthesis Example 2, and compound (1'-2-1) (1.77 g, 7.16 mmol), obtained in Dihydroxy Compound Example 1, as diol monomers were added to a reaction vessel. Then an aqueous (114 mL) solution of sodium hydroxide (960 mg, 23.9 mmol) and a 2 wt% aqueous solution of tetrabutylammonium bromide (4.75 mL) were added, and stirring was initiated. Trans-1,4-cyclohexanedicarboxylic acid dichloride (2.50 g, 12.0 mmol) as a dicarboxylic acid dichloride monomer, furthermore, was dissolved in chloroform (119 mL), followed by the addition of this solution to the reaction system. After the reaction system was stirred for 3 hours, the mixture in the system was added to methanol (500 mL). The solid that formed was collected by filtration, washed with methanol (200 mL) and water (200 mL) and then vacuum-dried, giving 4.13 g (percent yield: 91%) of polymer 86.

[Polymerization Example 2]

[Chem. 294]

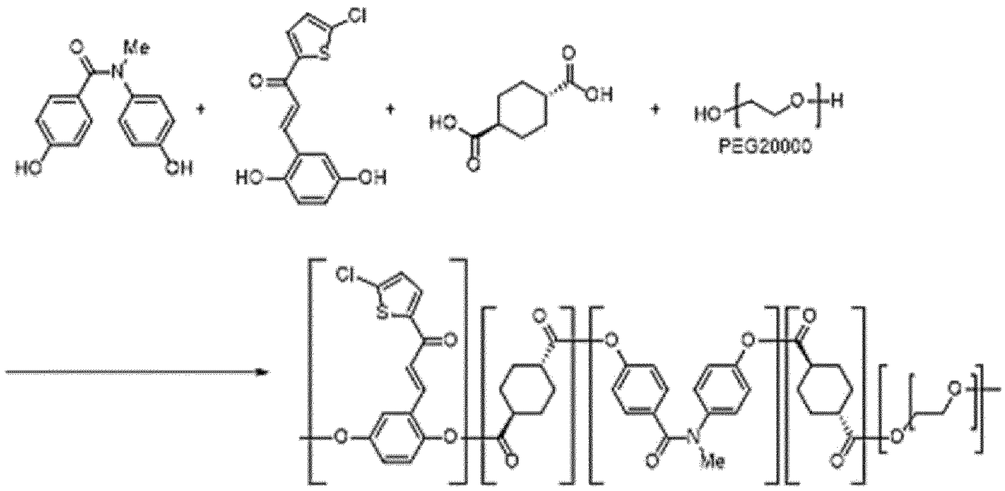

Polymer 87

In a nitrogen atmosphere, compound (2"A-1-1-DH) (1.44 g, 5.92 mmol), obtained in Synthesis Example 2, compound (1'-2-2) (3.22 g, 11.5 mmol), obtained in Dihydroxy Compound Example 2, trans-1,4-cyclohexanedicarboxylic acid (3.00 g, 17.4 mmol), PEG 20000 (0.801 mg) and pyridine (29.6 mL) were added to a reaction vessel. The reaction system was stirred at 30°C for 1 hour for dissolution, and then N,N'-diisopropylcarbodiimide (4.84 g, 38.3 mmol) was added dropwise. After the reaction system was stirred at 30°C for 3 hours, the mixture in the system was added to methanol (300 mL). The solid that formed was collected by filtration, washed with methanol (500 mL) and then vacuum-dried to give polymer 87 (actual yield: 4.53 g; percent yield: 58%).

[Polymerization Example 3]

[Chem. 295]

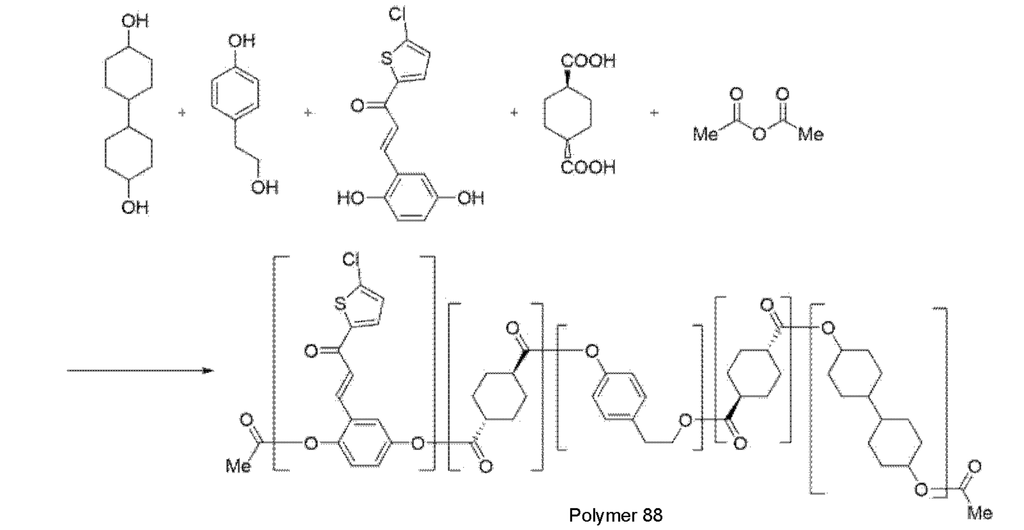

Polymer 88

In a nitrogen atmosphere, compound (1'-2-2) (21 g, 74 mmol), obtained in Dihydroxy Compound Example 2, 4,4-bicyclohexanol (1.8 g, 9.3 mmol), 4-(2-hydroxyethyl)phenol (4.5 g, 33 mmol), trans-1,4-cyclohexanedicarboxylic acid (20 g, 120 mmol) and pyridine (119 mL) were added to a reaction vessel. The reaction system was stirred at 30°C for 30 minutes for dissolution, and then N,N'-diisopropylcarbodiimide (32 g, 260 mmol) as an ester-linkage-forming condensing agent was added dropwise to initiate polymerization. The reaction system was stirred at 30°C for 3 hours, followed by the addition of acetic anhydride (12 g, 120 mmol) as a monocarboxylic acid derivative compound. After the reaction system was stirred at 30°C for 30 minutes, the mixture in the system was added to methanol (1400 mL). The solid that formed was collected by filtration, washed with methanol (2000 mL) and then vacuum-dried to give polymer 88 (actual yield: 31.6 g; percent yield: 73%).

[Polymerization Example 4]

[Chem. 296]

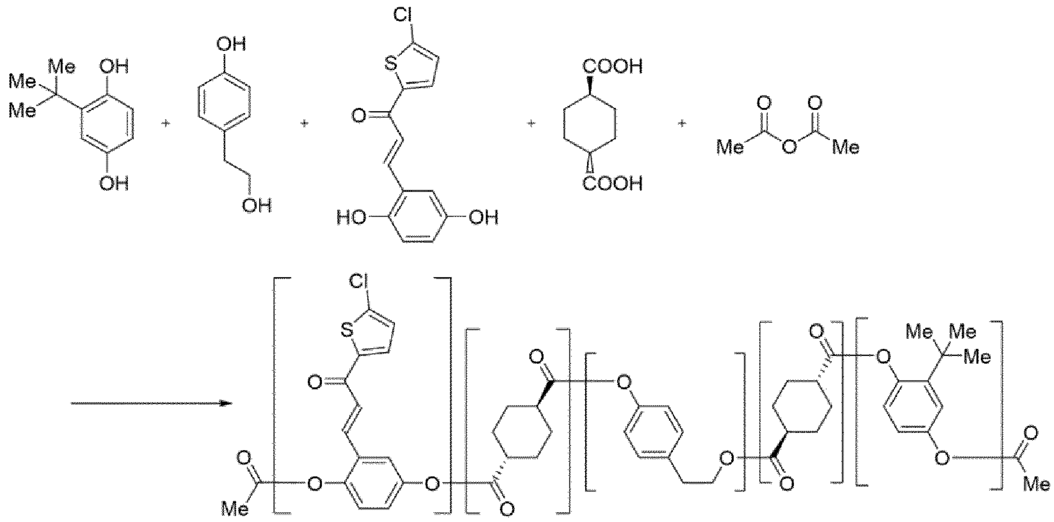

Polymer 89

In a nitrogen atmosphere, compound (1'-2-2) (0.98 g, 3.49 mmol), obtained in Dihydroxy Compound Example

2, 2-(tert-butyl)benzene-1,4-diol (0.15 g, 0.93 mmol), 4-(2-hydroxyethyl)phenol (0.19 g, 1.39 mmol), trans-1,4-cyclohexanedicarboxylic acid (1.00 g, 5.81 mmol) and pyridine (7.8 mL) were added to a reaction vessel. The reaction system was stirred at 30°C for 30 minutes for dissolution, and then N,N'-diisopropylcarbodiimide (1.61 g, 12.78 mmol) as an ester-linkage-forming condensing agent was added dropwise to initiate polymerization. The reaction system was stirred at 30°C for 3 hours, followed by the addition of acetic anhydride (0.6 g, 5.81 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 89 (actual yield: 1.49 g; percent yield: 70%).

[Polymerization Example 5]

[Chem. 297]

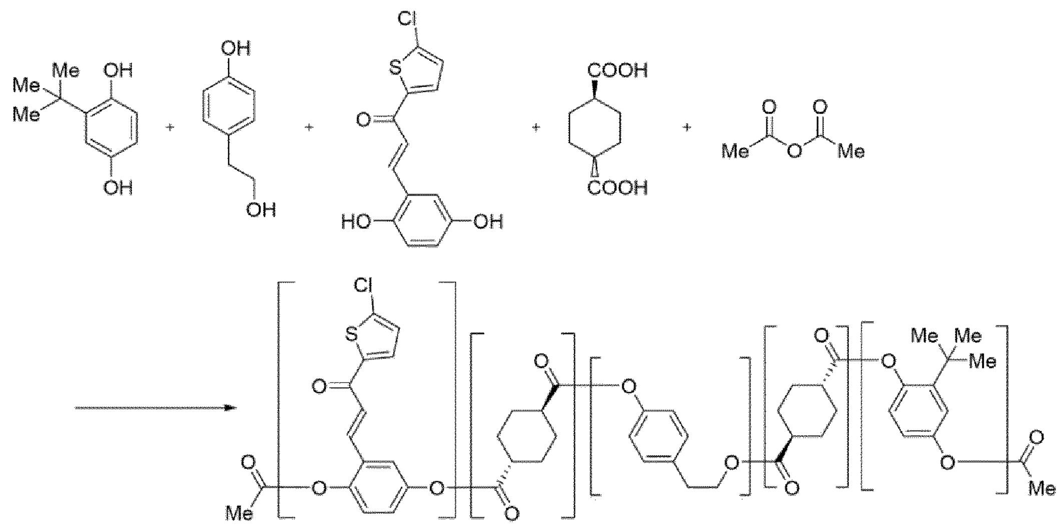

Polymer 90

In a nitrogen atmosphere, compound (1'-2-2) (0.98 g, 3.49 mmol), obtained in Dihydroxy Compound Example 2, 2-(tert-butyl)benzene-1,4-diol (0.19 g, 1.16 mmol), 4-(2-hydroxyethyl)phenol (0.16 g, 1.16 mmol), trans-1,4-cyclohexanedicarboxylic acid (1.00 g, 5.81 mmol) and pyridine (7.8 mL) were added to a reaction vessel. The reaction system was stirred at 30°C for 30 minutes for dissolution, and then N,N'-diisopropylcarbodiimide (1.61 g, 12.78 mmol) as an ester-linkage-forming condensing agent was added dropwise to initiate polymerization. The reaction system was stirred at 30°C for 3 hours, followed by the addition of acetic anhydride (0.6 g, 5.81 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 90 (actual yield: 1.47 g; percent yield: 69%).

[Polymerization Example 6]

[Chem. 298]

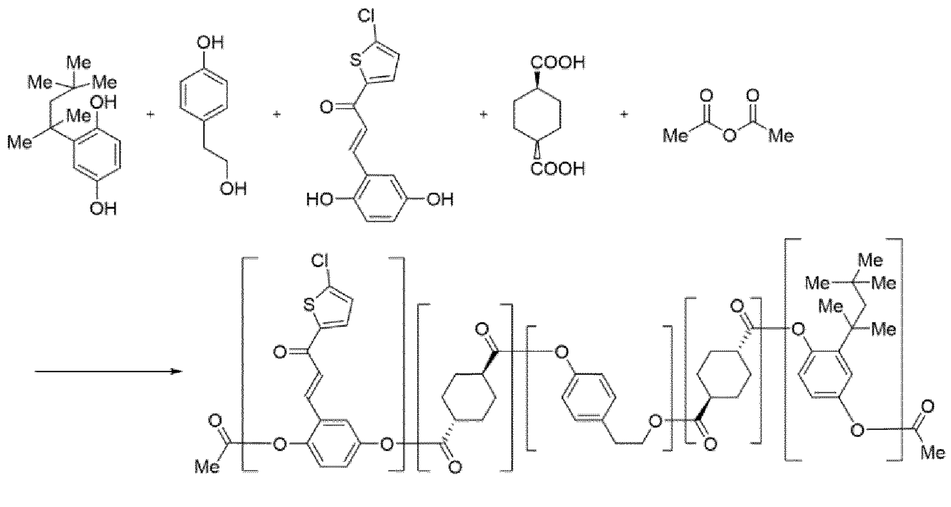

Polymer 91

In a nitrogen atmosphere, compound (1'-2-2) (0.91 g, 3.25 mmol), obtained in Dihydroxy Compound Example 2, 2-(2,4,4-trimethylpentan-2-yl)benzene-1,4-diol (0.27 g, 0.93 mmol), 4-(2-hydroxyethyl)phenol (0.22 g, 1.63 mmol), trans-1,4-cyclohexanedicarboxylic acid (1.00 g, 5.81 mmol) and pyridine (7.9 mL) were added to a reaction vessel. The reaction system was stirred at 30°C for 30 minutes for dissolution, and then N,N'-diisopropylcarbodiimide (1.61 g, 12.78 mmol) as an ester-linkage-forming condensing agent was added dropwise to initiate polymerization. The reaction system was stirred at 30°C for 3 hours, followed by the addition of acetic anhydride (0.6 g, 5.81 mmol) as a monocarboxylic acid derivative compound. After the reaction system was stirred at 30°C for 30 minutes, the mixture in the system was added to methanol (300 mL). The solid that formed was collected by filtration, washed with methanol (500 mL) and then vacuum-dried to give polymer 91 (actual yield: 1.35 g; percent yield: 63%).

[Example 86]

5.4% by weight of polymer 86 and 0.6% by weight of dimethyl phthalate (molecular weight: 194) as a thermal reorientation accelerator were dissolved in 94% by weight of 1,1,1,3,3,3-hexafluoro-2-propanol. This solution was cast onto a quartz glass substrate, spin coating was performed at 4000 rpm for 60 seconds, and the resulting coating was dried in an oven at 60°C for 30 minutes to give a thin film (thickness, 1.004 μm). The resulting thin film was irradiated with 100 mJ/cm$^2$ of linearly polarized ultraviolet light at 365 nm and then heated at 190°C, giving a thin film exhibiting a large amount of retardation with a good appearance. The amount of retardation is presented in Table 7.

[Example 87]

5.4% by weight of polymer 86 and 0.6% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 5000 rpm for 60 seconds, and the resulting coating was dried in an oven at 60°C for 30 minutes to give a thin film (thickness, 0.948 μm). The resulting thin film was irradiated with 100 mJ/cm$^2$ of linearly polarized ultraviolet light at 365 nm and then heated at 190°C, giving a thin film exhibiting a large amount of retardation with a good appearance. The amount of retardation is presented in Table 7.

[Example 88]

A thin film (thickness, 1.121 μm) was obtained in the same manner as in Example 86, except that ditridecyl phthalate (molecular weight: 531) was used as a thermal reorientation accelerator. The resulting thin film was irradiated with 100 mJ/cm$^2$ of linearly polarized ultraviolet light at 365 nm and then heated at 190°C, giving a thin film exhibiting a large amount of retardation with a good appearance. The amount of retardation is presented in Table 7.

[Example 89]

A thin film (thickness, 1.098 μm) was obtained in the same manner as in Example 86, except that tributyl trimellitate (molecular weight: 378) was used as a thermal reorientation accelerator. The resulting thin film was irradiated

with 100 mJ/cm$^2$ of linearly polarized ultraviolet light at 365 nm and then heated at 190°C, giving a thin film exhibiting a large amount of retardation with a good appearance. The amount of retardation is presented in Table 7.

[Example 90]

A thin film (thickness, 1.155 μm) was obtained in the same manner as in Example 86, except that trihexyl O-butyrylcitrate (molecular weight: 515) was used as a thermal reorientation accelerator. The resulting thin film was irradiated with 100 mJ/cm$^2$ of linearly polarized ultraviolet light at 365 nm and then heated at 190°C, giving a thin film exhibiting a large amount of retardation with a good appearance. The amount of retardation is presented in Table 7.

[Example 91]

5.7% by weight of polymer 86 and 0.3% by weight of diisodecyl adipate (molecular weight: 427) as a thermal reorientation accelerator were dissolved in 94% by weight of 1,1,1,3,3,3-hexafluoro-2-propanol. This solution was cast onto a quartz glass substrate, spin coating was performed at 5000 rpm for 60 seconds, and the resulting coating was dried in an oven at 60°C for 30 minutes to give a thin film (thickness, 1.021 μm). The resulting thin film was irradiated with 100 mJ/cm$^2$ of linearly polarized ultraviolet light at 365 nm and then heated at 190°C, giving a thin film exhibiting a large amount of retardation with a good appearance. The amount of retardation is presented in Table 7.

[Example 92]

A thin film (thickness, 0.943 μm) was obtained in the same manner as in Example 87, except that diisodecyl adipate (molecular weight: 427) was used as a thermal reorientation accelerator. The resulting thin film was irradiated with 100 mJ/cm$^2$ of linearly polarized ultraviolet light at 365 nm and then heated at 190°C, giving a thin film exhibiting a large amount of retardation with a good appearance. The amount of retardation is presented in Table 7.

[Example 93]

5.1 % by weight of polymer 86 and 0.9% by weight of diisodecyl adipate (molecular weight: 427) as a thermal reorientation accelerator were dissolved in 94% by weight of 1,1,1,3,3,3-hexafluoro-2-propanol. This solution was cast onto a quartz glass substrate, spin coating was performed at 2500 rpm for 60 seconds, and the resulting coating was dried in an oven at 60°C for 30 minutes to give a thin film (thickness, 0.981 μm). The resulting thin film was irradiated with 100 mJ/cm$^2$ of linearly polarized ultraviolet light at 365 nm and then heated at 190°C, giving a thin film exhibiting a large amount of retardation with a good appearance. The amount of retardation is presented in Table 7.

[Example 94]

4.5% by weight of polymer 86 and 1.5% by weight of diisodecyl adipate (molecular weight: 427) as a thermal reorientation accelerator were dissolved in 94% by weight of 1,1,1,3,3,3-hexafluoro-2-propanol. This solution was cast onto a quartz glass substrate, spin coating was performed at 5000 rpm for 60 seconds, and the resulting coating was dried in an oven at 60°C for 30 minutes to give a thin film (thickness, 0.896 μm). The resulting thin film was irradiated with 100 mJ/cm$^2$ of linearly polarized ultraviolet light at 365 nm and then heated at 190°C, giving a thin film exhibiting a large amount of retardation with a good appearance. The amount of retardation is presented in Table 7.

[Example 95]

A thin film (thickness, 1.019 μm) was obtained in the same manner as in Example 86, except that trimethyl phosphate (molecular weight: 140) was used as a thermal reorientation accelerator. The resulting thin film was irradiated with 100 mJ/cm$^2$ of linearly polarized ultraviolet light at 365 nm and then heated at 190°C, giving a thin film exhibiting a large amount of retardation with a good appearance. The amount of retardation is presented in Table 7.

[Example 96]

A thin film (thickness, 1.025 μm) was obtained in the same manner as in Example 87, except that a polyester plasticizer (ADK CIZER P-300 (molecular weight: 3000)) was used as a thermal reorientation accelerator. The resulting thin film was irradiated with 100 mJ/cm$^2$ of linearly polarized ultraviolet light at 365 nm and then heated at 190°C, giving a thin film exhibiting a large amount of retardation with a good appearance. The amount of retardation is presented in Table 7.

[Example 97]

A thin film (thickness, 1.052 μm) was obtained in the same manner as in Example 87, except that polyethylene glycol 4000 (molecular weight: 4000) was used as a thermal reorientation accelerator. The resulting thin film was irradiated with 100 mJ/$cm^2$ of linearly polarized ultraviolet light at 365 nm and then heated at 190°C, giving a thin film exhibiting a large amount of retardation with a good appearance. The amount of retardation is presented in Table 7.

[Example 98]

A thin film (thickness, 1.077 μm) was obtained in the same manner as in Example 87, except that polyethylene glycol 11000 (molecular weight: 11000) was used as a thermal reorientation accelerator. The resulting thin film was irradiated with 100 mJ/$cm^2$ of linearly polarized ultraviolet light at 365 nm and then heated at 190°C, giving a thin film exhibiting a large amount of retardation with a good appearance. The amount of retardation is presented in Table 7.

[Example 99]

A thin film (thickness, 1.141 μm) was obtained in the same manner as in Example 87, except that polyethylene glycol 20000 (molecular weight: 20000) was used as a thermal reorientation accelerator. The resulting thin film was irradiated with 100 mJ/$cm^2$ of linearly polarized ultraviolet light at 365 nm and then heated at 190°C, giving a thin film exhibiting a large amount of retardation with a good appearance. The amount of retardation is presented in Table 7.

[Example 100]

A thin film (thickness, 0.975 μm) was obtained in the same manner as in Example 87, except that bis[3-(3-tert-butyl-4-hydroxy-5-methylphenyl)propionic acid][ethylenebis(oxyethylene)] (molecular weight: 587) was used as a thermal reorientation accelerator. The resulting thin film was irradiated with 100 mJ/$cm^2$ of linearly polarized ultraviolet light at 365 nm and then heated at 190°C, giving a thin film exhibiting a large amount of retardation with a good appearance. The amount of retardation is presented in Table 8.

[Example 101]

4.5% by weight of polymer 86 and 0.5% by weight of tetrakis[3-(3',5'-di-tert-butyl-4'-hydroxyphenyl)propionic acid] (molecular weight: 1178) as a thermal reorientation accelerator were dissolved in 95% by weight of 1,1,1,3,3,3-hexafluoro-2-propanol. This solution was cast onto a quartz glass substrate, spin coating was performed at 3000 rpm for 60 seconds, and the resulting coating was dried in an oven at 60°C for 30 minutes to give a thin film (thickness, 0.986 μm). The resulting thin film was irradiated with 100 mJ/$cm^2$ of linearly polarized ultraviolet light at 365 nm and then heated at 190°C, giving a thin film exhibiting a large amount of retardation with a good appearance. The amount of retardation is presented in Table 8.

[Example 102]

A thin film (thickness, 1.067 μm) was obtained in the same manner as in Example 87, except that 2,4,8,10-tetraoxaspiro[5,5]undecane-3,9-diylbis(2-methylpropane-2,1-diyl) bis[3-[3-(tert-butyl)-4-hydroxy-5-methylphenyl]propanoate] (molecular weight: 741) was used as a thermal reorientation accelerator. The resulting thin film was irradiated with 100 mJ/$cm^2$ of linearly polarized ultraviolet light at 365 nm and then heated at 190°C, giving a thin film exhibiting a large amount of retardation with a good appearance. The amount of retardation is presented in Table 8.

[Example 103]

A thin film (thickness, 0.969 μm) was obtained in the same manner as in Example 87, except that triisodecyl phosphite (molecular weight: 503) was used as a thermal reorientation accelerator. The resulting thin film was irradiated with 100 mJ/$cm^2$ of linearly polarized ultraviolet light at 365 nm and then heated at 190°C, giving a thin film exhibiting a large amount of retardation with a good appearance. The amount of retardation is presented in Table 8.

[Example 104]

A thin film (thickness, 0.970 μm) was obtained in the same manner as in Example 87, except that didodecyl 3,3'-thiodipropionate (molecular weight: 515) was used as a thermal reorientation accelerator. The resulting thin film

was irradiated with 100 $mJ/cm^2$ of linearly polarized ultraviolet light at 365 nm and then heated at 190°C, giving a thin film exhibiting a large amount of retardation with a good appearance. The amount of retardation is presented in Table 8.

[Example 105]

A thin film (thickness, 1.033 μm) was obtained in the same manner as in Example 87, except that bis(1,2,2,6,6-pentamethyl-4-piperidyl) sebacate (molecular weight: 509) was used as a thermal reorientation accelerator. The resulting thin film was irradiated with 100 $mJ/cm^2$ of linearly polarized ultraviolet light at 365 nm and then heated at 190°C, giving a thin film exhibiting a large amount of retardation with a good appearance. The amount of retardation is presented in Table 8.

[Example 106]

5.4% by weight of polymer 87 and 0.6% by weight of dihexyl phthalate (molecular weight: 224) as a thermal reorientation accelerator were dissolved in 94% by weight of 1,1,1,3,3,3-hexafluoro-2-propanol. This solution was cast onto a quartz glass substrate, spin coating was performed at 2200 rpm for 60 seconds, and the resulting coating was dried in an oven at 60°C for 30 minutes to give a thin film (thickness, 1.033 μm). The resulting thin film was irradiated with 100 $mJ/cm^2$ of linearly polarized ultraviolet light at 365 nm and then heated at 150°C, giving a thin film exhibiting a large amount of retardation with a good appearance. The amount of retardation is presented in Table 9.

[Example 107]

A thin film (thickness, 1.084 μm) was obtained in the same manner as in Example 106, except that diphenyl phthalate (molecular weight: 318) was used as a thermal reorientation accelerator. The resulting thin film was irradiated with 100 $mJ/cm^2$ of linearly polarized ultraviolet light at 365 nm and then heated at 150°C, giving a thin film exhibiting a large amount of retardation with a good appearance. The amount of retardation is presented in Table 9.

[Example 108]

A thin film (thickness, 1.042 μm) was obtained in the same manner as in Example 106, except that diisodecyl phthalate (molecular weight: 447) was used as a thermal reorientation accelerator. The resulting thin film was irradiated with 100 $mJ/cm^2$ of linearly polarized ultraviolet light at 365 nm and then heated at 150°C, giving a thin film exhibiting a large amount of retardation with a good appearance. The amount of retardation is presented in Table 9.

[Example 109]

A thin film (thickness, 1.079 μm) was obtained in the same manner as in Example 106, except that ditridecyl phthalate (molecular weight: 531) was used as a thermal reorientation accelerator. The resulting thin film was irradiated with 100 $mJ/cm^2$ of linearly polarized ultraviolet light at 365 nm and then heated at 150°C, giving a thin film exhibiting a large amount of retardation with a good appearance. The amount of retardation is presented in Table 9.

[Example 110]

A thin film (thickness, 1.018 μm) was obtained in the same manner as in Example 106, except that diisodecyl adipate (molecular weight: 427) was used as a thermal reorientation accelerator. The resulting thin film was irradiated with 100 $mJ/cm^2$ of linearly polarized ultraviolet light at 365 nm and then heated at 150°C, giving a thin film exhibiting a large amount of retardation with a good appearance. The amount of retardation is presented in Table 9.

[Example 111]

A thin film (thickness, 1.036 μm) was obtained in the same manner as in Example 106, except that trihexyl phosphate (molecular weight: 350) was used as a thermal reorientation accelerator. The resulting thin film was irradiated with 100 $mJ/cm^2$ of linearly polarized ultraviolet light at 365 nm and then heated at 150°C, giving a thin film exhibiting a large amount of retardation with a good appearance. The amount of retardation is presented in Table 9.

[Example 112]

A thin film (thickness, 1.057 μm) was obtained in the same manner as in Example 106, except that triphenyl

phosphate (molecular weight: 326) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 9.

[Example 113]

5.7% by weight of polymer 88 and 0.3% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 2600 rpm for 60 seconds, and the resulting coating was dried in an oven at 60°C for 30 minutes to give a thin film (thickness, 0.996 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 10.

[Example 114]

5.4% by weight of polymer 88 and 0.6% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 2400 rpm for 60 seconds, and the resulting coating was dried in an oven at 60°C for 30 minutes to give a thin film (thickness, 1.015 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 10.

[Example 115]

5.1% by weight of polymer 88 and 0.9% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 2300 rpm for 60 seconds, and the resulting coating was dried in an oven at 60°C for 30 minutes to give a thin film (thickness, 1.006 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 10.

[Example 116]

4.8% by weight of polymer 88 and 1.2% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 2000 rpm for 60 seconds, and the resulting coating was dried in an oven at 60°C for 30 minutes to give a thin film (thickness, 1.125 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 10.

[Example 117]

A thin film (thickness, 1.015 μm) was obtained in the same manner as in Example 114, except that ditridecyl phthalate (molecular weight: 531) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 10.

[Example 118]

A thin film (thickness, 0.995 μm) was obtained in the same manner as in Example 114, except that bis(2-ethylhexyl) phthalate (molecular weight: 391) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 10.

[Example 119]

A thin film (thickness, 1.021 μm) was obtained in the same manner as in Example 114, except that tris(2-ethylhexyl) trimellitate (molecular weight: 547) was used as a thermal reorientation accelerator. The resulting thin film

was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 10.

[Example 120]

A thin film (thickness, 0.997 μm) was obtained in the same manner as in Example 114, except that diisodecyl adipate (molecular weight: 427) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 10.

[Example 121]

A thin film (thickness, 1.019 μm) was obtained in the same manner as in Example 114, except that tris(2-ethylhexyl) phosphate (molecular weight: 435) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 10.

[Example 122]

A thin film (thickness, 1.027 μm) was obtained in the same manner as in Example 114, except that bis(2-ethylhexyl) terephthalate (molecular weight: 391) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 10.

[Example 123]

A thin film (thickness, 1.005 μm) was obtained in the same manner as in Example 114, except that di 2-ethylhexyl 4,5-epoxycyclohexane-1,2-dicarboxylate (molecular weight: 394) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 10.

[Example 124]

A thin film (thickness, 1.005 μm) was obtained in the same manner as in Example 114, except that bis(2-ethylhexyl) 4-cyclohexene-1,2-dicarboxylate (molecular weight: 395) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 10.

[Example 125]

5.94% by weight of polymer 89 and 0.06% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 2700 rpm for 60 seconds, and the resulting coating was dried in an oven at 60°C for 30 minutes to give a thin film (thickness, 0.991 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 11.

[Example 126]

5.7% by weight of polymer 89 and 0.3% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 2600 rpm for 60 seconds, and the resulting coating was dried in an oven at 60°C for 30 minutes to give a thin film (thickness, 0.972 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 11.

[Example 127]

5.4% by weight of polymer 89 and 0.6% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 2500 rpm for 60 seconds, and the resulting coating was dried in an oven at 60°C for 30 minutes to give a thin film (thickness, 1.009 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 11.

[Example 128]

5.1% by weight of polymer 89 and 0.9% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 2300 rpm for 60 seconds, and the resulting coating was dried in an oven at 60°C for 30 minutes to give a thin film (thickness, 1.033 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 11.

[Example 129]

4.8% by weight of polymer 89 and 1.2% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 2000 rpm for 60 seconds, and the resulting coating was dried in an oven at 60°C for 30 minutes to give a thin film (thickness, 1.096 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 11.

[Example 130]

A thin film (thickness, 0.969 μm) was obtained in the same manner as in Example 103, except that bis(2-ethylhexyl) phthalate (molecular weight: 391) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 11.

[Example 131]

A thin film (thickness, 0.991 μm) was obtained in the same manner as in Example 127, except that diundecyl phthalate (molecular weight: 475) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 11.

[Example 132]

A thin film (thickness, 1.034 μm) was obtained in the same manner as in Example 127, except that ditridecyl phthalate (molecular weight: 531) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 11.

[Example 133]

A thin film (thickness, 0.992 μm) was obtained in the same manner as in Example 127, except that tris(2-ethylhexyl) trimellitate (molecular weight: 547) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 11.

[Example 134]

A thin film (thickness, 0.958 μm) was obtained in the same manner as in Example 127, except that diisodecyl

adipate (molecular weight: 427) was used as a thermal reorientation accelerator. The resulting thin film was irradiated with 100 $mJ/cm^2$ of linearly polarized ultraviolet light at 365 nm and then heated at 150°C, giving a thin film exhibiting a large amount of retardation with a good appearance. The amount of retardation is presented in Table 11.

[Example 135]

A thin film (thickness, 1.025 μm) was obtained in the same manner as in Example 127, except that tris(2-ethylhexyl) phosphate (molecular weight: 435) was used as a thermal reorientation accelerator. The resulting thin film was irradiated with 100 $mJ/cm^2$ of linearly polarized ultraviolet light at 365 nm and then heated at 150°C, giving a thin film exhibiting a large amount of retardation with a good appearance. The amount of retardation is presented in Table 11.

[Example 136]

A thin film (thickness, 0.950 μm) was obtained in the same manner as in Example 127, except that bis(2-ethylhexyl) terephthalate (molecular weight: 391) was used as a thermal reorientation accelerator. The resulting thin film was irradiated with 100 $mJ/cm^2$ of linearly polarized ultraviolet light at 365 nm and then heated at 150°C, giving a thin film exhibiting a large amount of retardation with a good appearance. The amount of retardation is presented in Table 11.

[Example 137]

A thin film (thickness, 0.980 μm) was obtained in the same manner as in Example 127, except that di 2-ethylhexyl 4,5-epoxycyclohexane-1,2-dicarboxylate (molecular weight: 394) was used as a thermal reorientation accelerator. The resulting thin film was irradiated with 100 $mJ/cm^2$ of linearly polarized ultraviolet light at 365 nm and then heated at 150°C, giving a thin film exhibiting a large amount of retardation with a good appearance. The amount of retardation is presented in Table 11.

[Example 138]

A thin film (thickness, 0.990 μm) was obtained in the same manner as in Example 127, except that bis(2-ethylhexyl) 4-cyclohexene-1,2-dicarboxylate (molecular weight: 395) was used as a thermal reorientation accelerator. The resulting thin film was irradiated with 100 $mJ/cm^2$ of linearly polarized ultraviolet light at 365 nm and then heated at 150°C, giving a thin film exhibiting a large amount of retardation with a good appearance. The amount of retardation is presented in Table 11.

[Example 139]

5.4% by weight of polymer 90 and 0.6% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 2500 rpm for 60 seconds, and the resulting coating was dried in an oven at 60°C for 30 minutes to give a thin film (thickness, 1.010 μm). The resulting thin film was irradiated with 100 $mJ/cm^2$ of linearly polarized ultraviolet light at 365 nm and then heated at 150°C, giving a thin film exhibiting a large amount of retardation with a good appearance. The amount of retardation is presented in Table 12.

[Example 140]

5.7% by weight of polymer 90 and 0.3% by weight of diisodecyl adipate (molecular weight: 427) as a thermal reorientation accelerator were dissolved in 94% by weight of 1,1,1,3,3,3-hexafluoro-2-propanol. This solution was cast onto a quartz glass substrate, spin coating was performed at 2600 rpm for 60 seconds, and the resulting coating was dried in an oven at 60°C for 30 minutes to give a thin film (thickness, 0.978 μm). The resulting thin film was irradiated with 100 $mJ/cm^2$ of linearly polarized ultraviolet light at 365 nm and then heated at 150°C, giving a thin film exhibiting a large amount of retardation with a good appearance. The amount of retardation is presented in Table 12.

[Example 141]

5.1 % by weight of polymer 90 and 0.9% by weight of diisodecyl adipate (molecular weight: 427) as a thermal reorientation accelerator were dissolved in 94% by weight of 1,1,1,3,3,3-hexafluoro-2-propanol. This solution was cast onto a quartz glass substrate, spin coating was performed at 2300 rpm for 60 seconds, and the resulting coating was

dried in an oven at 60°C for 30 minutes to give a thin film (thickness, 1.002 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 12.

[Example 142]

5.1% by weight of polymer 90 and 0.9% by weight of bis(2-ethylhexyl) terephthalate (molecular weight: 391) as a thermal reorientation accelerator were dissolved in 94% by weight of 1,1,1,3,3,3-hexafluoro-2-propanol. This solution was cast onto a quartz glass substrate, spin coating was performed at 2300 rpm for 60 seconds, and the resulting coating was dried in an oven at 60°C for 30 minutes to give a thin film (thickness, 0.962 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 12.

[Example 143]

5.7% by weight of polymer 90 and 0.3% by weight of bis(2-ethylhexyl) 4-cyclohexene-1,2-dicarboxylate (molecular weight: 395) as a thermal reorientation accelerator were dissolved in 94% by weight of 1,1,1,3,3,3-hexafluoro-2-propanol. This solution was cast onto a quartz glass substrate, spin coating was performed at 2600 rpm for 60 seconds, and the resulting coating was dried in an oven at 60°C for 30 minutes to give a thin film (thickness, 0.953 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 12.

[Example 144]

5.1% by weight of polymer 90 and 0.9% by weight of bis(2-ethylhexyl) 4-cyclohexene-1,2-dicarboxylate (molecular weight: 395) as a thermal reorientation accelerator were dissolved in 94% by weight of 1,1,1,3,3,3-hexafluoro-2-propanol. This solution was cast onto a quartz glass substrate, spin coating was performed at 2300 rpm for 60 seconds, and the resulting coating was dried in an oven at 60°C for 30 minutes to give a thin film (thickness, 0.967 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 12.

[Example 145]

5.94% by weight of polymer 91 and 0.06% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 2700 rpm for 60 seconds, and the resulting coating was dried in an oven at 60°C for 30 minutes to give a thin film (thickness, 1.035 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 146]

5.7% by weight of polymer 91 and 0.3% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 2600 rpm for 60 seconds, and the resulting coating was dried in an oven at 60°C for 30 minutes to give a thin film (thickness, 0.931 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 147]

5.4% by weight of polymer 91 and 0.6% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 2500 rpm for 60 seconds, and the resulting coating was dried in an oven at 60°C for 30 minutes to give a thin film (thickness, 0.994 μm). The resulting thin film was irradiated with 100 mJ/cm$^2$ of linearly polarized ultraviolet light at 365 nm and then heated at 150°C, giving a thin film exhibiting a

large amount of retardation with a good appearance. The amount of retardation is presented in Table 13.

[Example 148]

5.1% by weight of polymer 91 and 0.9% by weight of diisodecyl phthalate (molecular weight: 447) as a thermal reorientation accelerator were dissolved in 94% by weight of 1,1,1,3,3,3-hexafluoro-2-propanol. This solution was cast onto a quartz glass substrate, spin coating was performed at 2300 rpm for 60 seconds, and the resulting coating was dried in an oven at 60°C for 30 minutes to give a thin film (thickness, 1.049 μm). The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 149]

A thin film (thickness, 0.952 μm) was obtained in the same manner as in Example 147, except that bis(2-ethylhexyl) phthalate (molecular weight: 391) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 150]

A thin film (thickness, 0.970 μm) was obtained in the same manner as in Example 147, except that ditridecyl phthalate (molecular weight: 531) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 151]

A thin film (thickness, 0.954 μm) was obtained in the same manner as in Example 147, except that tris(2-ethylhexyl) trimellitate (molecular weight: 547) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 152]

A thin film (thickness, 0.973 μm) was obtained in the same manner as in Example 145, except that diisodecyl adipate (molecular weight: 427) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 153]

A thin film (thickness, 0.999 μm) was obtained in the same manner as in Example 146, except that diisodecyl adipate (molecular weight: 427) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 154]

A thin film (thickness, 0.953 μm) was obtained in the same manner as in Example 147, except that diisodecyl adipate (molecular weight: 427) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 155]

A thin film (thickness, 1.020 μm) was obtained in the same manner as in Example 148, except that diisodecyl adipate (molecular weight: 427) was used as a thermal reorientation accelerator. The resulting thin film was irradiated with 100 mJ/cm$^2$ of linearly polarized ultraviolet light at 365 nm and then heated at 150°C, giving a thin film exhibiting a

large amount of retardation with a good appearance. The amount of retardation is presented in Table 13.

[Example 156]

A thin film (thickness, 0.980 μm) was obtained in the same manner as in Example 147, except that tris(2-ethylhexyl) phosphate (molecular weight: 435) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 157]

A thin film (thickness, 0.989 μm) was obtained in the same manner as in Example 145, except that bis(2-ethylhexyl) terephthalate (molecular weight: 391) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 158]

A thin film (thickness, 0.966 μm) was obtained in the same manner as in Example 146, except that bis(2-ethylhexyl) terephthalate (molecular weight: 391) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 159]

A thin film (thickness, 1.037 μm) was obtained in the same manner as in Example 147, except that bis(2-ethylhexyl) terephthalate (molecular weight: 391) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 160]

A thin film (thickness, 1.025 μm) was obtained in the same manner as in Example 148, except that bis(2-ethylhexyl) terephthalate (molecular weight: 391) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 161]

A thin film (thickness, 0.966 μm) was obtained in the same manner as in Example 145, except that bis(2-ethylhexyl) 4-cyclohexene-1,2-dicarboxylate (molecular weight: 395) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 162]

A thin film (thickness, 0.943 μm) was obtained in the same manner as in Example 146, except that bis(2-ethylhexyl) 4-cyclohexene-1,2-dicarboxylate (molecular weight: 395) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 163]

A thin film (thickness, 1.000 μm) was obtained in the same manner as in Example 147, except that bis(2-ethylhexyl) 4-cyclohexene-1,2-dicarboxylate (molecular weight: 395) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Example 164]

A thin film (thickness, 1.011 μm) was obtained in the same manner as in Example 148, except that bis(2-ethylhexyl) 4-cyclohexene-1,2-dicarboxylate (molecular weight: 395) was used as a thermal reorientation accelerator. The resulting thin film was irradiated with 100 mJ/cm$^2$ of linearly polarized ultraviolet light at 365 nm and then heated at 150°C, giving a thin film exhibiting a large amount of retardation with a good appearance. The amount of retardation is presented in Table 13.

[Comparative Example 3]

6.0% by weight of polymer 86 was dissolved in 94% by weight of 1,1,1,3,3,3-hexafluoro-2-propanol. This solution was cast onto a quartz glass substrate, spin coating was performed at 5700 rpm for 60 seconds, and the resulting coating was dried in an oven at 60°C for 30 minutes to give a thin film (thickness, 0.974 μm). The resulting thin film was irradiated with 100 mJ/cm$^2$ of linearly polarized ultraviolet light at 365 nm and then heated at 190°C, without giving a thin film that was good in terms of appearance but exhibited a large amount of retardation. The amount of retardation is presented in Table 14.

[Comparative Example 4]

3.9% by weight of polymer 86 and 2.1% by weight of diisodecyl adipate (molecular weight: 427) as a thermal reorientation accelerator were dissolved in 94% by weight of 1,1,1,3,3,3-hexafluoro-2-propanol. This solution was cast onto a quartz glass substrate, spin coating was performed at 2500 rpm for 60 seconds, and the resulting coating was dried in an oven at 60°C for 30 minutes to give a thin film (thickness, 1.167 μm). The resulting thin film was irradiated with 100 mJ/cm$^2$ of linearly polarized ultraviolet light at 365 nm and then heated at 190°C, giving a thin film that exhibited a large amount of retardation but was inferior in terms of appearance. The amount of retardation is presented in Table 14.

[Comparative Example 5]

6.0% by weight of polymer 87 was dissolved in 94% by weight of 1,1,1,3,3,3-hexafluoro-2-propanol. This solution was cast onto a quartz glass substrate, spin coating was performed at 3000 rpm for 60 seconds, and the resulting coating was dried in an oven at 60°C for 30 minutes to give a thin film (thickness, 0.945 μm). The resulting thin film was irradiated with 100 mJ/cm$^2$ of linearly polarized ultraviolet light at 365 nm and then heated at 150°C, without giving a thin film that was good in terms of appearance but exhibited a large amount of retardation. The amount of retardation is presented in Table 14.

[Comparative Example 6]

6.0% by weight of polymer 88 was dissolved in 94% by weight of 1,1,1,3,3,3-hexafluoro-2-propanol. This solution was cast onto a quartz glass substrate, spin coating was performed at 2700 rpm for 60 seconds, and the resulting coating was dried in an oven at 60°C for 30 minutes to give a thin film (thickness, 1.017 μm). The resulting thin film was irradiated with 100 mJ/cm$^2$ of linearly polarized ultraviolet light at 365 nm and then heated at 150°C, without giving a thin film that was good in terms of appearance but exhibited a large amount of retardation. The amount of retardation is presented in Table 14.

[Comparative Example 7]

6.0% by weight of polymer 89 was dissolved in 94% by weight of 1,1,1,3,3,3-hexafluoro-2-propanol. This solution was cast onto a quartz glass substrate, spin coating was performed at 2700 rpm for 60 seconds, and the resulting coating was dried in an oven at 60°C for 30 minutes to give a thin film (thickness, 1.031 μm). The resulting thin film was irradiated with 100 mJ/cm$^2$ of linearly polarized ultraviolet light at 365 nm and then heated at 150°C, without giving a thin film that

was good in terms of appearance but exhibited a large amount of retardation. The amount of retardation is presented in Table 14.

[Comparative Example 8]

6.0% by weight of polymer 90 was dissolved in 94% by weight of 1,1,1,3,3,3-hexafluoro-2-propanol. This solution was cast onto a quartz glass substrate, spin coating was performed at 2700 rpm for 60 seconds, and the resulting coating was dried in an oven at 60°C for 30 minutes to give a thin film (thickness, 1.000 μm). The resulting thin film was irradiated with 100 mJ/$cm^2$ of linearly polarized ultraviolet light at 365 nm and then heated at 150°C, without giving a thin film that was good in terms of appearance but exhibited a large amount of retardation. The amount of retardation is presented in Table 14.

[Comparative Example 9]

6.0% by weight of polymer 91 was dissolved in 94% by weight of 1,1,1,3,3,3-hexafluoro-2-propanol. This solution was cast onto a quartz glass substrate, spin coating was performed at 2700 rpm for 60 seconds, and the resulting coating was dried in an oven at 60°C for 30 minutes to give a thin film (thickness, 1.006 μm). The resulting thin film was irradiated with 100 mJ/$cm^2$ of linearly polarized ultraviolet light at 365 nm and then heated at 150°C, without giving a thin film that was good in terms of appearance but exhibited a large amount of retardation. The amount of retardation is presented in Table 14.

[Table 7]

| | Photoreactive reverse wavelength dispersion polymer | | Thermal reorientation accelerator | | | | | Heating temperature (°C) | Retardation after heating Re (nm) | Re (450)/Re (550) | Appearance of the thin film |
|---|---|---|---|---|---|---|---|---|---|---|---|
| | Type | % by weight | Type of accelerator | Structural classification | Name | % by weight | Molecular weight | | | | |

(continued)

| | Photoreactive reverse wavelength dispersion polymer | | Thermal reorientation accelerator | | | | | Heating temperature (°C) | Retardation after heating Re (nm) | Re (450)/ Re (550) | Appearance of the thin film |
|---|---|---|---|---|---|---|---|---|---|---|---|
| | Type | % by weight | Type of accelerator | Structural classification | Name | % by weight | Molecular weight | | | | |
| Example 86 | Polymer 86 | 90 | Plasticizer | Phthalates | Dimethyl phthalate | 10 | 194 | 190 | 14.5 | 0.959 | Good |
| Example 87 | | 90 | | | Diisodecyl phthalate | 10 | 447 | | 16.4 | 0.954 | Good |
| Example 88 | | 90 | | | Ditridecyl phthalate | 10 | 531 | | 23.4 | 0.962 | Good |
| Example 89 | | 90 | | Trimellitates | Tributyl trimellitate | 10 | 378 | | 13.7 | 0.961 | Good |
| Example 90 | | 90 | | Citrates | Trihexyl O-butyrylcitrate | 10 | 515 | | 22.8 | 0.964 | Good |
| Example 91 | | 95 | | Adipates | Diisodecyl adipate | 5 | 427 | | 12.2 | 0.959 | Good |
| Example 92 | | 90 | | | | 10 | | | 22.2 | 0.954 | Good |
| Example 93 | | 85 | | | | 15 | | | 19.6 | 0.955 | Good |
| Example 94 | | 75 | | | | 25 | | | 15.8 | 0.952 | Good |
| Example 95 | | 90 | | Phosphates | Trimethyl phosphate | 10 | 140 | | 13.5 | 0.959 | Good |
| Example 96 | | 90 | | Polyesters | ADK CIZER P-300 | 10 | 3000 | | 17.8 | 0.967 | Good |
| Example 97 | | 90 | | Ethers | Polyethylene glycol 4000 | 10 | 4000 | | 12.0 | 0.964 | Good |
| Example 98 | | 90 | | | Polyethylene glycol 11000 | 10 | 11000 | | 12.8 | 0.963 | Good |
| Example 99 | | 90 | | | Polyethylene glycol 20000 | 10 | 20000 | | 18.5 | 0.967 | Good |

[Table 8]

| | Photoreactive reverse wavelength dispersion polymer | | Thermal reorientation accelerator | | | | | Heating temperature (°C) | Retardation after heating Re (nm) | Re (450)/Re (550) | Appearance of the thin film |
|---|---|---|---|---|---|---|---|---|---|---|---|
| | Type | % by weight | Type of accelerator | Structural classification | Name | % by weight | Molecular weight | | | | |
| Example 100 | Polymer 86 | 90 | Antioxidant | Hindered-phenol | Bis[3-(3-tert-butyl-4-hydroxy-5-methylphenyl) propionic acid] [ethylenebis (oxyethylene) ] | 10 | 587 | 190 | 8.6 | 0.954 | Good |
| Example 101 | | 90 | | | Tetrakis[3-(3',5'-di-tert-butyl-4'-hydroxyphenyl) propionic acid] | 10 | 1178 | | 6.0 | 0.946 | Good |
| Example 102 | | 90 | | | 2,4,8,10-tetraoxaspiro [5,5]undecane-3,9-diylbis(2-methylpropane-2,1-diyl) bis[3-[3-(tert-butyl)-4-hydroxy-5-methylphenyl] propanoate] | 10 | 741 | | 7.3 | 0.956 | Good |
| Example 103 | | 90 | | Phosphorus | Triisodecyl phosphite | 10 | 503 | | 11.5 | 0.964 | Good |
| Example 104 | | 90 | | Sulfur | Didodecyl 3,3'-thiodipropionate | 10 | 515 | | 17.3 | 0.957 | Good |
| Example 105 | | 90 | Photostabilizer | Hinderedamine | Bis(1,2,2,6,6-pentamethyl-4-piperidyl) sebacate | 10 | 509 | | 18.6 | 0.983 | Good |

[Table 9]

| | Photoreactive reverse wavelength dispersion polymer | | Thermal reorientation accelerator | | | | | Heating temperature (°C) | Retardation after heating Re (nm) | Re (450)/ Re (550) | Appearance of the thin film |
|---|---|---|---|---|---|---|---|---|---|---|---|
| | Type | % by weight | Type of accelerator | Structural classification | Name | % by weight | Molecular weight | | | | |
| Example 106 | Polymer 87 | 90 | Plasticizer | Phthalates | Dihexyl phthalate | 10 | 334 | 150 | 55.3 | 0.924 | Good |
| Example 107 | | 90 | | | Diphenyl phthalate | 10 | 318 | | 46.5 | 0.926 | Good |
| Example 108 | | 90 | | | Diisodecyl phthalate | 10 | 447 | | 62.4 | 0.922 | Good |
| Example 109 | | 90 | | | Ditridecyl phthalate | 10 | 531 | | 50.3 | 0.924 | Good |
| Example 110 | | 90 | | Adipates | Diisodecyl adipate | 10 | 427 | | 53.6 | 0.926 | Good |
| Example 111 | | 90 | | Phosphates | Trihexyl phosphate | 10 | 350 | | 51.1 | 0.926 | Good |
| Example 112 | | 90 | | | Triphenyl phosphate | 10 | 326 | | 49.2 | 0.926 | Good |

[Table 10]

| | Photoreactive reverse wavelength dispersion polymer | | Thermal reorientation accelerator | | | | | Heating temperature (°C) | Retardation afterheating Re (nm) | Re (450)/ Re (550) | Appearance of the thin film |
|---|---|---|---|---|---|---|---|---|---|---|---|
| | Type | % by weight | Type of accelerator | Structural classification | Name | % by weight | Molecular weight | | | | |
| Example 113 | Polymer 88 | 95 | Plasticizer | Phthalates | Diisodecyl phthalate | 5 | 447 | 150 | 37.8 | 0.860 | Good |
| Example 114 | | 90 | | | | 10 | | | 44.1 | 0.851 | Good |
| Example 115 | | 85 | | | | 15 | | | 42.5 | 0.856 | Good |
| Example 116 | | 80 | | | | 20 | | | 43.1 | 0.856 | Good |
| Example 117 | | 90 | | | Bis(2-ethylhexyl) phthalate | 10 | 391 | | 42.9 | 0.861 | Good |
| Example 118 | | 90 | | | Ditridecyl phthalate | 10 | 531 | | 41.9 | 0.858 | Good |
| Example 119 | | 90 | | Trimellitates | Tris(2-ethylhexyl) trimellitate | 10 | 547 | | 42.7 | 0.850 | Good |
| Example 120 | | 90 | | Adipates | Diisodecyl adipate | 10 | 427 | | 40.7 | 0.866 | Good |
| Example 121 | | 90 | | Phosphates | Tris(2-ethylhexyl) phosphate | 10 | 435 | | 37.3 | 0.871 | Good |
| Example 122 | | 90 | | Other carboxylates | Bis(2-ethylhexyl) terephthalate | 10 | 391 | | 44.4 | 0.859 | Good |
| Example 123 | | 90 | | Epoxidized esters | Di 2-ethylhexyl 4,5-epoxycyclohexane-1,2-dicarboxylate | 10 | 394 | | 45.9 | 0.855 | Good |
| Example 124 | | 90 | | Other carboxylates | Bis(2-ethylhexyl) 4-cyclohexene-1,2-dicarboxylate | 10 | 395 | | 35.3 | 0.863 | Good |

[Table 11]

| | Photoreactive reverse wavelength dispersion polymer | | Thermal reorientation accelerator | | | | | Heating temperature (°C) | Retardation afterheating Re (nm) | Re (450)/ Re (550) | Appearance of the thin film |
|---|---|---|---|---|---|---|---|---|---|---|---|
| | Type | % by weight | Type of accelerator | Structural classification | Name | % by weight | Molecular weight | | | | |

(continued)

| | Photoreactive reverse wavelength dispersion polymer | | Thermal reorientation accelerator | | | | | Heating temperature (°C) | Retardation after heating Re (nm) | Re (450)/ Re (550) | Appearance of the thin film |
|---|---|---|---|---|---|---|---|---|---|---|---|
| | Type | % by weight | Type of accelerator | Structural classification | Name | % by weight | Molecular weight | | | | |
| Example 125 | Polymer 89 | 99 | Plasticizer | Phthalates | Diisodecyl phthalate | 1 | 447 | 150 | 35.7 | 0.863 | Good |
| Example 126 | | 95 | | | | 5 | | | 41.3 | 0.871 | Good |
| Example 127 | | 90 | | | | 10 | | | 48.9 | 0.870 | Good |
| Example 128 | | 85 | | | | 15 | | | 46.7 | 0.870 | Good |
| Example 129 | | 80 | | | | 20 | | | 44.6 | 0.866 | Good |
| Example 130 | | 90 | | | Bis(2-ethylhexyl) phthalate | 10 | 391 | | 45.4 | 0.870 | Good |
| Example 131 | | 90 | | | Diundecyl phthalate | 10 | 475 | | 44.3 | 0.867 | Good |
| Example 132 | | 90 | | | Ditridecyl phthalate | 10 | 531 | | 46.9 | 0.859 | Good |
| Example 133 | | 90 | | Trimellitates | Tris(2-ethylhexyl) trimellitate | 10 | 547 | | 41.1 | 0.861 | Good |
| Example 134 | | 90 | | Adipates | Diisodecyl adipate | 10 | 427 | | 43.1 | 0.868 | Good |
| Example 135 | | 90 | | Phosphates | Tris(2-ethylhexyl) phosphate | 10 | 435 | | 46.0 | 0.868 | Good |
| Example 136 | | 90 | | Other carboxylates | Bis(2-ethylhexyl) terephthalate | 10 | 391 | | 42.5 | 0.863 | Good |
| Example 137 | | 90 | | Epoxidized esters | Di 2-ethylhexyl 4,5-epoxycyclohexane-1,2-dicarboxylate | 10 | 394 | | 42.6 | 0.870 | Good |

(continued)

| | Photoreactive reverse wavelength dispersion polymer | | Thermal reorientation accelerator | | | | | Heating temperature (°C) | Retardation afterheating Re (nm) | Re (450)/ Re (550) | Appearance of the thin film |
|---|---|---|---|---|---|---|---|---|---|---|---|
| | Type | % by weight | Type of accelerator | Structural classification | Name | % by weight | Molecular weight | | | | |
| Example 138 | | 90 | | Other carboxylates | Bis(2-ethylhexyl) 4-cyclohexene-1,2-dicarboxylate | 10 | 395 | | 45.6 | 0.872 | Good |

[Table 12]

| | Photoreactive reverse wavelength dispersion polymer | | Thermal reorientation accelerator | | | | | Heating temperature (°C) | Retardation after heating Re (nm) | Re (450)/ Re (550) | Appearance of the thin film |
|---|---|---|---|---|---|---|---|---|---|---|---|
| | Type | % by weight | Type of accelerator | Structural classification | Name | % by weight | Molecular weight | | | | |
| Example 139 | Polymer 90 | 90 | Plasticizer | Phthalates | Diisodecyl phthalate | 10 | 447 | 150 | 47.1 | 0.866 | Good |
| Example 140 | | 95 | | Adipates | Diisodecyl adipate | 5 | 427 | | 38.6 | 0.867 | Good |
| Example 141 | | 85 | | | | 15 | | | 42.8 | 0.862 | Good |
| Example 142 | | 85 | | Other carboxylates | Bis(2-ethylhexyl) terephthalate | 15 | 391 | | 41.8 | 0.861 | Good |
| Example 143 | | 95 | | | Bis(2-ethylhexyl) 4-cyclohexene-1,2-dicarboxylate | 5 | 395 | | 36.4 | 0.870 | Good |
| Example 144 | | 85 | | | | 15 | | | 44.0 | 0.862 | Good |

[Table 13]

| | Photoreactive reverse wavelength dispersion polymer | | Thermal reorientation accelerator | | | | | Heating temperature (°C) | Retardation after heating Re (nm) | Re (450)/ Re (550) | Appearance of the thin film |
|---|---|---|---|---|---|---|---|---|---|---|---|
| | Type | % by weight | Type of accelerator | Structural classification | Name | % by weight | Molecular weight | | | | |
| Example 145 | Polymer 91 | 99 | Plasticizer | Phthalates | Diisodecyl phthalate | 1 | 447 | 150 | 37.6 | 0.865 | Good |
| Example 146 | | 95 | | | | 5 | | | 40.2 | 0.874 | Good |
| Example 147 | | 90 | | | | 10 | | | 47.0 | 0.871 | Good |
| Example 148 | | 85 | | | | 15 | | | 44.6 | 0.872 | Good |
| Example 149 | | 90 | | | Bis(2-ethylhexyl) phthalate | 10 | 391 | | 43.9 | 0.869 | Good |
| Example 150 | | 90 | | | Ditridecyl phthalate | 10 | 531 | | 35.5 | 0.862 | Good |
| Example 151 | | 90 | | Trimellitates | Tris(2-ethylhexyl) trimellitate | 10 | 547 | | 36.0 | 0.863 | Good |
| Example 152 | | 99 | | Adipates | Diisodecyl adipate | 1 | 427 | | 37.7 | 0.869 | Good |
| Example 153 | | 95 | | | | 5 | | | 45.4 | 0.865 | Good |
| Example 154 | | 90 | | | | 10 | | | 43.6 | 0.868 | Good |
| Example 155 | | 85 | | | | 15 | | | 44.7 | 0.870 | Good |
| Example 156 | | 90 | | Phosphates | Tris(2-ethylhexyl) phosphate | 10 | 435 | | 42.5 | 0.869 | Good |
| Example 157 | | 99 | | Other carboxylates | Bis(2-ethylhexyl) terephthalate | 1 | 391 | | 40.1 | 0.871 | Good |

(continued)

| | Photoreactive reverse wavelength dispersion polymer | | Thermal reorientation accelerator | | | | | Heating temperature (°C) | Retardation after heating Re (nm) | Re (450)/ Re (550) | Appearance of the thin film |
|---|---|---|---|---|---|---|---|---|---|---|---|
| | Type | % by weight | Type of accelerator | Structural classification | Name | % by weight | Molecular weight | | | | |
| Example 158 | | 95 | | | | 5 | | | 42.3 | 0.876 | Good |
| Example 159 | | 90 | | | | 10 | | | 41.5 | 0.862 | Good |
| Example 160 | | 85 | | | | 15 | | | 42.5 | 0.874 | Good |
| Example 161 | | 99 | | | Bis(2-ethylhexyl) 4-cyclohexene-1,2-dicarboxylate | 1 | 395 | | 40.1 | 0.873 | Good |
| Example 162 | | 95 | | | | 5 | | | 40.2 | 0.876 | Good |
| Example 163 | | 90 | | | | 10 | | | 43.8 | 0.870 | Good |
| Example 164 | | 85 | | | | 15 | | | 43.7 | 0.871 | Good |

[Table 14]

| | Photoreactive reverse wavelength dispersion polymer | | Thermal reorientation accelerator | | | | | Heating temperature (°C) | Retardation after heating Re (nm) | Re (450)/ Re (550) | Appearance of the thin film |
|---|---|---|---|---|---|---|---|---|---|---|---|
| | Type | % by weight | Type of accelerator | Structural classification | Name | % by weight | Molecular weight | | | | |
| Comparative Example 3 | Polymer 86 | 100 | - | - | - | - | - | 190 | 4.3 | 0.959 | Good |
| Comparative Example 4 | | 65 | Plasticizer | Adipates | Diisodecyl adipate | 35 | 427 | 190 | 7.3 | 0.953 | Inferior |
| Comparative Example 5 | Polymer 87 | 100 | - | - | - | - | - | 150 | 31.0 | 0.926 | Good |
| Comparative Example 6 | Polymer 88 | 100 | - | - | - | - | - | 150 | 29.6 | 0.851 | Good |
| Comparative Example 7 | Polymer 89 | 100 | - | - | - | - | - | 150 | 30.3 | 0.865 | Good |
| Comparative Example 8 | Polymer 90 | 100 | - | - | - | - | - | 150 | 27.9 | 0.861 | Good |
| Comparative Example 9 | Polymer 91 | 100 | - | - | - | - | - | 150 | 32.4 | 0.863 | Good |

[In Tables 7 to 14, Re (450)/Re (550) represents the ratio between the in-plane retardation at 450 nm and the in-plane retardation at 550 nm.]

Industrial Applicability

By using the polymer according to the present invention, a reverse wavelength dispersion retardation film can be formed without requiring an alignment film. The present invention, therefore, is highly industrially applicable.

## Claims

1. A main chain polymer comprising, in a polymer main chain thereof, a photoreactive reverse wavelength dispersion unit that exhibits both functions of photoreactivity and reverse wavelength dispersion of birefringence, wherein:

   the photoreactive reverse wavelength dispersion unit has a structure represented by chemical formula (1) below.

   [Chem. 1]

   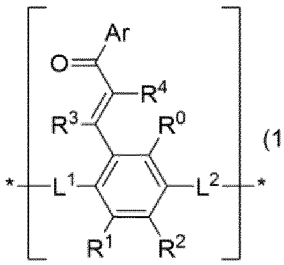

   (1)

   [In chemical formula (1) above, $L^1$ and $L^2$ may be the same or different from each other, each representing a carbonyl group, an ester linkage, an amide linkage, an ether linkage or a single bond.
   * represents a site of binding with another structure in the main chain polymer.
   Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.
   $R^0$, $R^1$, $R^2$, $R^3$ and $R^4$ each independently represent a hydrogen atom, a halogen atom, a C1 to C8 alkyl group or a group represented by chemical formula (Z1) below.]

   [Chem. 2]

   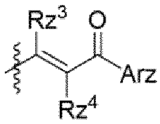

   (Z1)

   [In chemical formula (Z1) above, $Rz^3$ and $Rz^4$ each independently represent a hydrogen atom, a halogen atom or a C1 to C8 alkyl group.
   Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent. A wavy-lined portion represents a site of binding with a portion in formula (1) excluding R°, $R^1$, $R^2$, $R^3$ or $R^4$.]

2. The main chain polymer according to Claim 1, wherein in chemical formula (1), Ar is any of chemical formulae (Ar-1) to (Ar-7) below.

[Chem. 3]

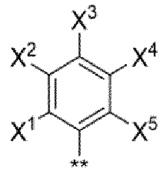

(Ar-1)

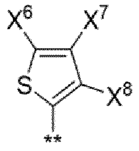

(Ar-2)

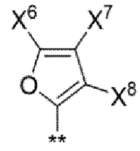

(Ar-3)

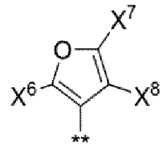

(Ar-4)

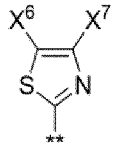

(Ar-5)

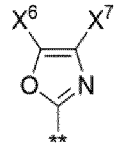

(Ar-6)

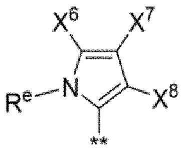

(Ar-7)

[In chemical formulae (Ar-1) to (Ar-7) above, $X^1$, $X^2$, $X^3$, $X^4$, $X^5$, $X^6$, $X^7$ and $X^8$ each independently represent a hydrogen atom or a C1 to C8 alkyl group, a C1 to C6 alkoxy group, a halogen atom, a nitro group, a cyano group, a C1 to C6 alkylthio group or a C2 to C8 dialkylamino group.
$R^e$ represents a hydrogen atom or a C1 to C10 alkyl group.
** represents a site of binding with the other portion in chemical formula (1), excluding Ar.]

**3.** The main chain polymer according to Claim 1, further comprising at least one type of structure selected from the group consisting of chemical formulae (2A), (2B), (2C) and (2D) below.

[Chem. 4]

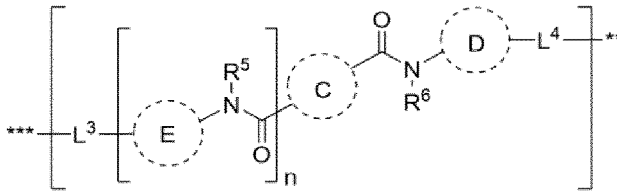

(2A)

[In chemical formula (2A) above, ring C, ring D and ring E each independently represent a ring system selected from the group consisting of a monocyclic aromatic ring system, a polycyclic aromatic ring system, an annulated aromatic ring system and an aliphatic hydrocarbon ring system whose ring-constituting atoms are atoms selected from the group consisting of a carbon atom, a nitrogen atom, an oxygen atom and a sulfur atom, with the monocyclic aromatic ring system, polycyclic aromatic ring system, annulated aromatic ring system and aliphatic hydrocarbon ring system optionally having a substituent.
$R^5$ and $R^6$ may be the same or different from each other, each representing a group selected from the group consisting of a hydrogen atom, a substituted or unsubstituted C1 to C20 alkyl group, a substituted or unsubstituted C3 to C8 cycloalkyl group and a substituted or unsubstituted C3 to C12 aromatic group.
n is 0 or 1.
$L^3$ and $L^4$ may be the same or different from each other, each representing a carbonyl group, an ester linkage, an amide linkage, an ether linkage or a single bond.
*** represents a site of binding with another structure in the main chain polymer.]

[Chem. 5]

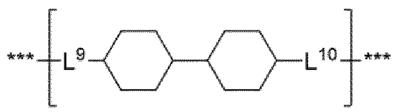

(2B)

[In chemical formula (2B) above, $L^9$ and $L^{10}$ may be the same or different from each other, each representing a carbonyl group, an ester linkage, an amide linkage, an ether linkage or a single bond.
*** represents a site of binding with another structure in the main chain polymer.]

[Chem. 6]

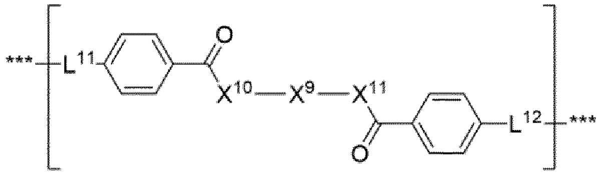

(2C)

[In chemical formula (2C) above, $X^9$ represents a C1 to C10 alkylene chain or a single bond.
$X^{10}$ represents -O- or -N($R^c$)-.
$X^{11}$ represents -O- or -N($R^d$)-.
$R^c$ and $R^d$ may be the same or different from each other, each representing a hydrogen atom or a C1 to C10 alkyl group.
$L^{11}$ and $L^{12}$ may be the same or different from each other, each representing a carbonyl group, an ester linkage, an amide linkage, an ether linkage or a single bond.
*** represents a site of binding with another structure in the main chain polymer.]

[Chem. 7]

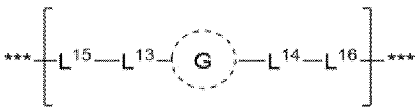

(2D)

[In chemical formula (2D) above, ring G represents a ring system selected from the group consisting of a monocyclic aromatic ring system, a polycyclic aromatic ring system, an annulated aromatic ring system, a spiro ring system and an aliphatic hydrocarbon ring system whose ring-constituting atoms are atoms selected from the group consisting of a carbon atom, a nitrogen atom, an oxygen atom and a sulfur atom, with the monocyclic aromatic ring system, polycyclic aromatic ring system, annulated aromatic ring system, spiro ring system and aliphatic hydrocarbon ring system optionally having a substituent.
$L^{13}$ and $L^{14}$ may be the same or different from each other, each representing a single bond or a C1 to C6 alkylene chain.
$L^{15}$ and $L^{16}$ may be the same or different from each other, each representing a carbonyl group, an ester linkage, an amide linkage, an ether linkage or a single bond.
*** represents a site of binding with another structure in the main chain polymer.]

4. The main chain polymer according to any one of Claims 1 to 3, wherein at least one end of the main chain polymer is a monocarboxylic acid ester represented by chemical formula (2') below.

[Chem. 8]

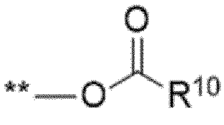

(2')

[In chemical formula (2') above, $R^{10}$ represents a group selected from the group consisting of a substituted or unsubstituted C1 to C20 alkyl group, a substituted or unsubstituted C3 to C8 cycloalkyl group and a substituted or unsubstituted C3 to C12 aromatic group.
** represents a site of binding with another structure in the main chain polymer.]

5. A resin composition comprising 70% to 99.99% by weight of the main chain polymer according to any one of Claims

1 to 3 and 0.01% to 30% by weight of a thermal reorientation accelerator.

6. A resin composition comprising 70% to 99.99% by weight of the main chain polymer according to Claim 4 and 0.01% to 30% by weight of a thermal reorientation accelerator.

7. A method for producing the main chain polymer according to any first of Claims 1 to 3, the method comprising polymerizing a source composition containing a dihydroxy compound represented by chemical formula (1') below.

[Chem. 9]

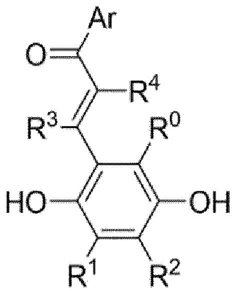

(1')

[In chemical formula (1') above, $R^0$, $R^1$, $R^2$, $R^3$ and $R^4$ each independently represent a hydrogen atom, a halogen atom, a C1 to C8 alkyl group or a group represented by chemical formula (Z1) below. Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent.]

[Chem. 10]

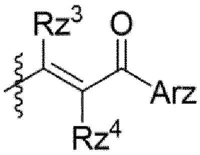

(Z1)

[In chemical formula (Z1) above, $Rz^3$ and $Rz^4$ each independently represent a hydrogen atom, a halogen atom or a C1 to C8 alkyl group.
Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent. A wavy-lined portion represents a site of binding with a portion in formula (1) excluding R°, $R^1$, $R^2$, $R^3$ or $R^4$.]

8. A method for producing the main chain polymer according to Claim 4, the method comprising, when polymerizing a source composition containing a dihydroxy compound represented by chemical formula (1'), further adding a monocarboxylic acid derivative compound represented by any of chemical formula (2-1) or (2-2) below.

[Chem. 11]

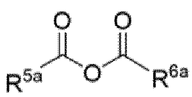

(2-1)

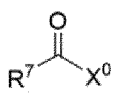

(2-2)

[In chemical formula (2-1) or (2-2) above, $R^{5a}$, $R^{6a}$ and $R^7$ each independently represent a group selected from the group consisting of a substituted or unsubstituted C1 to C20 alkyl group, a substituted or unsubstituted C3 to C8 cycloalkyl group and a substituted or unsubstituted C3 to C12 aromatic group.]

**9.** An optical film comprising the main chain polymer according to any one of Claims 1 to 3 or the resin composition according to Claim 5.

**10.** An optical film comprising the main chain polymer according to Claim 4 or the resin composition according to Claim 6.

**11.** The optical film according to Claim 9 or 10, wherein retardations (Re) meet formula (I) below.

$$Re\ (450) \leq Re\ (550)\ \ldots \qquad (I)$$

(In formula (I), Re (450) represents an in-plane retardation value measured at a wavelength of 450 nm, and Re (550) represents an in-plane retardation value measured at a wavelength of 550 nm.)

**12.** The optical film according to Claim 10, wherein a yellowness index (YI) at a film thickness of 5 $\mu$m is 5% or less.

**13.** A method for producing the optical film according to any one of Claims 9 to 12, the method comprising irradiation with any one type of ultraviolet radiation out of polarized ultraviolet radiation and obliquely incident ultraviolet radiation irradiation.

**14.** The production method according to Claim 13, further comprising a heat treatment step.

**15.** A multilayer film comprising the optical film according to any one of Claims 9 to 12.

**16.** A dihydroxy compound represented by chemical formula (1') below.

[Chem. 12]

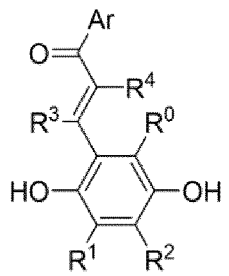

(1')

[In chemical formula (1') above, $R^0$, $R^1$, $R^2$, $R^3$ and $R^4$ each independently represent a hydrogen atom, a halogen atom or a C1 to C8 alkyl group or a group represented by chemical formula (Z1) below. Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent..]

[Chem. 13]

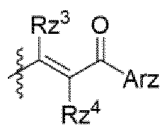

(Z1)

[In chemical formula (Z1) above, $Rz^3$ and $Rz^4$ each independently represent a hydrogen atom, a halogen atom or a C1 to C8 alkyl group.

Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent. A wavy-lined portion represents a site of binding with a portion in formula (1) excluding R°, $R^1$, $R^2$, $R^3$ or $R^4$.]

**17.** A method for producing a dihydroxy compound, the method comprising allowing a dihydroxy compound indicated by chemical formula (3) below and a ketone indicated by chemical formula (4') below to react together to give a dihydroxy compound represented by chemical formula (1') below.

[Chem. 14]

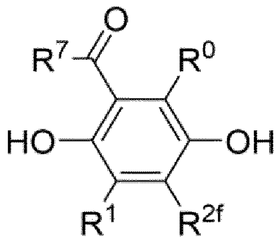

**(3)**

[In chemical formula (3) above, R°, $R^1$ and $R^7$ each independently represent a hydrogen atom, a halogen atom or a C1 to C8 alkyl group.
$R^{2f}$ represents a hydrogen atom, a halogen atom, a C1 to C8 alkyl group or a formyl group.]

[Chem. 15]

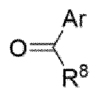

**(4')**

[In chemical formula (4') above, $R^8$ represents a C1 to C8 alkyl group or a C1 to C6 haloalkyl group.
Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system and annulated aromatic ring system optionally having a substituent.]

[Chem. 16]

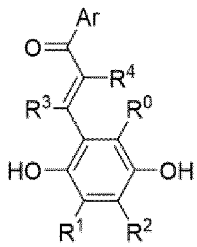

**(1')**

[In chemical formula (1') above, Ar represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system and annulated aromatic ring system optionally having a substituent.
R°, $R^1$, $R^2$, $R^3$ and $R^4$ each independently represent a hydrogen atom, a halogen atom or a C1 to C8 alkyl group or a group represented by chemical formula (Z1) below.]

[Chem. 17]

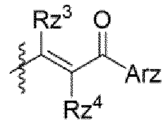

(Z1)

[In formula (Z1) above, $Rz^3$ and $Rz^4$ each independently represent a hydrogen atom, a halogen atom or a C1 to C8 alkyl group.

Arz represents a ring system selected from the group consisting of a monocyclic aromatic ring system, a polycyclic aromatic ring system and an annulated aromatic ring system whose ring-constituting atoms are atoms selected from the group consisting of a carbon atom, a nitrogen atom, an oxygen atom and a sulfur atom, with the monocyclic aromatic ring system, polycyclic aromatic ring system or annulated aromatic ring system optionally having a substituent. A wavy-lined portion represents a site of binding with a portion in formula (1) excluding R°, $R^1$, $R^2$, $R^3$ or $R^4$.]

**INTERNATIONAL SEARCH REPORT**

International application No.
**PCT/JP2022/047334**

**A. CLASSIFICATION OF SUBJECT MATTER**

***C08G 85/00***(2006.01)i; ***C07D 307/46***(2006.01)i; ***C07D 333/16***(2006.01)i; ***C07D 333/28***(2006.01)i; ***C07D 333/32***(2006.01)i; ***C07D 339/06***(2006.01)i; ***C08G 63/00***(2006.01)i; ***C08G 63/137***(2006.01)i; ***C08G 63/19***(2006.01)i; ***C08G 63/547***(2006.01)i; ***C08G 63/682***(2006.01)i; ***C08G 63/685***(2006.01)i; ***C08G 63/688***(2006.01)i; ***C08G 63/78***(2006.01)i; ***C08G 69/00***(2006.01)i; ***C08G 69/44***(2006.01)i; ***C08K 5/00***(2006.01)i; ***C08L 67/02***(2006.01)i; ***C08L 77/12***(2006.01)i; ***C08L 101/12***(2006.01)i; ***G02B 1/04***(2006.01)i; ***G02B 5/30***(2006.01)i; ***H05B 33/02***(2006.01)i; ***H10K 50/00***(2023.01)i

FI: C08G85/00; C07D307/46; C07D333/16; C07D333/28; C07D333/32 CSP; C07D339/06; C08G63/00; C08G63/137; C08G63/19; C08G63/682; C08G63/685; C08G63/688; C08G63/78; C08G69/00; C08G69/44; C08K5/00; C08L67/02; C08L77/12; C08L101/12; G02B1/04; G02B5/30; H05B33/02; H05B33/14 A; C08G63/547

According to International Patent Classification (IPC) or to both national classification and IPC

**B. FIELDS SEARCHED**

Minimum documentation searched (classification system followed by classification symbols)

C08G85/00; C07D307/46; C07D333/16; C07D333/28; C07D333/32; C07D339/06; C08G63/00; C08G63/137; C08G63/19; C08G63/547; C08G63/682; C08G63/685; C08G63/688; C08G63/78; C08G69/00; C08G69/44; C08K5/00; C08L67/02; C08L77/12; C08L101/12; G02B1/04; G02B5/30; H05B33/02; H10K50/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | KIM, Beom-Tae et al. Synthesis of Dihydroxylated Chalcone Derivatives with Diverse Substitution Patterns and Their Radical Scavenging Ability toward DPPH Free Radicals. Bull. Korean Chem. Soc. 2008, vol. 29, no. 6, 1125-1130<br>p. 1129, etc. | 16-17 |
| A | | 1-15 |
| X | HWANG, Ki-Jun et al. Synthesis of Heterocyclic Chalcone Derivatives and Their Radical Scavenging Ability Toward 2,2-Diphenyl-1-Picrylhydrazyl (DPPH) Free Radicals. Bull. Korean Chem. Soc. 2012, vol. 33, no. 8, 2585-2591<br>table 2, etc. | 16-17 |
| A | | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 January 2023** | **14 February 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No. **PCT/JP2022/047334**

**C. DOCUMENTS CONSIDERED TO BE RELEVANT**

| Category* | Citation of document, with indication, where appropriate, of the relevant passages | Relevant to claim No. |
|---|---|---|
| X | JP 2012-100668 A (SEOUL NATIONAL UNIV INDUSTRY FOUNDATION) 31 May 2012 (2012-05-31) table 1, etc. | 16-17 |
| A | | 1-15 |
| X | US 2013/0178533 A1 (LEE, Jung-Weon) 11 July 2013 (2013-07-11) table 1, etc. | 16-17 |
| A | | 1-15 |

Form PCT/ISA/210 (second sheet) (January 2015)

**INTERNATIONAL SEARCH REPORT**
**Information on patent family members**

International application No.
**PCT/JP2022/047334**

| Patent document cited in search report | Publication date (day/month/year) | Patent family member(s) | Publication date (day/month/year) |
|---|---|---|---|
| JP 2012-100668 A | 31 May 2012 | US 2010/0068730 A1<br>table 1, etc.<br>US 2012/0282619 A1<br>EP 2099440 A1<br>EP 2601943 A1<br>KR 10-2008-0052391 A<br>CN 101528210 A<br>CN 102199113 A | |
| US 2013/0178533 A1 | 11 July 2013 | KR 10-2012-0022504 A<br>KR 10-2012-0023524 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 8160430 A
- JP 2003505561 W
- WO 2010150748 A
- JP 6172556 B
- JP 6754845 B
- JP 2002226858 A
- WO 2014017497 A
- JP 2018188529 A
- JP 2021028375 A
- JP 2021028384 A
- JP 5325733 B

### Non-patent literature cited in the description

- *Macromolecules,* 2018, vol. 51 (23), 9430-9441